# EUROPEAN PATENT SPECIFICATION

(11) **EP 4 140 990 B1**
(45) Date of publication and mention of the grant of the patent: **19.03.2025**
(21) Application number: 22187136.1
(22) Date of filing: 27.07.2022
(51) Int. Cl.: C07D 307/91, C07D 409/04, C07D 405/10, H10K 85/60, H10K 50/00

(54) **BENZO[B]NAPHTHO[2,1-D]FURAN COMPOUND AND ORGANIC LIGHT EMITTING DEVICE USING SAME**
BENZO[B]NAPHTHO[2,1-D]FURAN-VERBINDUNG UND ORGANISCHE LICHTEMITTIERENDE VORRICHTUNG DAMIT
COMPOSÉ DE BENZO[B]NAPHTHO[2,1-D]FURANE ET DISPOSITIF ÉLECTROLUMINESCENT ORGANIQUE L'UTILISANT

(30) Priority: 25.08.2021 KR 20210112426
(43) Date of publication of application: 01.03.2023
(73) Proprietor: LT Materials Co., Ltd., Yongin-City 17118 (KR)
(72) Inventor: KIM, Ji-Un, 17118 Yongin-City, Gyeonggi-Do (KR); LEE, Nam-Jin, 17118 Yongin-City, Gyeonggi-Do (KR); JEONG, Won-Jang, 17118 Yongin-City, Gyeonggi-Do (KR); KIM, Dong-Jun, 17118 Yongin-City, Gyeonggi-Do (KR)
(74) Representative: Goddar, Heinz J.

(56) References cited:
- EP-A1- 4 349 821
- EP-A2- 3 757 095
- WO-A1-2021/060865
- WO-A1-2022/039408

## Description

### TECHNICAL FIELD

The present application relates to a heterocyclic compound and an organic light emitting device using the same.

### BACKGROUND ART

An electroluminescence device is a kind of self-emitting type display device, and has an advantage in that the viewing angle is wide, the contrast is excellent, and the response speed is fast.

An organic light emitting device has a structure in which an organic thin film is disposed between two electrodes. When a voltage is applied to an organic light emitting device having the structure, electrons and holes injected from the two electrodes combine with each other in an organic thin film to make a pair, and then, emit light while being extinguished. The organic thin film may be composed of a single layer or multi layers, if necessary.

A material for the organic thin film may have a light emitting function, if necessary. For example, as the material for the organic thin film, it is also possible to use a compound, which may itself constitute a light emitting layer alone, or it is also possible to use a compound, which may serve as a host or a dopant of a host-dopant-based light emitting layer. In addition, as a material for the organic thin film, it is also possible to use a compound, which may perform a function such as hole injection, hole transport, electron blocking, hole blocking, electron transport or electron injection.

In order to improve the performance, service life, or efficiency of the organic light emitting device, there is a continuous need for developing a material for an organic thin film.

### [Related Art Document]

### [Patent Document]

US Patent No. 4,356,429
EP 3 757 095 A2 discloses a heterocyclic compound represented by the following Chemical Formula 1: 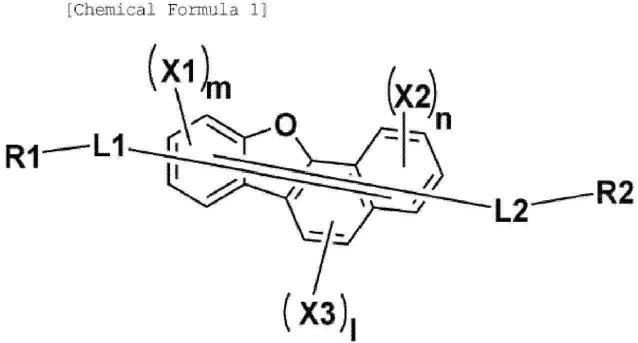
L1 is a direct bond; a substituted or unsubstituted arylene group having 6 to 60 carbon atoms; or a substituted or unsubstituted heteroarylene group having 2 to 60 carbon atoms,
L2 is a direct bond; or a substituted or unsubstituted arylene group having 6 to 60 carbon atoms,
R1 is a substituted or unsubstituted aryl group having 6 to 60 carbon atoms; a substituted or unsubstituted heteroaryl group having 2 to 60 carbon atoms; or a substituted or unsubstituted amine group,
R2 is a substituted or unsubstituted aryl group having 6 to 60 carbon atoms; or a substituted or unsubstituted amine group,
X1 to X3 are each independently hydrogen; deuterium; a halogen group; a substituted or unsubstituted alkyl group having 61 to 60 carbon atoms; a substituted or unsubstituted cycloalkyl group having 3 to 60 carbon atoms; a substituted or unsubstituted aryl group having 6 to 60 carbon atoms; or a substituted or unsubstituted heterocyclic group having 2 to 60 carbon atoms,
m and n are each an integer of 1 to 4, and when m and n are 2 or greater, substituents in the parentheses are the same as or different from each other,
1 is 1 or 2, and when 1 is 2, substituents in the parentheses are the same as or different from each other, and
m, n and 1 are m+n+1≤8.

WO 2021/060865A1 discloses a heterocyclic compound represented by the following Chemical Formula 1: 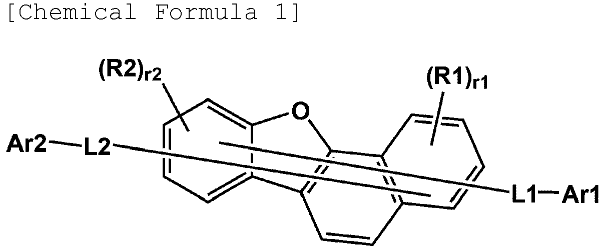 wherein, in Chemical Formula 1,
L1 and L2 are each independently a direct bond; a substituted or unsubstituted C6 to C60 arylene group; or a substituted or unsubstituted divalent C2 to C60 heterocyclic group,
Ar1 is a substituted or unsubstituted C2 to C60 heterocyclic group including N,
Ar2 is -N(R106) (R107); or a substituted or unsubstituted C2 to C60 heterocyclic group,
R1 and R2 are each independently hydrogen; deuterium; a halogen group; a substituted or unsubstituted C1 to C60 alkyl group; a substituted or unsubstituted C3 to C60 cycloalkyl group; a substituted or unsubstituted C6 to C60 aryl group; or a substituted or unsubstituted C2 to C60 heterocyclic group,
R106 and R107 are each independently hydrogen; deuterium; a halogen group; an alkyl group; an alkenyl group; an alkoxy group; a cycloalkyl group; an aryl group; or a heterocyclic group,
r1 and r2 are each an integer of 1 to 4, and
when r1 and r2 are each 2 or greater, substituents in the parentheses are the same as or different from each other.

### SUMMARY OF THE INVENTION

The present specification has been made in an effort to provide a heterocyclic compound and an organic light emitting device including the same.

The present application provides a heterocyclic compound according to claim 1.

Further, another exemplary embodiment of the present application provides an organic light emitting device including a first electrode, a second electrode and an organic material layer having one or more layers provided between the first electrode and the second electrode, in which one or more layers of the organic material layer include the heterocyclic compound of the invention.

A heterocyclic compound according to an exemplary embodiment of the present application can be used as a material for an organic material layer of an organic light emitting device. The heterocyclic compound can be used as a material for a hole injection layer, a hole transport layer, a light emitting layer, an electron transport layer, an electron injection layer, a charge generation layer, and the like in an organic light emitting device. In particular, the heterocyclic compound can be used as a material for a hole transport layer or hole transport auxiliary layer of an organic light emitting device. In addition, when the heterocyclic compound is used for an organic light emitting device, the driving voltage of the device can be lowered, the light efficiency of the device can be improved, and the service life characteristics of the device can be improved due to the thermal stability of the compound.

### BRIEF DESCRIPTION OF THE DRAWINGS

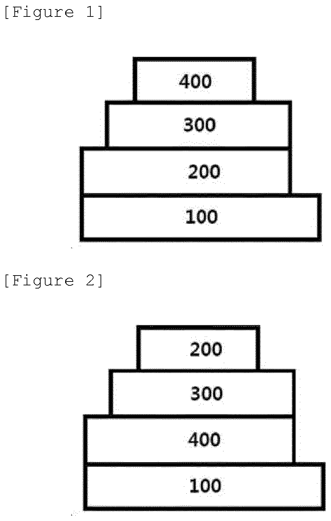

FIGS. 1 to 3 each are views schematically illustrating a stacking structure of an organic light emitting device according to an exemplary embodiment of the present application.

### DETAILED DESCRIPTION

Hereinafter, the present application will be described in detail.

The heterocyclic compound may have a structure in which two specific substituents including an amine group in naphthobenzofuran are substituted to delocalize the highest occupied molecular orbital (HOMO) energy level, thereby increasing the hole transport ability and stabilizing the HOMO energy.

Further, by bonding an amine moiety to a substituent having enhanced hole characteristics, the planarity of the amine derivative and the glass transition temperature may be increased to enhance the thermal stability of the compound. Moreover, the heterocyclic compound has a structure in which only one amine group is substituted, the molecular weight is lower than that in the case where two amine groups are substituted or the amine group is not possessed as the substituent, an appropriate HOMO for hole injection and transport may be formed, and thermal stability may be achieved by allowing the material to have a spatial structure.

In addition, the heterocyclic compound may improve the hole transfer ability and may also enhance the stability of the molecule by adjusting band gap and Ti value (energy level value of the triplet state).

Furthermore, since a substituent other than an amine group corresponds to a specific substituent or an aryl group having a specific carbon number, it is possible to form a HOMO in which holes are more easily injected and transported than the case of having other substituents.

Due to the characteristics, when the material of is used as a material for the hole transport layer in the organic light emitting device, the driving voltage of the device may be lowered and the light emitting efficiency of the device may be improved. Further, when the heterocyclic compound is used as a material for the hole transport auxiliary layer, the transfer of electrons from the opposite side of the electron transport layer may be effectively prevented, thereby improving the efficiency of the device.

In addition, the service life of the device using the heterocyclic compound may be improved by the thermal stability of the heterocyclic compound .

In the present specification, the term "substitution" means that a hydrogen atom bonded to a carbon atom of a compound is changed into another substituent, and a position to be substituted is not limited as long as the position is a position at which the hydrogen atom is substituted, that is, a position at which the substituent may be substituted, and when two or more substituents are substituted, the two or more substituents may be the same as or different from each other.

In the present specification, "substituted or unsubstituted" means being unsubstituted or substituted with one or more substituents selected from the group consisting of deuterium; a cyano group; a halogen group; a straight-chained or branched alkyl having 1 to 60 carbon atoms; a straight-chained or branched alkenyl having 2 to 60 carbon atoms; a straight-chained or branched alkynyl having 2 to 60 carbon atoms; a monocyclic or polycyclic cycloalkyl having 3 to 60 carbon atoms; a monocyclic or polycyclic heterocycloalkyl having 2 to 60 carbon atoms; a monocyclic or polycyclic aryl having 6 to 60 carbon atoms; a monocyclic or polycyclic heteroaryl having 2 to 60 carbon atoms; -SiRR'R"; -P(=O)RR'; an alkylamine having 1 to 20 carbon atoms; a monocyclic or polycyclic arylamine having 6 to 60 carbon atoms; and a monocyclic or polycyclic heteroarylamine having 2 to 60 carbon atoms, or being unsubstituted or substituted with a substituent to which two or more substituents selected from the above exemplified substituents are linked, and means that R, R' and R" are the same as or different from each other, and are each independently a substituted or unsubstituted alkyl having 1 to 60 carbon atoms; a substituted or unsubstituted aryl having 6 to 60 carbon atoms; or a substituted or unsubstituted heteroaryl having 2 to 60 carbon atoms.

In the present specification, "when a substituent is not indicated in the structure of a chemical formula or compound" means that a hydrogen atom is bonded to a carbon atom. However, since deuterium (²H) is an isotope of hydrogen, some hydrogen atoms may be deuterium.

In an exemplary embodiment of the present application, "when a substituent is not indicated in the structure of a chemical formula or compound" may mean that all the positions that may be reached by the substituent are hydrogen or deuterium. That is, deuterium is an isotope of hydrogen, and some hydrogen atoms may be deuterium which is an isotope, and in this case, the content of deuterium may be 0% to 100%.

In an exemplary embodiment of the present application, in "the case where a substituent is not indicated in the structure of a chemical formula or compound", when the content of deuterium is 0%, the content of hydrogen is 100%, and all the substituents do not explicitly exclude deuterium such as hydrogen, hydrogen and deuterium may be mixed and used in the compound.

In an exemplary embodiment of the present application, deuterium is one of the isotopes of hydrogen, is an element that has a deuteron composed of one proton and one neutron as a nucleus, and may be represented by hydrogen-2, and the element symbol may also be expressed as D or 2H.

In an exemplary embodiment of the present application, the isotope means an atom with the same atomic number (Z), but different mass numbers (A), and the isotope may be interpreted as an element which has the same number of protons, but different number of neutrons.

In an exemplary embodiment of the present application, when the total number of substituents of a basic compound is defined as T1 and the number of specific substituents among the substituents is defined as T2, the content T% of the specific substituent may be defined as T2/T1×100 = T%.

That is, in an example, the deuterium content of 20% in a phenyl group represented by 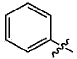 may be represented by 20% when the total number of substituents that the phenyl group can have is 5 (T1 in the formula) and the number of deuteriums among the substituents is 1 (T2 in the formula). That is, a deuterium content of 20% in the phenyl group may be represented by the following structural formula.

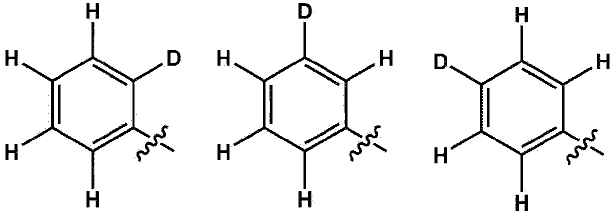

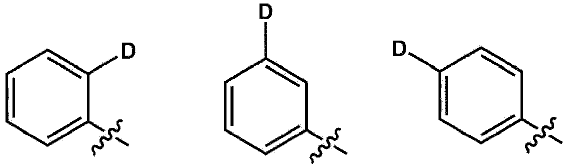

Further, in an exemplary embodiment of the present application, "a phenyl group having a deuterium content of 0%" may mean a phenyl group that does not include a deuterium atom, that is, has five hydrogen atoms.

In the present specification, the halogen may be fluorine, chlorine, bromine or iodine.

In the present specification, the alkyl group includes a straight-chain or branched-chain having 1 to 60 carbon atoms, and may be additionally substituted with another substituent. The number of carbon atoms of the alkyl group may be 1 to 60, specifically 1 to 40, and more specifically 1 to 20. Specific examples thereof include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethyl-butyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like, but are not limited thereto.

In the present specification, the alkenyl group includes a straight-chain or branched-chain having 2 to 60 carbon atoms, and may be additionally substituted with another substituent. The number of carbon atoms of the alkenyl group may be 2 to 60, specifically 2 to 40, and more specifically 2 to 20. Specific examples thereof include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like, but are not limited thereto.

In the present specification, the alkynyl group includes a straight-chain or branched-chain having 2 to 60 carbon atoms, and may be additionally substituted with another substituent. The number of carbon atoms of the alkynyl group may be 2 to 60, specifically 2 to 40, and more specifically 2 to 20.

In the present specification, an alkoxy group may be straight-chained, branched, or cyclic. The number of carbon atoms of the alkoxy group is not particularly limited, but is preferably 1 to 20. Specific examples thereof include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and the like, but are not limited thereto.

In the present specification, the cycloalkyl group includes a monocycle or polycycle having 3 to 60 carbon atoms, and may be additionally substituted with another substituent. Here, the polycycle means a group in which a cycloalkyl group is directly linked to or fused with another cyclic group. Here, another cyclic group may also be a cycloalkyl group, but may also be another kind of cyclic group, for example, a heterocycloalkyl group, an aryl group, a heteroaryl group, and the like. The number of carbon atoms of the cycloalkyl group may be 3 to 60, specifically 3 to 40, and more specifically 5 to 20. Specific examples thereof include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group, and the like, but are not limited thereto.

In the present specification, the heterocycloalkyl group includes O, S, Se, N, or Si as a heteroatom, includes a monocycle or polycycle having 2 to 60 carbon atoms, and may be additionally substituted with another substituent. Here, the polycycle means a group in which a heterocycloalkyl group is directly linked to or fused with another cyclic group. Here, another cyclic group may also be a heterocycloalkyl group, but may also be another kind of cyclic group, for example, a cycloalkyl group, an aryl group, a heteroaryl group, and the like. The number of carbon atoms of the heterocycloalkyl group may be 2 to 60, specifically 2 to 40, and more specifically 3 to 20.

In the present specification, the aryl group includes a monocycle or polycycle having 6 to 60 carbon atoms, and may be additionally substituted with another substituent. Here, the polycycle means a group in which an aryl group is directly linked to or fused with another cyclic group. Here, another cyclic group may also be an aryl group, but may also be another kind of cyclic group, for example, a cycloalkyl group, a heterocycloalkyl group, a heteroaryl group, and the like. The aryl group includes a spiro group. The number of carbon atoms of the aryl group may be 6 to 60, specifically 6 to 40, and more specifically 6 to 25. Specific examples of the aryl group include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused cyclic group thereof, and the like, but are not limited thereto.

In the present specification, a phosphine oxide group is represented by -P(=O)R101R102, and R101 and R102 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the phosphine oxide group include a diphenylphosphine oxide group, dinaphthylphosphine oxide, and the like, but are not limited thereto.

In the present specification, a silyl group includes Si and is a substituent to which the Si atom is directly linked as a radical, and is represented by -SiR104R105R106, and R104 to R106 are the same as or different from each other, and may be each independently a substituent composed of at least one of hydrogen; deuterium; a halogen group; an alkyl group; an alkenyl group; an alkoxy group; a cycloalkyl group; an aryl group; and a heterocyclic group. Specific examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but are not limited thereto.

In the present specification, the fluorenyl group may be substituted, and adjacent substituents may be bonded to each other to form a ring.

In the present specification, the spiro group is a group including a spiro structure, and may have 15 to 60 carbon atoms. For example, the spiro group may include a structure in which a 2,3-dihydro-1H-indene group or a cyclohexane group is spiro-bonded to a fluorenyl group. Specifically, the spiro group may include any one of the groups of the following structural formulae.

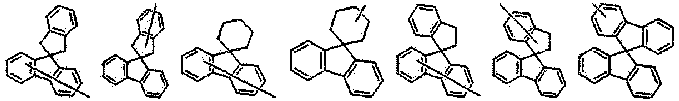

In the present specification, the heteroaryl group includes S, O, Se, N, or Si as a heteroatom, includes a monocycle or polycycle having 2 to 60 carbon atoms, and may be additionally substituted with another substituent. Here, the polycycle means a group in which a heteroaryl group is directly linked to or fused with another cyclic group. Here, another cyclic group may also be a heteroaryl group, but may also be another kind of cyclic group, for example, a cycloalkyl group, a heterocycloalkyl group, an aryl group, and the like. The number of carbon atoms of the heteroaryl group may be 2 to 60, specifically 2 to 40, and more specifically 3 to 25. Specific examples of the heteroaryl group include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a quinozolilyl group, a naphthyridyl group, an acridinyl group, a phenanthridinyl group, an imidazopyridinyl group, a diaza naphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi (dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepin group, a 9,10-dihydroacridinyl group, a phenanthrazinyl group, a phenothiazinyl group, a phthalazinyl group, a naphthylidinyl group, a phenanthrolinyl group, a benzo[c][1,2,5]thiadiazolyl group, a 5,10-dihydrodibenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group, and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and the number of carbon atoms thereof is not particularly limited, but is preferably 1 to 30. Specific examples of the amine group include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group, and the like, but are not limited thereto.

In the present specification, an arylene group means that there are two bonding positions in an aryl group, that is, a divalent group. The above-described description on the aryl group may be applied to the arylene group, except that the arylene groups are each a divalent group. Further, a heteroarylene group means that there are two bonding positions in a heteroaryl group, that is, a divalent group. The above-described description on the heteroaryl group may be applied to the heteroarylene group, except for a divalent heteroarylene group.

In the present specification, the "adjacent" group may mean a substituent substituted with an atom directly linked to an atom in which the corresponding substituent is substituted, a substituent disposed to be sterically closest to the corresponding substituent, or another substituent substituted with an atom in which the corresponding substituent is substituted. For example, two substituents substituted at the ortho position in a benzene ring and two substituents substituted with the same carbon in an aliphatic ring may be interpreted as groups which are "adjacent" to each other.

A heterocyclic compound according to the present application is represented by claim 1. More specifically, the heterocyclic compound may be used as a material for an organic material layer of an organic light emitting device by the structural characteristics of the core structure and the substituent as described above.

In an exemplary embodiment of the present application, the deuterium content of the heterocyclic compound represented may be 0% to 100%.

In an exemplary embodiment of the present application, the deuterium content of the heterocyclic compound may be 10% to 100%.

In an exemplary embodiment of the present application, the deuterium content of the heterocyclic compound may be 20% to 100%.

In an exemplary embodiment of the present application, the deuterium content of the heterocyclic compound may be 30% to 100%.

In an exemplary embodiment of the present application, the deuterium content of the heterocyclic compound may be 40% to 100%.

According to the present application, the heterocyclic compound is represented by any one of the following compounds.

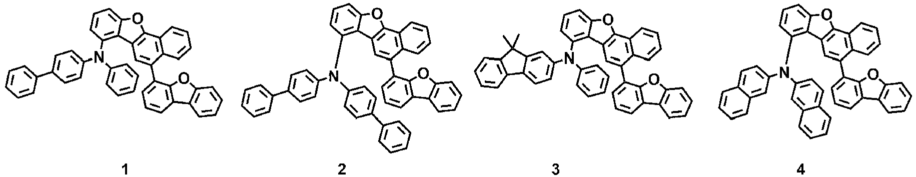

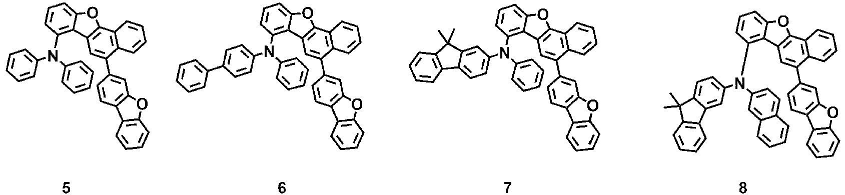

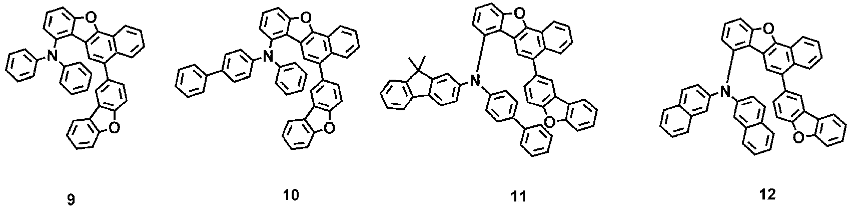

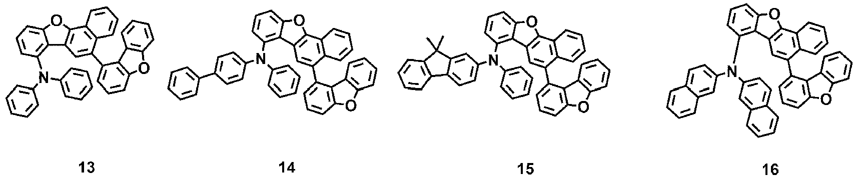

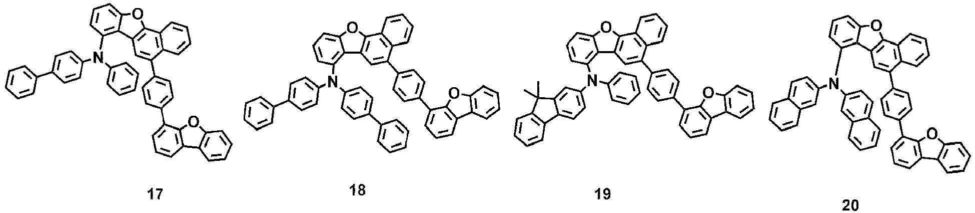

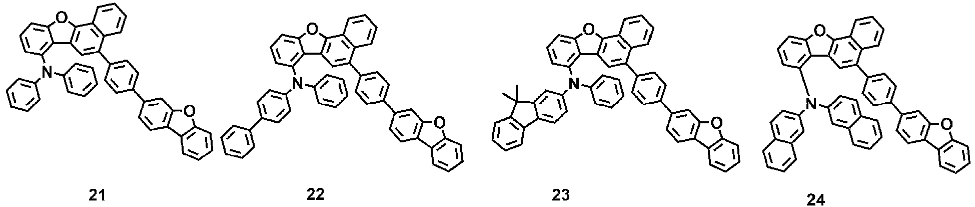

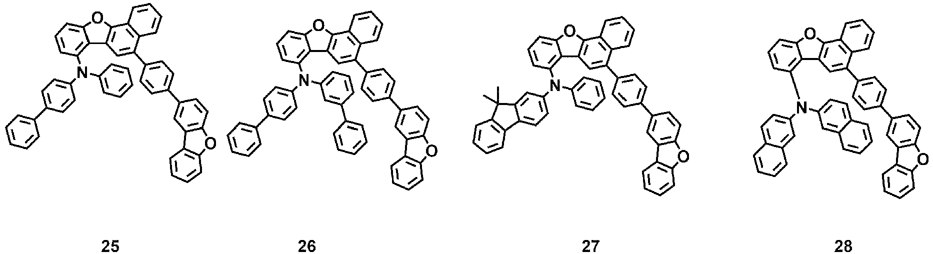

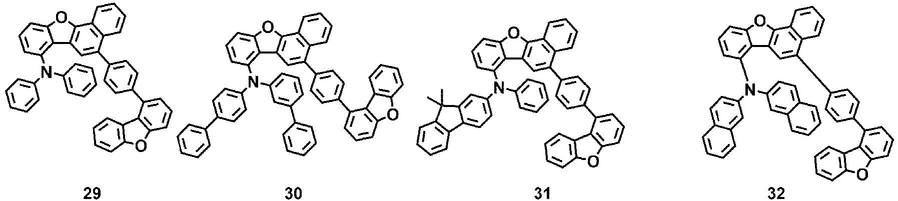

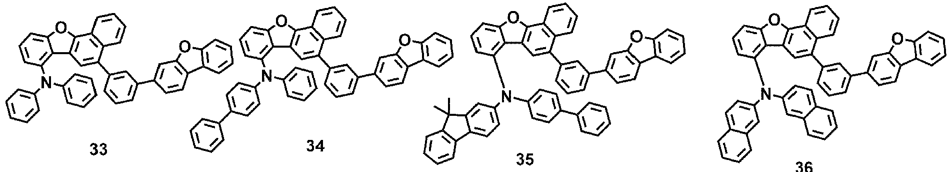

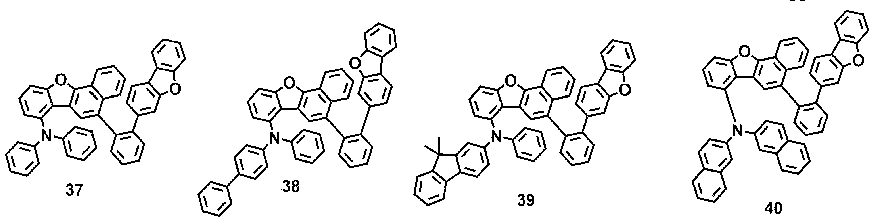

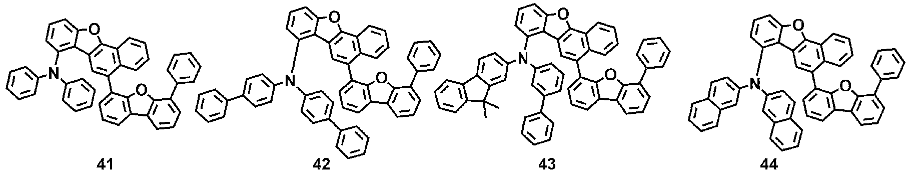

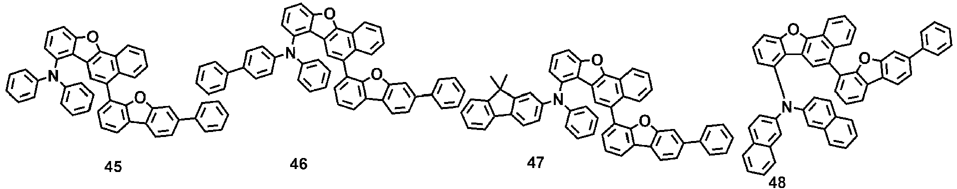

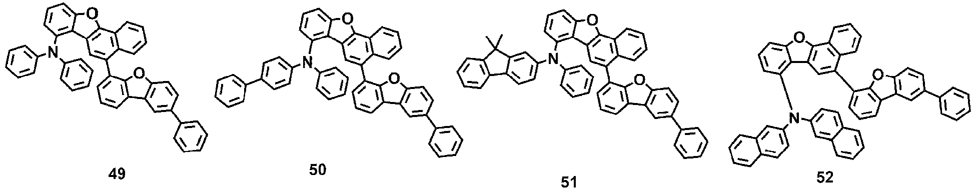

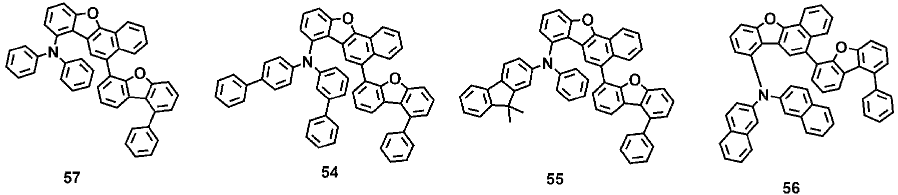

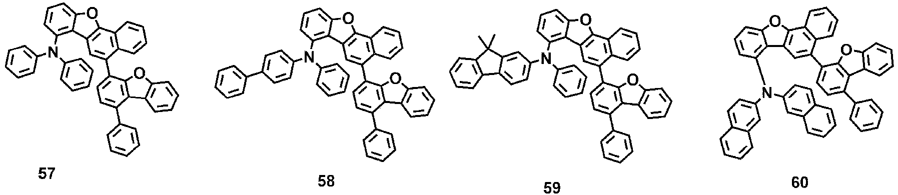

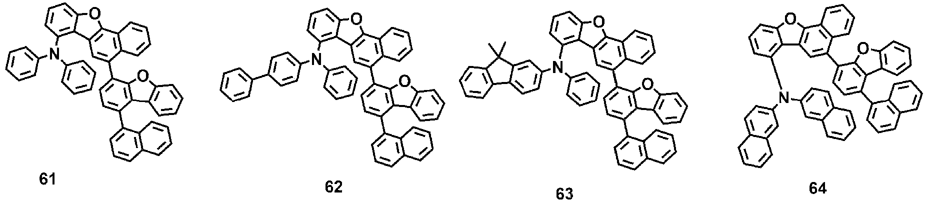

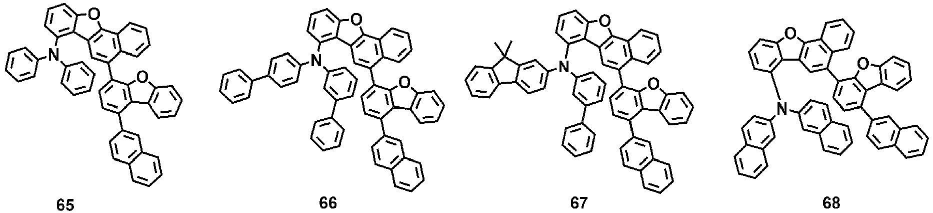

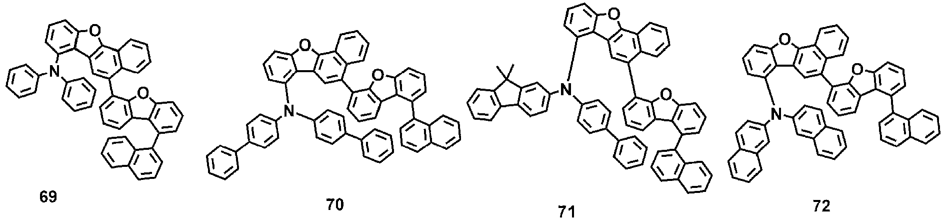

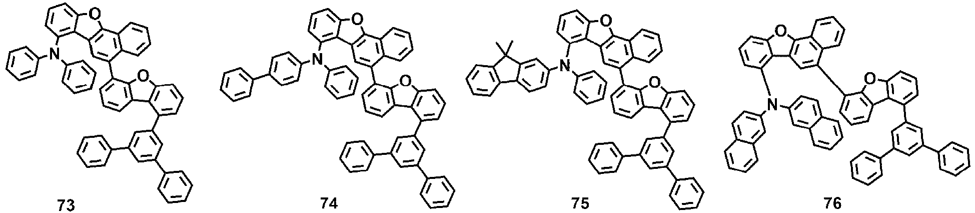

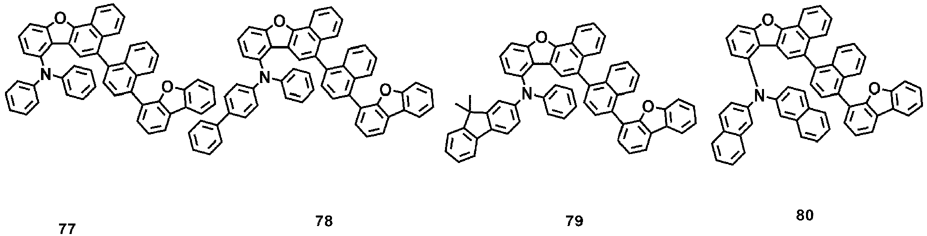

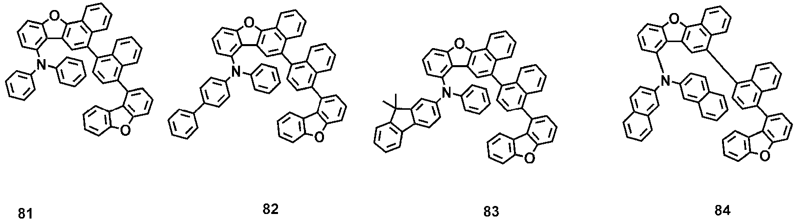

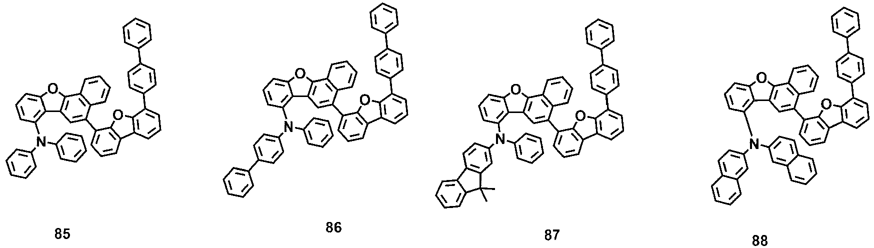

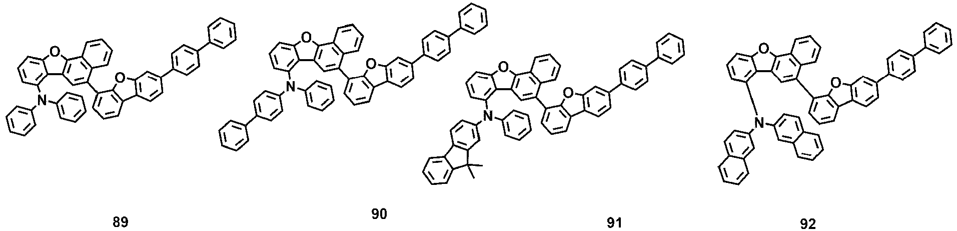

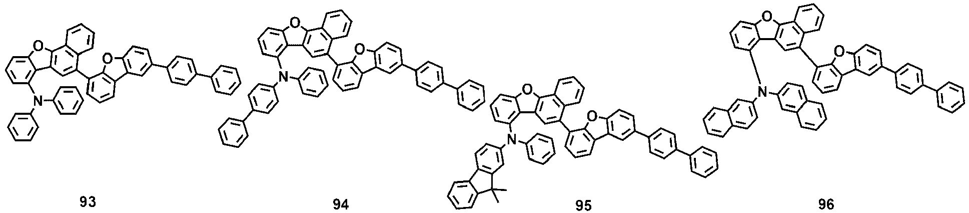

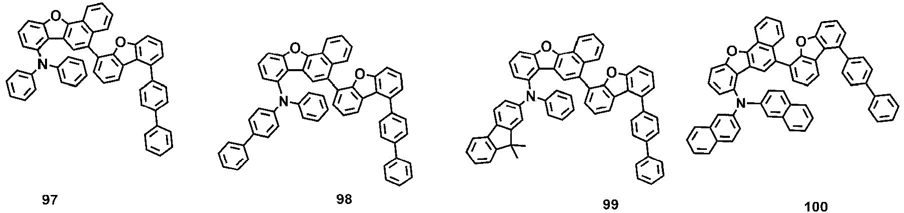

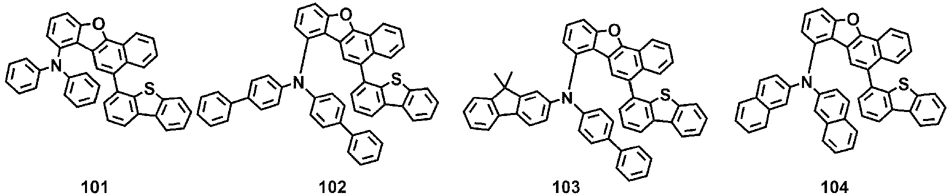

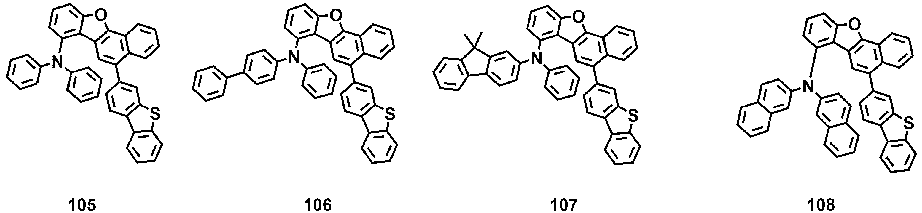

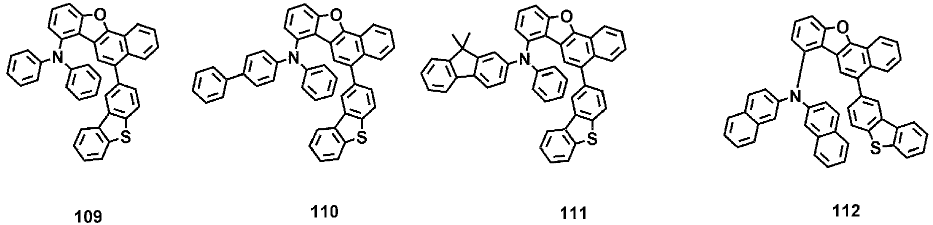

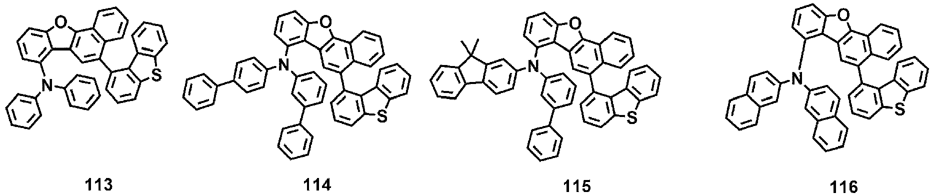

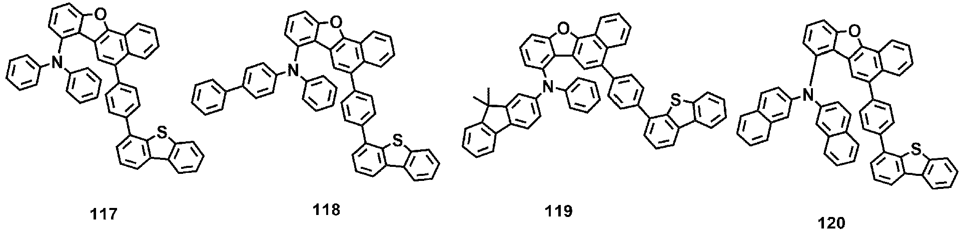

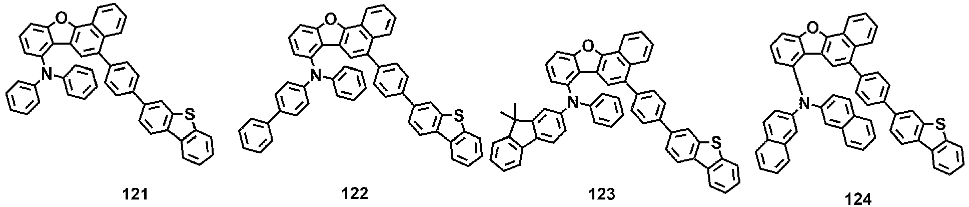

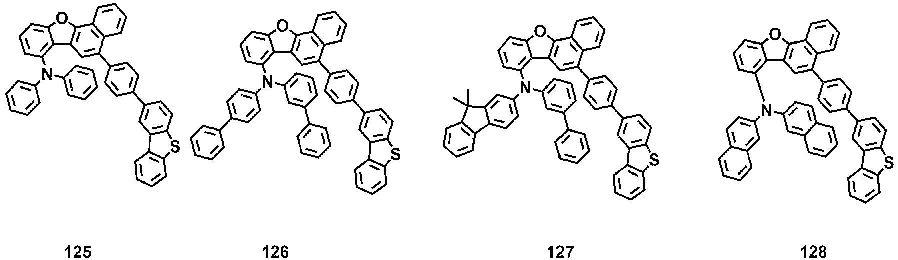

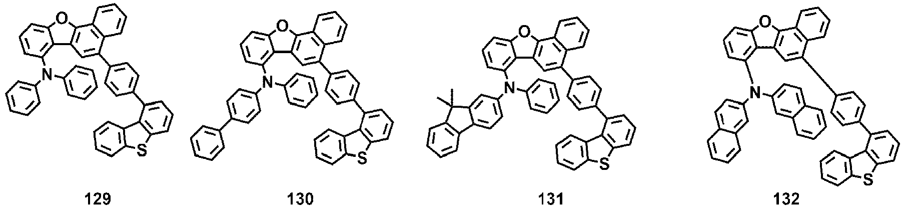

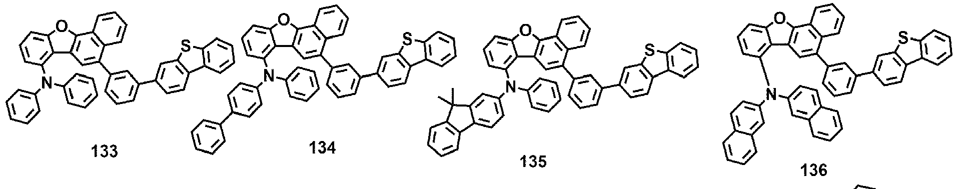

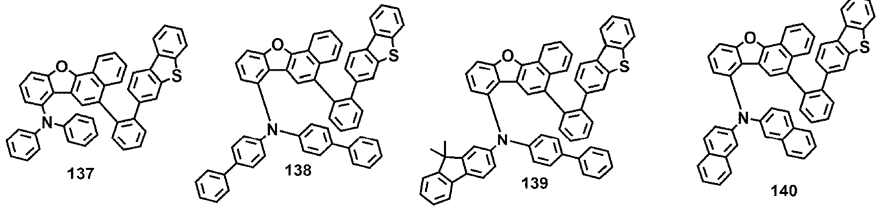

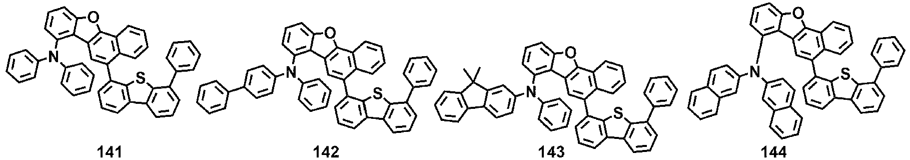

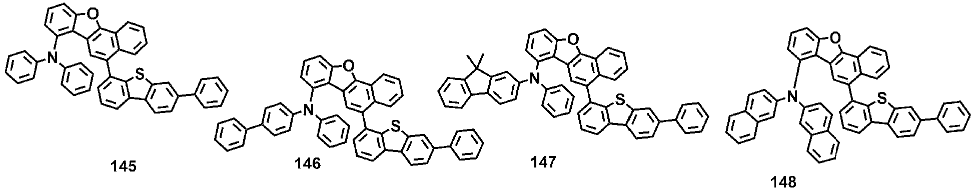

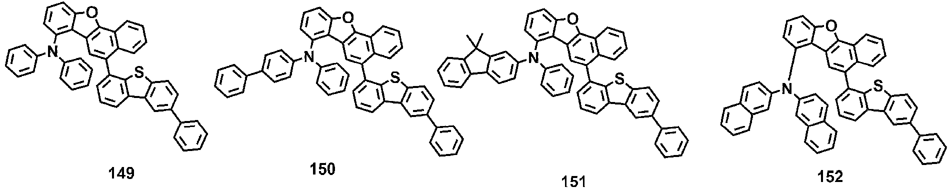

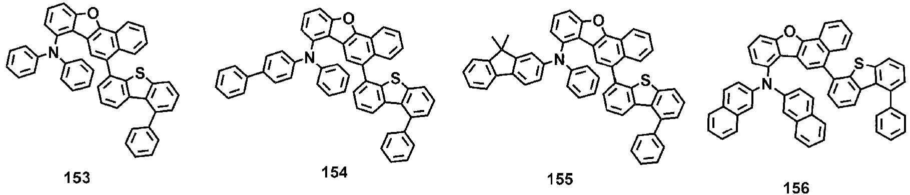

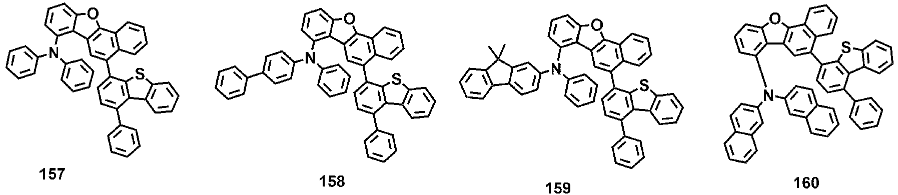

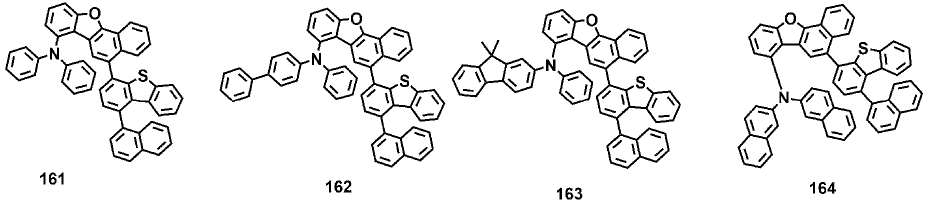

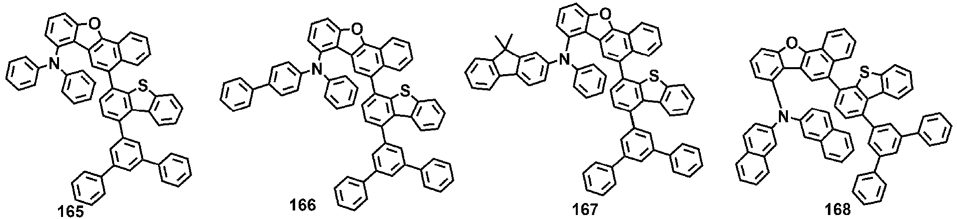

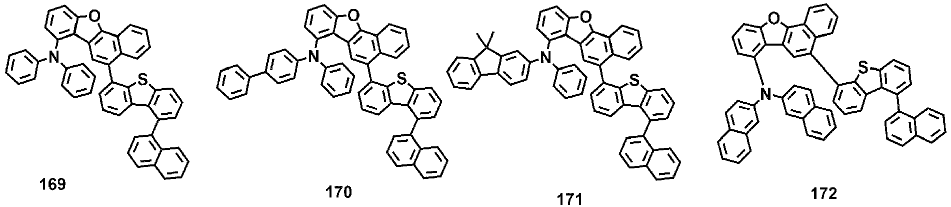

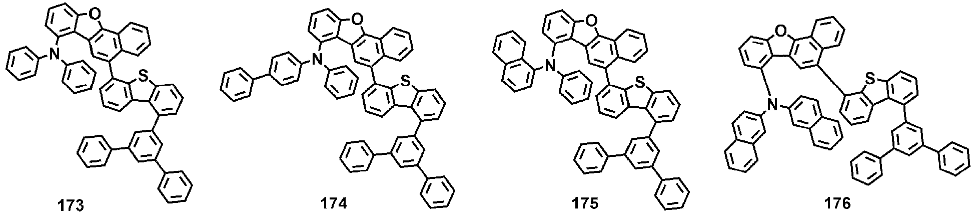

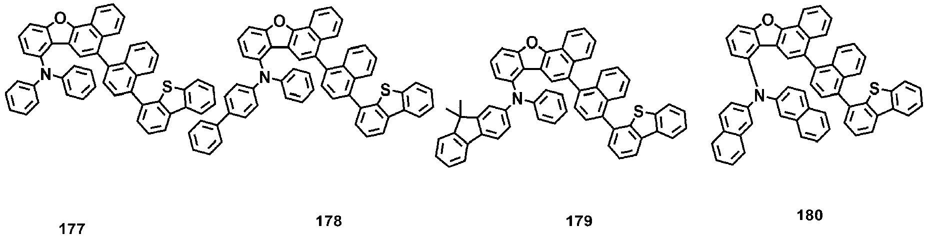

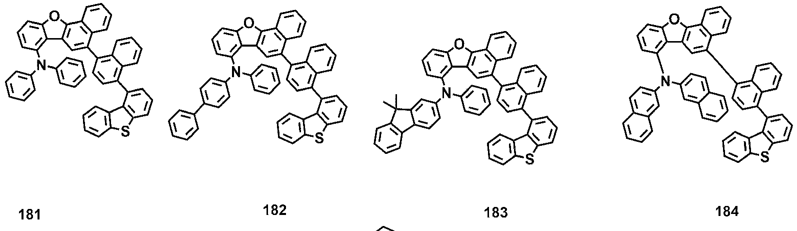

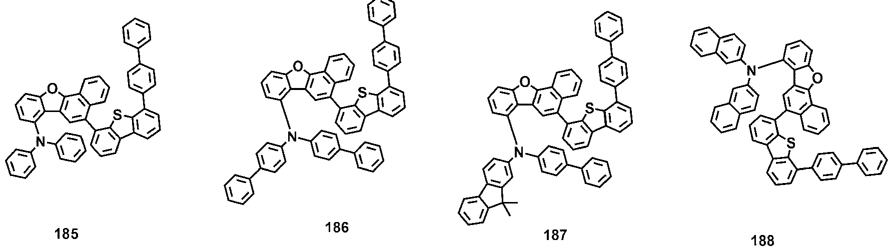

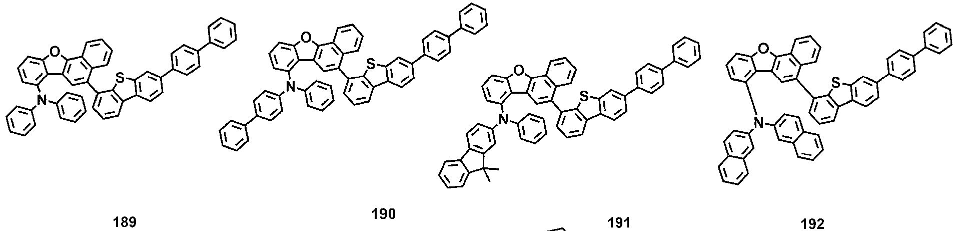

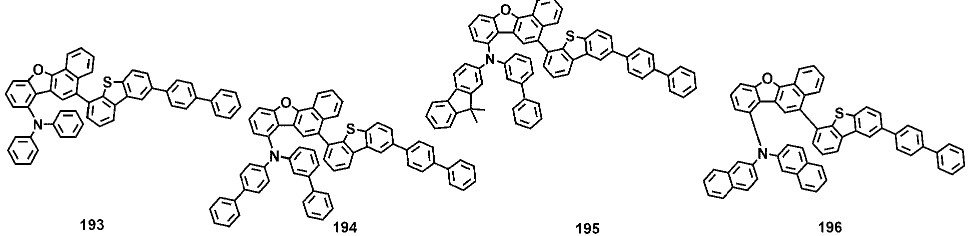

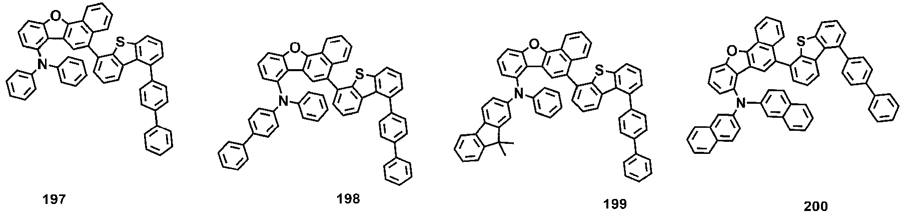

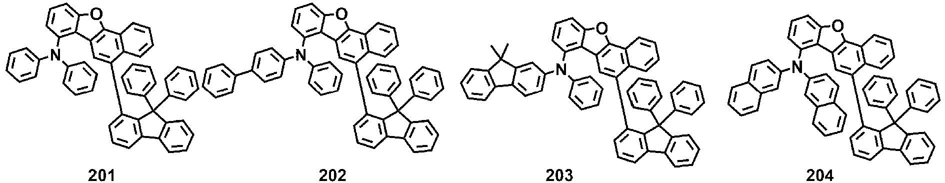

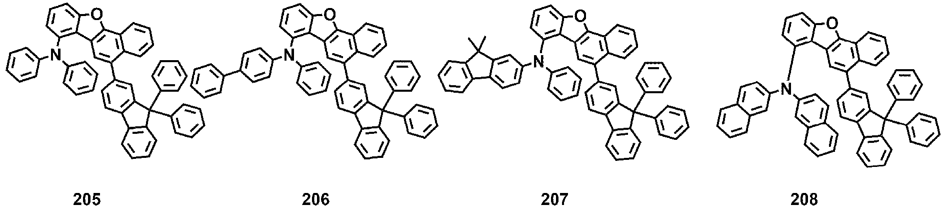

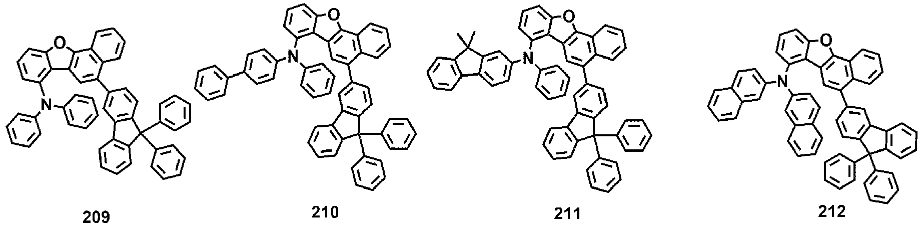

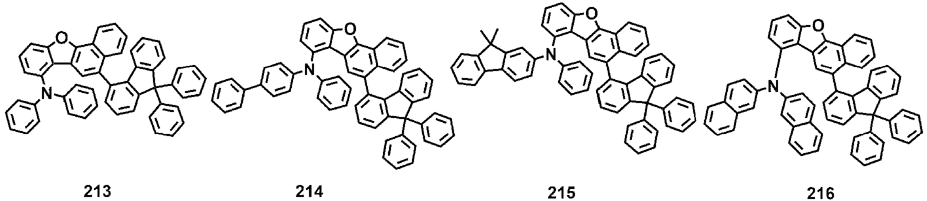

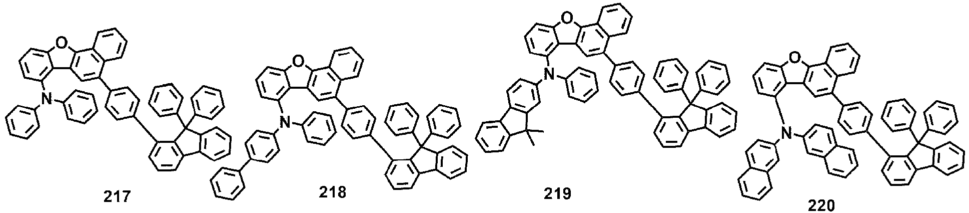

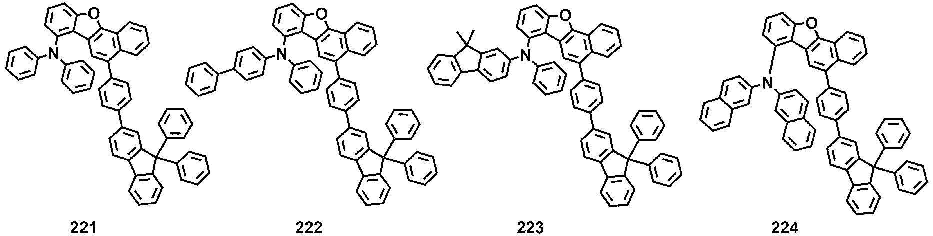

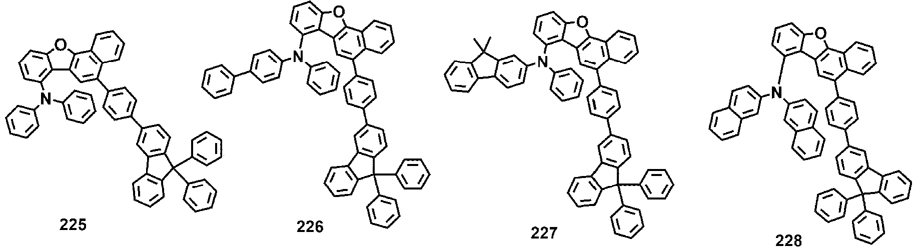

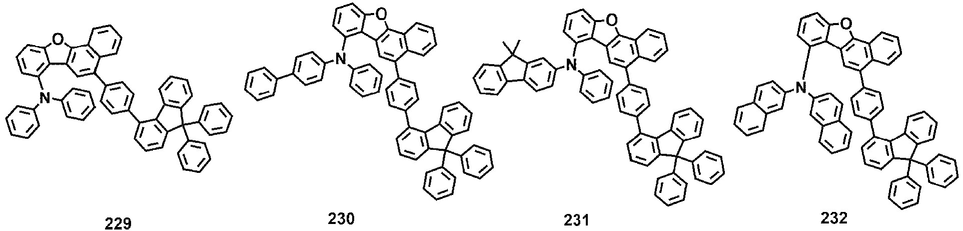

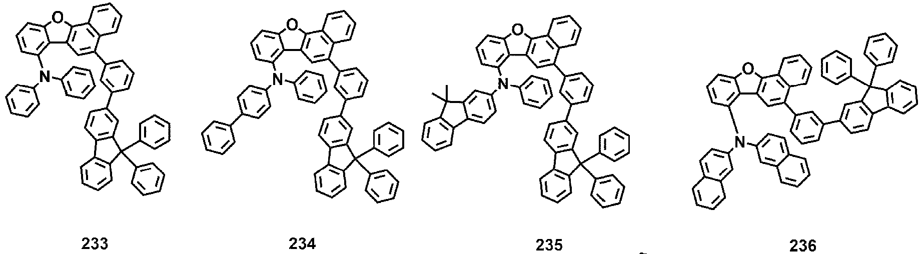

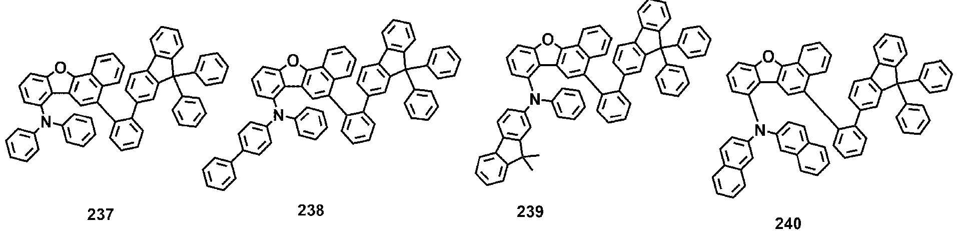

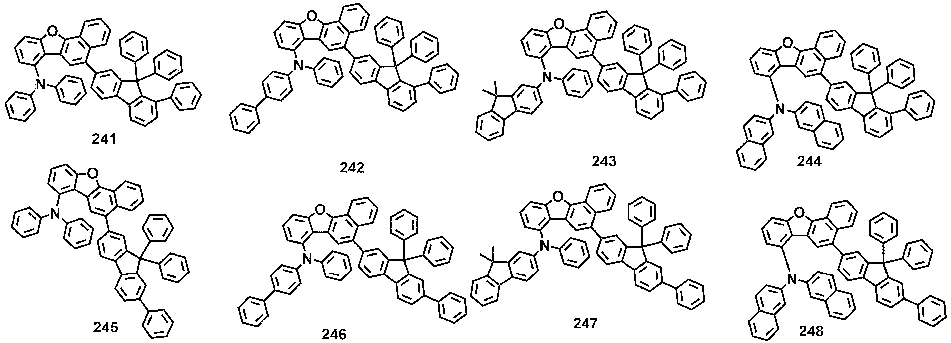

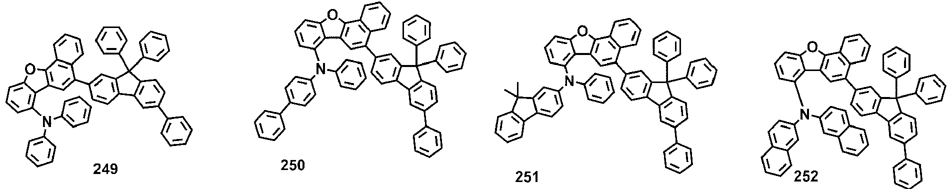

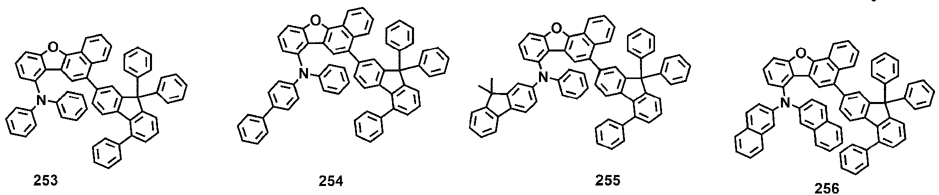

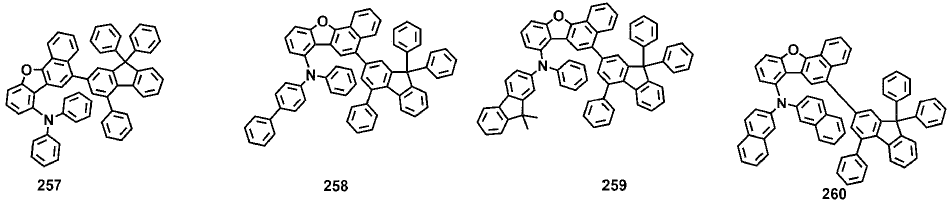

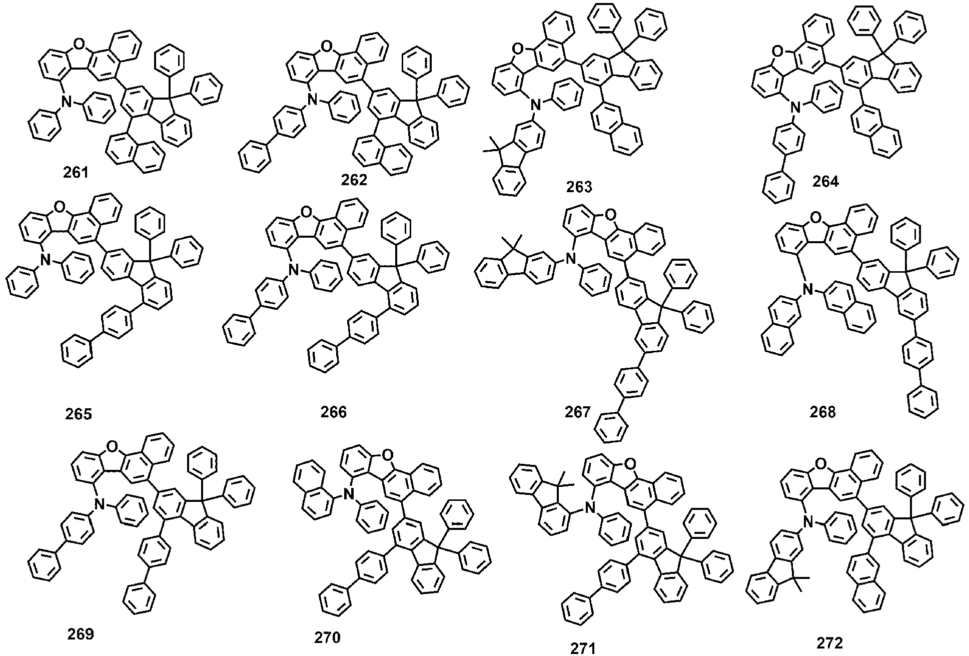

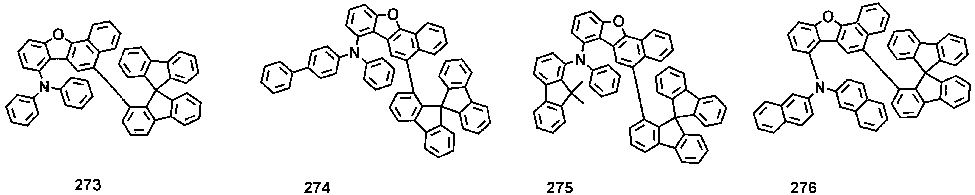

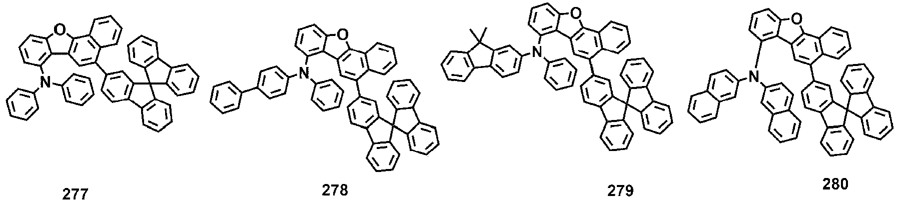

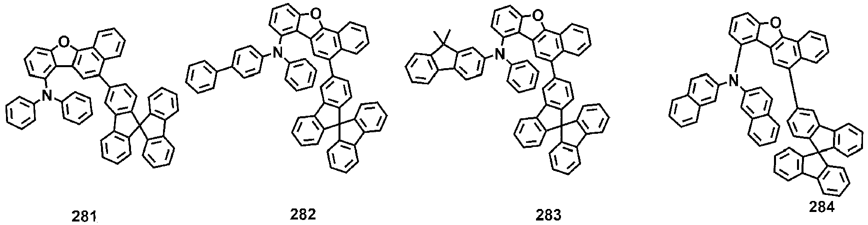

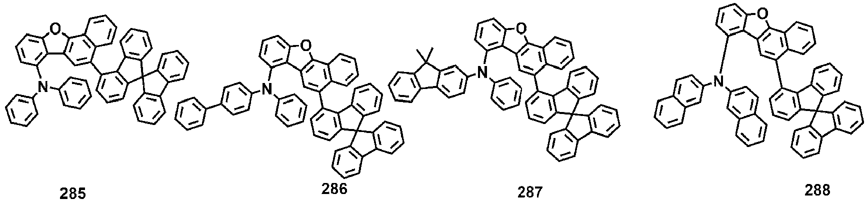

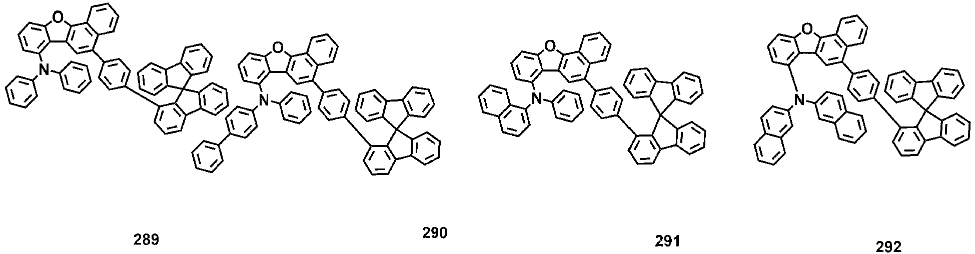

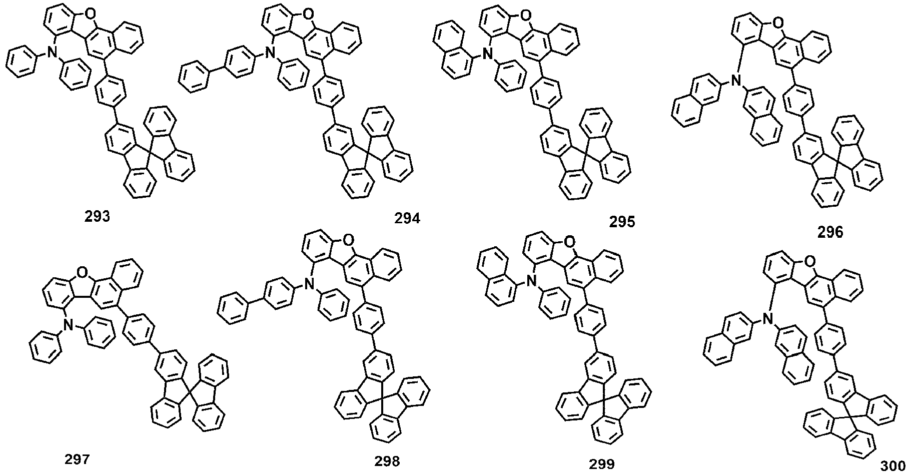

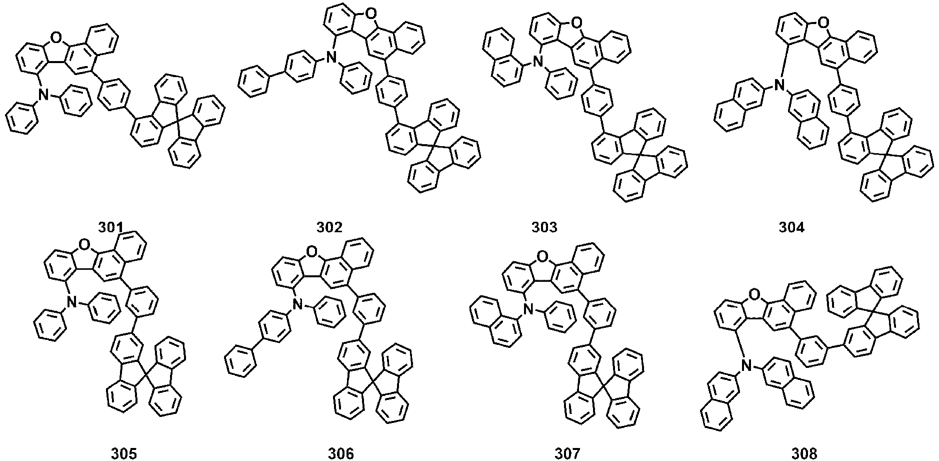

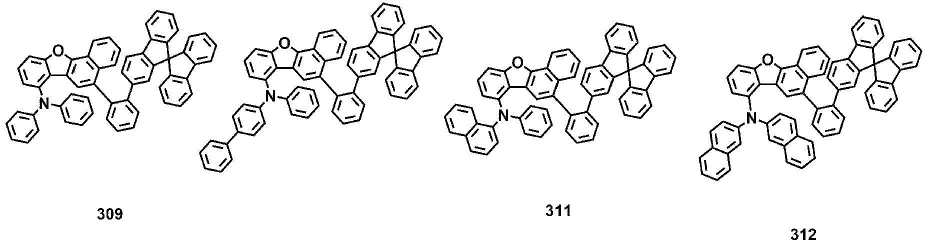

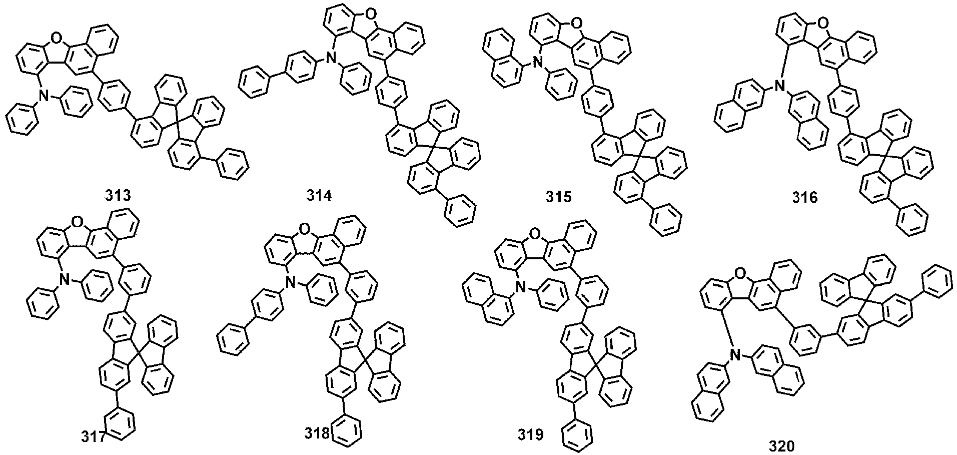

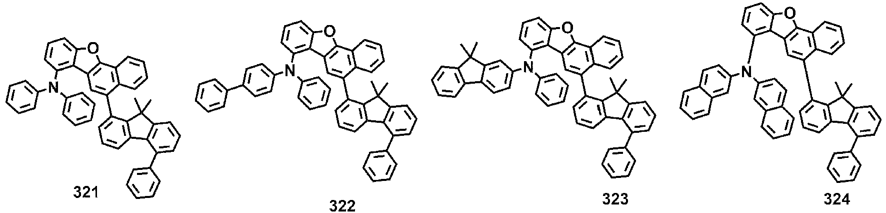

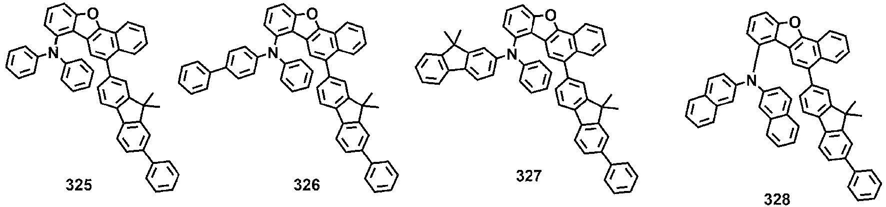

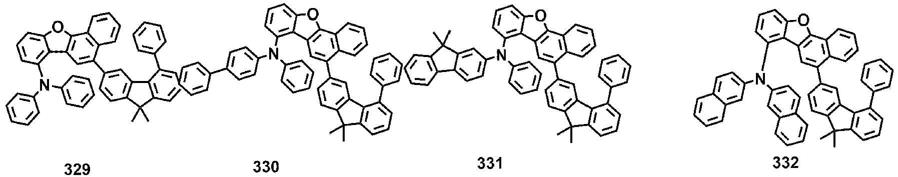

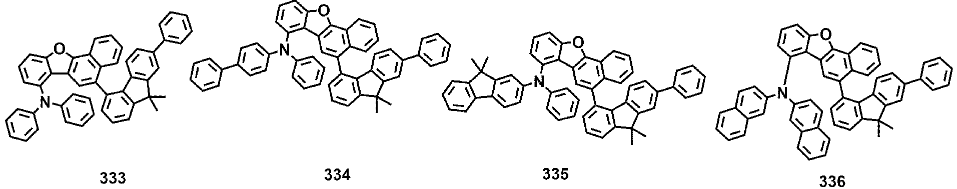

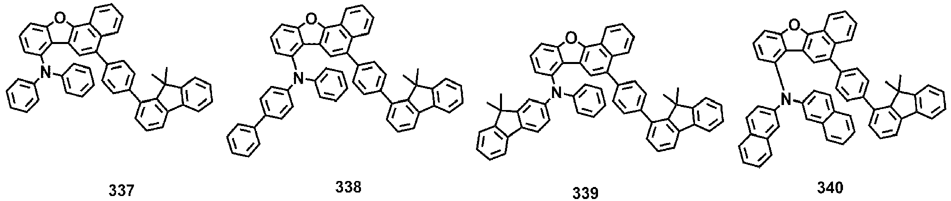

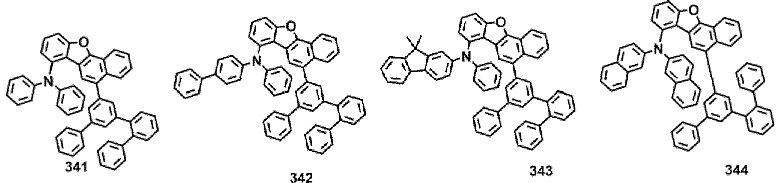

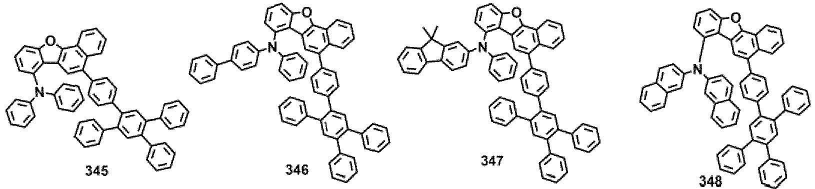

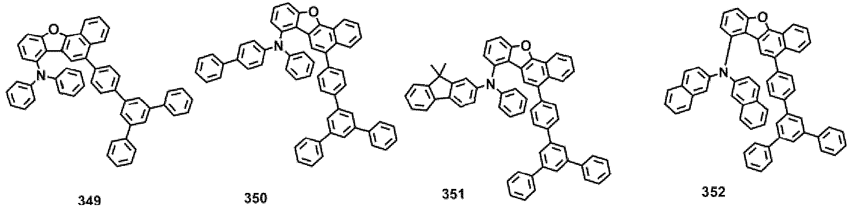

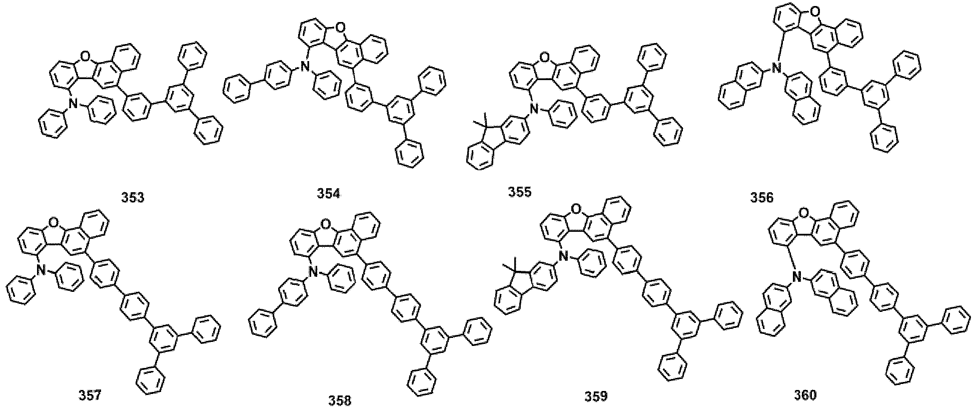

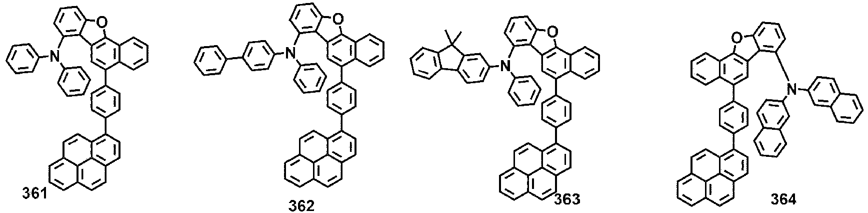

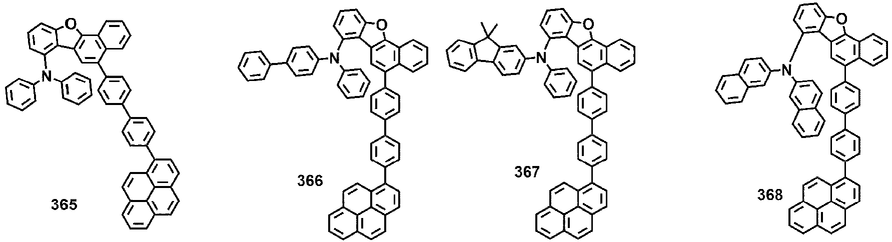

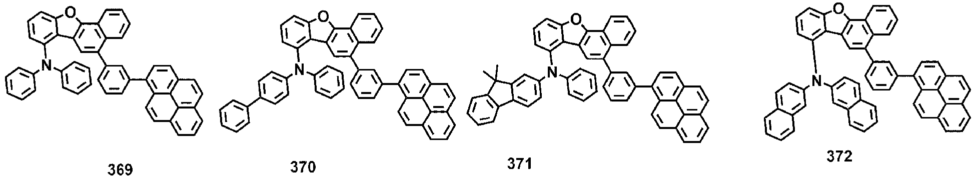

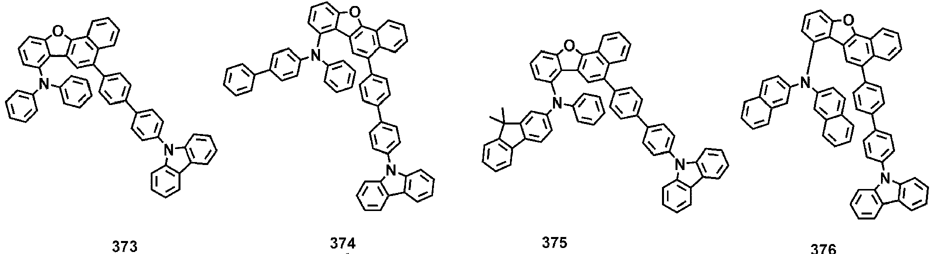

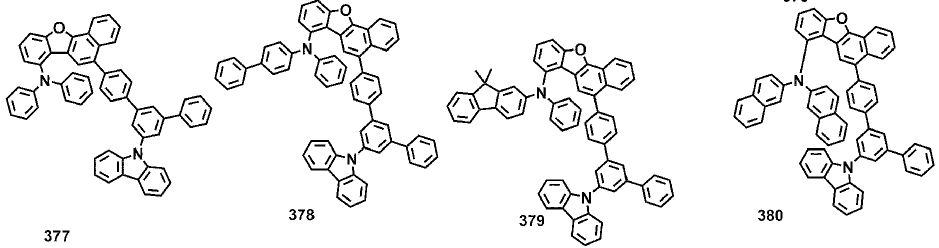

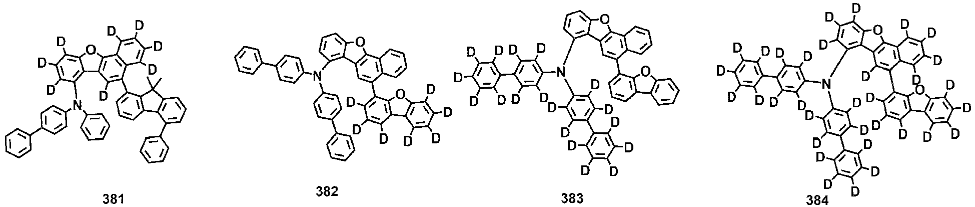

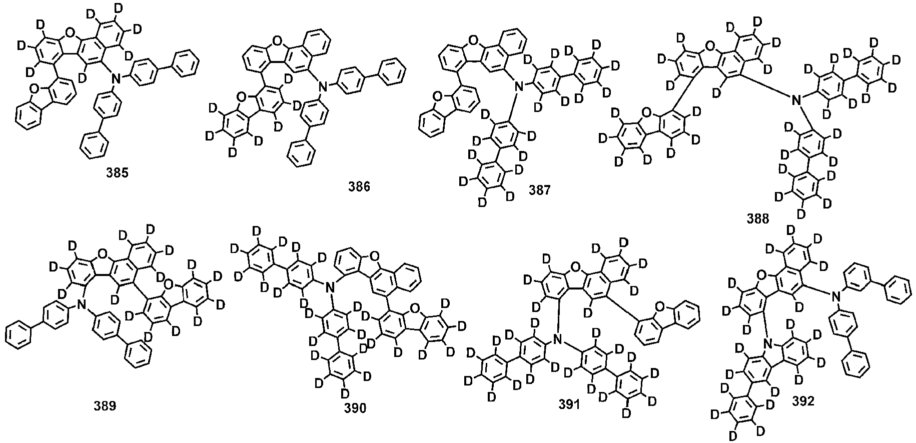

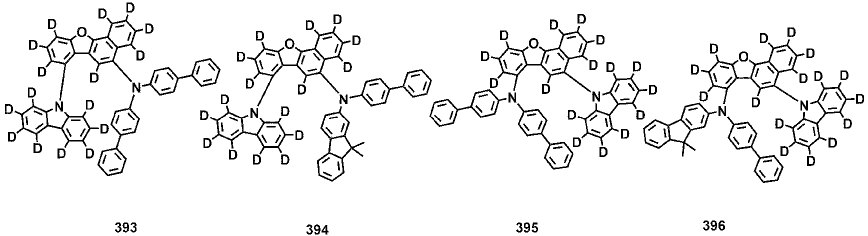

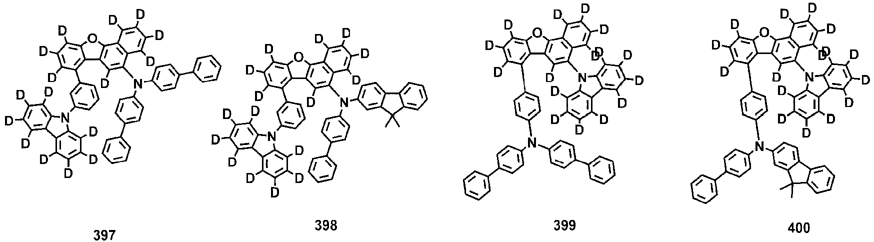

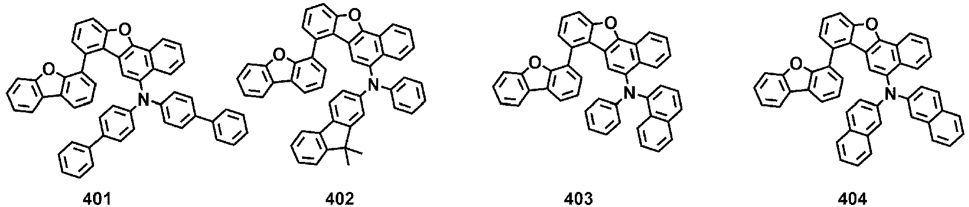

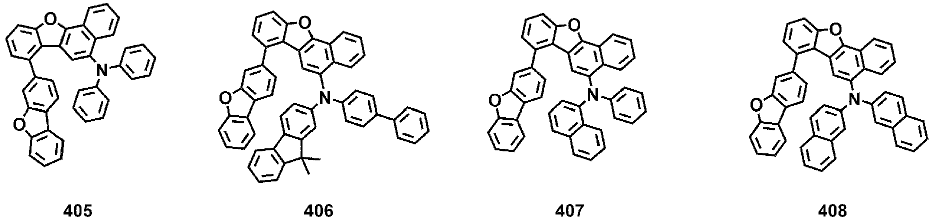

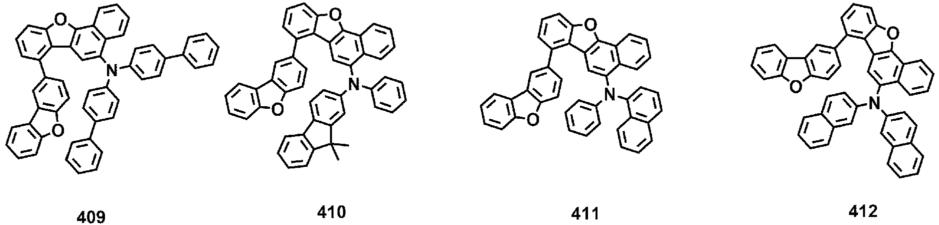

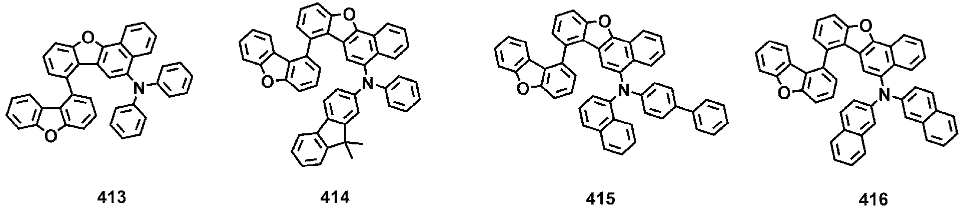

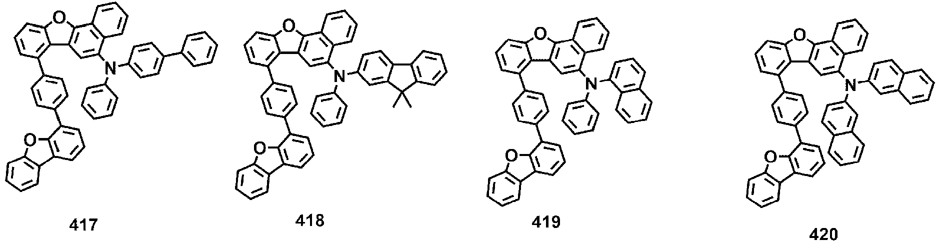

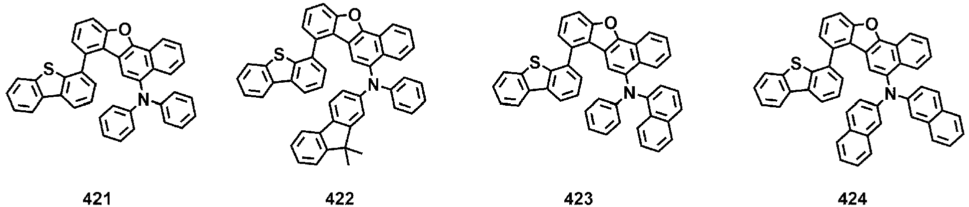

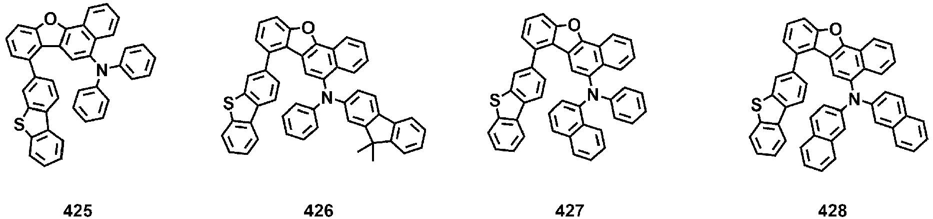

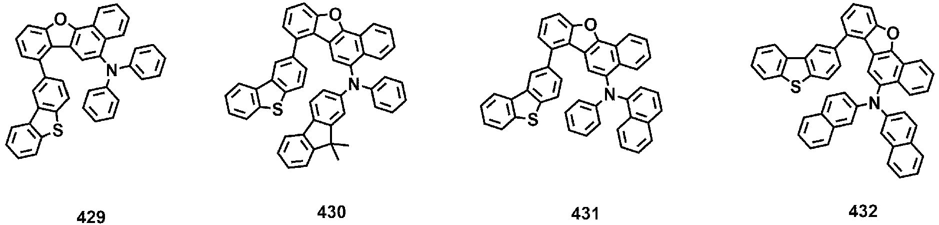

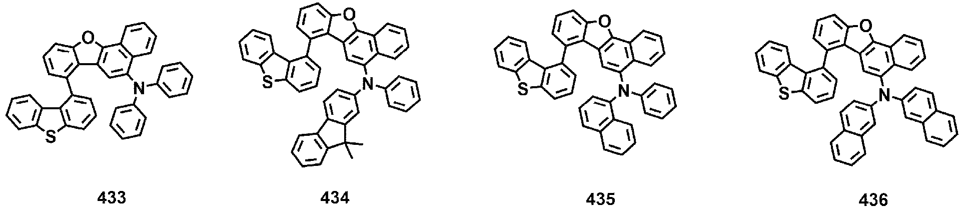

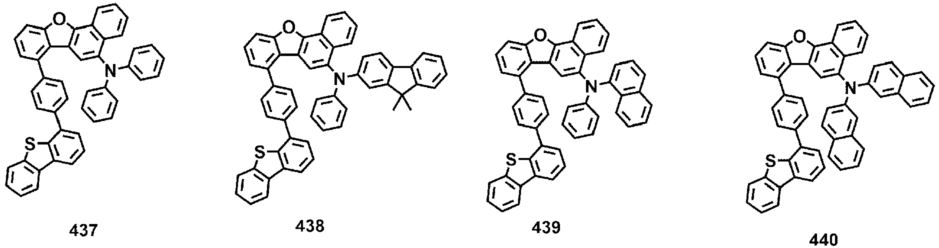

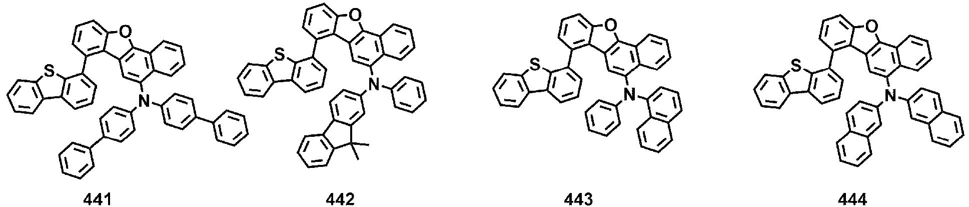

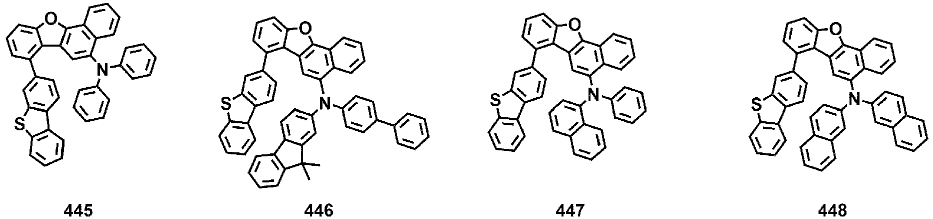

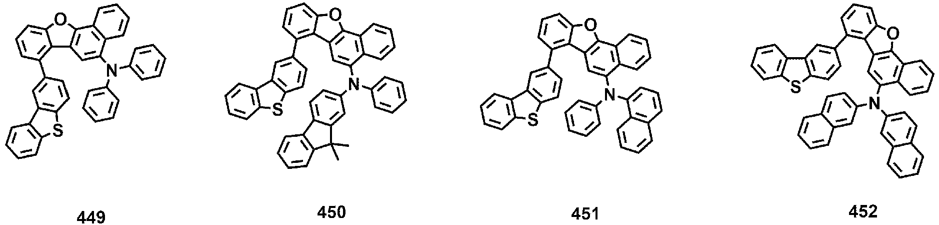

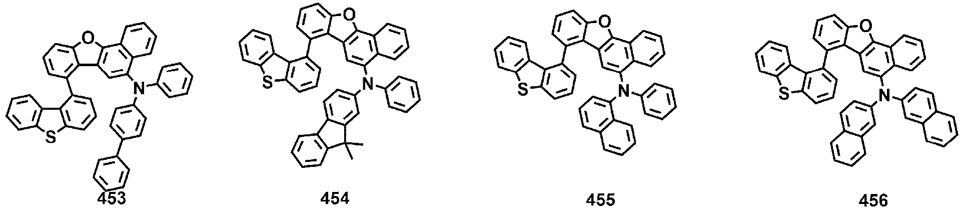

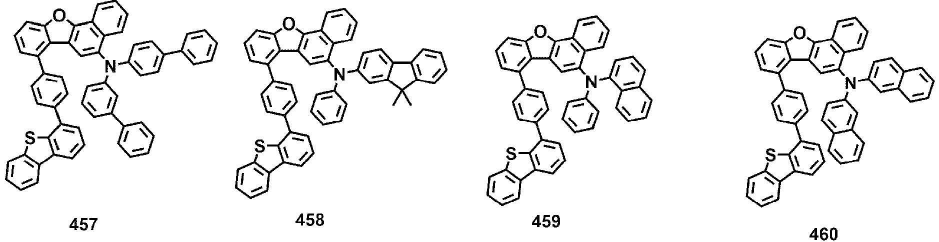

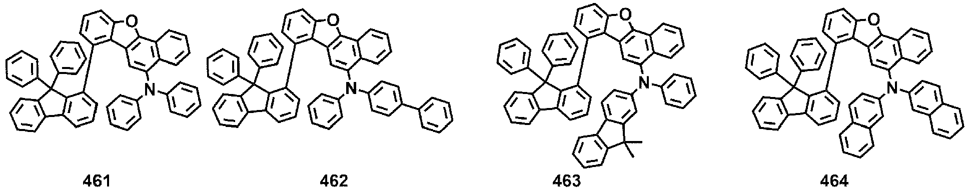

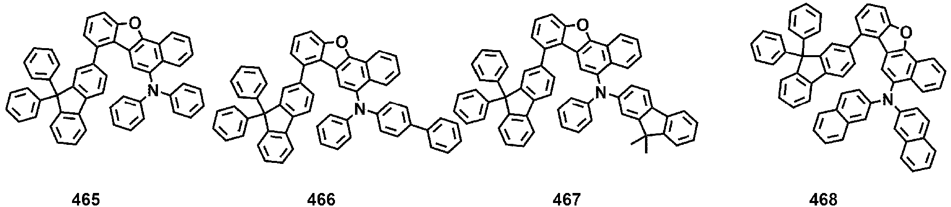

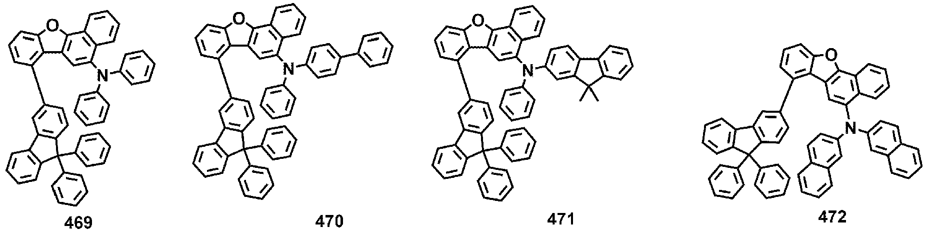

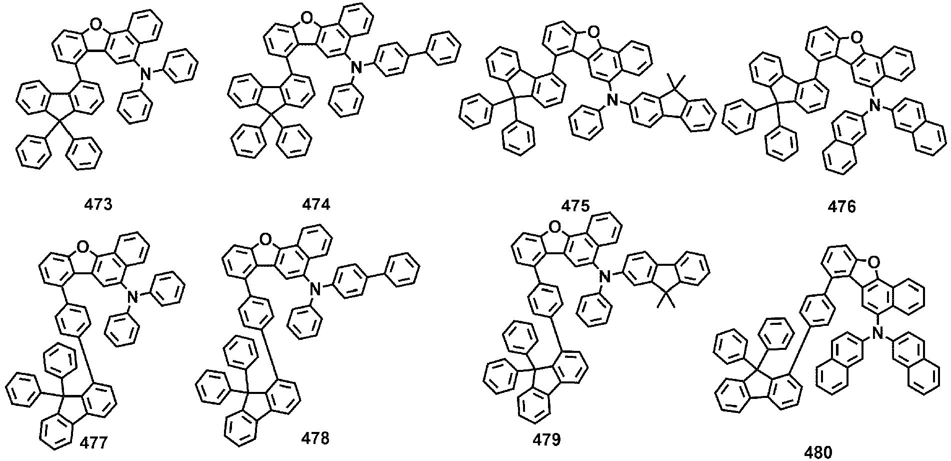

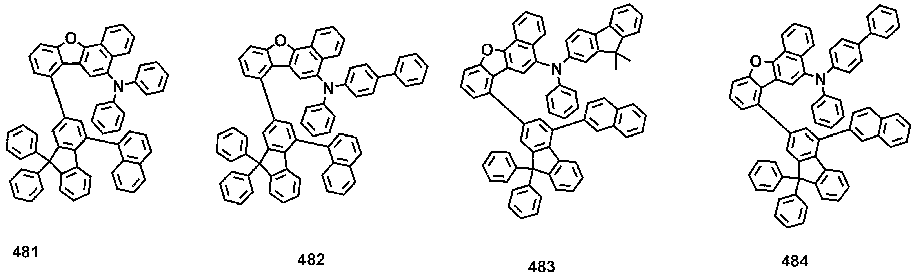

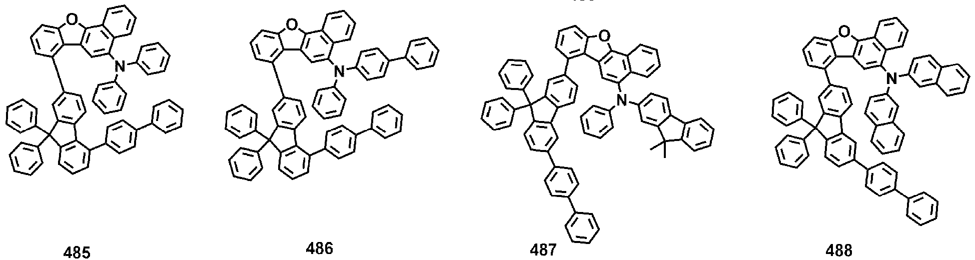

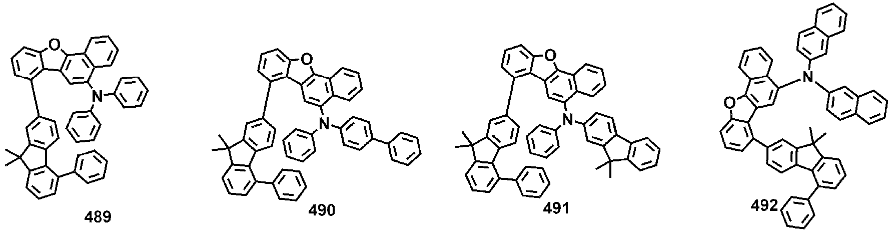

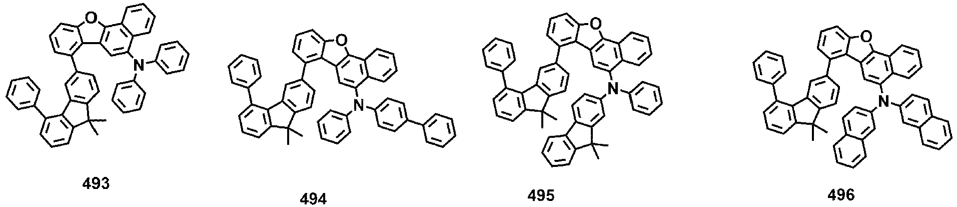

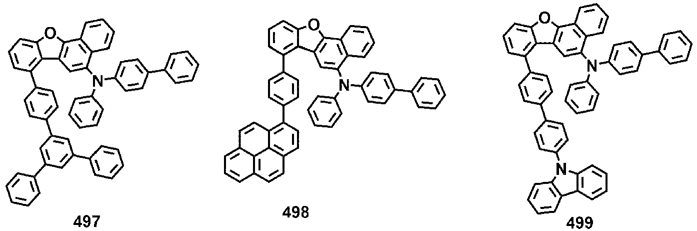

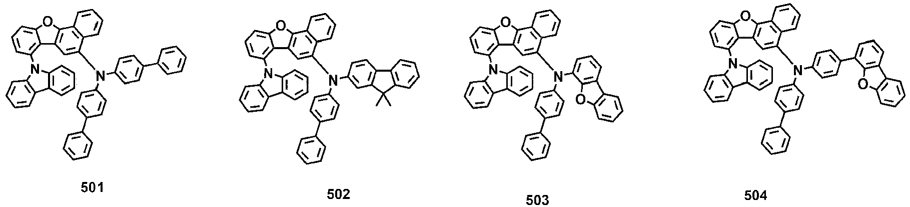

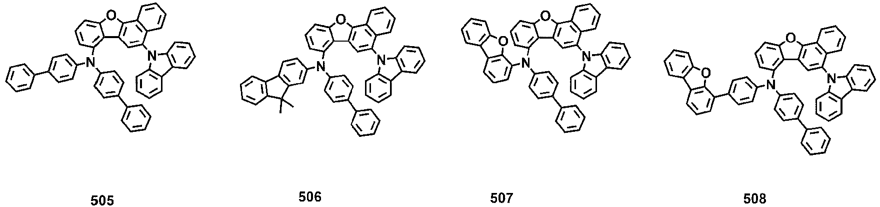

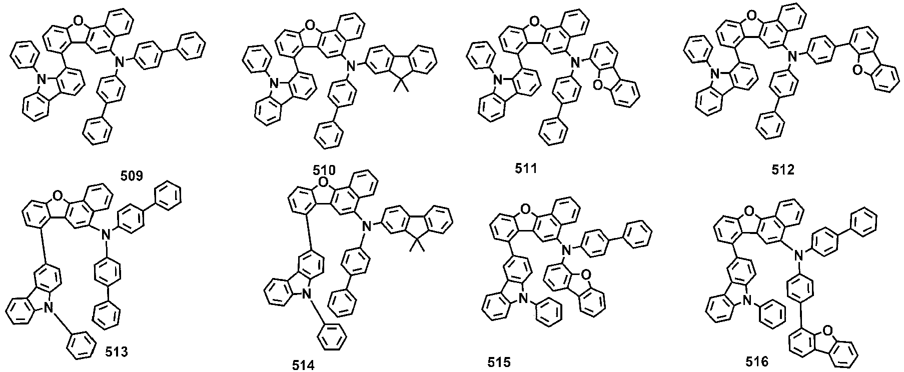

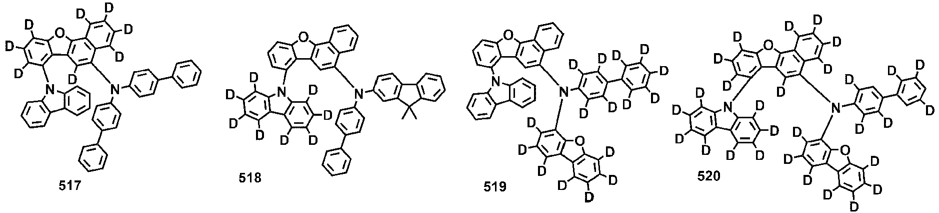

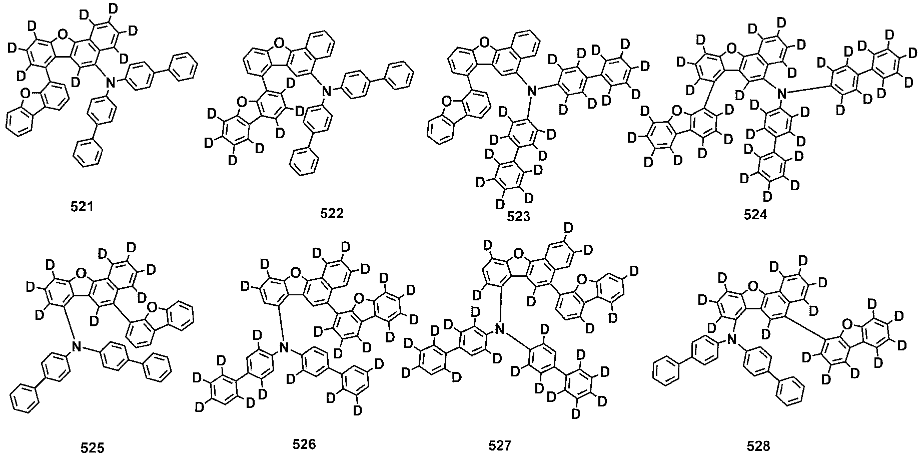

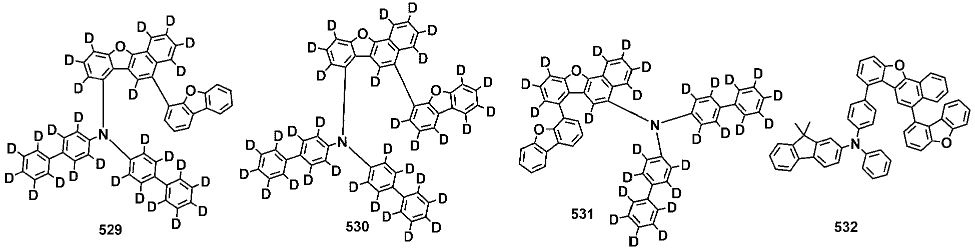

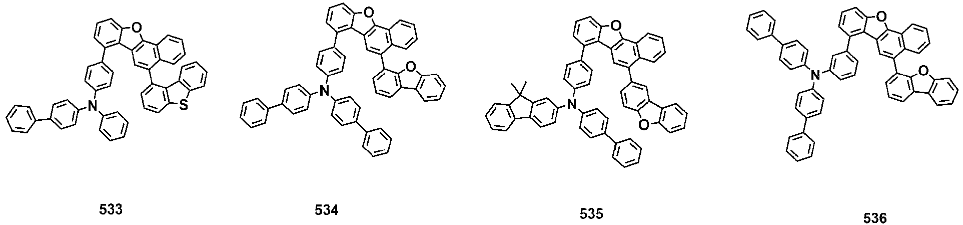

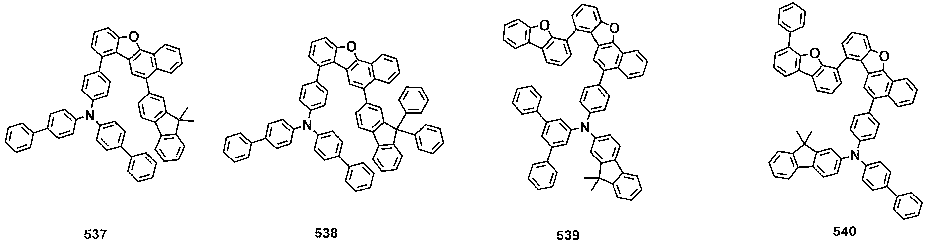

Meanwhile, the heterocyclic compound has a high glass transition temperature (Tg) and thus has excellent thermal stability. The increase in thermal stability becomes an important factor for providing a device with driving stability.

The heterocyclic compound according to an exemplary embodiment of the present application may be prepared by a multi-step chemical reaction. Some intermediate compounds are first prepared, and the compound may be prepared from the intermediate compounds. More specifically, the heterocyclic compound according to an exemplary embodiment of the present application may be prepared based on the Preparation Examples to be described below.

Another exemplary embodiment of the present application provides an organic light emitting device including the heterocyclic compound . The "organic light emitting device" may be expressed by terms such as "organic light emitting diode", "organic light emitting diodes (OLEDs)", "OLED device", and "organic electroluminescence device".

The heterocyclic compound may be formed as an organic material layer by not only a vacuum deposition method, but also a solution application method when an organic light emitting device is manufactured. Here, the solution application method means spin coating, dip coating, inkjet printing, screen printing, a spray method, roll coating, and the like, but is not limited thereto.

Specifically, the organic light emitting device according to an exemplary embodiment of the present application includes a first electrode, a second electrode and an organic material layer having one or more layers provided between the first electrode and the second electrode, in which one or more layers of the organic material layer include the heterocyclic compound . When the organic material layer includes the heterocyclic compound , the light emitting efficiency and service life of the organic light emitting device are excellent.

In an exemplary embodiment of the present application, the first electrode may be a positive electrode, and the second electrode may be a negative electrode.

In another exemplary embodiment, the first electrode may be a negative electrode, and the second electrode may be a positive electrode.

The organic material layer may include a hole transport auxiliary layer having one or more layers. In the present specification, the "hole transport auxiliary layer" is a layer disposed between the hole transport layer and the light emitting layer of the organic light emitting device, means a functional layer used for the purpose of improving the brightness and efficiency and service life characteristics of the organic light emitting device by preventing electrons from crossing from the opposite side of the electron transport layer, and may be referred to as an "electron protection layer" or a "prime layer". A material constituting the hole transport auxiliary layer may be determined by a light emitting material for the light emitting layer.

The organic material layer of the organic light emitting device according to an exemplary embodiment of the present application includes a hole transport auxiliary layer having one or more layers, and the hole transport auxiliary layer includes the heterocyclic compound . When the hole transport auxiliary layer among the organic material layers includes the heterocyclic compound , the light emitting efficiency and service life of the organic light emitting device are better.

The organic material layer of the organic light emitting device according to an exemplary embodiment of the present application includes a hole transport layer having one or more layers, and the hole transport layer includes the heterocyclic compound . When the hole transport layer among the organic material layers includes the heterocyclic compound , the light emitting efficiency and service life of the organic light emitting device are better.

The organic light emitting device of the present invention may further include one or two or more layers selected from the group consisting of a light emitting layer, a hole injection layer, a hole transport layer, an electron injection layer, an electron transport layer, a hole auxiliary layer, and a hole blocking layer.

The organic light emitting device according to an exemplary embodiment of the present application may be manufactured by typical manufacturing methods and materials of the organic light emitting device, except that the above-described heterocyclic compound is used to form an organic material layer.

FIGS. 1 to 3 exemplify the stacking sequence of the electrodes and the organic material layer of the organic light emitting device according to an exemplary embodiment of the present application. However, the scope of the present application is not intended to be limited by these drawings, and the structure of the organic light emitting device known in the art may also be applied to the present application.

According to FIG. 1, an organic light emitting device in which a positive electrode 200, an organic material layer 300, and a negative electrode 400 are sequentially stacked on a substrate 100 is illustrated. However, the organic light emitting device is not limited only to such a structure, and as in FIG. 2, an organic light emitting device in which a negative electrode, an organic material layer, and a positive electrode are sequentially stacked on a substrate may also be implemented.

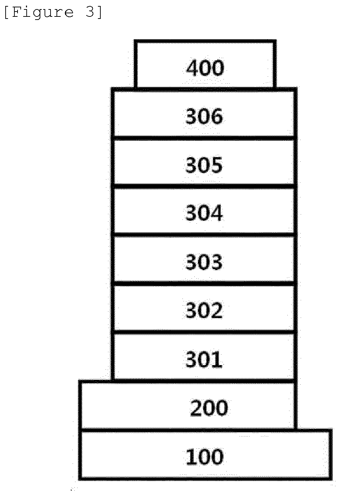

FIG. 3 exemplifies a case where an organic material layer is a multilayer. An organic light emitting device according to FIG. 3 includes a hole injection layer 301, a hole transport layer 302, a light emitting layer 303, a hole blocking layer 304, an electron transport layer 305, and an electron injection layer 306. However, the scope of the present application is not limited by the stacking structure as described above, and if necessary, the other layers except for the light emitting layer may be omitted, and another necessary functional layer may be further added.

The heterocyclic compound represented by Chemical Formula 1 may alone constitute one or more layers of the hole transport layer or electron blocking layer of the organic light emitting device. However, the heterocyclic compound may be mixed with another material, if necessary, to constitute a hole transport layer or an electron blocking layer.

In the organic light emitting device according to an exemplary embodiment of the present application, materials other than the heterocyclic compound will be exemplified below, but these materials are illustrative only and are not for limiting the scope of the present application, and may be replaced with materials publicly known in the art.

As a positive electrode material, materials having a relatively high work function may be used, and a transparent conductive oxide, a metal or a conductive polymer, and the like may be used. Specific examples of the positive electrode material include: a metal such as vanadium, chromium, copper, zinc, and gold, or an alloy thereof; a metal oxide such as zinc oxide, indium oxide, indium tin oxide (ITO), and indium zinc oxide (IZO); a combination of a metal and an oxide, such as ZnO:Al or SnO₂:Sb; a conductive polymer such as poly(3-methylthiophene), poly[3,4-(ethylene-1,2-dioxy)thiophene] (PEDT), polypyrrole, and polyaniline; and the like, but are not limited thereto.

As a negative electrode material, materials having a relatively low work function may be used, and a metal, a metal oxide, or a conductive polymer, and the like may be used. Specific examples of the negative electrode material include: a metal such as magnesium, calcium, sodium, potassium, titanium, indium, yttrium, lithium, gadolinium, aluminum, silver, tin, and lead, or an alloy thereof; a multi-layer structured material, such as LiF/Al or LiO_{2/}Al; and the like, but are not limited thereto.

As a hole injection material, a publicly-known hole injection material may also be used, and it is possible to use, for example, a phthalocyanine compound such as copper phthalocyanine disclosed in US Patent No. 4,356,429 or starburst-type amine derivatives described in the document [Advanced Material, 6, p. 677 (1994)], for example, tris(4-carbazoyl-9-ylphenyl)amine (TCTA), 4,4',4"-tri[phenyl(m-tolyl)amino]triphenylamine (m-MTDATA), 1,3,5-tris[4-(3-methylphenylphenylamino)phenyl]benzene (m-MTDAPB), polyaniline/dodecylbenzenesulfonic acid or poly(3,4-ethylenedioxythiophene)/poly(4-styrenesulfonate), which is a soluble conductive polymer, polyaniline/camphor sulfonic acid or polyaniline/poly(4-styrene-sulfonate), and the like.

As a hole transporting material, a pyrazoline derivative, an arylamine-based derivative, a stilbene derivative, a triphenyldiamine derivative, and the like may be used, and a low-molecular weight or polymer material may also be used.

As an electron transporting material, it is possible to use an oxadiazole derivative, anthraquinodimethane and a derivative thereof, benzoquinone and a derivative thereof, naphthoquinone and a derivative thereof, anthraquinone and a derivative thereof, tetracyanoanthraquinodimethane and a derivative thereof, a fluorenone derivative, diphenyldicyanoethylene and a derivative thereof, a diphenoquinone derivative, a metal complex of 8-hydroxyquinoline and a derivative thereof, and the like, and a low-molecular weight material and a polymer material may also be used.

As an electron injection material, for example, LiF is representatively used in the art, but the present application is not limited thereto.

As a light emitting material, a red, green, or blue light emitting material may be used, and if necessary, two or more light emitting materials may be mixed and used. Further, a fluorescent material may also be used as the light emitting material, but may also be used as a phosphorescent material. As the light emitting material, it is also possible to use alone a material which emits light by combining holes and electrons each injected from a positive electrode and a negative electrode, but materials in which a host material and a dopant material are involved in light emission together may also be used.

The organic light emitting device according to an exemplary embodiment of the present application may be a top emission type, a bottom emission type, or a dual emission type according to the material to be used.

The heterocyclic compound according to an exemplary embodiment of the present application may act even in organic electronic devices including organic solar cells, organic photoconductors, organic transistors, and the like, based on the principle similar to those applied to organic light emitting devices.

Hereinafter, the present specification will be described in more detail through Examples.

### <Preparation Example 1> Preparation of Compound 2

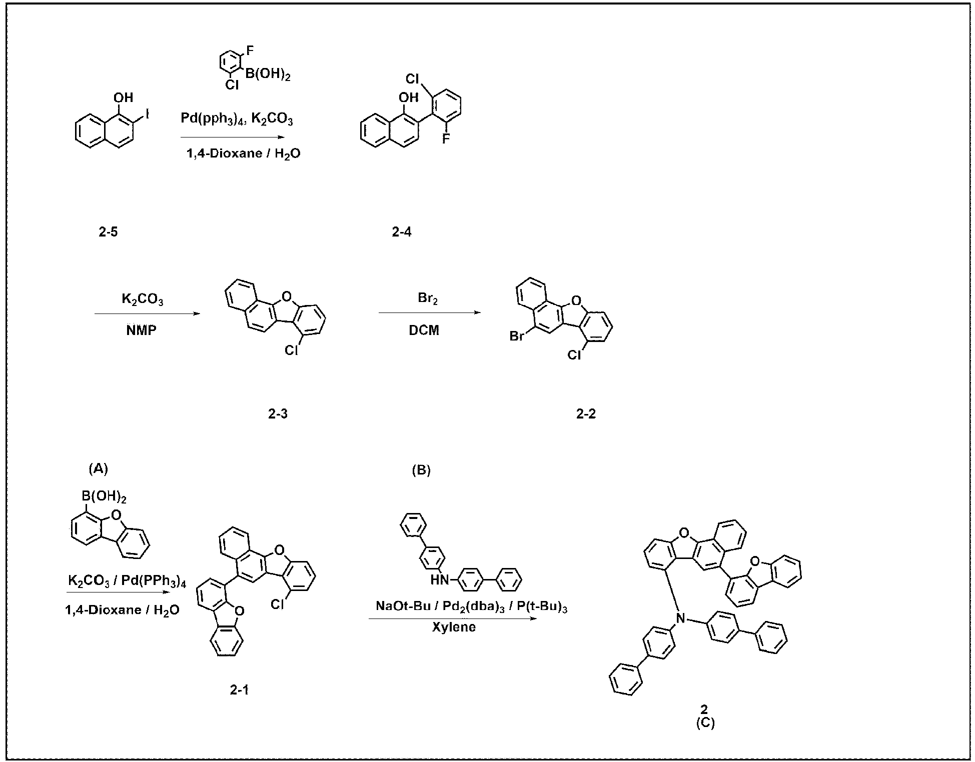

### (1) Preparation of Compound 2-4

A compound 2-5(2-iodonaphthalen-1-ol) (47.0 g, 174.75 mmol), (2-chloro-6-fluorophenyl)boronic acid (34.2 g, 192.22 mmol) , Pd(pph₃)₄ (201.93 g, 174.75 mmol), and K₂CO₃ (24.15 g, 174.75 mmol) were added to a mixture of 1,4-dioxane (750 mL) and water (150 mL) and the resulting mixture was stirred at 120°C for 4 hours (h). The temperature was lowered to room temperature, an aqueous layer was separated, and an organic layer was separated by washing the organic layer once more with water. A slurry was produced by introducing anhydrous magnesium sulfate into the collected organic layer, and then filtered and concentrated under reduced pressure. The compound in an oil state was separated by silica chromatography with a combination of hexane and ethyl acetate to obtain Compound 2-4 (2-(2-chloro-6-fluorophenyl)naphthalen-1-ol) (43.4 g, 159.3 mmol, 91% yield).

### (2) Preparation of Compound 2-3

Compound 2-4 (2-(2-chloro-6-fluorophenyl)naphthalen-1-ol) (43.4 g, 159.3 mmol) and K₂CO₃ (66.04 g, 477.89 mmol) were added to N-methyl-2-pyrrolidone (hereinafter, NMP) (460 mL), and the resulting mixture was stirred at 140°C for 3 hours. Thereafter, after about 1 hour, the reaction product was cooled to room temperature and slowly introduced into 500 ml of water. A precipitated solid was filtered, and after the filtered solid was dissolved in tetrahydrofuran (hereinafter, THF), the resulting solution was treated with anhydrous magnesium sulfate, filtered, and then concentrated under reduced pressure. The concentrated compound was slurried with a small amount of tetrahydrofuran and an excessive amount of hexane and filtered. In order to purify the filtered compound, the filtered compound was separated by silica chromatography with hexane and ethyl acetate to prepare Compound 2-3 (7-chloronaphtho[1,2-b]benzofuran) (37.6 g, 148.83 mmol, 93.431% yield).

### (3) Preparation of Compound 2-2

After Compound 2-3 (7-chloronaphtho[1,2-b]benzofuran) (37.6 g, 148.83 mmol) was dissolved in dichloromethane (hereinafter, DCM) (400 mL), the resulting solution was stirred in an iced water bath, then bromine (26.43 g, 163.71 mmol) was added dropwise thereto using a needle, and then the resulting mixture was stirred at room temperature for 3 hours to obtain Compound 2-2 (5-bromo-7-chloronaphtho[1,2-b]benzofuran)) (48.6 g, 146.7 mmol, 98.564% yield).

### (4) Preparation of Compound 2-1

Compound 2-2 (5-bromo-7-chloronaphtho[1,2-b]benzofuran)) (48.6 g, 146.57 mmol), dibenzo[b,d]furan-4-ylboronic acid (A) (32.63 g, 153.89 mmol), Pd(pph₃)₄ (8.47 g, 7.33 mmol), and K₂CO₃ (60.77 g, 439.7 mmol) were added to a mixture of 1,4-dioxane (800 mL) and distilled water (200 mL), and the resulting mixture was reacted under reflux for 5 hours. After the reaction was completed, 100 ml of methylene chloride (hereinafter, MC) and 150 ml of water were added to the reaction solution for work up, and then the resulting product was put into a separatory funnel to separate the organic layer. After the organic layer was dried over anhydrous MgSO₄ and the solvent was removed by a rotary evaporator, the organic layer was slurried by acetone and hexane to obtain Compound 2-1 (7-chloro-5-dibenzofuran-4-yl-naphtho[1,2-b]benzofuran) (55 g, 131.31 mmol, 89.588% yield).

### (5) Preparation of Compound 2(C)

Compound 2-1 (7-chloro-5-dibenzofuran-4-yl-naphtho[1,2-b]benzofuran) (55.0 g, 131.31 mmol), bis(4-biphenylyl)amine (B) (43.06 g, 131.31 mmol), Pd₂(dba)₃ (6.01 g, 6.57 mmol), Xphos (6.26 g, 13.13 mmol) and NaOt-Bu (37.86 g, 393.92 mmol) were added to xylene (1000 mL), and the resulting mixture was stirred under reflux at 125°C for 5 hours. After the reaction was completed, MC was added to the reaction solution and dissolved, then extraction with distilled water was performed, the organic layer was dried over anhydrous magnesium sulfate, the solvent was removed by a rotary evaporator, and then the residue was purified by column chromatography (MC:hexane = 1:3) to obtain Compound 2 (5-dibenzofuran-4-yl-N,N-bis(4-phenylphenyl)naphtho[1,2-b]benzofuran-7-amine) (C) (67 g, 95.195 mmol, 72.499% yield).

Target Compound C in the following Table 1 was synthesized in the same manner as in Preparation Example 1, except that Intermediate A and Intermediate B in the following Table 1 were used instead of dibenzo[b,d]furan-4-ylboronic acid and bis(4-biphenylyl)amine), respectively, in Preparation Example 1.

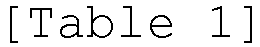

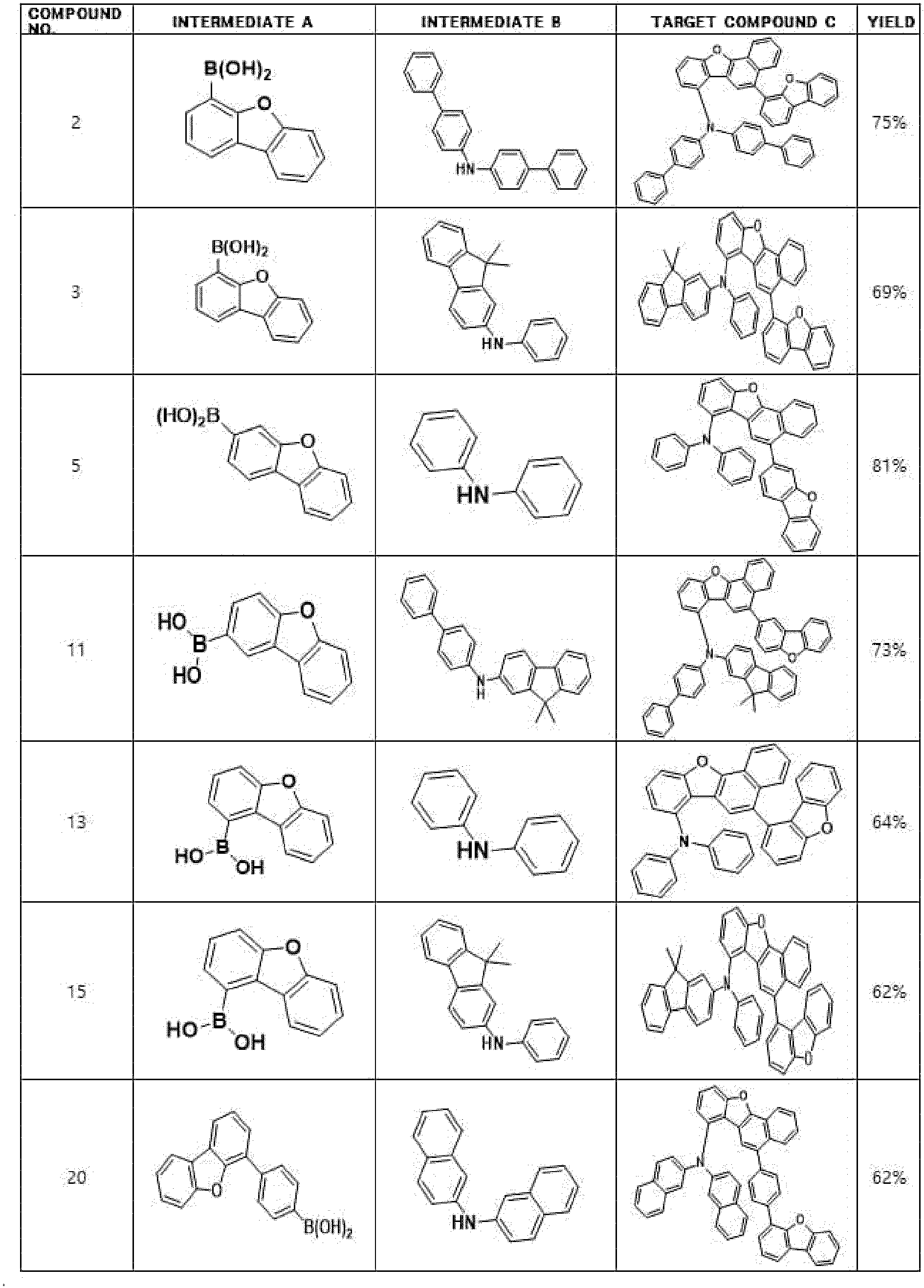

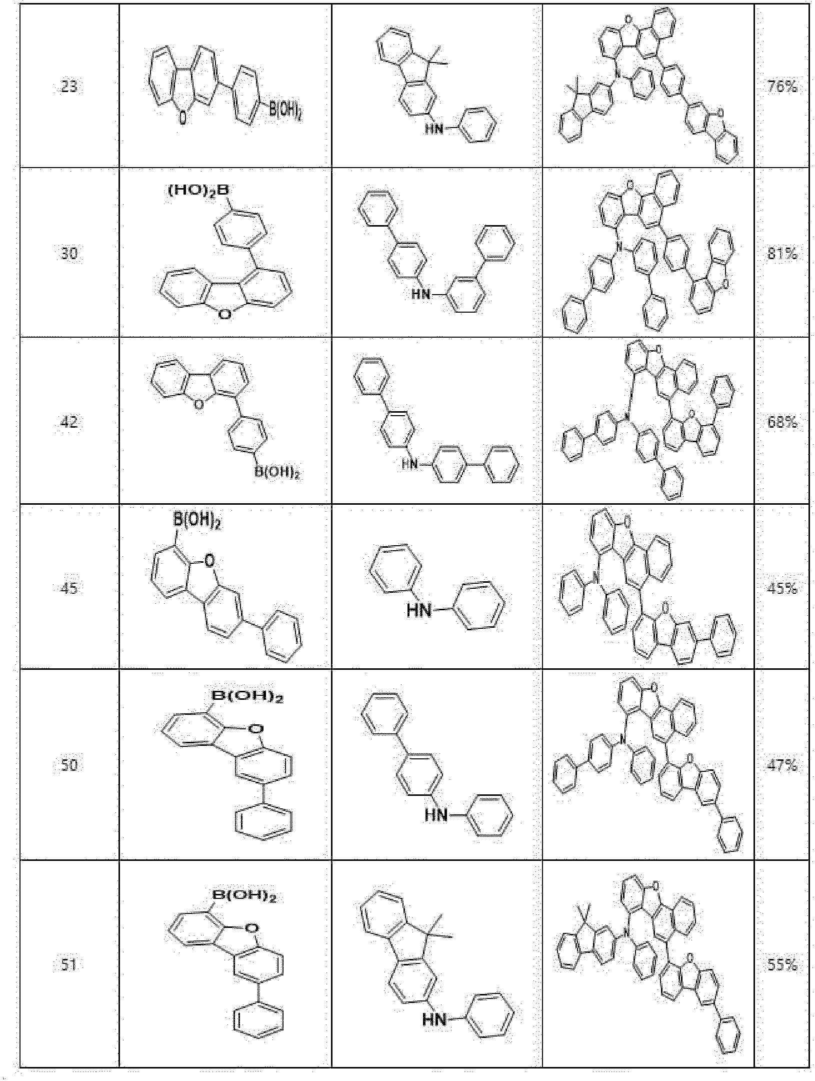

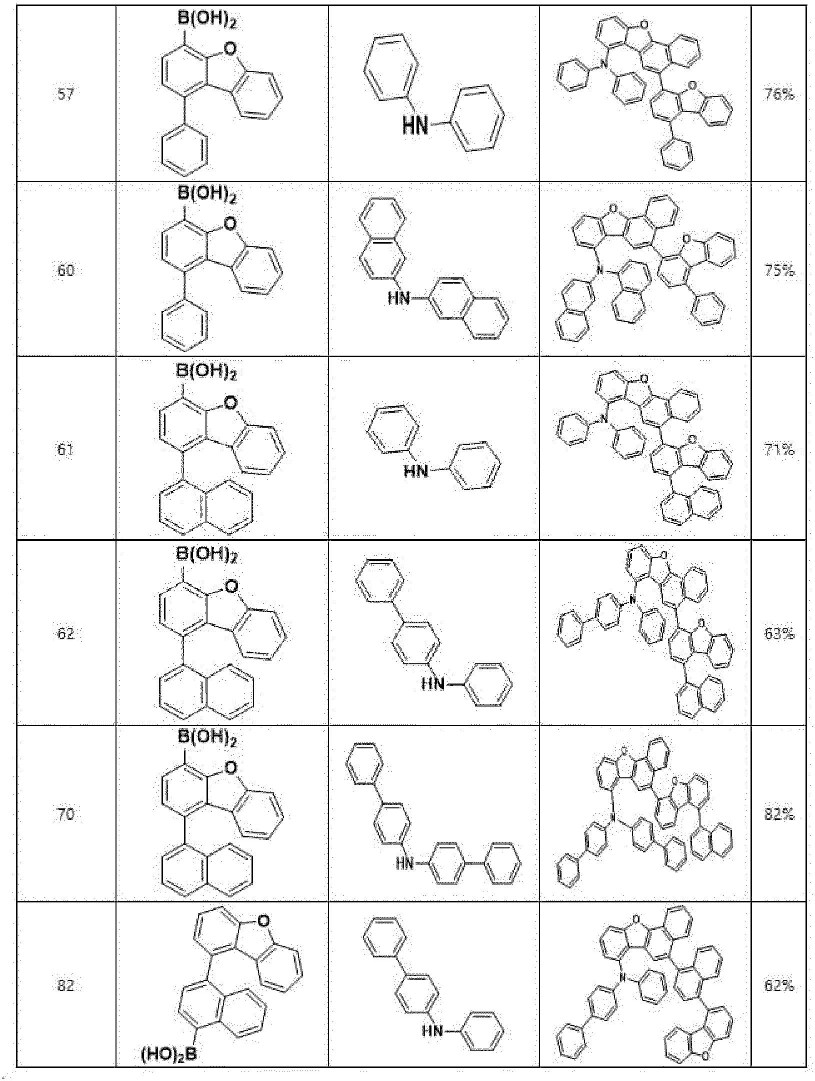

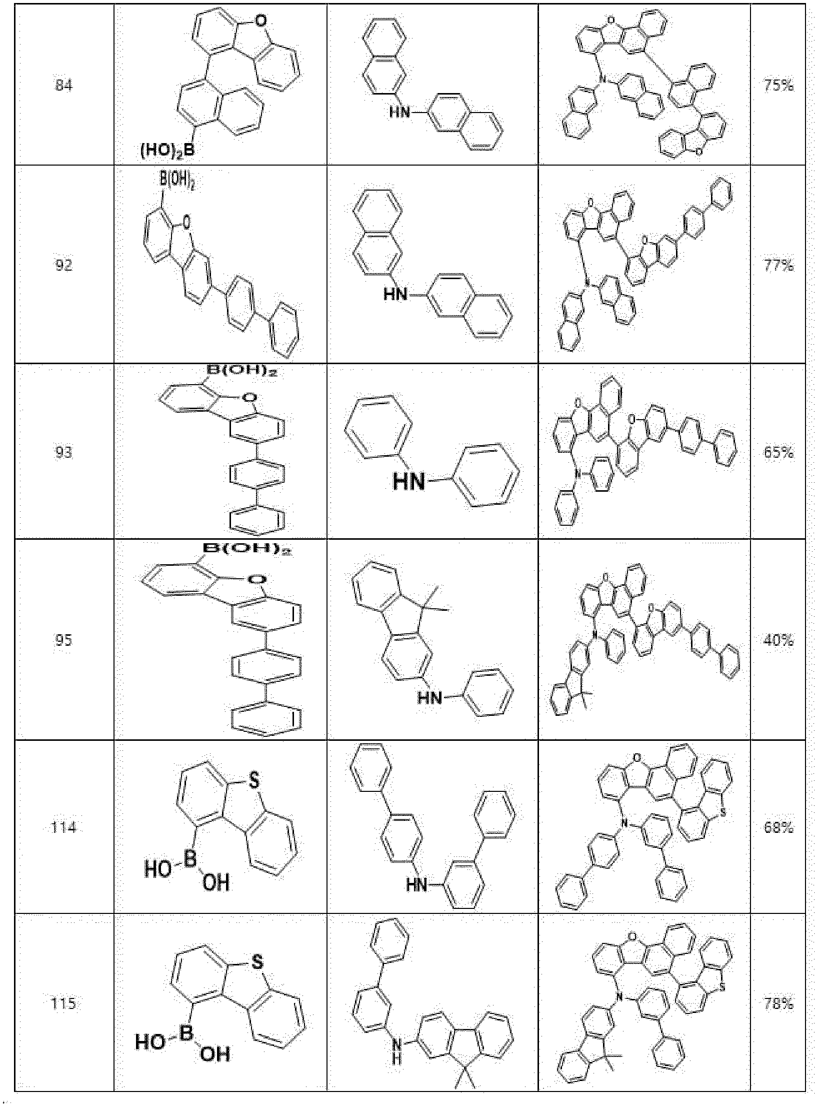

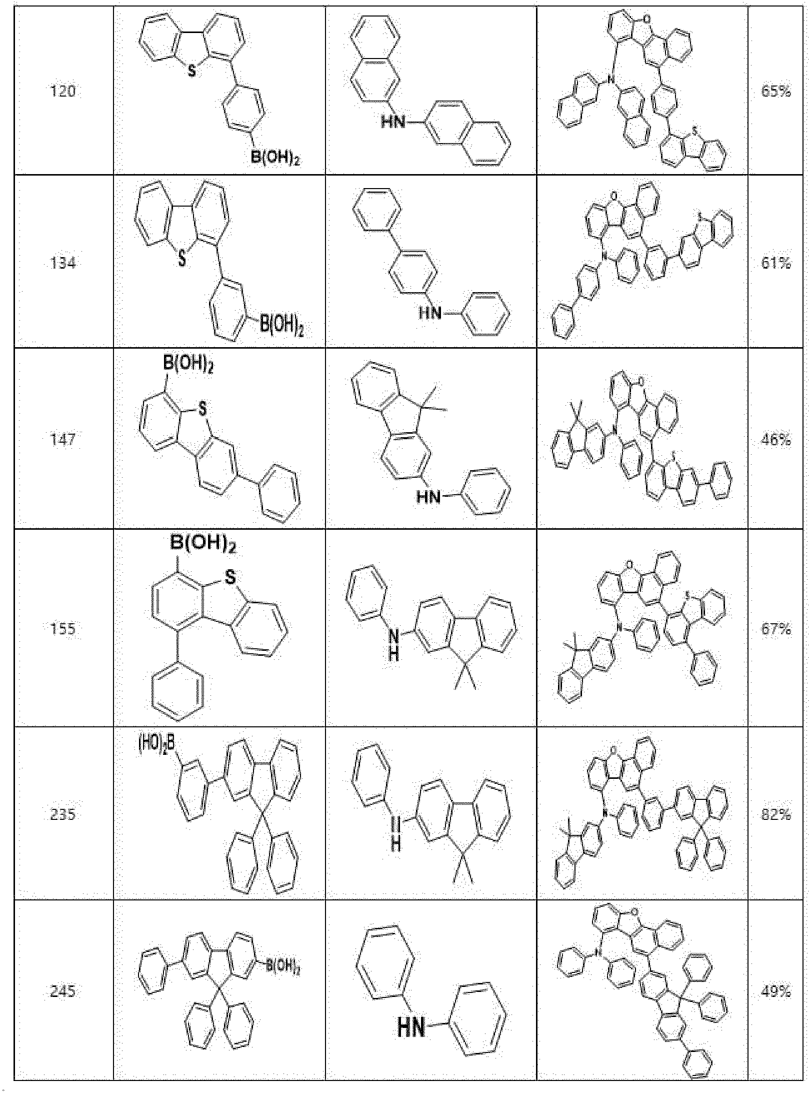

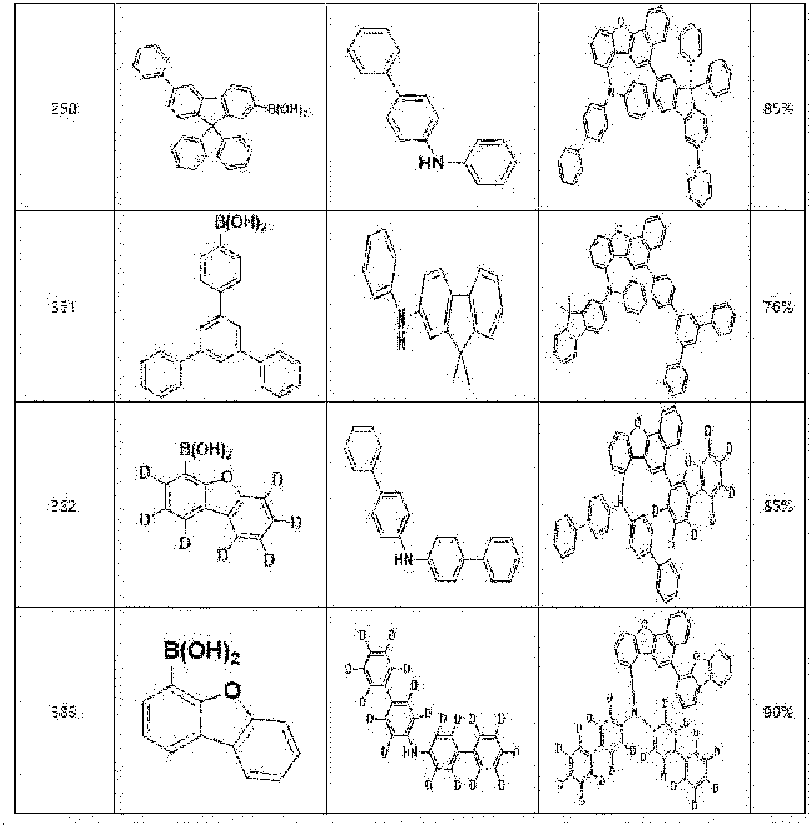

### <Preparation Example 2> Preparation of Compound 418(F)

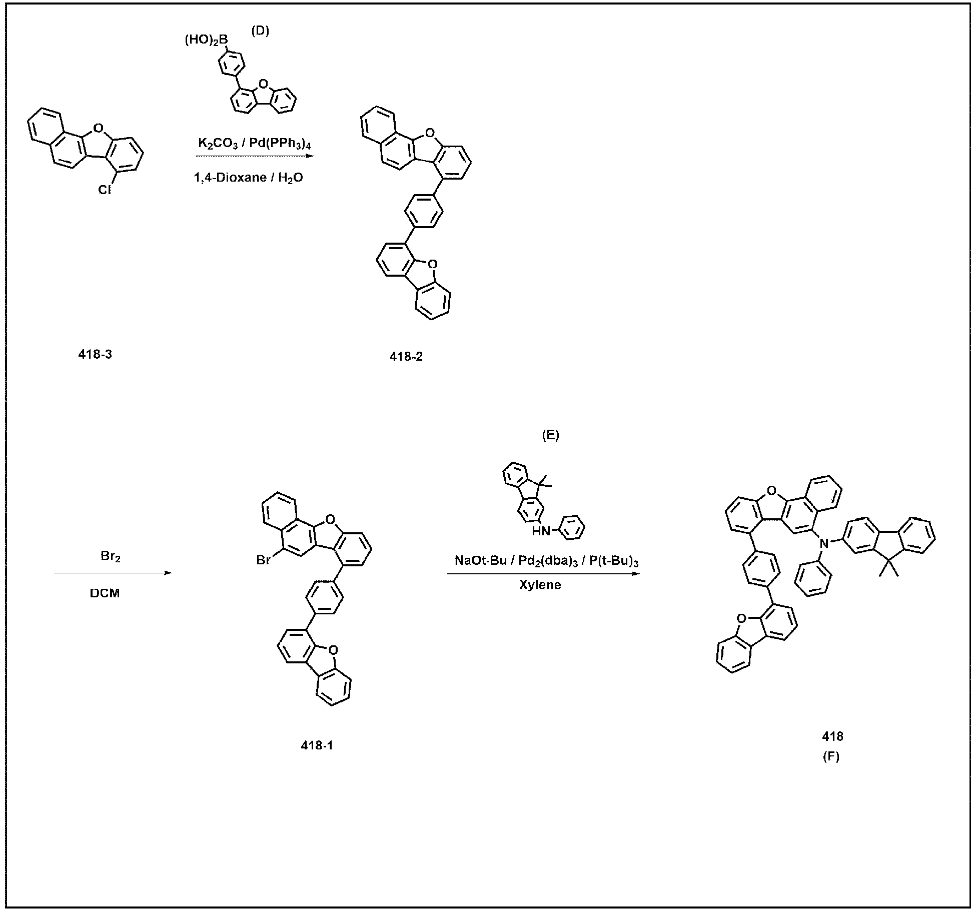

### (1) Preparation of Compound 418-2

Compound 418-3 (7-chloronaphtho[1,2-b]benzofuran) (50.0 g, 197.86 mmol), 4-dibenzofuran-4-yl-phenyl-boronic acid (D) (64.89 g, 220.73 mmol), Pd₂(dba)₃ (9.06 g, 9.89 mmol), Xphos (9.43 g, 19.79 mmol), and K₂CO₃ (82.03 g, 593.59 mmol) were added to a mixture of 1,4-dioxane (1000 mL) and distilled water (250 mL), and the resulting mixture was stirred at 120°C for 4 hours. The temperature was lowered to room temperature, an aqueous layer was separated, and an organic layer was separated by washing the organic layer once more with water. A slurry was produced by introducing anhydrous magnesium sulfate into the collected organic layer, and then filtered and concentrated under reduced pressure. The compound in an oil state was separated by silica chromatography with a combination of hexane and ethyl acetate to obtain Compound 418-2 (7-(4-dibenzofuran-4-ylphenyl)naphtho[1,2-b]benzofuran) (80 g, 173.72 mmol, 87.796% yield).

### (2) Preparation of Compound 418-1

After Compound 418-2 (7-(4-dibenzofuran-4-ylphenyl)naphtho[1,2-b]benzofuran) (80.0 g, 173.72 mmol) was dissolved in DCM (800 mL), the resulting solution was stirred in an iced water bath, then bromine (26.43 g, 163.71 mmol) was added dropwise thereto using a needle, and then the resulting mixture was stirred at room temperature for 3 hours to obtain Compound 418-1 (5-bromo-7-(4-dibenzofuran-4-ylphenyl)naphtho[1,2-b]benzofuran) (80 g, 148.31 mmol, 85.373% yield).

### (3) Preparation of Compound 418

Compound 418-1 (5-bromo-7-(4-dibenzofuran-4-ylphenyl)naphtho[1,2-b]benzofuran) (80 g, 148.31 mmol), 9,9-dimethyl-N-phenyl-9H-fluoren-2-amine (E) (46.56 g, 163.14 mmol), Pd₂(dba)₃ (6.79 g, 7.42 mmol), Xphos (7.07 g, 14.83 mmol) and NaOt-Bu (42.76 g, 444.92 mmol) were added to xylene (1200 mL), and the resulting mixture was stirred under reflux at 125°C for 5 hours. After the reaction was completed, MC was added to the reaction solution and dissolved, then extraction with distilled water was performed, the organic layer was dried over anhydrous magnesium sulfate, the solvent was removed by a rotary evaporator, and then the residue was purified by column chromatography (MC:hexane = 1:3) to obtain Compound 418 (7-(4-dibenzofuran-4-ylphenyl)-N-(9,9-dimethylfluoren-2-yl)-N-phenyl-naphtho[1,2-b]benzofuran-5-amine) (F) (55 g, 73.936 mmol, 49.853% yield).

Target Compound F was synthesized in the same manner as in Preparation Example 2, except that Intermediate D and Intermediate were used instead of 4-dibenzofuran-4-yl-phenyl-boronic acid and 9,9-dimethyl-N-phenyl-9H-fluoren-2-amine, respectively, in Preparation Example 2.

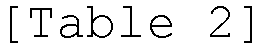

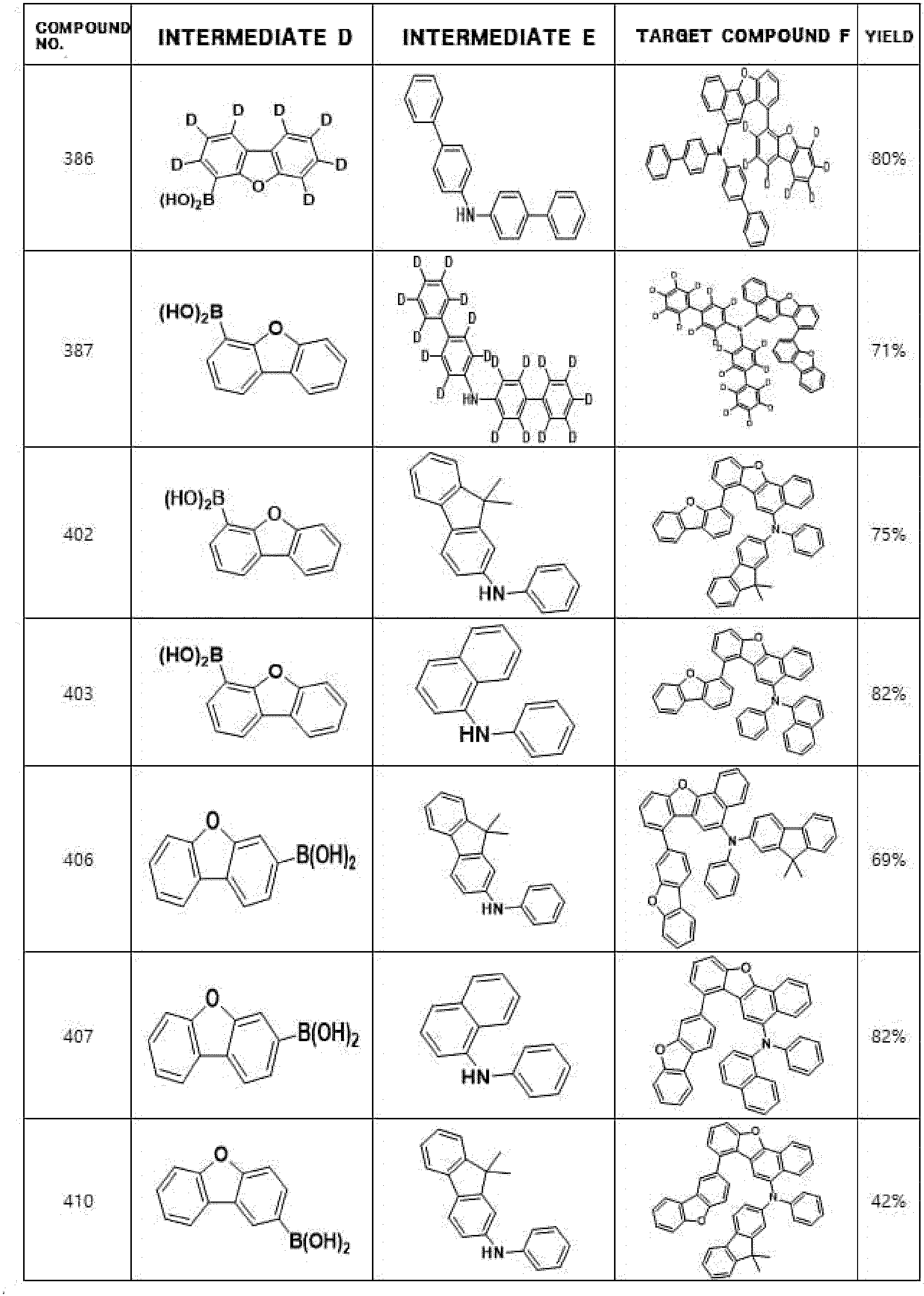

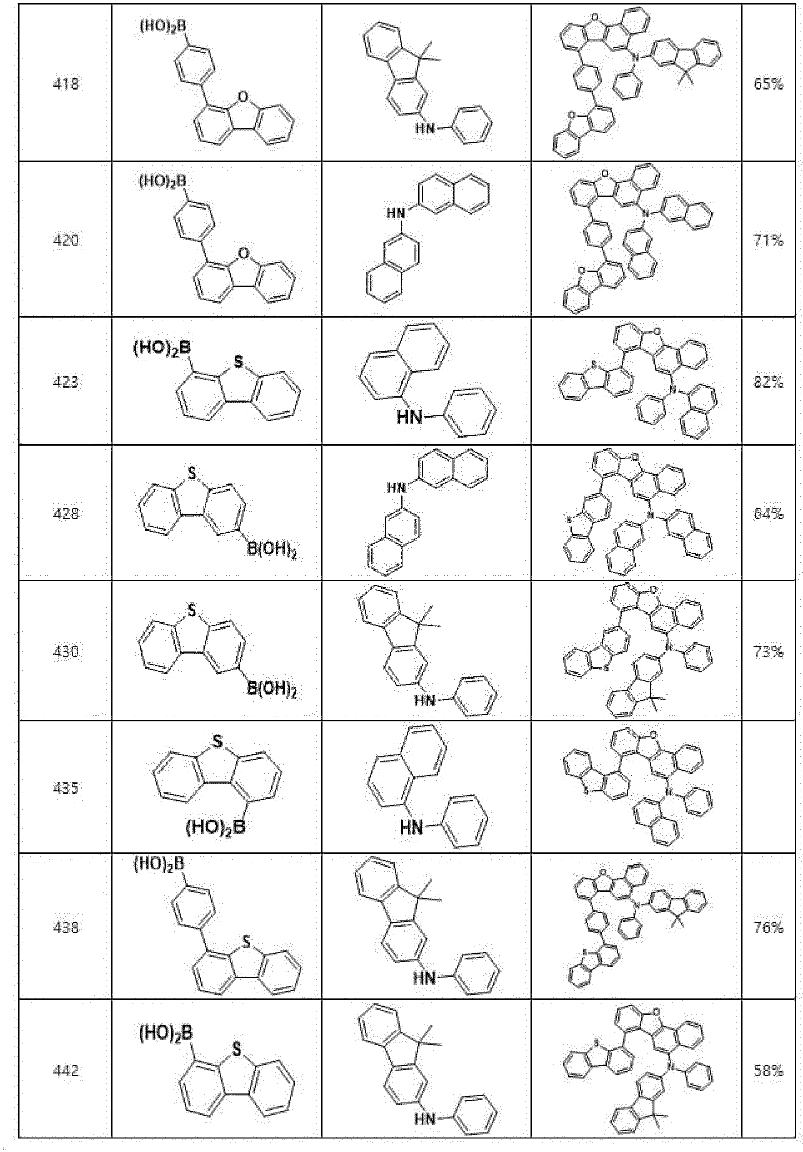

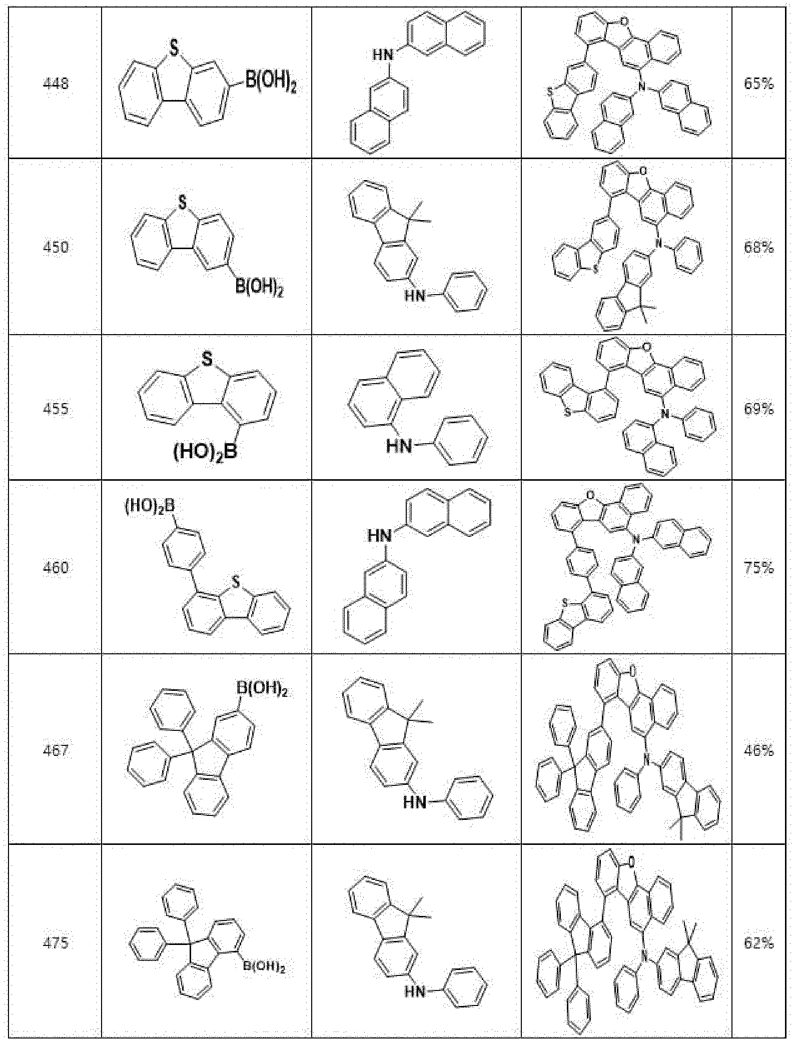

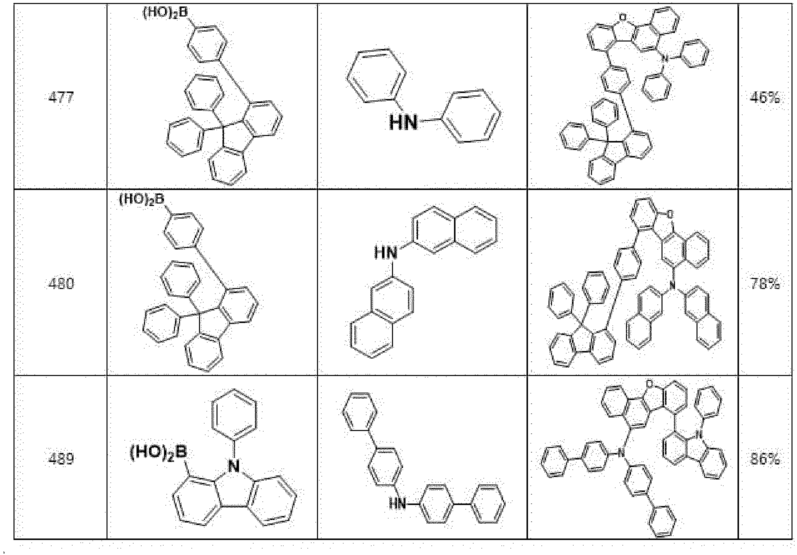

### <Preparation Example 3> Preparation of Compound 384

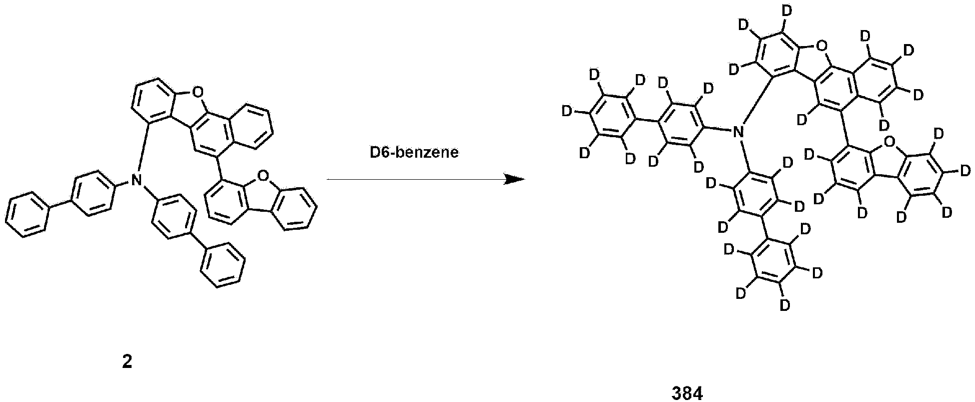

After Compound 2 (10.0 g, 14.21 mmol) and trifluoromethanesulfonic acid (8.78 mL, 99.46 mmol) were dissolved in D₆-benzene (100 ml), the resulting solution was stirred at 60°C for 4 hours. After the reaction was completed, the resulting product was neutralized with an aqueous K₃PO₄ solution at room temperature, and then extracted with dichloromethane (DCM) and distilled water (H₂O).

The reaction product extracted once again was purified by column chromatography (DCM:Hex = 1:1) and recrystallized with methanol to obtain Target Compound 384 (9 g, 12.211 mmol, 85.945% yield).

Here, Hex means hexane, and DCM:Hex means a volume ratio.

### <Preparation Example 4> Preparation of Compound 524

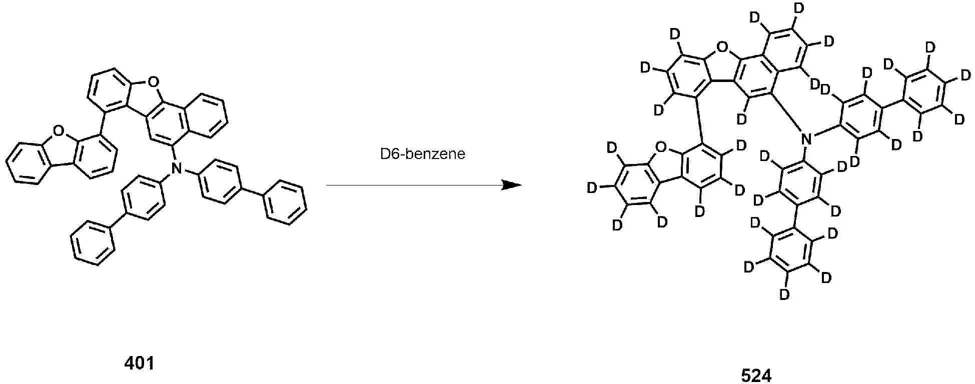

After Compound 401 (10.0 g, 14.21 mmol) and trifluoromethanesulfonic acid (8.78 mL, 99.46 mmol) were dissolved in D₆-benzene (100 ml), the resulting solution was stirred at 60°C for 4 hours. After the reaction was completed, the resulting product was neutralized with an aqueous K₃PO₄ solution at room temperature, and then extracted with dichloromethane (DCM) and water (H₂O).

The reaction product extracted once again was purified by column chromatography (dichloromethane:hexane = a 1:1 volume ratio) and recrystallized with methanol, and the resulting product was extracted with dichloromethane/H₂O. The reaction product was purified by column chromatography (DCM:Hex = 1:1) and recrystallized with methanol to obtain Target Compound 524 (8 g, 10.854 mmol, 76.395% yield).

Here, Hex means hexane, and DCM:Hex means a volume ratio.

### <Preparation Example 5> Preparation of Compound 534 (F)

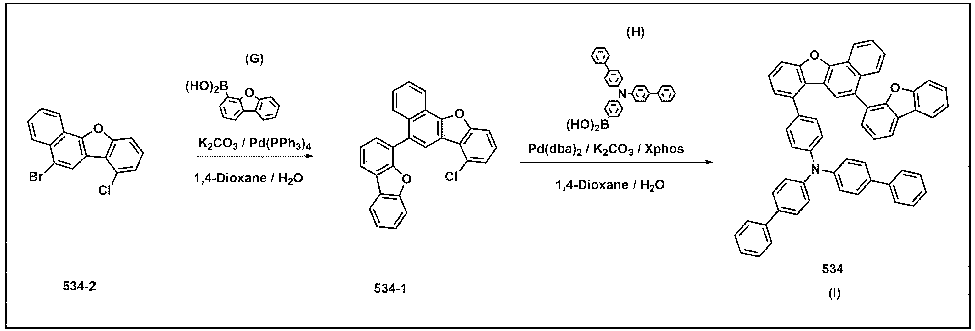

### (1) Preparation of Compound 534-1

Compound 534-1 was prepared in the same manner as in the method of preparing Compound 2-1 in Preparation Example 1. That is, Compound 534-2 is the same as Compound 2-2 in Preparation Example 1, and Compound 534-1 is the same as Compound 2-1 in Preparation Example 1.

### (2) Preparation of Compound 534

Compound 534-1, 4-(dibiphenyl-4-ylamino)phenylboronic acid (H) (11.71 g, 26.26 mmol), 7-chloro-5-dibenzofuran-4-yl-naphtho[1,2-b]benzofuran (10.0 g, 23.87 mmol), Pd(dba)₂ (0.41 g, 1.19 mmol), Xphos (1.29 mL, 2.39 mmol), and potassium carbonate (9.9 g, 71.62 mmol were added to 1,4-dioxane (200 mL) and water (50 mL), and the resulting mixture was stirred at 120°C for 4 hours, and then extracted with water and MC to concentrate the organic layer. Silica gel was added to the concentrated crude mixture, concentrated and adsorbed, and then separated by column chromatography (MC:Hex = 1:4). After the separated solution was concentrated, and then slurried with methanol (MeOH), a solid was filtered to obtain Compound 534 (N-[4-(5-dibenzofuran-4-ylnaphtho[1,2-b]benzofuran-7-yl)phenyl]-4-phenyl-N-(4-phenylphenyl)aniline). (15 g,19.233 mmol, 80.56% yield)

Target Compound I in the following Table 3 was synthesized in the same manner as in Preparation Example 5, except that Intermediate G in the following Table 3 was used instead of dibenzo[b,d]furan-4-ylboronic acid (D), and Intermediate H in the following Table 3 was used instead of 4-(dibiphenyl-4-ylamino)phenylboronic acid (H), in Preparation Example 5.

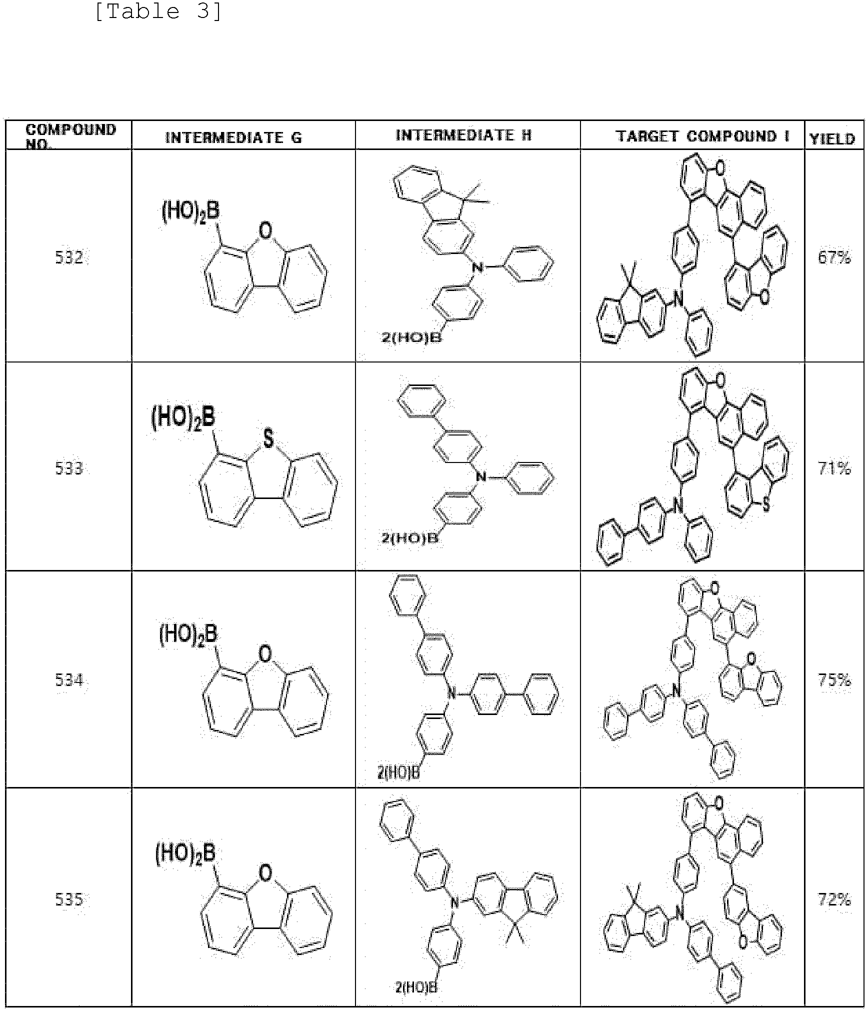

Compounds were prepared in the same manner as in the Preparation Examples, and the synthesis confirmation results thereof are shown in the following Tables 4 and 5. Specifically, Table 4 shows the measured values of ¹H NMR(CDCl₃, 200 Mz), and Table 5 shows the measured values of field desorption mass spectrometry (FD-MS).

**[Table 4]**

| Compound | ¹H NMR(CDCl₃, 200Mz) |
|---|---|
| 2 | δ= 8.97(1H, d), 8.18(1H, d), 7.98~8.08(3H, m), 7.75(4H, d), 7.25~7.55(23H, m) |
| 3 | δ= 8.97(1H, d), 8.18(1H, d), 7.98~8.08 (3H, m), 7.86~7.90 (2H, d), 7.51~7.64(5H, m), 7.24~7.39(11H, m) 7.00~7.08(3H, m), 6.91(2H, d),1.69 (6H, s) |
| 5 | δ= 8.97(1H, d), 8.18(1H, d), 7.98~8.08 (3H, m), 7.76~7.70 (2H, t), 7.54~7.64(3H, m), 7.05~7.39(9H, m), 6.91~7.09(7.H, m) |
| 11 | δ= 8.97(1H, d), 8.18(1H, d), 7.98(1H,d) 7.86-7.96(7H, m), 7.28-7.63(19H, m), 7.16(1H, d), 6.91(1H, d) |
| 15 | δ= 8.97(1H, d), 8.18(1H, d), 7.98(1H,d), 7.00~7.90 (3H, m), 7.53~7.69 (6H, m), 7.00~7.08(3H, m), 6.91 (2H, d),1.69 (6H, s) |
| 20 | δ= 8.97(1H, d), 8.18(1H, d), 7.98~-8.08(3H, m), 7.25~7.64 (12H, m), 7.11(2H, s), 6.91(2H, d) |
| 23 | δ= 8.97(1H, d), 8.18(1H, d), 7.98~8.03(2H, m), 7.76~7.90 (4H, m), 7.16~7.38 (15H, m), 7.08 (3H, m), 6.91 (1H, d) |
| 30 | δ= 8.97 (1H, d), 8.18 (1H, d), 7.98 (1H, d), 7.18~7.82 (31H, m), 6.91(1H, d) |
| 42 | δ= 8.97 (1H, d), 8.18 (1H, d), 8.02~8.08 (4H, m), 7.75(4H, d), 7.34~7.64 (25H, m), 7.25(1H, m), 6.91(1H, d) |
| 45 | δ= 8.97(1H, d), 8.18 (1H, d), 8.02~8.08(3H, m), 7.75~7.82 (4H, m), 7.24~7.64 (13H, m), 6.91~7.08 (7H, m) |
| 50 | δ= 8.97(1H, d), 8.18(1H, d), 8.02~8.08(2H, m), 7.75~7.88 (7H, m), 7.24~7.64 (18H, m), 7.00~7.08 (3H, m), 6.91(1H, d) |
| 51 | δ= 8.97(1H, d), 8.18(1H, d), 8.02~8.08(2H, m), 7.75~7.83 (3H, m), 7.24~7.64 (16H, m), 7.00~7.08 (3H, m), 6.91(1H, d) |
| 57 | δ= 8.97 (1H, d), 8.18(1H, d), 7.98~8.08(2H, m), 7.79(2H, d), 7.24~7.64 (15H, m), 6.91~7.08 (7H, m) |
| 60 | δ= 8.97 (1H, d), 8.18 (1H, d), 7.98~8.08(3H, m), 7.25~7.78(25H, m), 7.11(2H, s), 6.91(1H, d) |
| 61 | δ= 8.97(1H, d), 8.50(1H, d), 8.18~8.20(2H, t), 8.07~8.95(4H, m), 7.77 (1H, t), 7.52~7.64 (4H, m), 7.24~7.39 (10H, m), 6.91~7.08 (7H, m) |
| 62 | δ= 8.97(1H, d), 8.50(1H, d), 8.18~8.20(2H, t), 7.98~8.08(4H, m), 7.77(1H, t), 7.24~7.64 (19H, m), 7.00~7.08 (3H, m), 6.91(1H, d) |
| 70 | δ= 8.97(1H, d), 8.50(1H, d), 8.18~8.20(2H, t), 8.02~8.09(3H, m), 7.34~7.77 (18H, m), 7.25 (1H, m), 6.91 (1H, d) |
| 82 | δ= 8.97~9.00(2H, m), 8.18(1H, d), 7.98 (1H, d), 7.24~7.75 (26H, m), 7.08 (3H, m), 6.91(1H, d) |
| 84 | δ= 8.97~9.00(3H, m), 8.18(1H, d), 7.98 (1H, d), 7.24~7.75 (26H, m), 7.11 (2H, s), 6.91(1H, d) |
| 92 | δ= 8.97(1H, d), 8.18(1H, d), 8.02~8.03(3H, m), 7.25~7.82 (29H, m), 7.11 (2H, s), 6.91(1H, d) |
| 93 | δ= 8.97 (1H, d), 8.18(1H, d), 8.02~8.08(2H, m), 7.75~7.88 (5H, m), 7.25~7.64 (17H, m), 7.00~7.08 (6H, m), |
| 95 | δ= 8.97 (1H, d), 8.18(1H, d), 8.02~8.08(2H, m), 7.75~7.88 (5H, m), 7.25~7.64 (22H, m), 7.00~7.08 (3H, m), 6.91(1H, d) |
| 114 | δ= 8.97 (1H, d), 8.45(1H, d), 8.18 (1H, d), 8.03(1H, d), 7.25~7.75(27H, m), 6.91 (1H, d) |
| 115 | δ= 8.97 (1H, d), 8.45(1H, d), 8.18 (1H, d), 8.03(1H, d), 7.17~7.93(4H, m), 7.26~7.64(21H, m), |
| 120 | δ= 8.97(1H, d), 8.55(1H, d), 8.45(1H, d), 8.32(1H, d), 8.18 (1H, d), 7.93(1H, d), 7.25~7.78(24H, m), 7.11 (2H, s), 6.91 (1H, d) |
| 134 | δ= 8.97 (1H, d), 8.45 (1H, d), 8.18~8.24(4H, m), 7.93~7.94 (2H, d), 7.24~7.64 (21H, m), 6.91~7.08(4H, m) |
| 147 | δ= 8.97 (1H, d), 8.55(1H, d), 8.32(1H, d), ), 8.18(1H, d), ), 8.03(1H, d), 7.79~7.94(5H, m), 7.55~7.70(5H, m), 7.25~7.46(12H, m), 7.00~7.08(3H, m) 6.91 (1H, d), 1.69(6H, s) |
| 155 | δ= 8.97 (1H, d), 8.55(1H, d), 8.32 (1H, d), 8.18 (1H, d), 8.03(1H, d), 7.79~7.94(5H, m), 7.08~7.46(12H, m),7.00~7.08(3H, m) |
| 235 | δ= 8.97 (1H, d), 8.18 (1H, d), 8.09(1H, d), 7.86~7.96(5H, m) 7.55~7.76(6H, m), 7.08~7.38(24H, m), 6.91(1H, d), 1.69(6H, s) |
| 245 | δ= 8.97(1H, d), 8.18(1H, d), 8.09(2H, d), 7.89(2H, s), 7.75~7.78 (4H, m), 7.59~7.64 (2H, m), 7.08~7.49(26H, m), 6.91(1H, d) |
| 250 | δ= 8.97(1H, d), 8.18(1H, d), 8.09(1H, d), 7.89(1H, s), 7.18~7.75(36H, m), 6.91(1H, d) |
| 351 | δ= 8.97(1H, d), 8.18(1H, d), 8.04(3H, s), 7.86~7.90(2H, m),7.75(4H, t), 7.16~7.64 (22H, m),7.00~7.08(3H, m), 6.91(1H, d) |
| 382 | δ= 8.97 (1H, d), 8.18(1H, d), 7.75(4H, t), 7.34~7.64(28H, m), 7.25(1H, d), 6.91(1H, d) |
| 383 | δ= 8.97(1H, d), 8.18(1H, d), 7.98~8.08(3H, m), 7.51~7.64(4H, m), 7.25~7.39(5H, m), 6.91(1H, d) |
| 386 | δ= 8.12~8.22(3H, m), 7.37~7.82(23H, m) |
| 387 | δ= = 7.98~8.22(6H, m),7.82(1H, d) 7.50~7.69(6H, m),7.31~7.39(2H, d) |
| 402 | δ= 7.98~8.22(6H, m), 7.82~7.90(3H, m), 7.50~7.69(5H, m), 7.24~7.39(8H, m), 7.00~7.08(3H, m) |
| 403 | δ= 7.98~8.22 (6H, m), 7.81~7.82(2H, m), 7.24~7.69(14H, m), 7.00~7.08 (3H, m) |
| 406 | δ= 8.12~8.22(3H, m), 7.98~8.03(2H, m), 7.76~7.90(5H, m), 7.50~7.69(6H, m), 7.16~7.39(8H, m), 7.00~7.08(3H, m) |
| 407 | δ= 8.12~8.22(3H, m), 7.24~7.69(17H, m), 7.00~7.08 (3H, m) |
| 410 | δ= 8.97 (1H, d), 8.18 (1H, d), 7.98~8.08(3H, m), 7.51~7.64(4H, m), 7.25~7.39(5H, m), 6.91(1H, d) |
| 418 | δ= 7.98~8.22(6H, m), 7.82~7.90(3H, m), 7.55~7.69(3H, m), 7.16~7.39(12H, m), 7.00~7.08 (3H, m) |
| 420 | δ= 8.08~8.22(5H, m), 7.25~7.82(25H, m), 7.11 (2H, s) |
| 423 | δ= 8.55(1H, d), 8.45(1H, d), 8.32(1H, d), 7.82~7.93(4H, m), 7.49~7.70(8H, m), 7.24~7.38 (10H, m), 7.00~7.08(3H, m) |
| 428 | δ= 8.55(1H, d), 8.45(1H, d), 8.32(1H, d), 7.82~7.93(4H, m), 7.49~7.70(8H, m), 7.24~7.38 (10H, m), 7.11 (2H, s) 7.00~7.08(3H, m) |
| 430 | δ= 8.55(1H, d), 8.45(1H, d), 8.32(1H, d), 7.82~7.93(4H, m), 7.49~7.70(8H, m), 7.11 (2H, s), 6.91(1H, d) |
| 435 | δ= 8.55(1H, d), 8.45(1H, d), 8.32(1H, d), 8.12~8.22(5H, m), 7.93(1H, d), 7.81~7.82(2H, t), 7.49~7.70(8H, m), 7.24~7.38(10H, m), 6.91(1H, d) |
| 438 | δ= 8.55(1H, d), 8.45(1H, d), 8.32(1H, d), 7.82~7.93(4H, m), 7.49~7.70(8H, m), 7.24~7.38 (10H, m), 7.00~7.08 (3H, m) |
| 450 | δ= 8.97(1H, d), 8.18 (1H, d), 8.02~8.03(3H, m), 7.25~7.82 (29H, m), 7.11 (2H, s), 6.91(1H, d) |
| 455 | δ= 8.97(1H, d), 8.18 (1H, d), 8.02~8.03(3H, m), 7.25~7.82 (29H, m), 7.11 (2H, s), ), 7.00~7.08 (3H, m), 6.91(1H, d) |
| 480 | δ= 8.55(1H, d), 8.45(1H, d), 8.32(1H, d), 8.12~8.22(5H, m), 7.49~7.70(11H, m), 7.00~7.08(3H, m) |
| 489 | δ= 8.55(1H, d), 8.45(1H, d), 8.32(1H, d), 8.12~8.22(5H, m), 7.93(1H, d), 7.81~7.82(2H, t), 7.49~7.70(11H, m), 7.24~7.25(6H, m), 7.00~7.08 (3H, m) |
| 534 | δ= 8.97 (1H, d), 8.18(1H, d), 7.98~8.03 (2H, m), 7.31~7.69(30H, m) |
| 535 | δ= 8.97(1H, d), 8.18(1H, d), 7.98(1H, d), 7.28~7.83(31H, m, 7.16(1H, d), 1.69(6H, s) |

**[Table 5]**

| Compound | FD-MS | Compound | FD-MS |
|---|---|---|---|
| 2 | Molecular Weight: 703.84 | 147 | Molecular Weight: 759.97 |
| 3 | Molecular Weight: 667.81 | 155 | Molecular Weight: 759.97 |
| 5 | Molecular Weight: 551.64 | 235 | Molecular Weight: 894.13 |
| 11 | Molecular Weight: 743.91 | 245 | Molecular Weight: 777.97 |
| 15 | Molecular Weight: 667.81 | 250 | Molecular Weight: 854.06 |
| 20 | Molecular Weight: 727.86 | 351 | Molecular Weight: 806.02 |
| 23 | Molecular Weight: 743.91 | 382 | Molecular Weight: 710.88 |
| 30 | Molecular Weight: 779.94 | 383 | Molecular Weight: 710.88 |
| 42 | Molecular Weight: 779.94 | 386 | Molecular Weight: 710.88 |
| 45 | Molecular Weight: 627.74 | 387 | Molecular Weight: 721.95 |
| 50 | Molecular Weight: 703.84 | 402 | Molecular Weight: 667.81 |
| 51 | Molecular Weight: 743.91 | 403 | Molecular Weight: 601.70 |
| 57 | Molecular Weight: 627.74 | 406 | Molecular Weight: 667.81 |
| 60 | Molecular Weight: 727.86 | 407 | Molecular Weight: 743.91 |
| 61 | Molecular Weight: 677.80 | 410 | Molecular Weight: 667.81 |
| 62 | Molecular Weight: 753.90 | 418 | Molecular Weight: 743.91 |
| 70 | Molecular Weight: 830.00 | 420 | Molecular Weight: 727.86 |
| 82 | Molecular Weight: 753.90 | 423 | Molecular Weight: 617.77 |
| 84 | Molecular Weight: 777.92 | 428 | Molecular Weight: 667.83 |
| 92 | Molecular Weight: 803.96 | 430 | Molecular Weight: 683.87 |
| 93 | Molecular Weight: 703.84 | 435 | Molecular Weight: 617.77 |
| 95 | Molecular Weight: 820.00 | 438 | Molecular Weight: 759.97 |
| 114 | Molecular Weight: 719.90 | 450 | Molecular Weight: 683.87 |
| 115 | Molecular Weight: 759.97 | 455 | Molecular Weight: 617.77 |
| 120 | Molecular Weight: 743.92 | 480 | Molecular Weight: 878.09 |
| 134 | Molecular Weight: 719.90 | 489 | Molecular Weight: 693.86 |

### <Experimental Example 1>

### (1) Manufacture of organic light emitting device

### Comparative Example 1

Trichloroethylene, acetone, ethanol, and distilled water were each sequentially used to ultrasonically wash a transparent electrode indium tin oxide (ITO) thin film obtained from glass for OLED (manufactured by Samsung-Corning Co., Ltd.) for 5 minutes, and then the ITO thin film was placed in isopropanol, stored, and then used. Next, the ITO substrate was disposed in a substrate folder of a vacuum deposition apparatus, and the following 4,4',4"-tris(N,N-(2-naphthyl)-phenylamino)triphenyl amine (2-TNATA) was placed in a cell in the vacuum deposition apparatus.

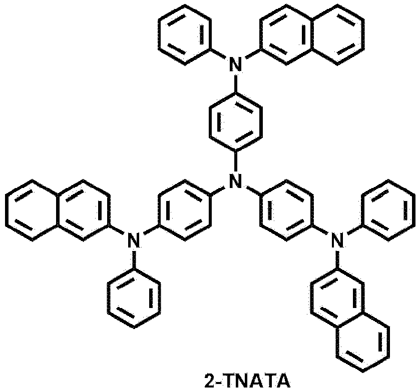

Subsequently, air in the chamber was evacuated until the degree of vacuum in the chamber reached 10⁻⁶ torr, and then a hole injection layer having a thickness of 600 Å was deposited on the ITO substrate by applying current to the cell to evaporate 2-TNATA. A hole transport layer having a thickness of 300 Å was deposited on the hole injection layer by placing the following N,N'-bis(α-naphthyl)-N,N'-diphenyl-4,4'-diamine (NPB) in another cell in the vacuum deposition apparatus and applying current to the cell to evaporate NPB.

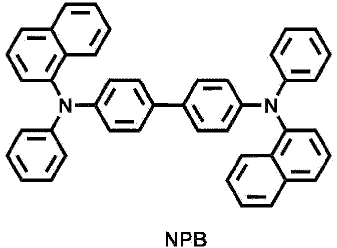

The hole injection layer and the hole transport layer were formed as described above, and then a blue light emitting material having the following structure as a light emitting layer was deposited thereon. Specifically, a blue light emitting host material H1 was vacuum deposited to have a thickness of 200 Å on one cell in the vacuum deposition apparatus, and a blue light emitting dopant material D1 was vacuum deposited thereon in an amount of 5% based on the host material.

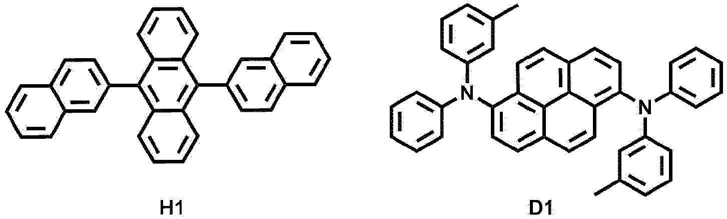

Subsequently, a compound having the following structural formula E1 as an electron transport layer was deposited to have a thickness of 300 Å.

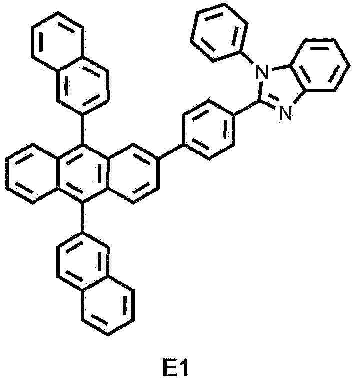

An OLED device was manufactured by depositing lithium fluoride (LiF) as an electron injection layer to have a thickness of 10 Å and allowing the Al negative electrode to have a thickness of 1,000 Å. Meanwhile, all the organic compounds required for manufacturing an OLED device were subjected to vacuum sublimed purification under 10⁻⁶ to 10⁻⁸ torr for each material, and used for the manufacture of OLED.

### Comparative Examples 2 to 7 and Examples 1 to 21

An organic electroluminescence device was manufactured in the same manner as in Comparative Example 1, except that the compound in the following Table 6 was used instead of NPB used when a hole transport layer was formed in Comparative Example 1.

Compounds HT1 to HT6 in the following Table 3 are as follows.

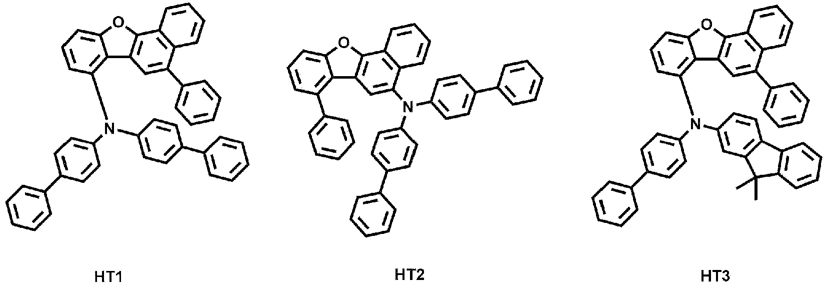

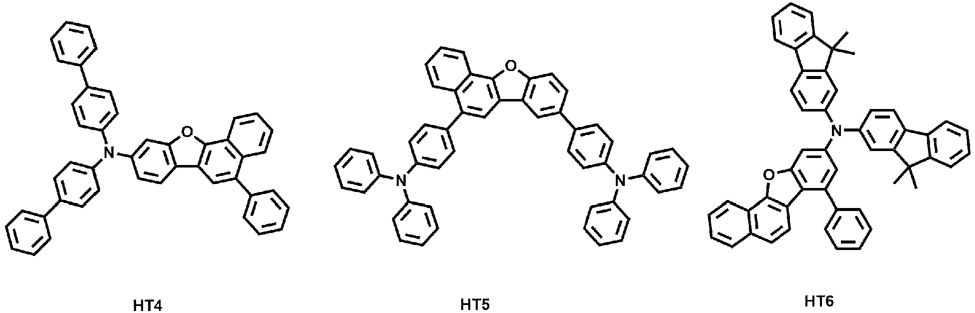

### (2) Evaluation of organic light emitting device

For the organic light emitting device manufactured as described above, the electroluminescence (EL) characteristics were measured using M7000 manufactured by McScience Inc., and the measurement results were used to measure T₉₅ through a lifetime measurement device (M6000) manufactured by McScience Inc., when the reference luminance was 700 cd/m².

The results of measuring the driving voltage, light emitting efficiency, color coordinate (CIE), and service life (T₉₅. unit: time (h)) of the blue organic light emitting device manufactured according to the present invention are shown as in Table 6.

**[Table 6]**

| | Compound | Driving voltage (V) | Light emitting efficiency (cd/A) | CIE (x, y) | Service life (T₉₅) |
|---|---|---|---|---|---|
| Example 1 | 2 | 4.20 | 6.60 | (0.134, 0.100) | 53 |
| Example 2 | 11 | 4.16 | 6.64 | (0.134, 0.100) | 49 |
| Example 3 | 60 | 4.43 | 6.69 | (0.134, 0.100) | 47 |
| Example 4 | 114 | 4.35 | 6.70 | (0.134, 0.101) | 48 |
| Example 5 | 115 | 4.24 | 6.68 | (0.134, 0.101) | 50 |
| Example 6 | 155 | 4.22 | 6.73 | (0.134, 0.100) | 49 |
| Example 7 | 235 | 4.23 | 6.70 | (0.134, 0.100) | 49 |
| Example 8 | 351 | 4.10 | 6.68 | (0.134, 0.100) | 50 |
| Example 9 | 382 | 4.18 | 6.60 | (0.134, 0.100) | 53 |
| Example 10 | 383 | 4.19 | 6.81 | (0.134, 0.101) | 49 |
| Example 11 | 386 | 4.23 | 6.70 | (0.134, 0.100) | 52 |
| Example 12 | 387 | 4.35 | 6.70 | (0.134, 0.101) | 48 |
| Example 13 | 406 | 4.16 | 6.64 | (0.134, 0.100) | 49 |
| Example 14 | 418 | 4.20 | 6.60 | (0.134, 0.100) | 53 |
| Example 15 | 438 | 4.20 | 6.60 | (0.134, 0.100) | 53 |
| Example 16 | 489 | 4.35 | 6.70 | (0.134, 0.101) | 48 |
| Example 17 | 518 | 4.65 | 6.96 | (0.134, 0.101) | 50 |
| Example 18 | 524 | 4.17 | 6.85 | (0.134, 0.101) | 51 |
| Example 19 | 527 | 4.23 | 6.76 | (0.134, 0.101) | 49 |
| Example 20 | 529 | 4.59 | 6.54 | (0.134, 0.101) | 45 |
| Example 21 | 531 | 4.36 | 6.31 | (0.134, 0.101) | 47 |
| Comparative Example 1 | NPB | 5.16 | 6.00 | (0.134, 0.101) | 21 |
| Comparative Example 2 | HT1 | 5.19 | 6.08 | (0.134, 0.101) | 20 |
| Comparative Example 3 | HT2 | 5.20 | 6.04 | (0.134, 0.101) | 18 |
| Comparative Example 4 | HT3 | 5.20 | 6.02 | (0.134, 0.101) | 23 |
| Comparative Example 5 | HT4 | 5.20 | 6.05 | (0.134, 0.101) | 24 |
| Comparative Example 6 | HT5 | 5.20 | 6.03 | (0.134, 0.101) | 22 |
| Comparative Example 7 | HT6 | 5.20 | 6.05 | (0.134, 0.101) | 25 |

As can be seen from the results in Table 6, the organic light emitting device using a hole transport layer material of the blue organic light emitting device of the present invention has a low driving voltage and remarkably improved light emitting efficiency and service life compared to the Comparative Examples.

In particular, for the compound of the present invention, it could be confirmed that when an amine derivative was used as a hole transport layer, the unshared electron pair of amine could enhance the flow of holes and improve the hole transfer capacity of the hole transport layer, and the binding of the amine moiety to the substituent with enhanced hole characteristics also enhanced the planarity and glass transition temperature of the amine derivative to enhance the thermal stability of the compound.

Further, since the bandgap and the energy level value of the triple state (T1 value) are adjusted to improve the hole transfer capacity and improve the stability of the molecule, it could be confirmed that the driving voltage of the device is lowered, the light efficiency of the device is improved, and service life characteristics of the device are improved by the thermal stability of the compound.

Specifically, it could be confirmed that when a compound having two substituents at one phenyl of a naphthobenzofuran core, that is, an amine group in which one or more aryl groups are substituted and an aryl group, or an amine group in which one or more aryl groups are substituted and a heteroaryl group including O or S as in the compound of the present application is used for a hole transport layer, the light emitting efficiency and service life are excellent because the substituted aryl group in the amine group delocalizes the highest occupied molecular orbital (HOMO) energy level of the compound to stabilize the HOMO energy of the compound.

### (1) Manufacture of organic light emitting device

### Comparative Examples 8 to 14 and Examples 22 to 42

A glass substrate, in which ITO was thinly coated to have a thickness of 1,500 Å, was ultrasonically washed with distilled water. When the washing with distilled water was finished, the glass substrate was ultrasonically washed with a solvent such as acetone, methanol, and isopropyl alcohol, dried and then was subjected to UVO treatment for 5 minutes using UV in a UV cleaning machine. Thereafter, the substrate was transferred to a plasma washing machine (PT), and then was subjected to plasma treatment in a vacuum state for an ITO work function and in order to remove a residual film, and was transferred to a thermal deposition apparatus for organic deposition.

Subsequently, air in the chamber was evacuated until the degree of vacuum in the chamber reached 10⁻⁶ torr, and then a hole injection layer having a thickness of 600 Å was deposited on the ITO substrate by applying current to the cell to evaporate 2-TNATA.

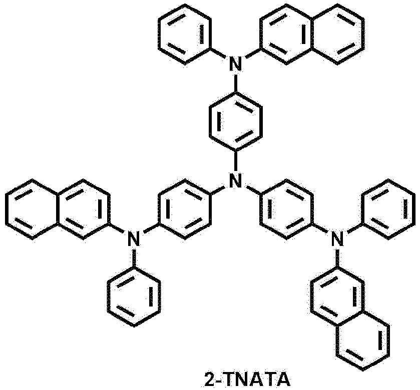

A hole transport layer having a thickness of 300 Å was deposited on the hole injection layer by placing the following N,N'-bis(α-naphthyl)-N,N'-diphenyl-4,4'-diamine (NPB) in another cell in the vacuum deposition apparatus and applying current to the cell to evaporate NPB.

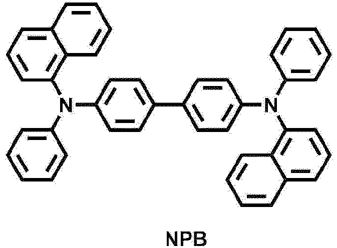

Thereafter, a prime layer was formed by depositing the compounds shown in the following Table 7 to 100 Å.

Compounds HT1 to HT6 in the following Table 7 are as follows.

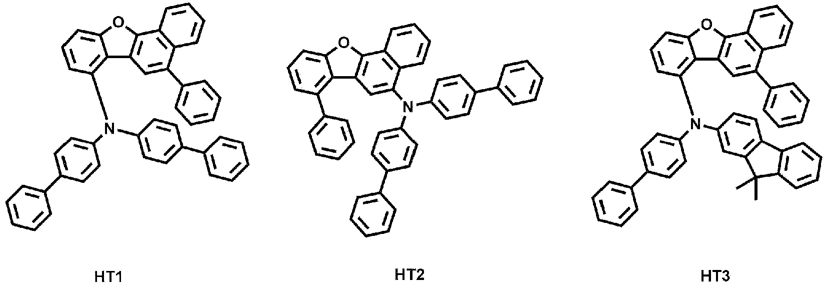

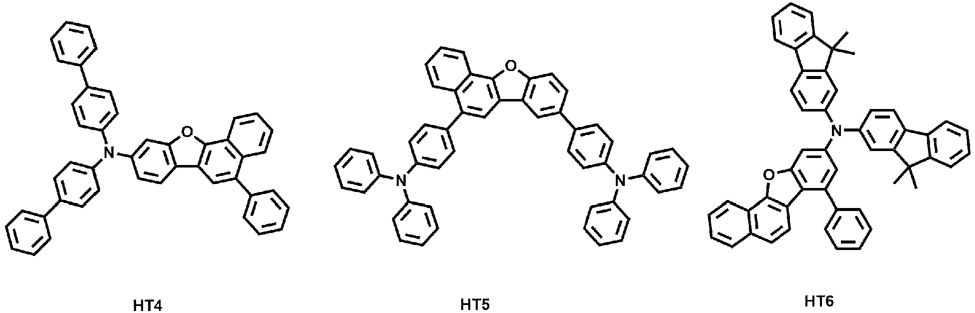

A light emitting layer was thermally vacuum deposited thereon as follows. The light emitting layer was deposited by depositing a compound of 9-[4-(4,6-diphenyl-1,3,5-triazin-2-yl)phenyl]-9'-phenyl-3,3'-Bi-9H-carbazole as a host to 400 Å and doping the deposited layer with Ir(ppy)₃ as a green phosphorescent dopant by 7% of the thickness of the light emitting layer. Thereafter, BCP as a hole blocking layer was deposited to have a thickness of 60 Å, and Alq₃ as an electron transport layer was deposited to have a thickness of 200 Å thereon.

Finally, an organic light emitting device was manufactured by depositing lithium fluoride (LiF) to have a thickness of 10 Å on the electron transport layer to form an electron injection layer, and then depositing an aluminum (Al) negative electrode to have a thickness of 1,200 Å on the electron injection layer to form a negative electrode.

Meanwhile, all the organic compounds required for manufacturing an organic light emitting device were subjected to vacuum sublimed purification under 10⁻⁸ to 10⁻⁶ torr for each material, and used for the manufacture of the organic light emitting device.

### (2) Evaluation of organic light emitting device

For the organic light emitting devices of Comparative Examples 8 to 14 and Examples 22 to 42 manufactured as above, electroluminescent light emission (EL) characteristics were measured using M7000 manufactured by McScience Inc., and with the measurement results, a service life T₉₀ (unit: h, hour), which was the time when the luminance became 90% compared to the initial luminance when the reference luminance was 6,000 cd/m², was measured using a service life measurement device (M6000) manufactured by McScience Inc, and the measurement results are shown in the following Table 7.

**[Table 7]**

| | Compound | Driving voltage (V) | Light emitting efficiency (cd/A) | Service life (T₉₅) |
|---|---|---|---|---|
| Example 22 | 2 | 4.30 | 6.80 | 250 |
| Example 23 | 11 | 4.25 | 6.85 | 250 |
| Example 24 | 60 | 4.29 | 6.90 | 220 |
| Example 25 | 114 | 4.29 | 6.87 | 253 |
| Example 26 | 115 | 4.30 | 6.86 | 253 |
| Example 27 | 155 | 4.29 | 6.88 | 243 |
| Example 28 | 235 | 4.31 | 6.90 | 248 |
| Example 29 | 351 | 4.27 | 6.91 | 250 |
| Example 30 | 382 | 4.29 | 6.87 | 251 |
| Example 31 | 383 | 4.31 | 6.88 | 248 |
| Example 32 | 386 | 4.32 | 6.92 | 256 |
| Example 33 | 387 | 4.30 | 6.92 | 249 |
| Example 34 | 406 | 4.23 | 6.93 | 253 |
| Example 35 | 418 | 4.28 | 6.85 | 252 |
| Example 36 | 438 | 4.29 | 6.87 | 252 |
| Example 37 | 489 | 4.27 | 6.85 | 262 |
| Example 38 | 518 | 4.19 | 6.93 | 275 |
| Example 39 | 524 | 4.36 | 6.92 | 285 |
| Example 40 | 527 | 4. 68 | 6.82 | 243 |
| Example 41 | 529 | 4.52 | 6.86 | 265 |
| Example 42 | 531 | 4.32 | 6.88 | 255 |
| Comparative Example 8 | NPB | 5.32 | 6.18 | 172 |
| Comparative Example 9 | HT1 | 5.36 | 6.14 | 171 |
| Comparative Example 10 | HT2 | 5.30 | 6.18 | 182 |
| Comparative Example 11 | HT3 | 5.33 | 6.16 | 167 |
| Comparative Example 12 | HT4 | 5.30 | 6.10 | 178 |
| Comparative Example 13 | HT5 | 5.20 | 6.22 | 168 |
| Comparative Example 14 | HT6 | 5.19 | 6.22 | 149 |

From the results of Table 7, it could be confirmed that during the formation of the hole transport auxiliary layer, in the case of the organic light emitting devices of Examples 22 to 42, in which the compound according to the present application was used, electrons were effectively prevented from crossing from the opposite side of the electron transport layer by having a structure in which two specific substituents including an amine group in naphthobenzofuran was substituted to delocalize the highest occupied molecular orbital (HOMO) energy level, thereby stabilizing the HOMO energy, and as a result, during the formation of the hole transport auxiliary layer, the light emitting efficiencies and service lives thereof were better than those of the organic light emitting devices of Comparative Examples 8 to 14 in which the compound according to the present application was not used.

Specifically, it could be confirmed that an amine group and a specific aryl group such as a fluorene group and a pyrene group and an aryl group or heteroaryl group composed of 21 to 60 carbon atoms in naphthobenzofuran effectively prevented electrons from crossing from the opposite side of the electron transport layer by having a structure in which the substituent was bi-substituted to delocalize the highest occupied molecular orbital (HOMO) energy level, thereby stabilizing the HOMO energy, and as a result, during the formation of the prime layer, the light emitting efficiencies and service lives thereof were better than those of the organic light emitting devices of the Comparative Examples in which the compound according to the present application was not used.

## Claims

1. A heterocyclic compound represented by any one of the following compounds:

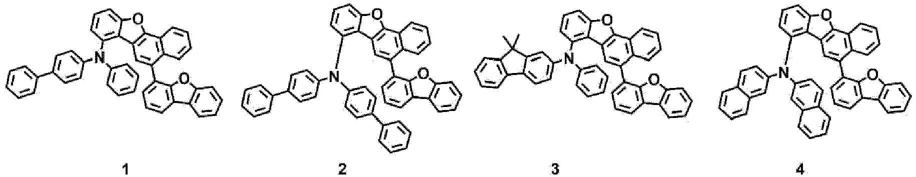

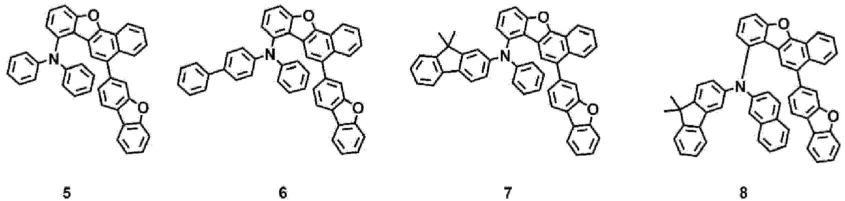

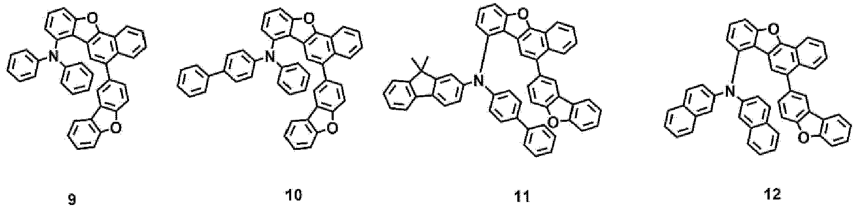

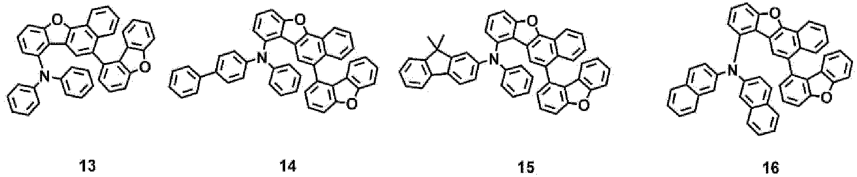

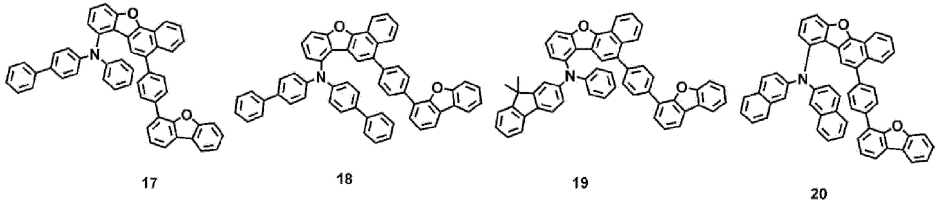

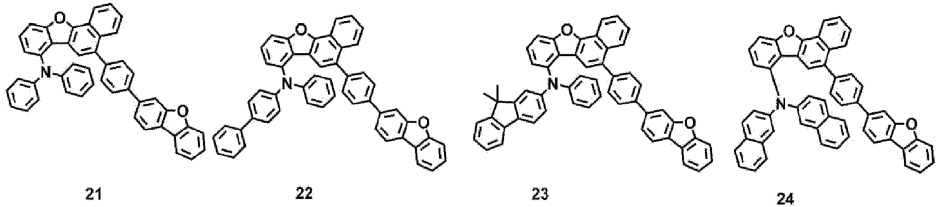

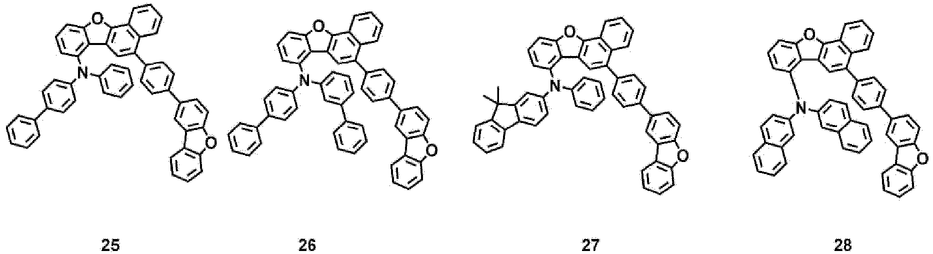

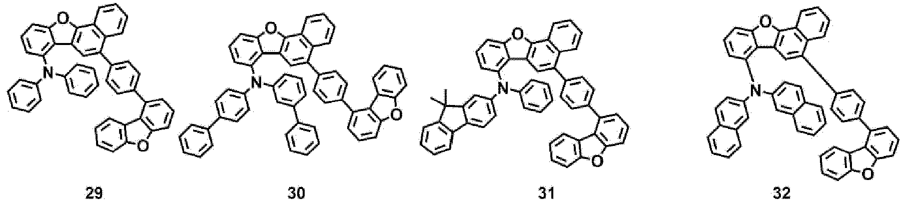

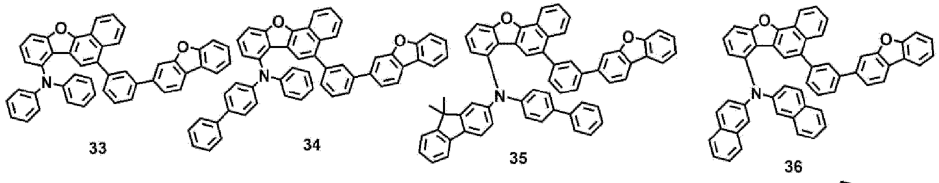

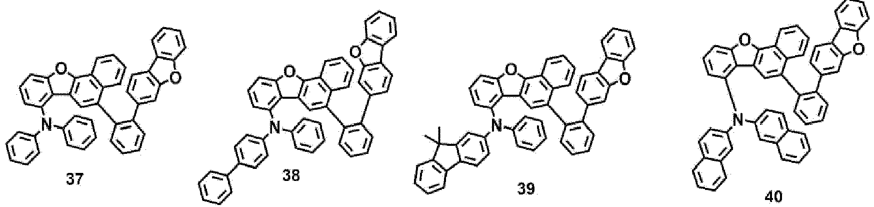

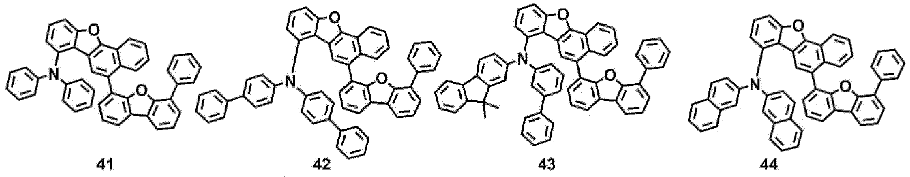

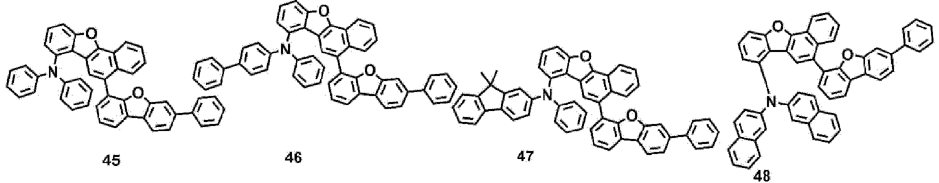

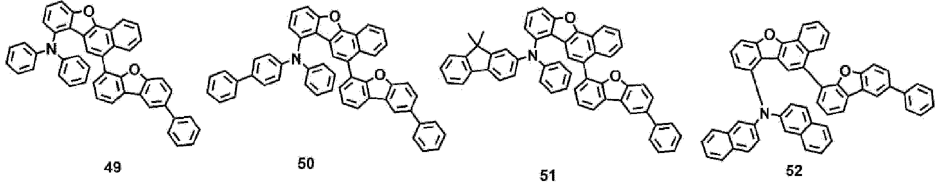

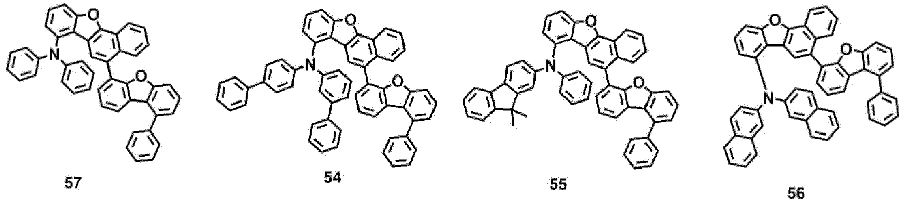

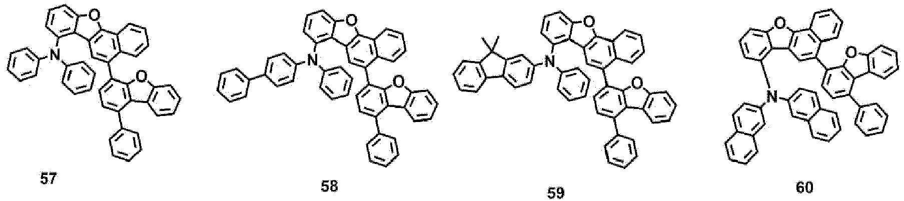

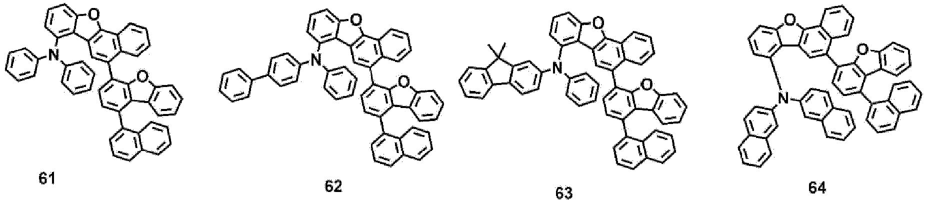

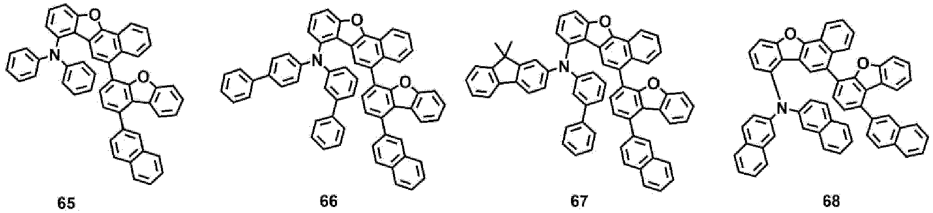

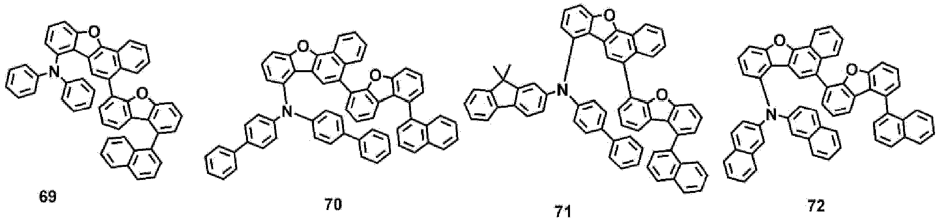

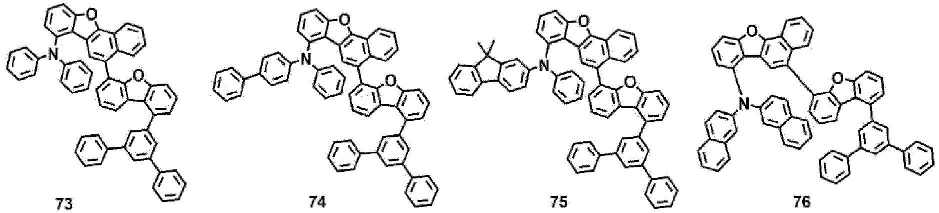

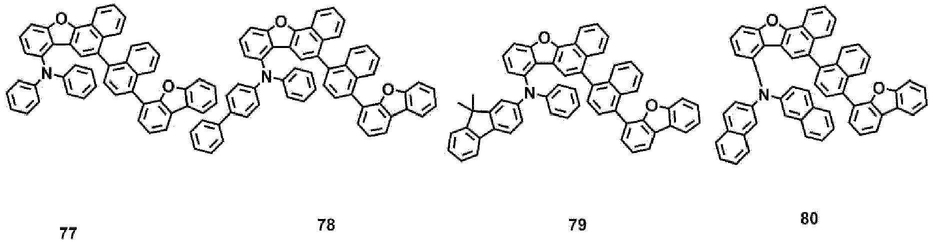

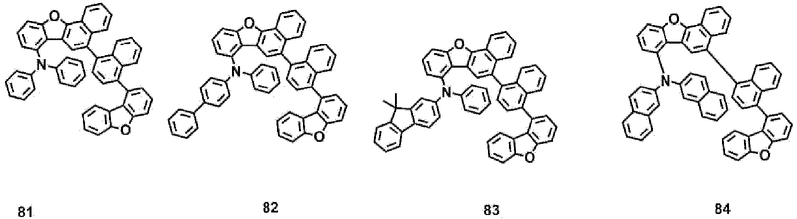

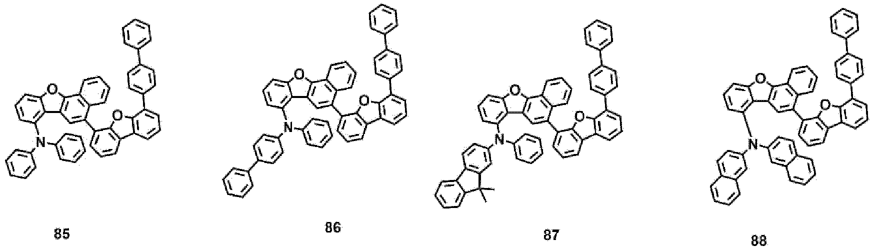

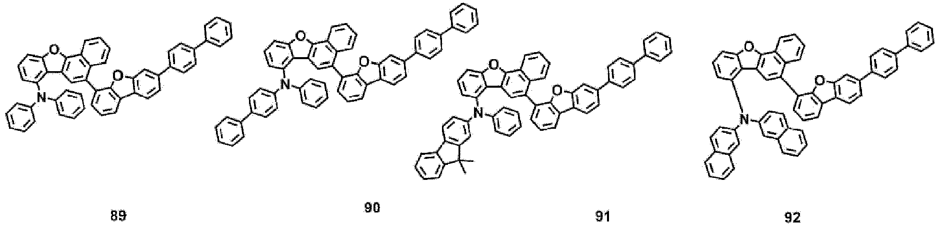

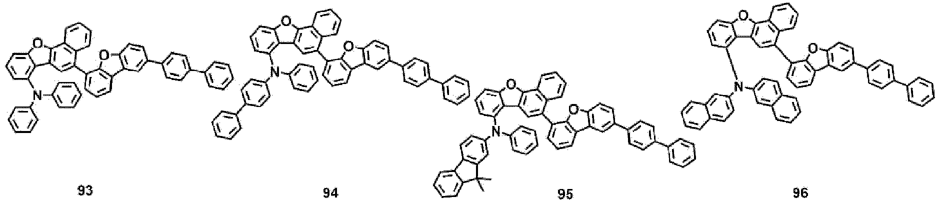

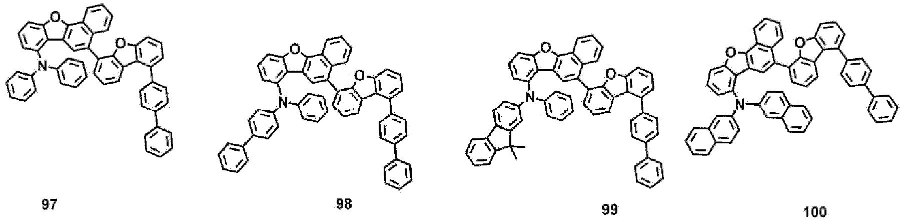

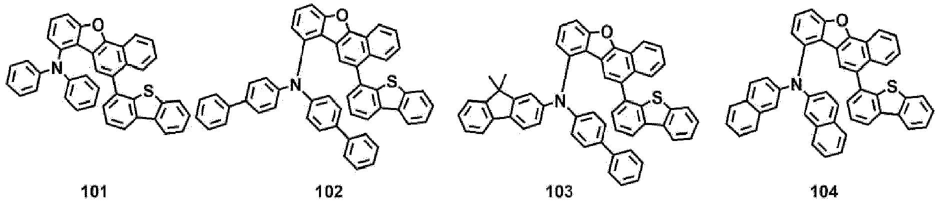

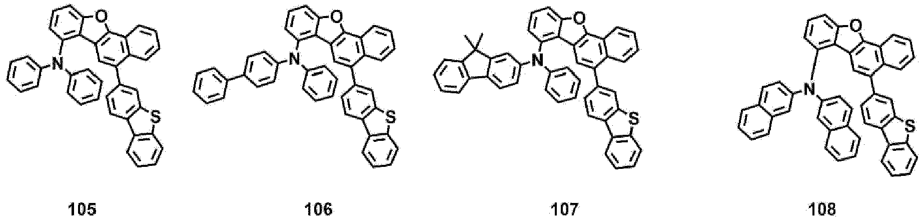

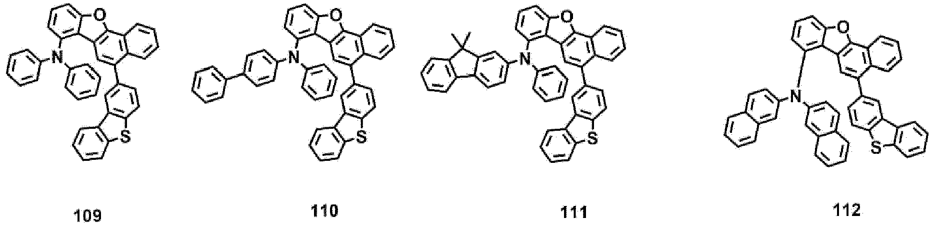

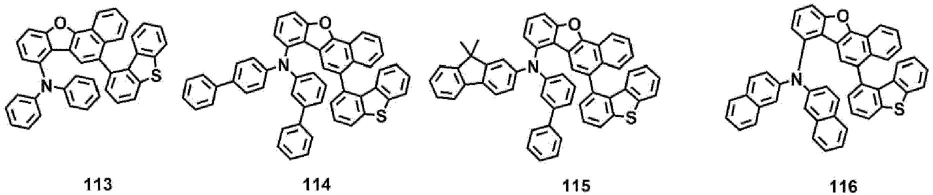

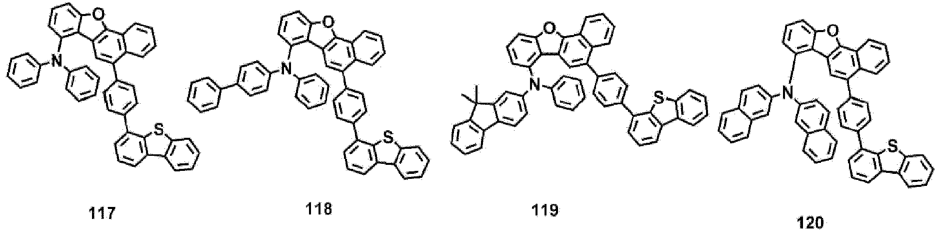

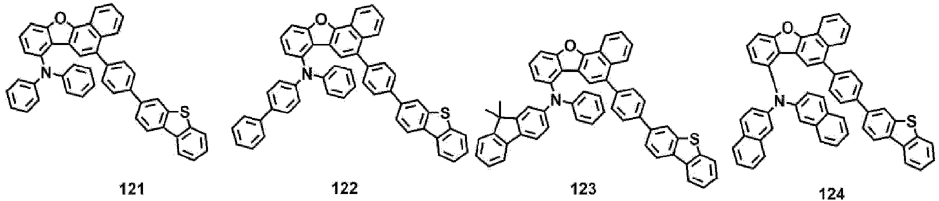

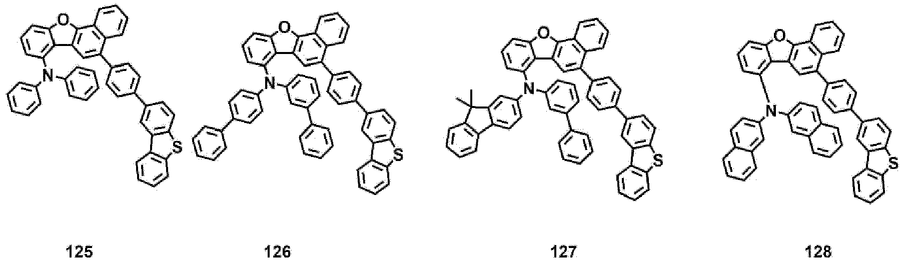

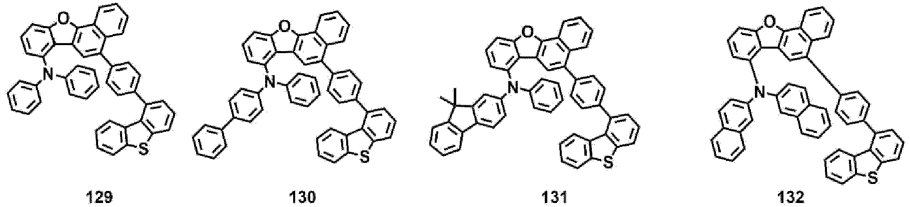

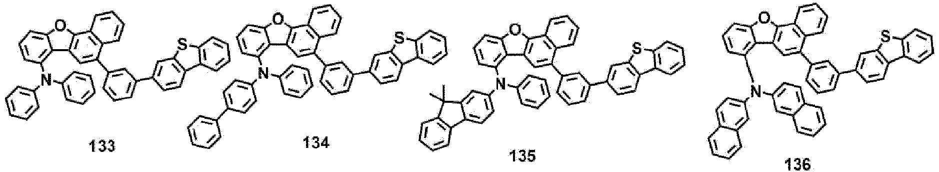

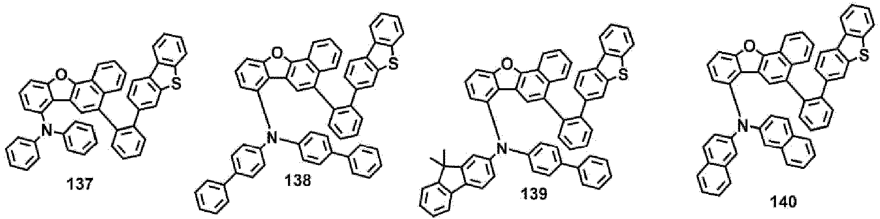

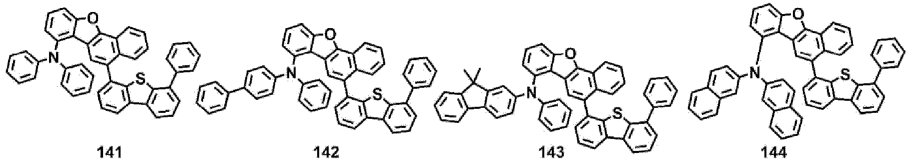

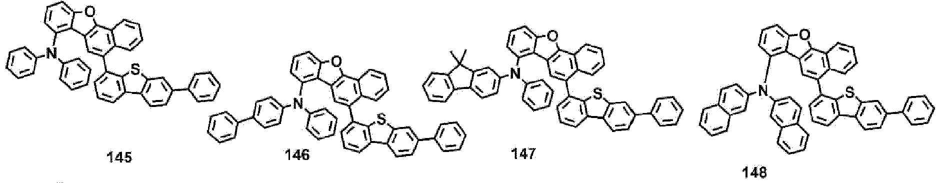

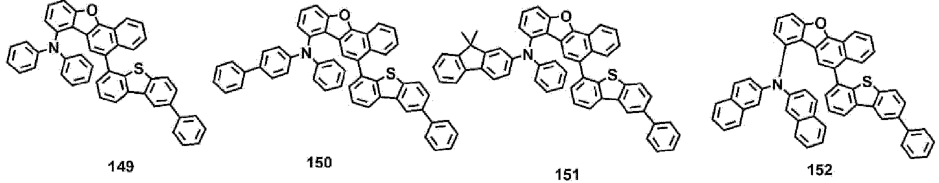

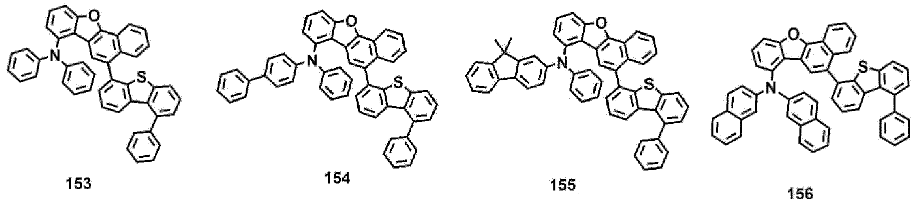

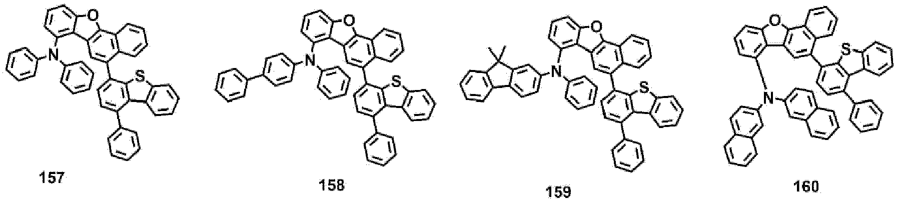

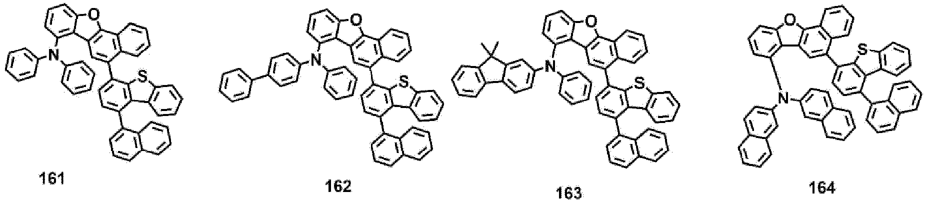

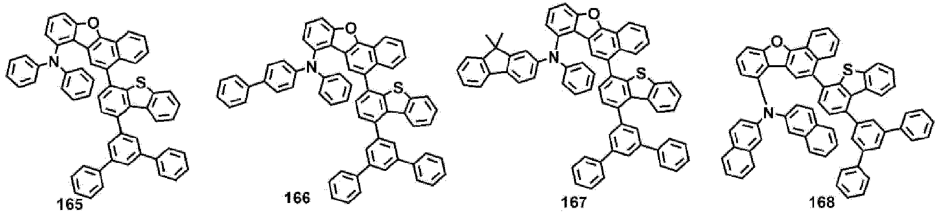

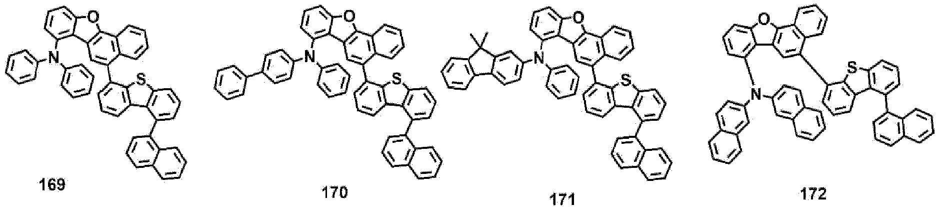

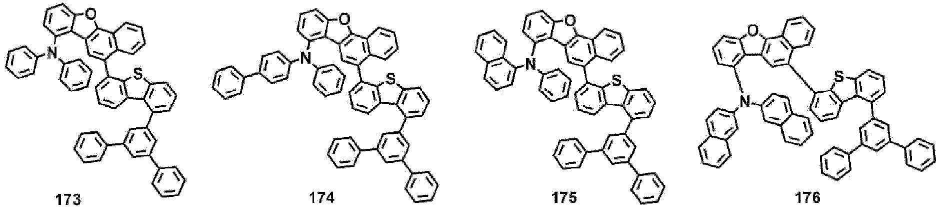

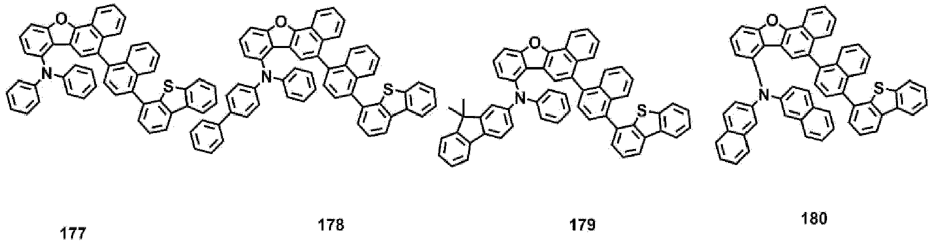

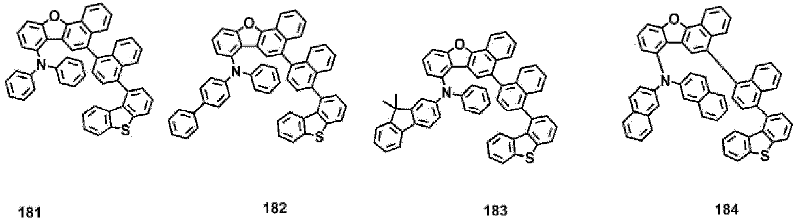

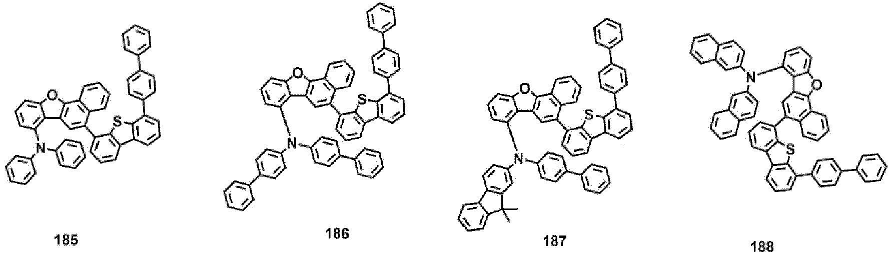

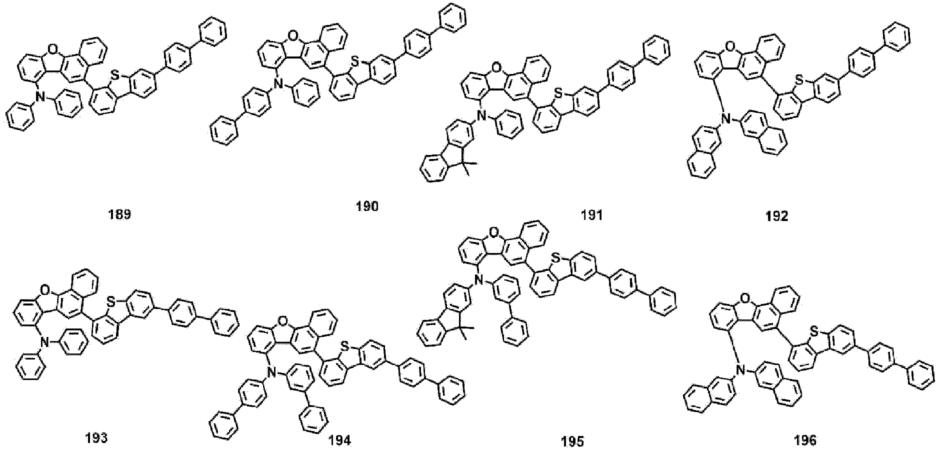

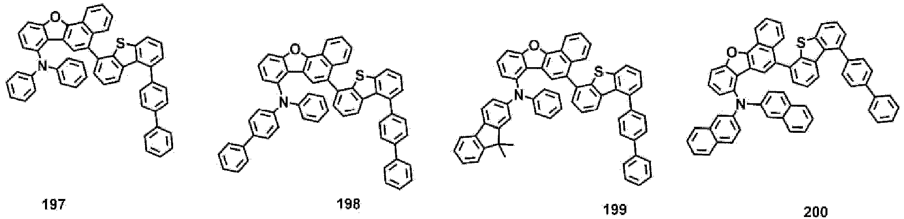

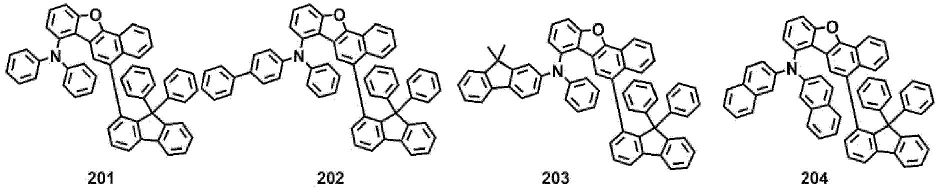

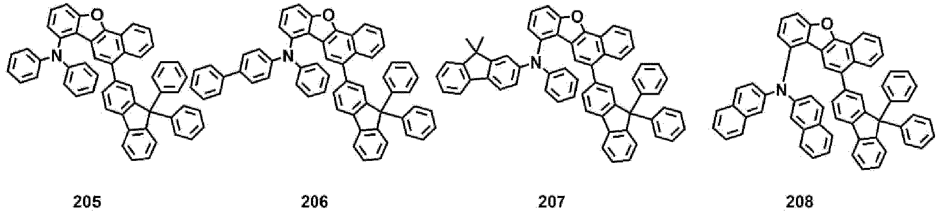

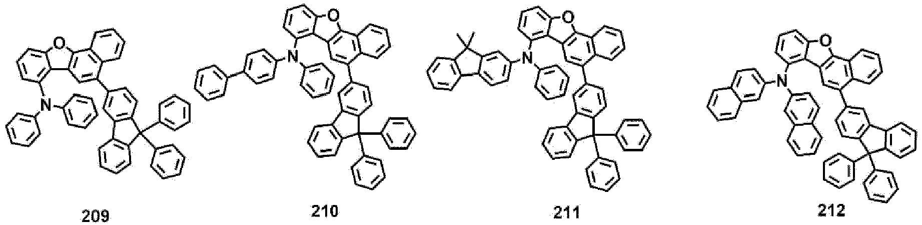

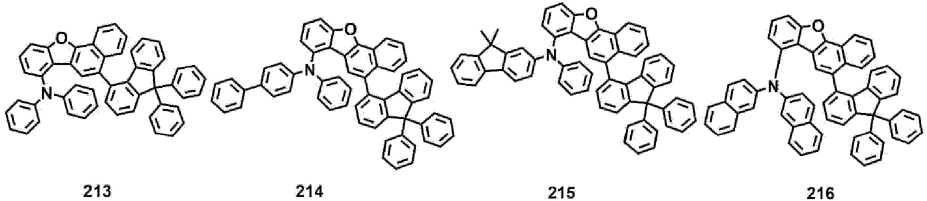

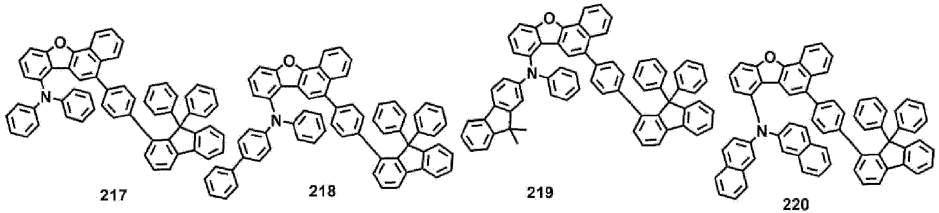

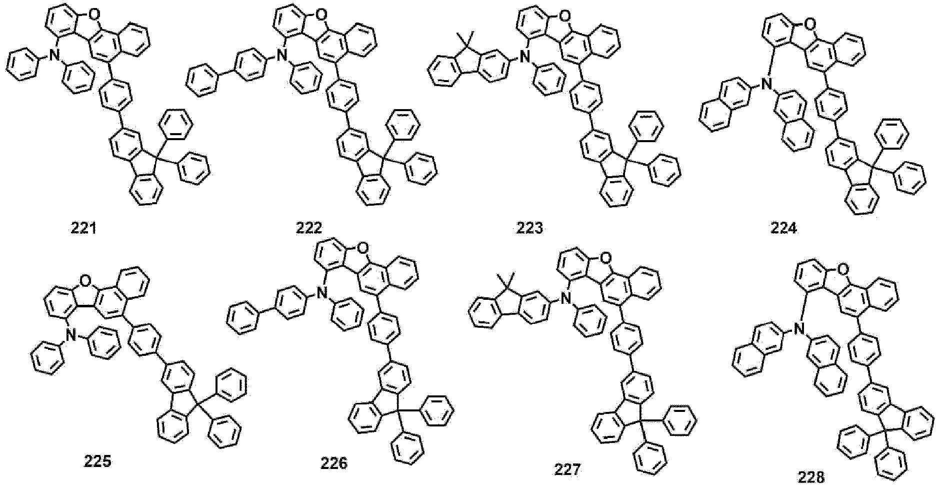

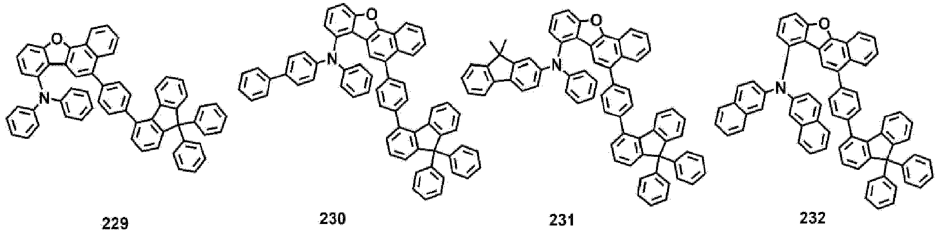

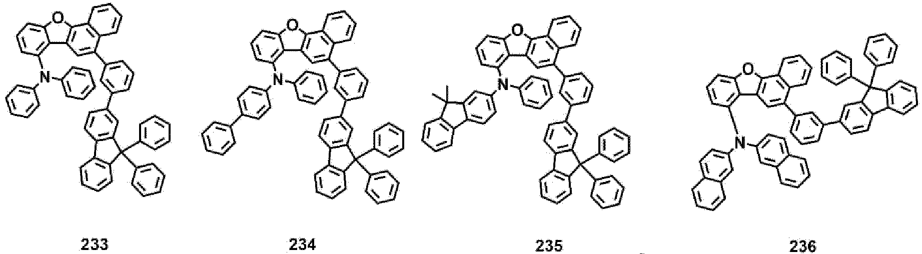

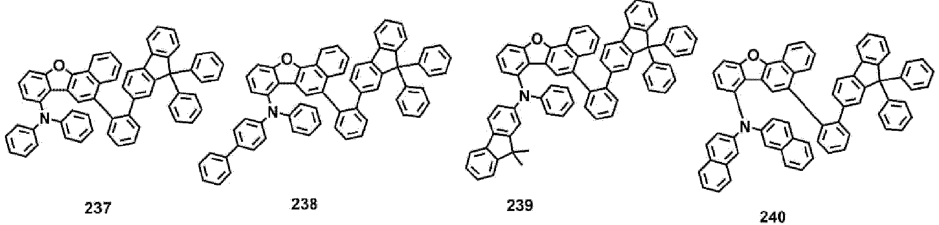

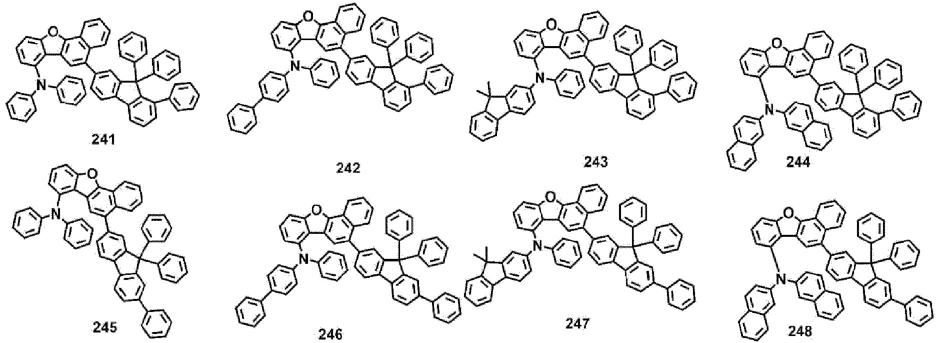

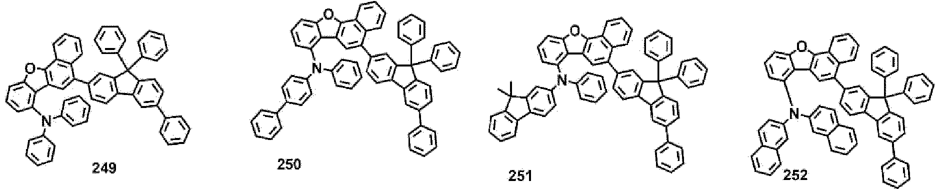

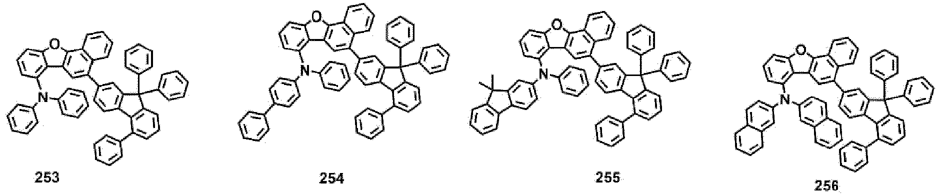

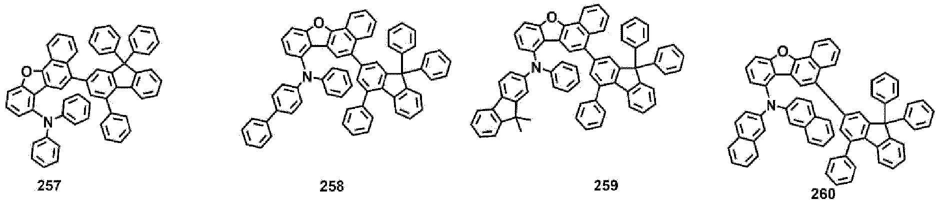

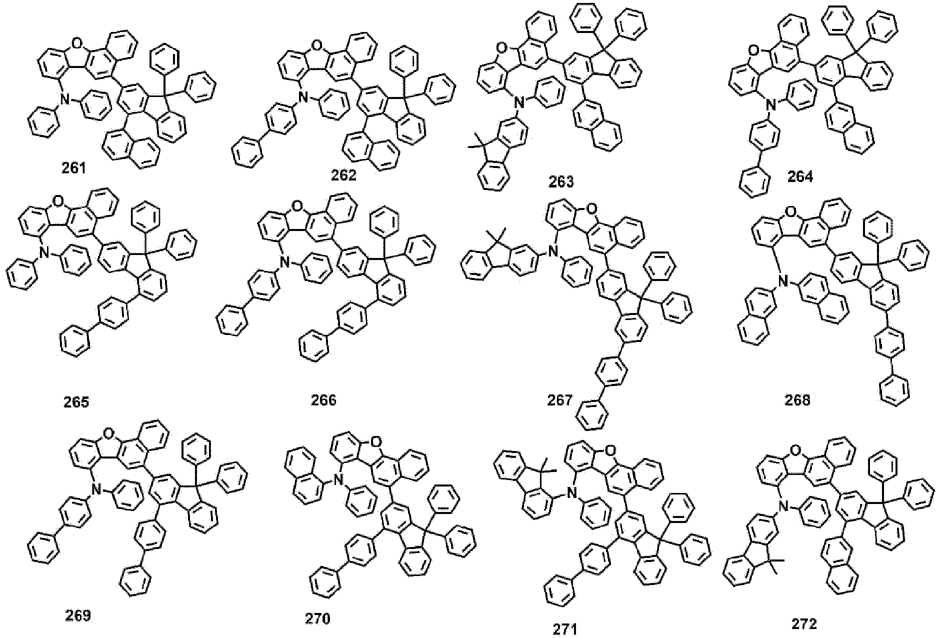

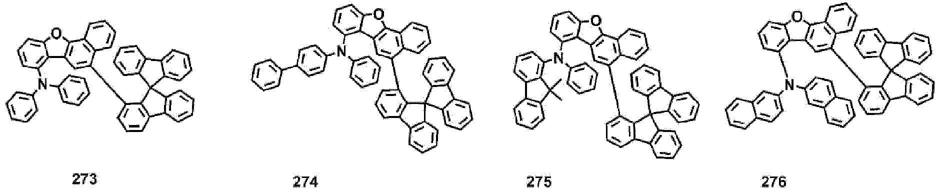

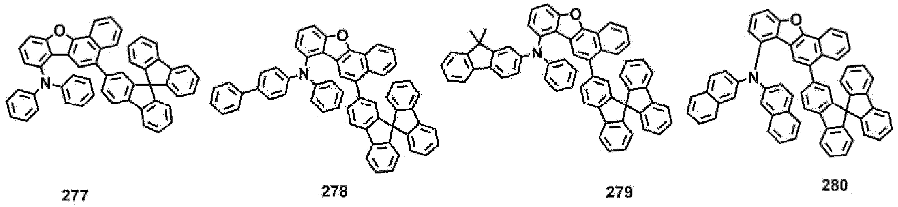

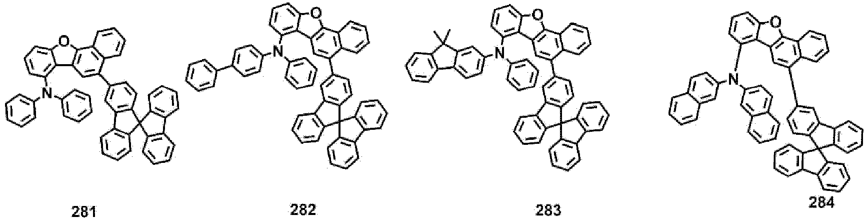

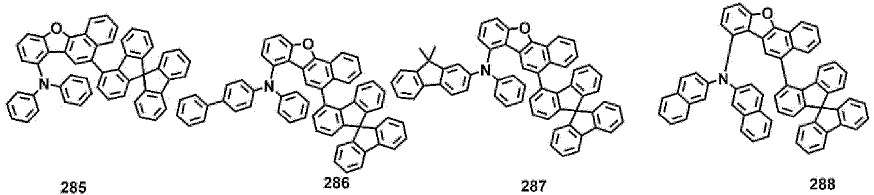

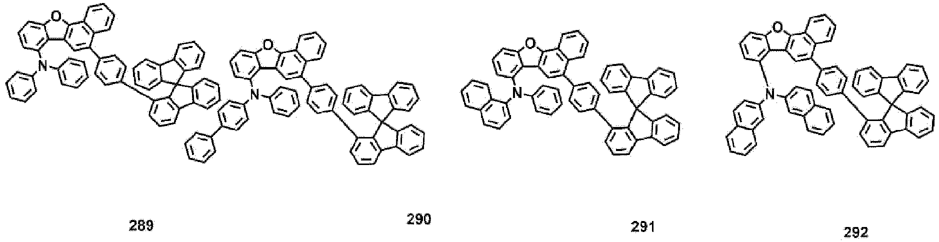

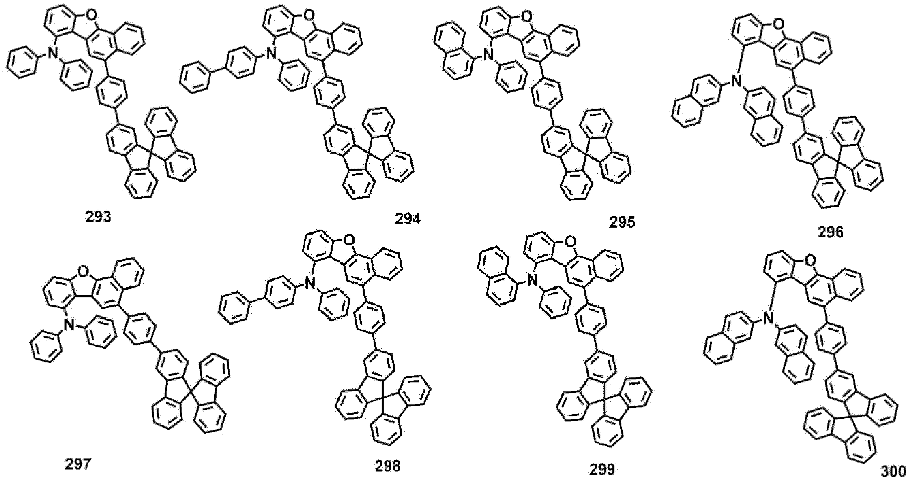

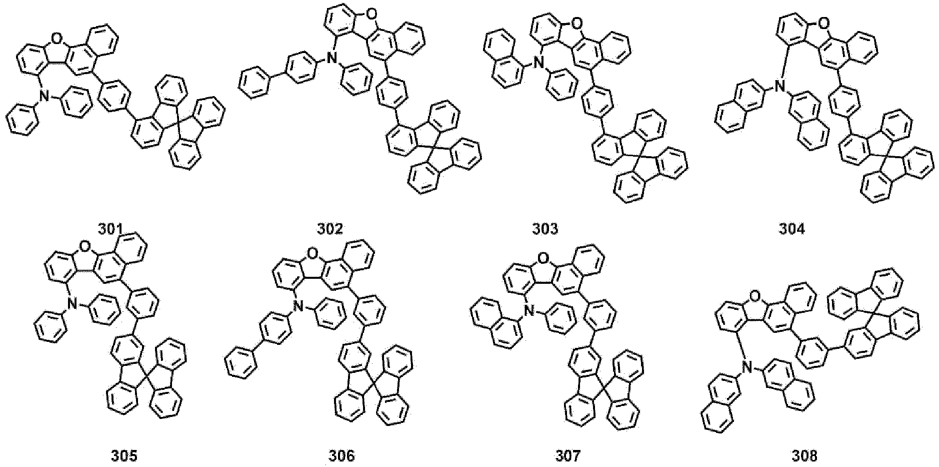

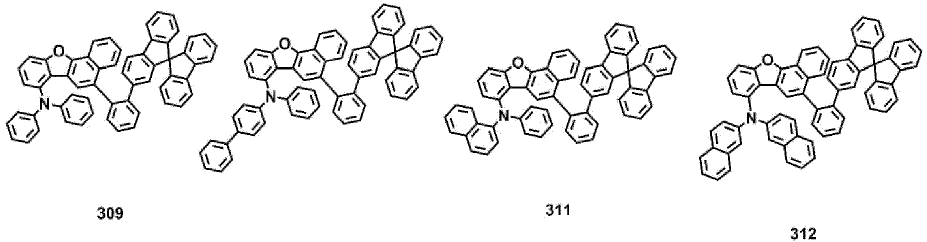

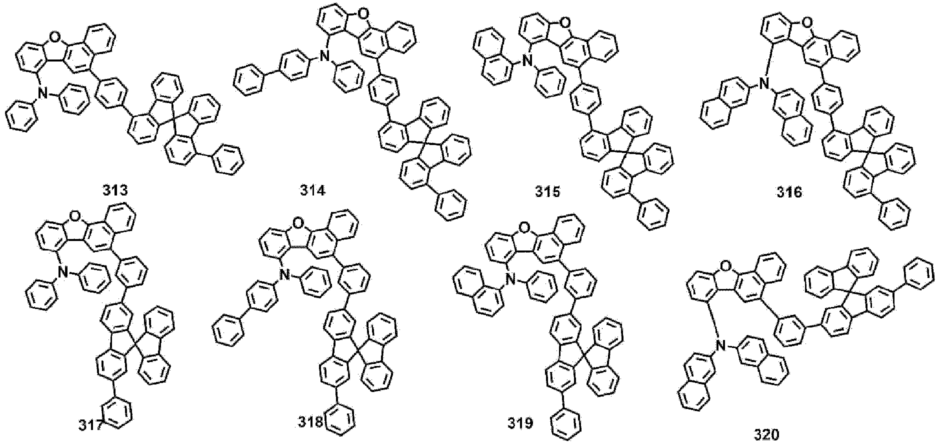

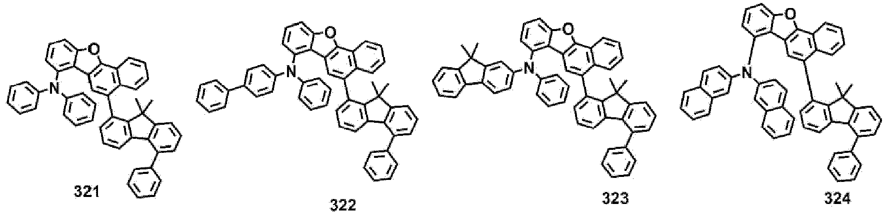

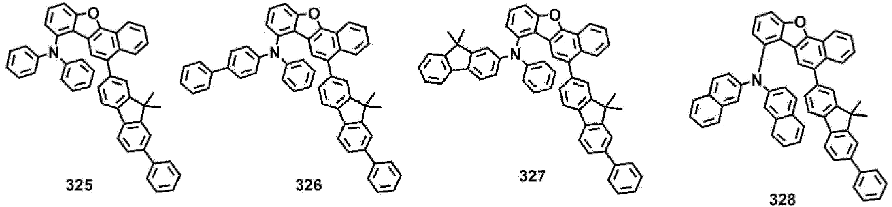

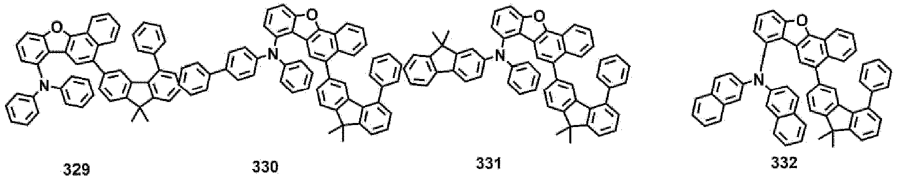

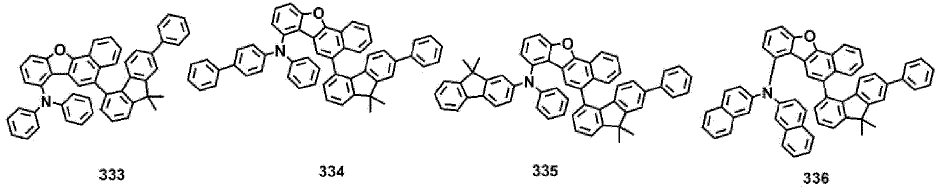

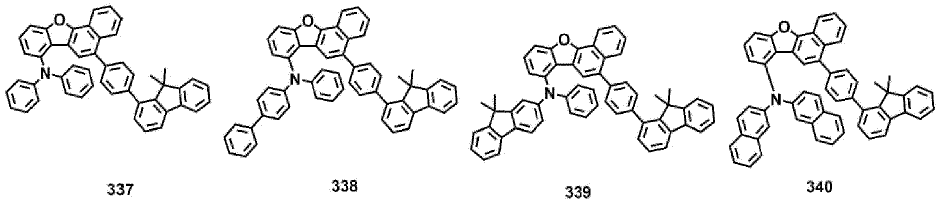

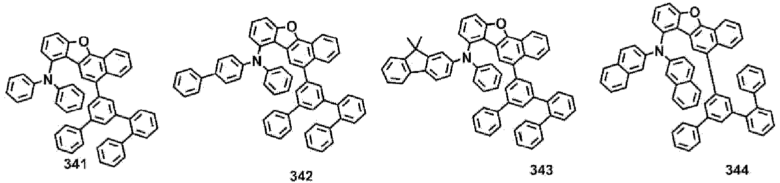

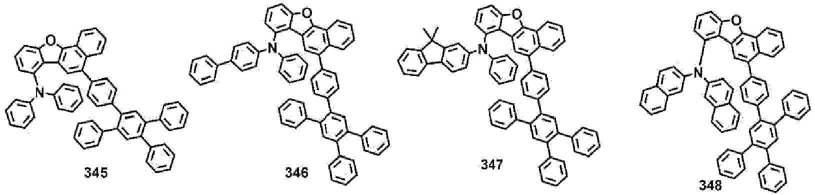

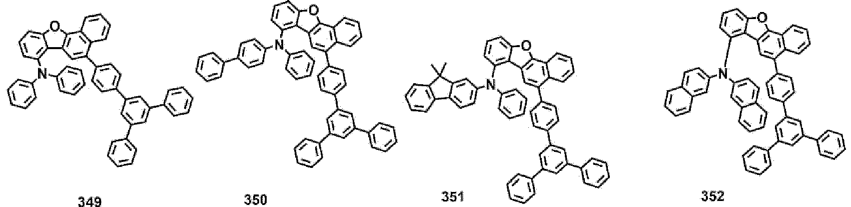

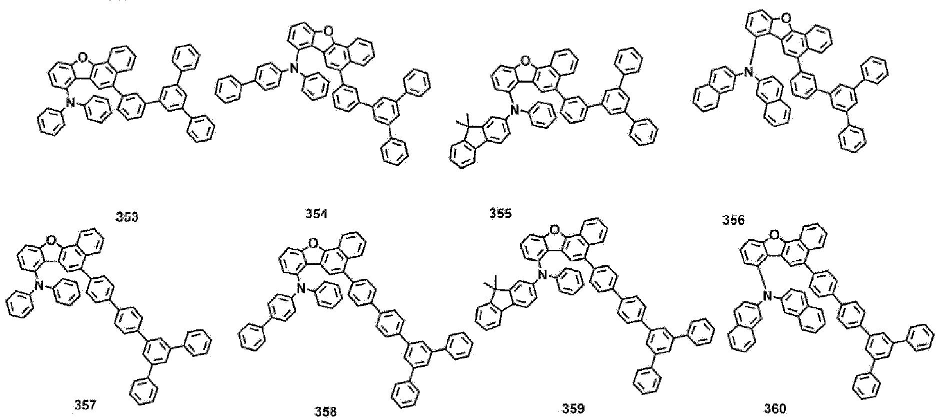

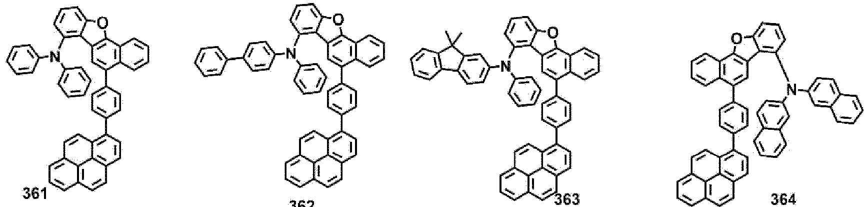

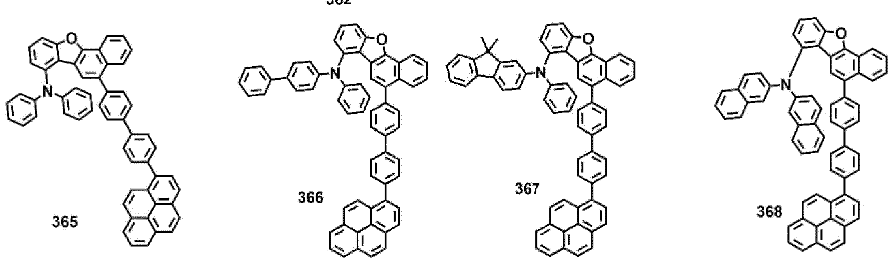

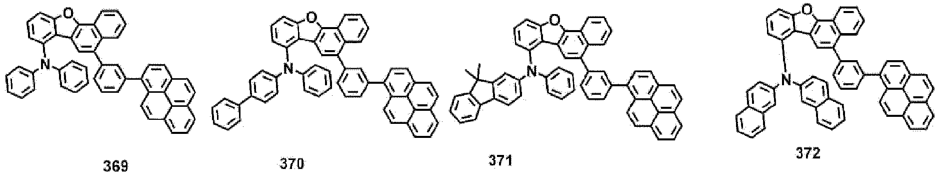

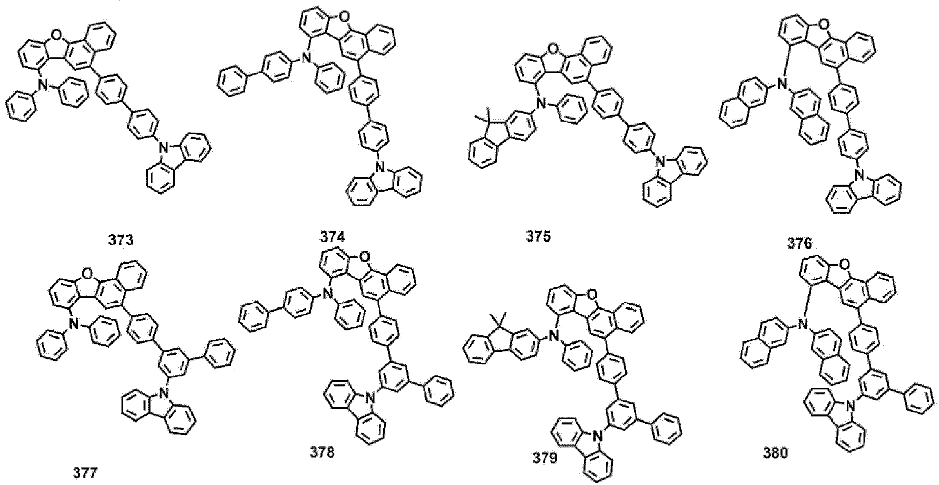

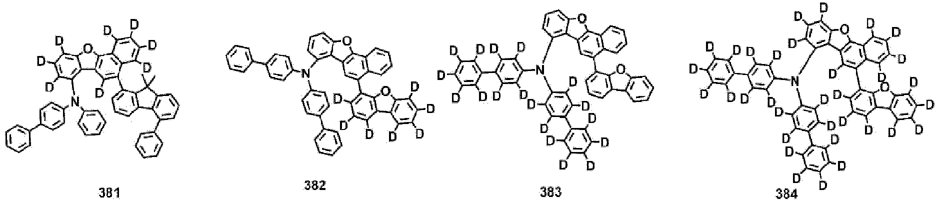

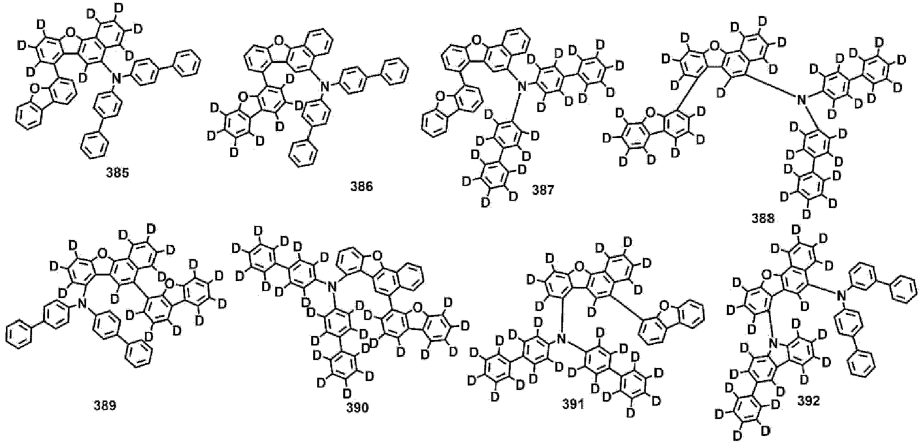

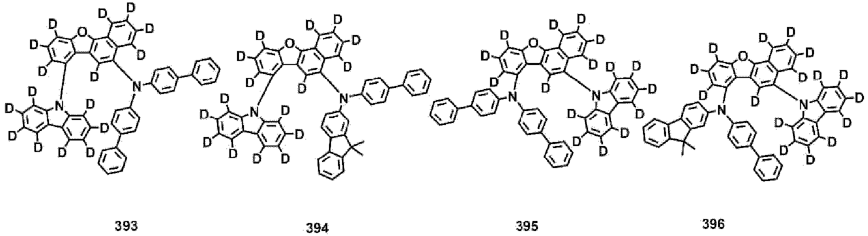

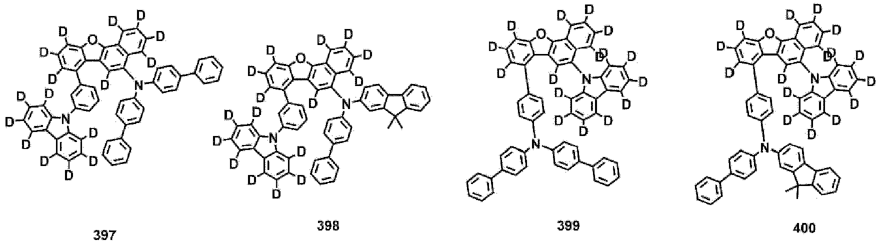

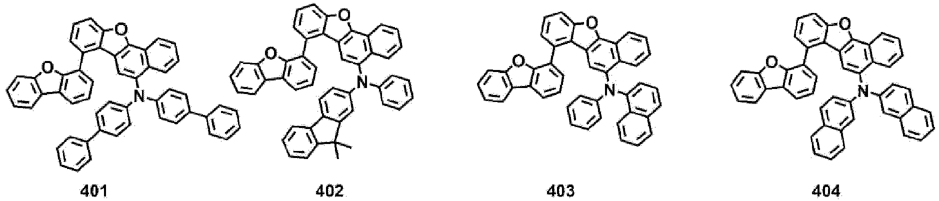

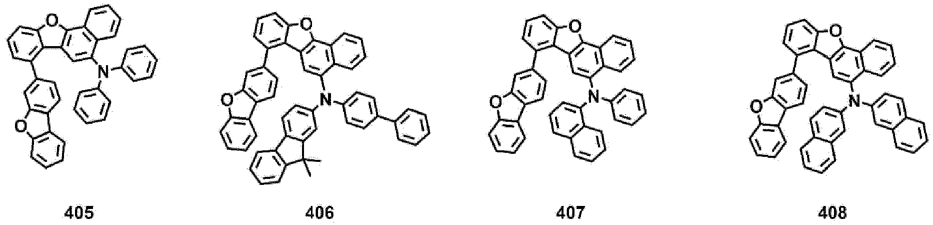

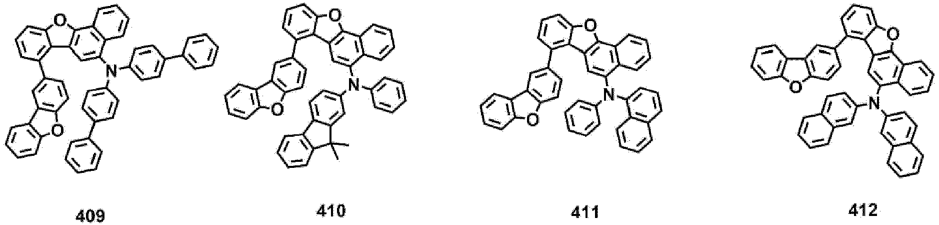

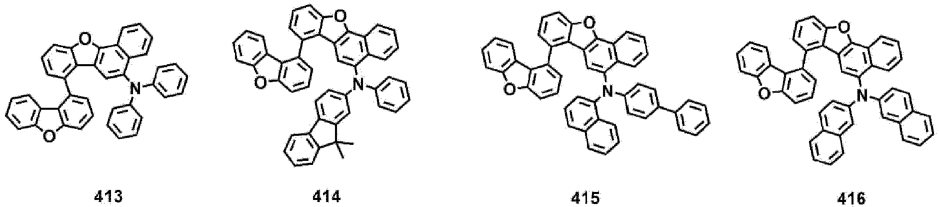

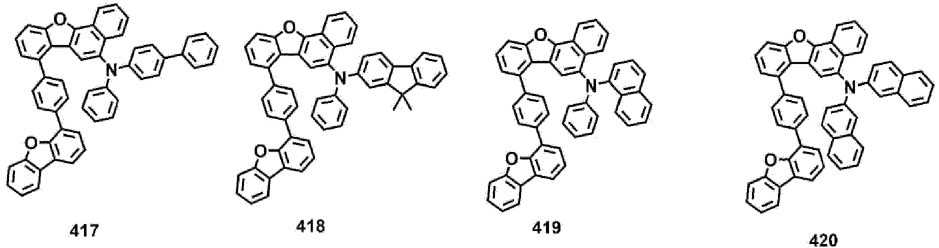

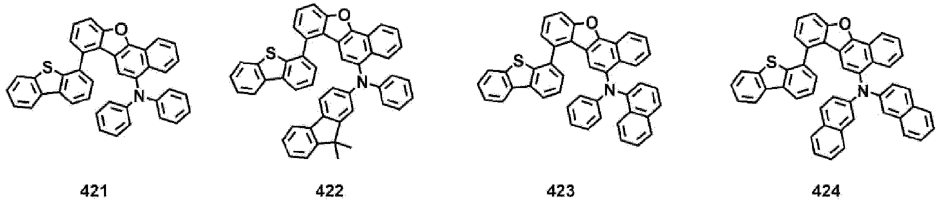

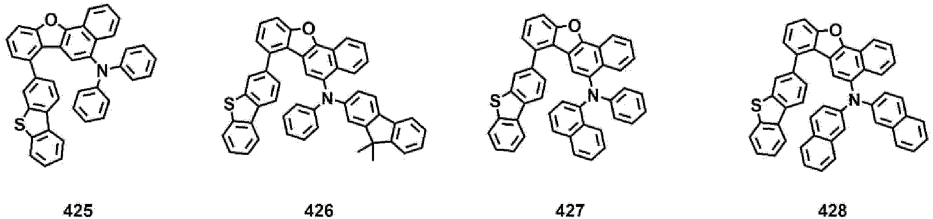

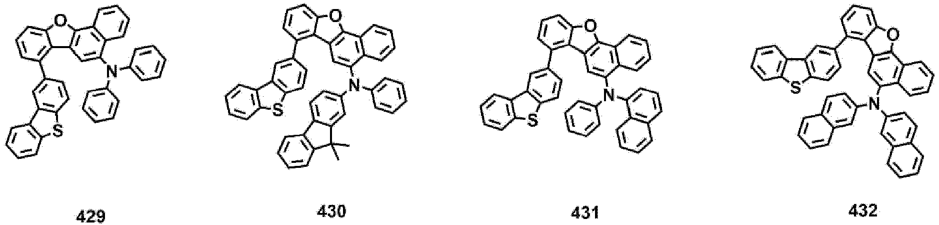

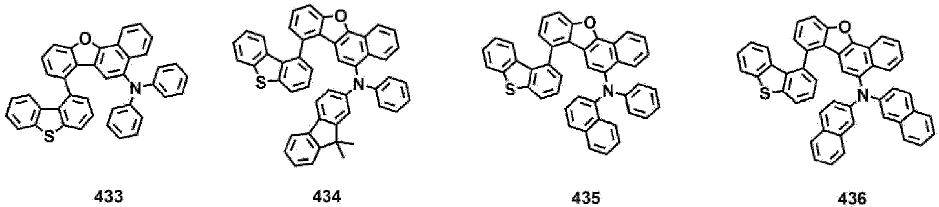

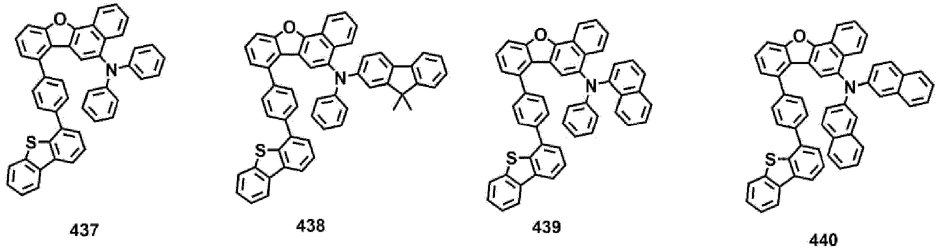

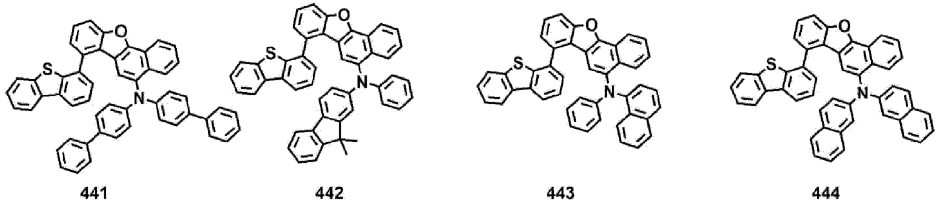

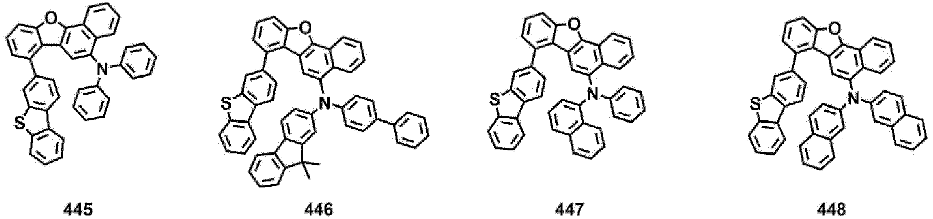

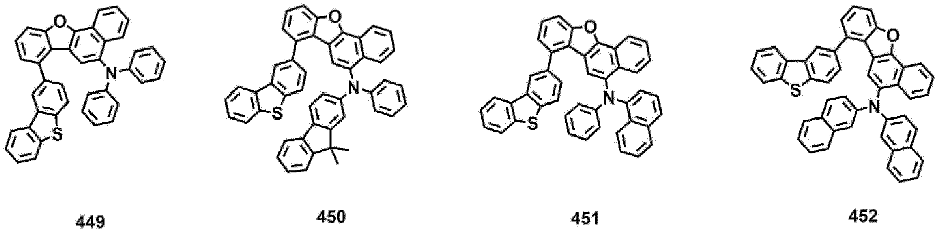

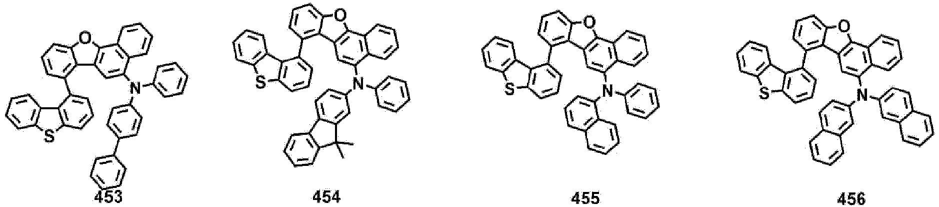

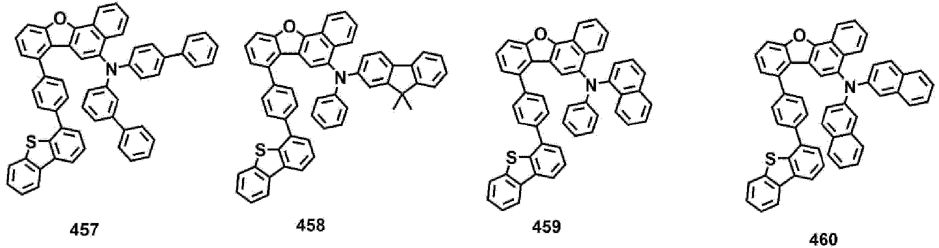

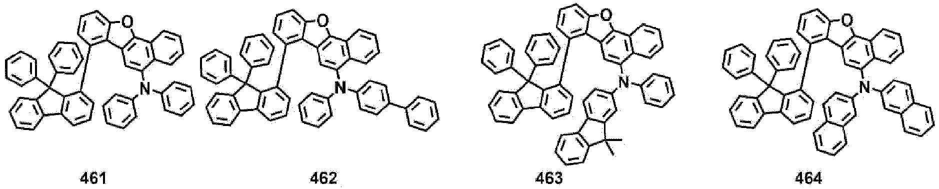

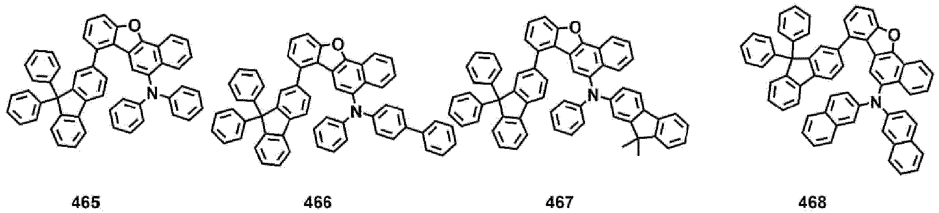

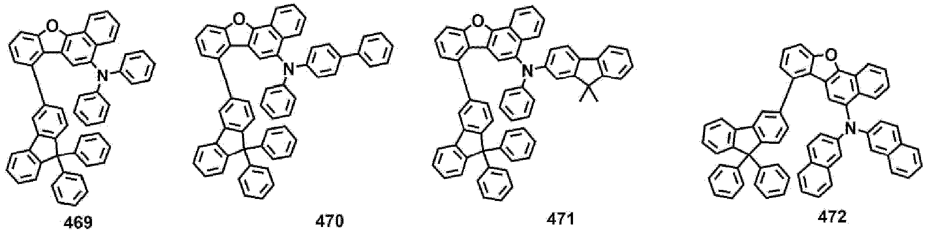

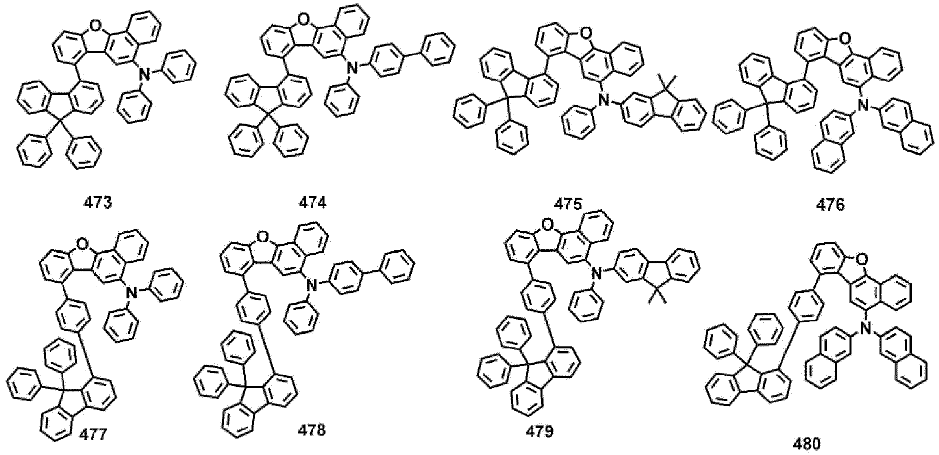

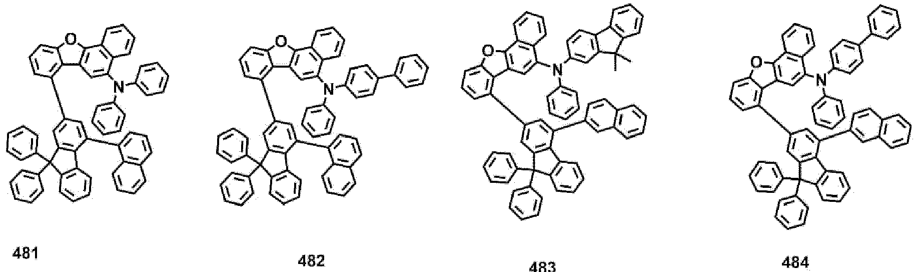

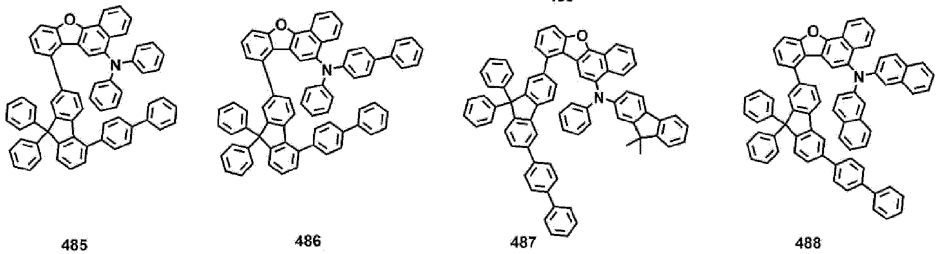

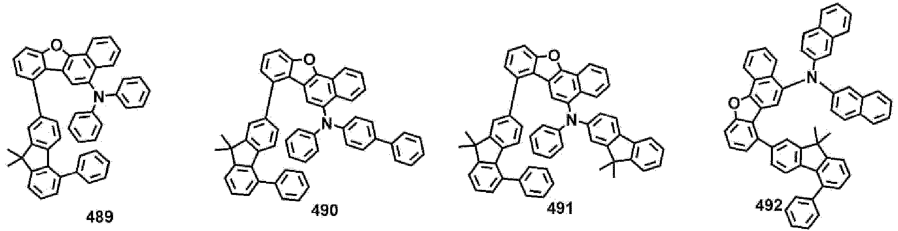

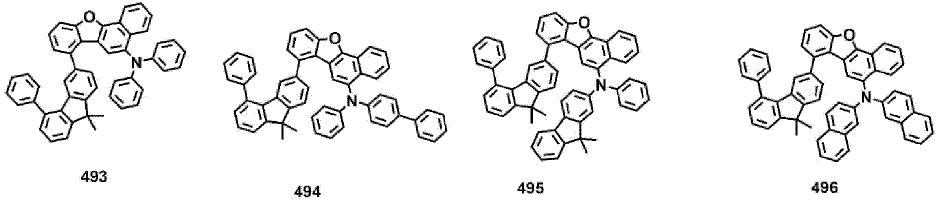

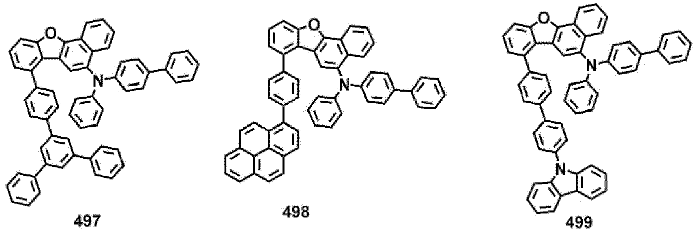

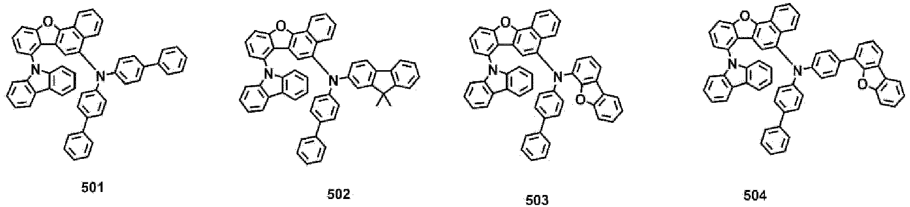

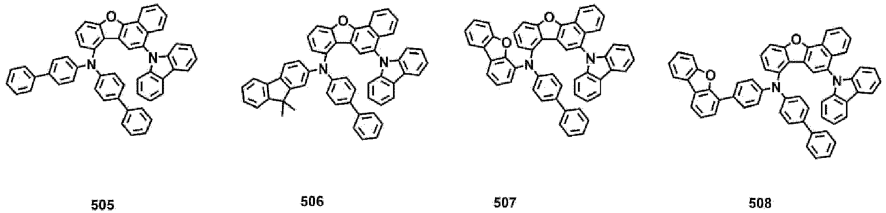

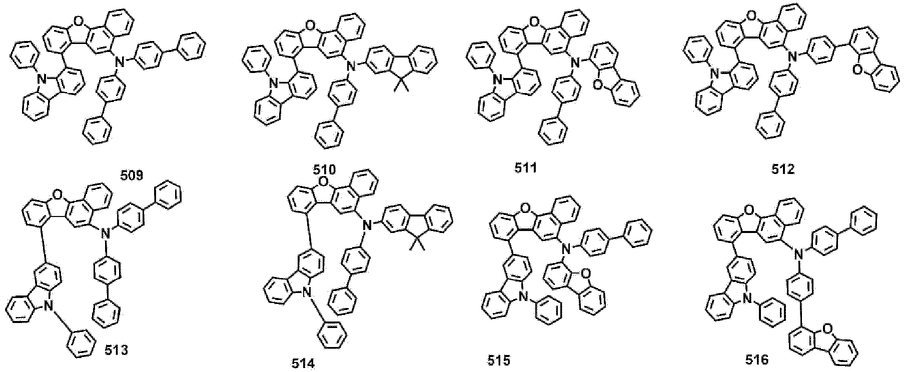

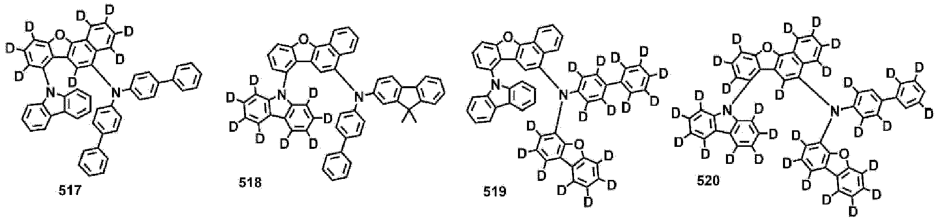

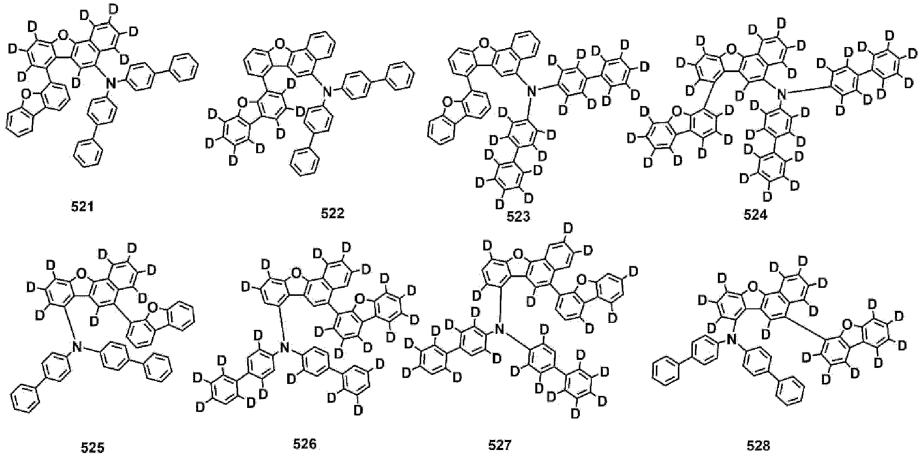

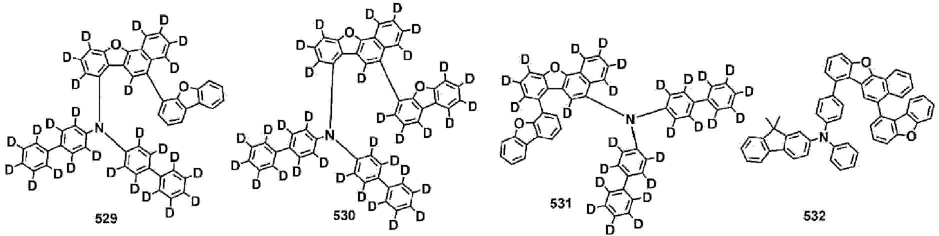

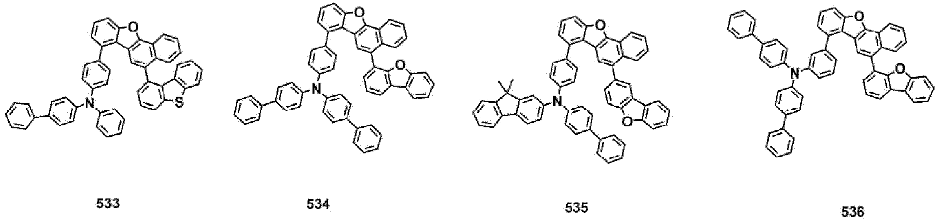

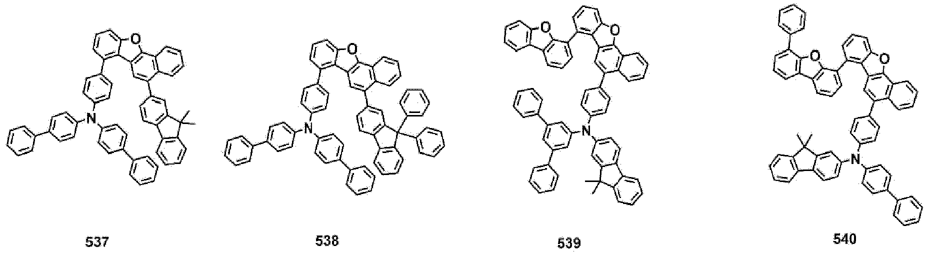

2. An organic light emitting device comprising a first electrode, a second electrode, and an organic material layer having one or more layers provided between the first electrode and the second electrode, wherein one or more layers of the organic material layer comprise the heterocyclic compound of claim 1.

3. The organic light emitting device of claim 2, wherein the organic material layer comprises a hole transport layer having one or more layers, and the hole transport layer comprises the heterocyclic compound.

4. The organic light emitting device of claim 2, wherein the organic material layer comprises a hole transport auxiliary layer having one or more layers, and the hole transport auxiliary layer comprises the heterocyclic compound.

5. The organic light emitting device of claim 2, further comprising one or two or more layers selected from the group consisting of a light emitting layer, a hole injection layer, a hole transport layer, an electron injection layer, an electron transport layer, a hole auxiliary layer, and a hole blocking layer.

## Patentansprüche

1. Heterocyclische Verbindung, dargestellt durch eine der folgenden Verbindungen:

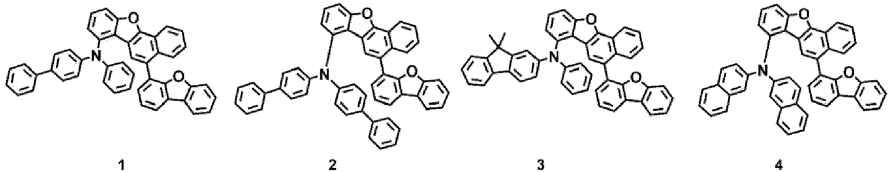

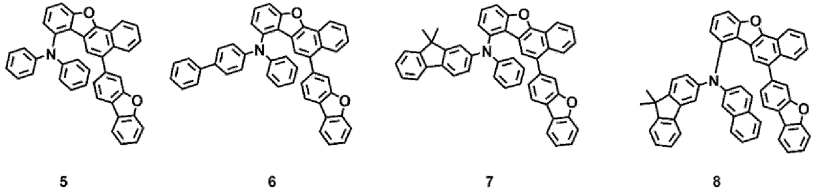

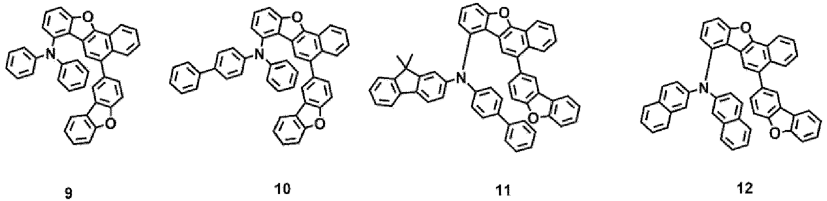

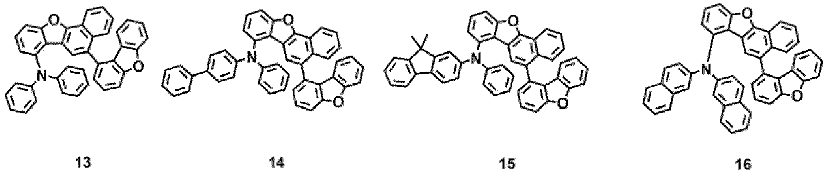

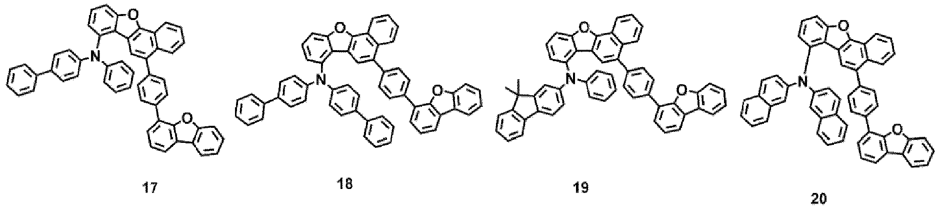

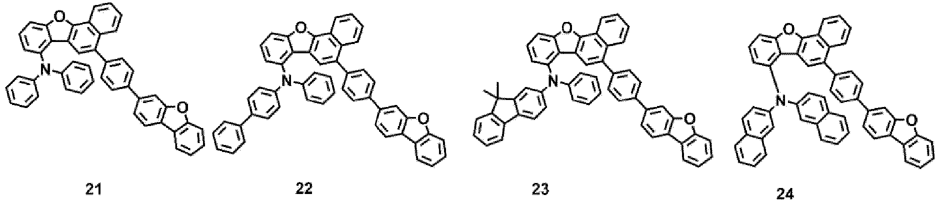

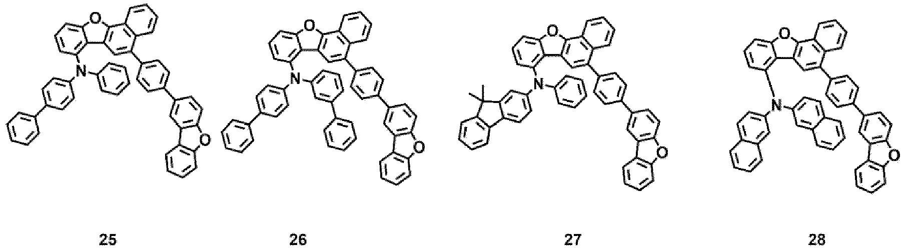

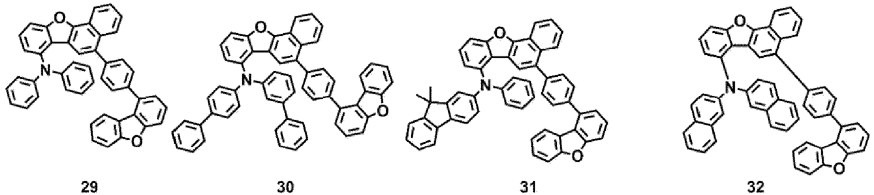

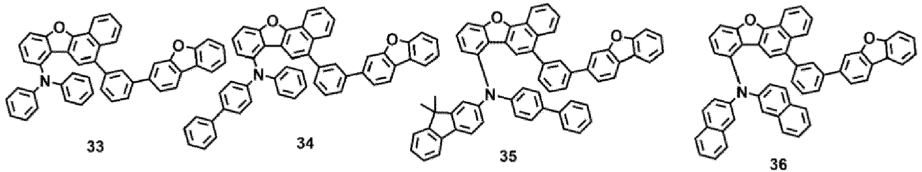

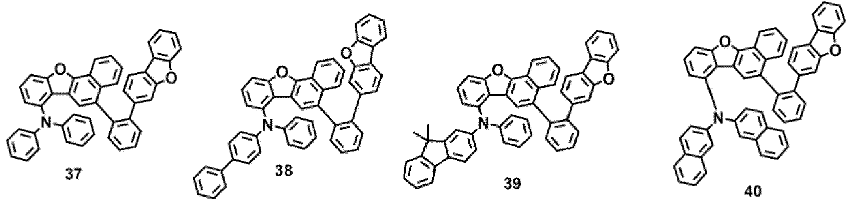

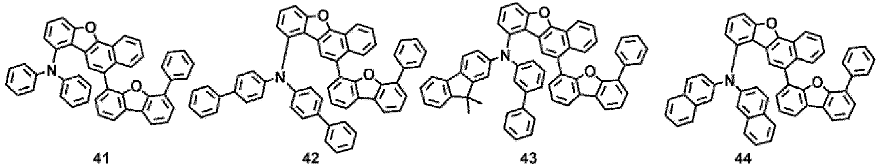

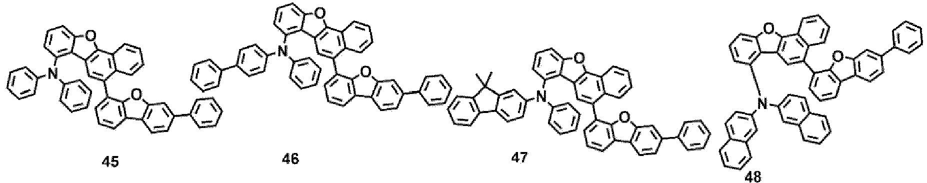

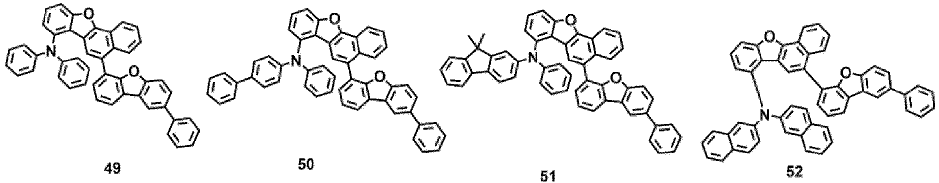

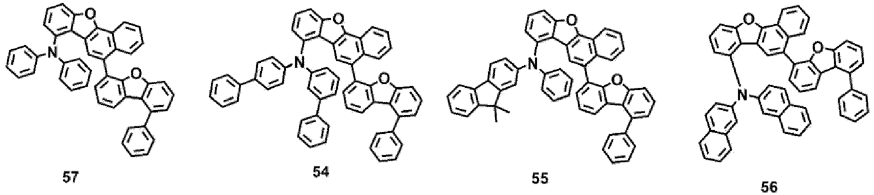

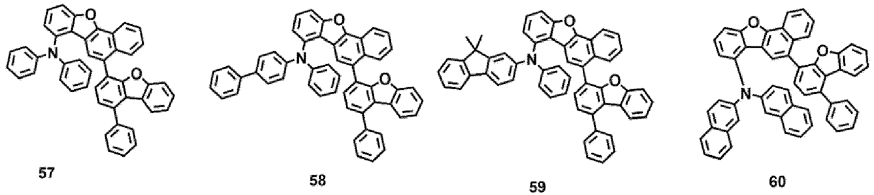

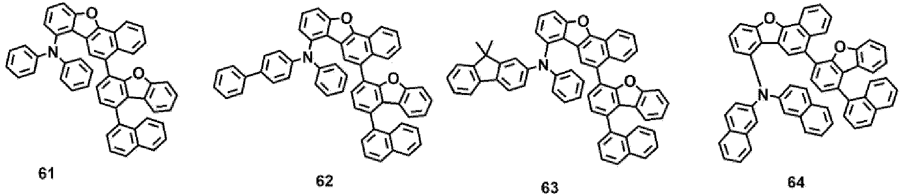

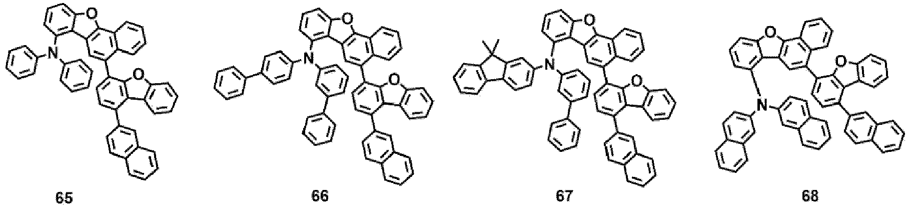

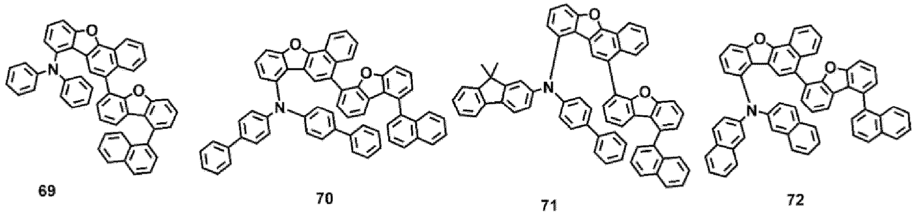

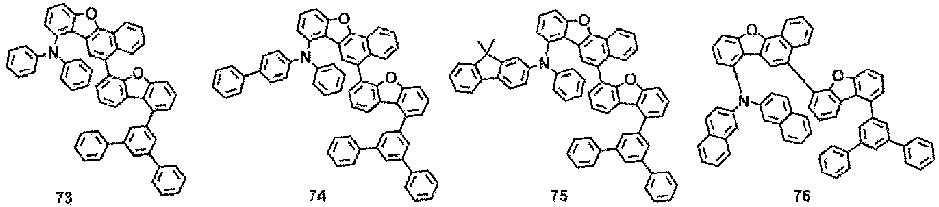

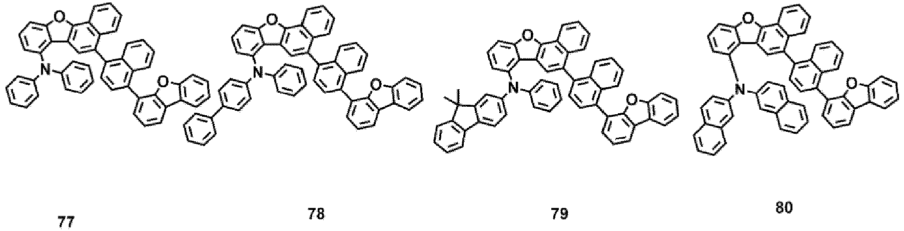

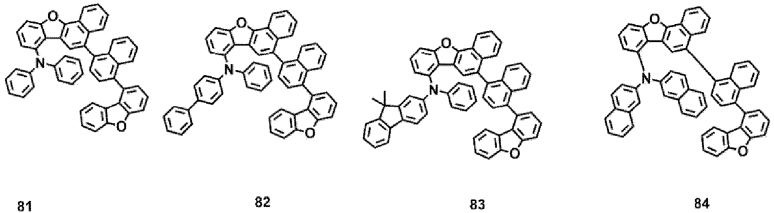

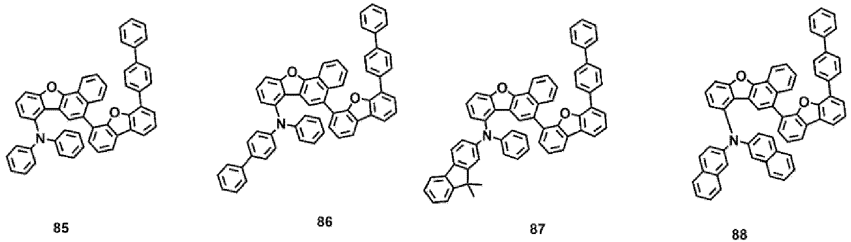

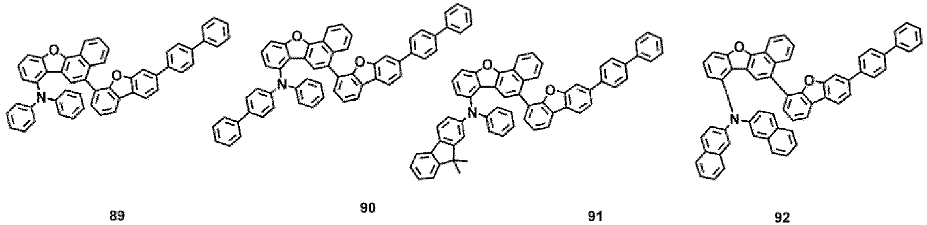

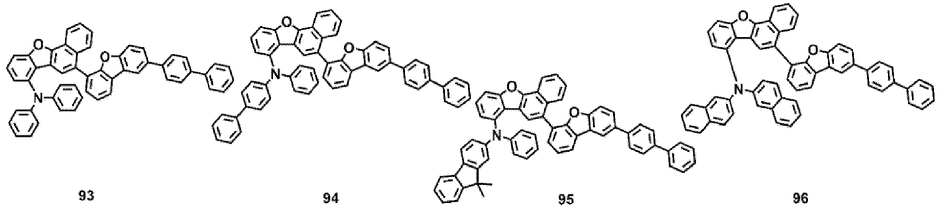

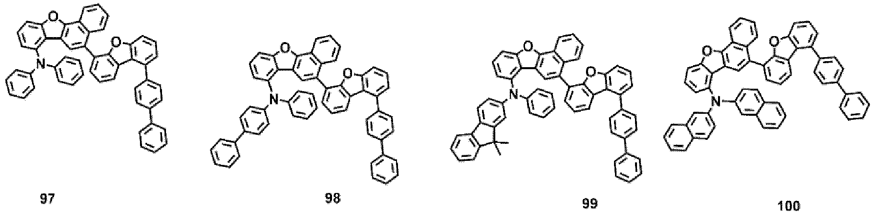

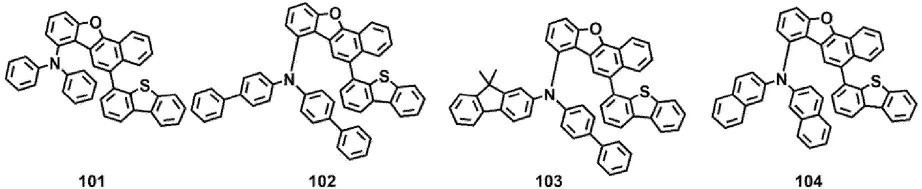

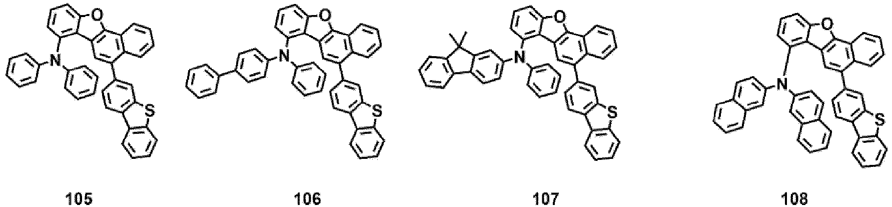

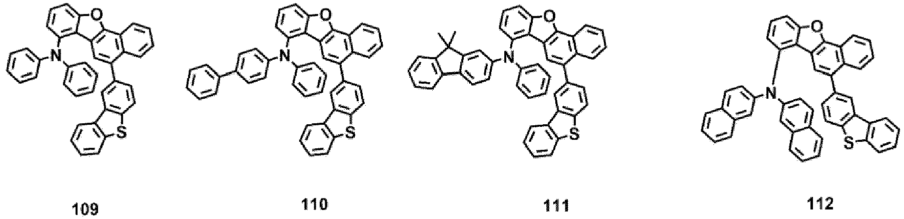

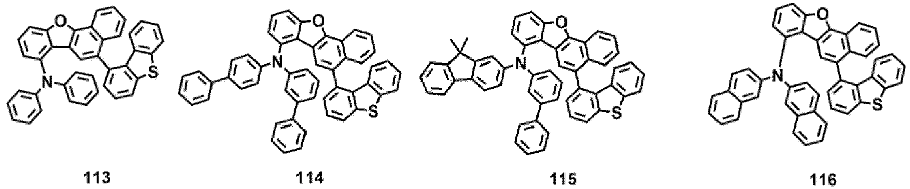

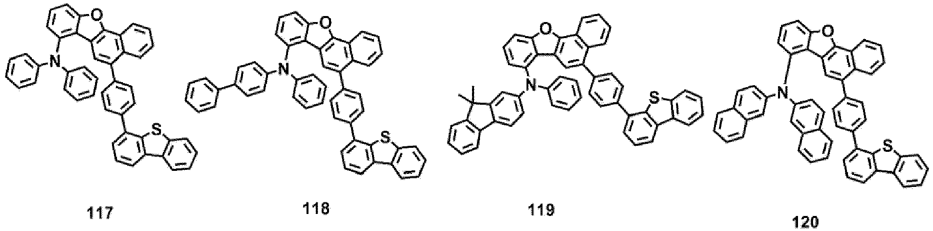

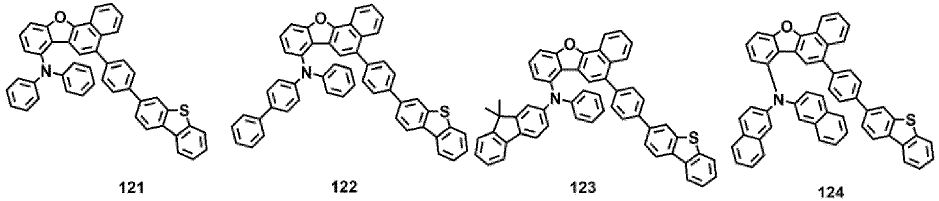

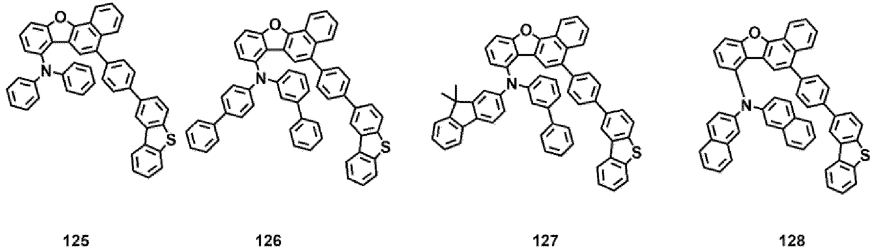

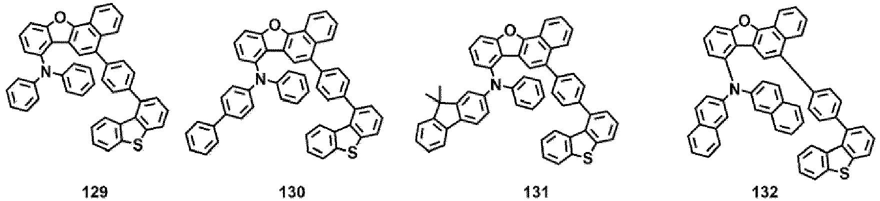

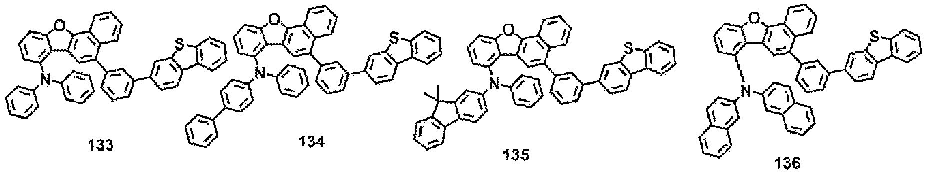

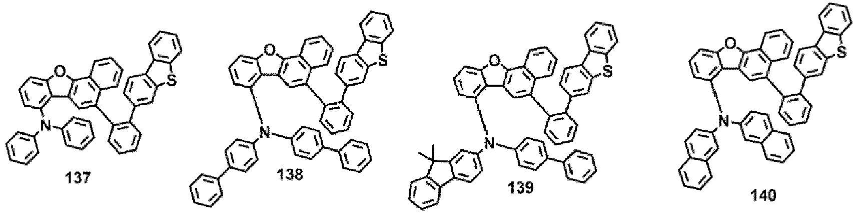

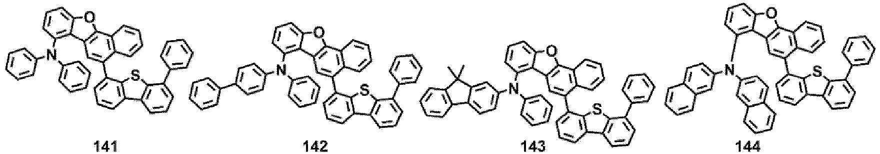

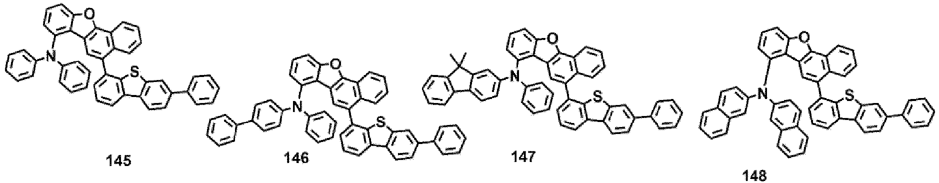

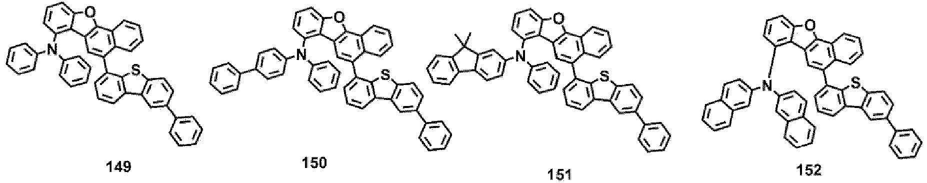

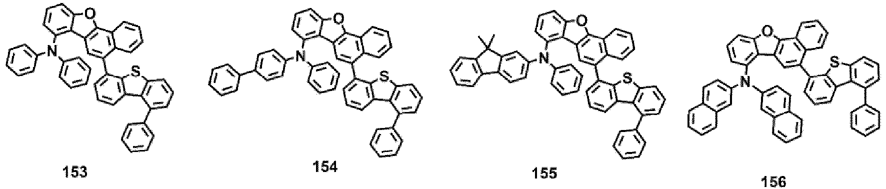

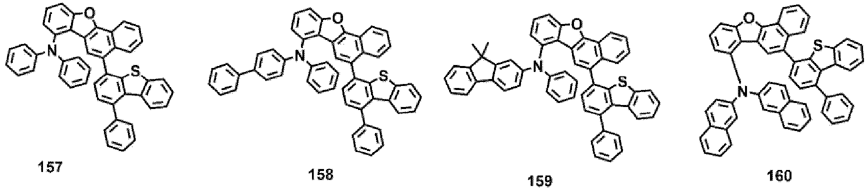

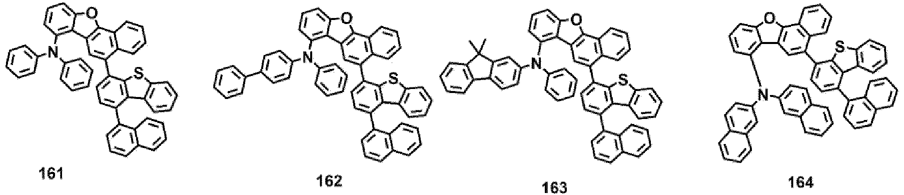

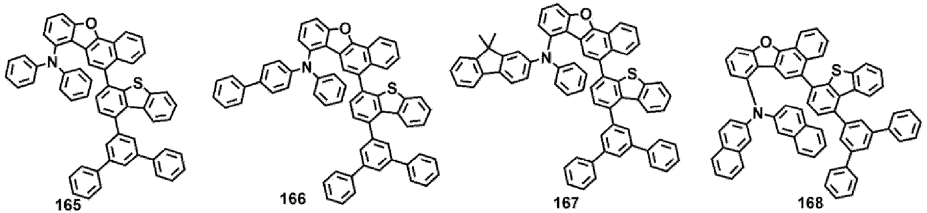

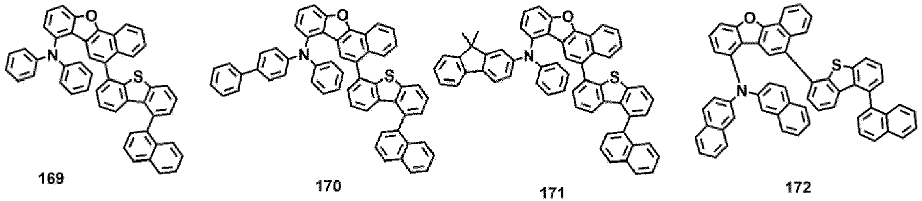

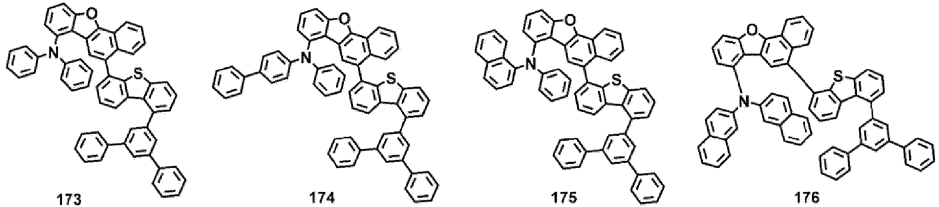

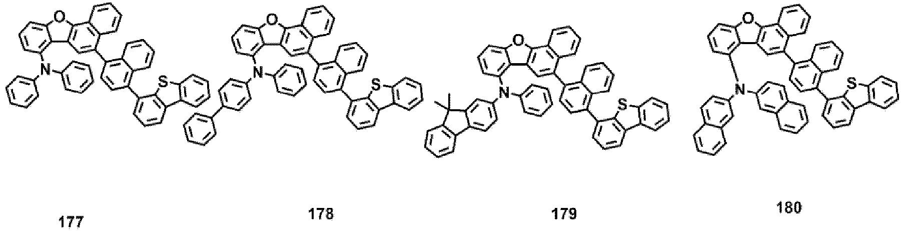

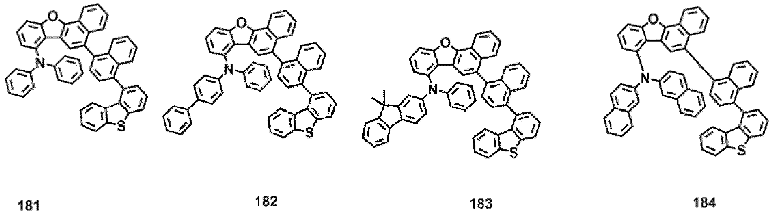

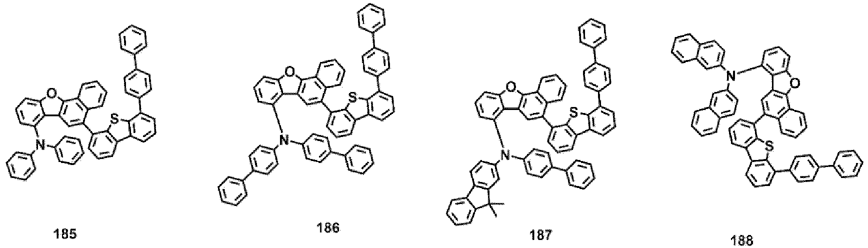

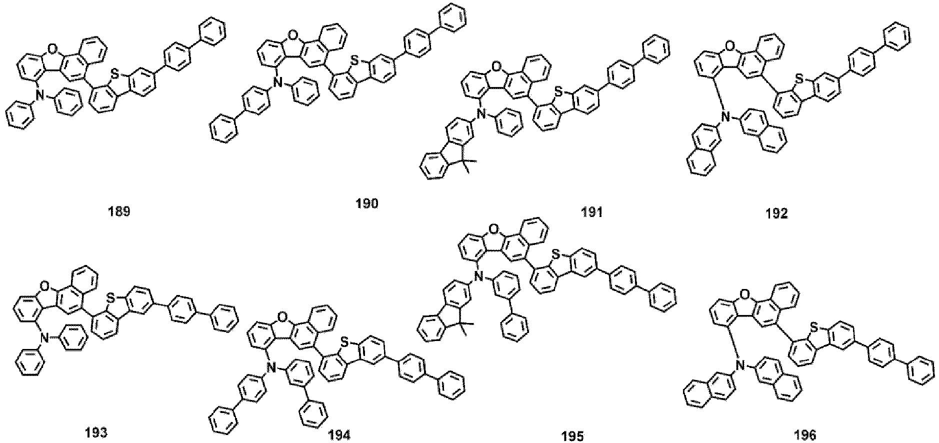

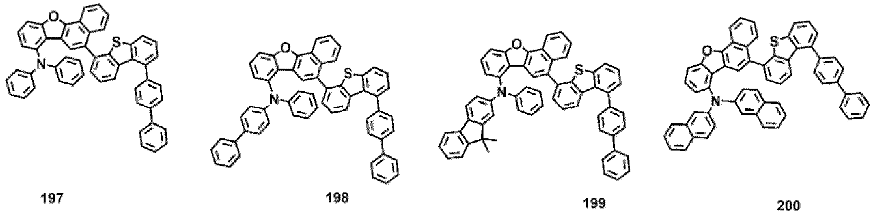

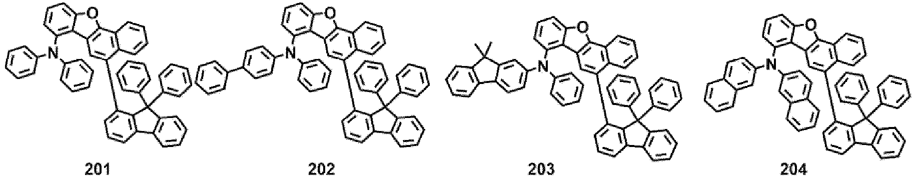

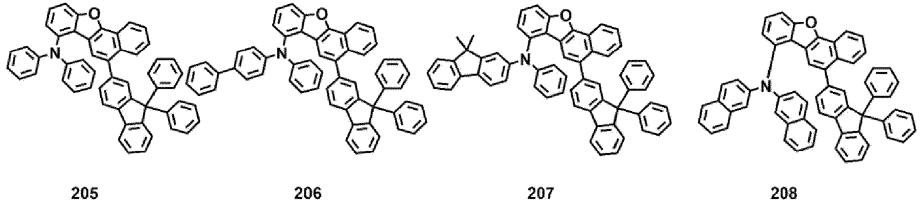

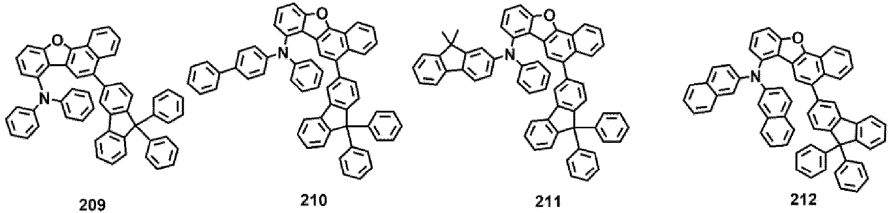

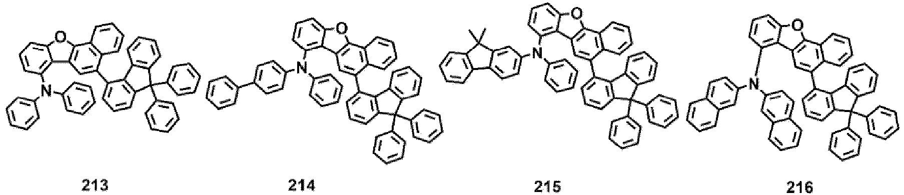

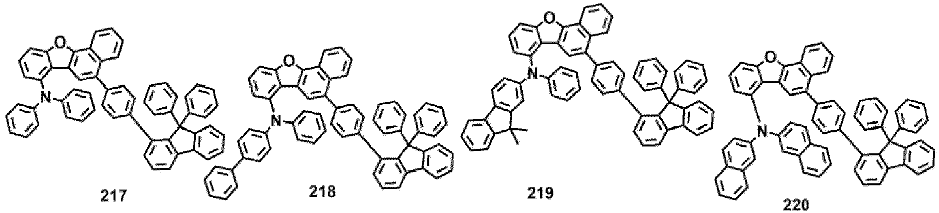

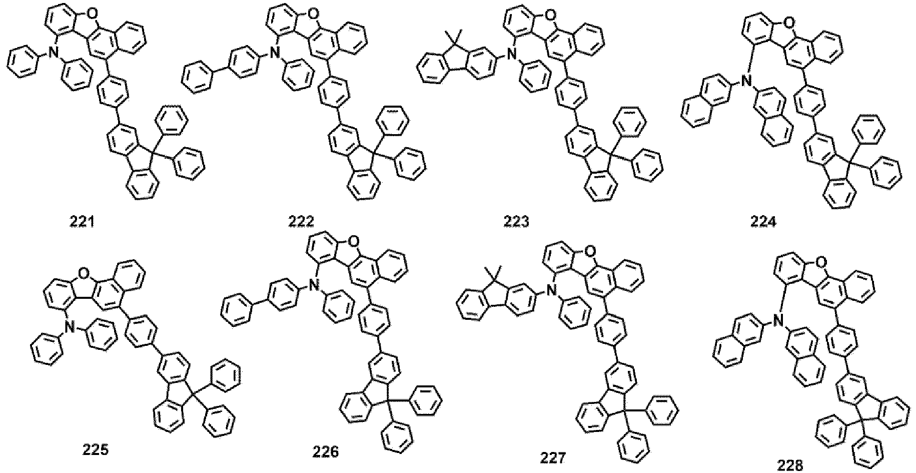

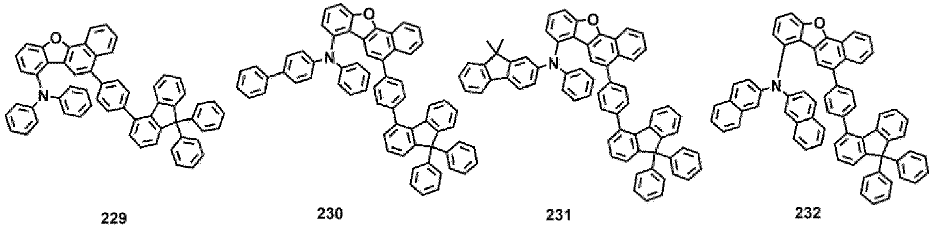

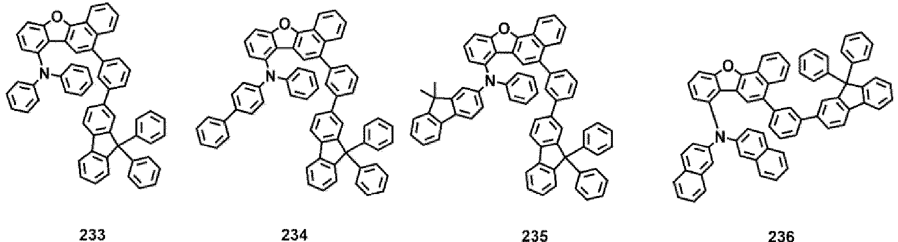

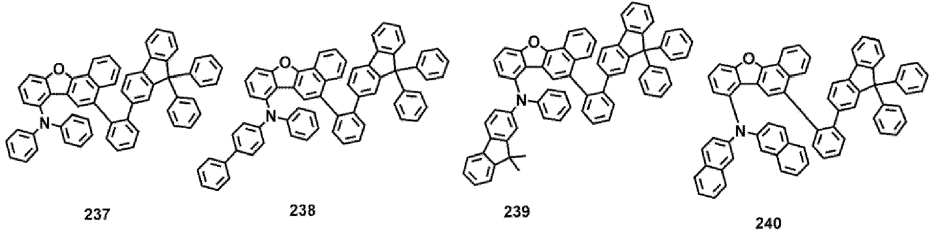

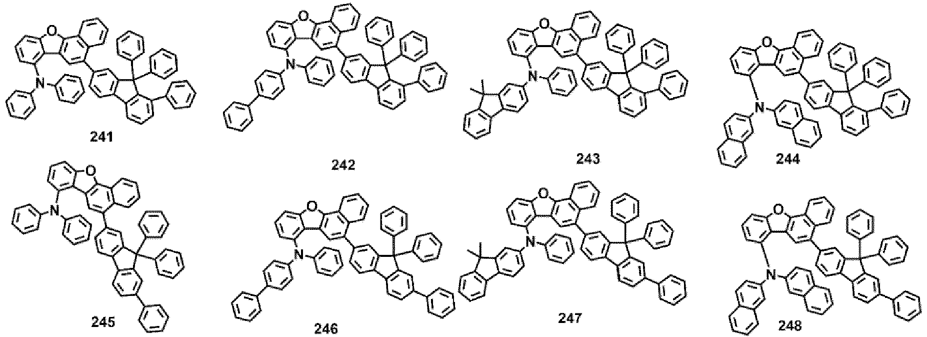

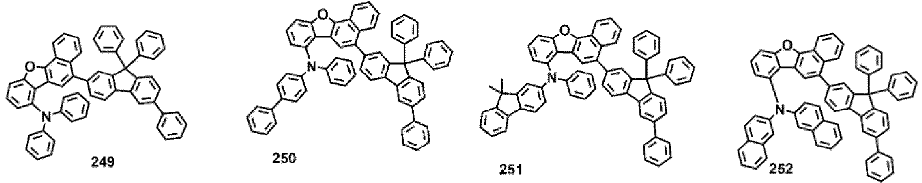

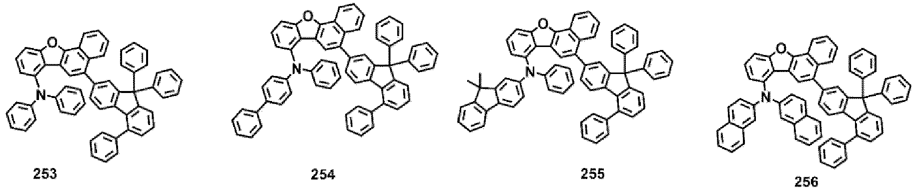

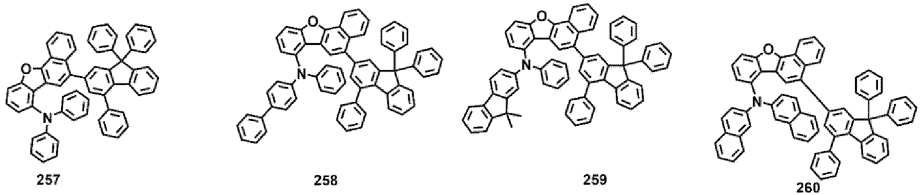

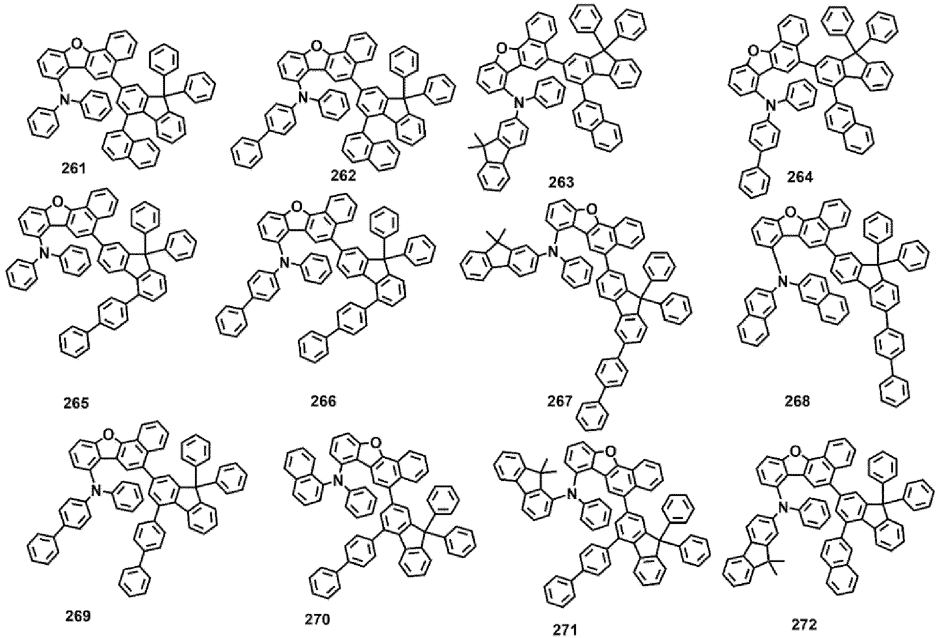

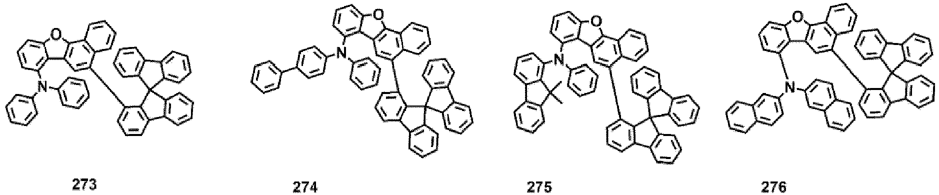

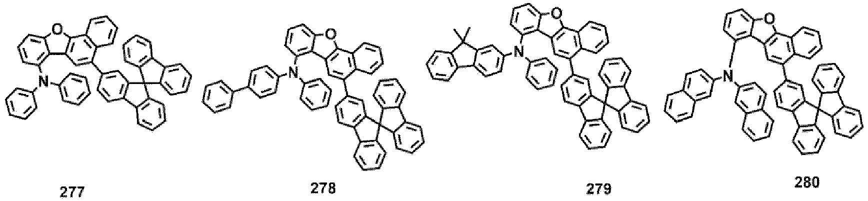

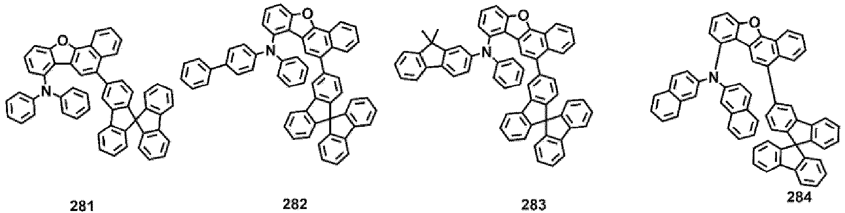

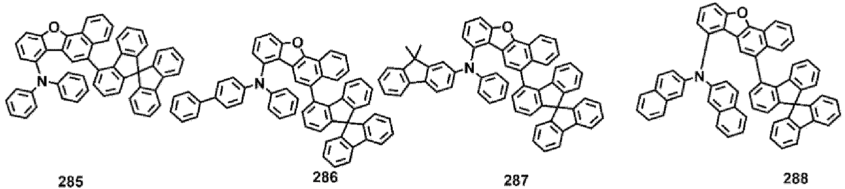

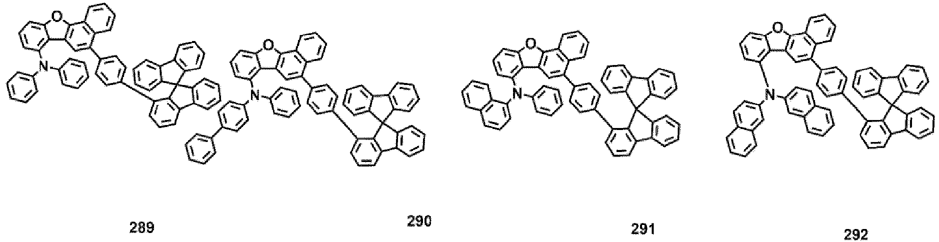

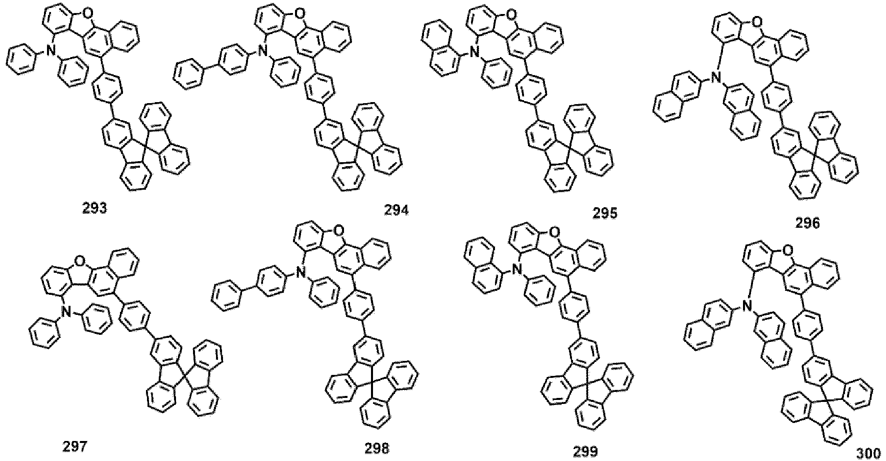

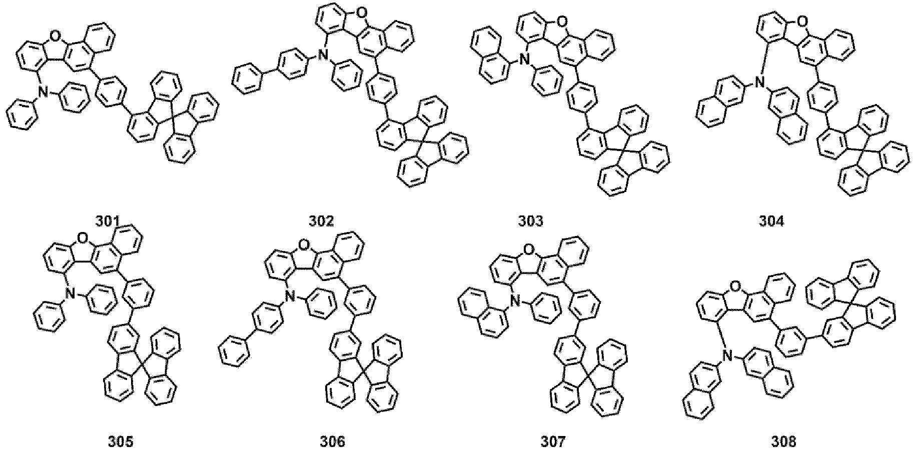

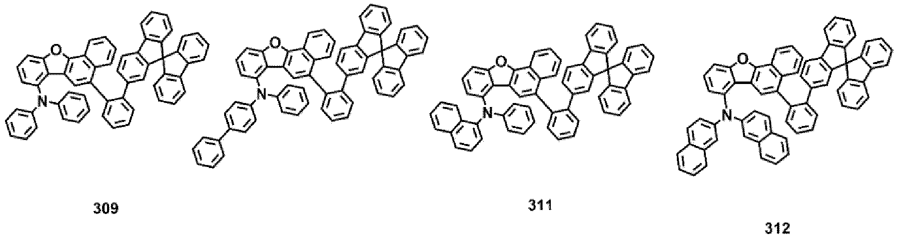

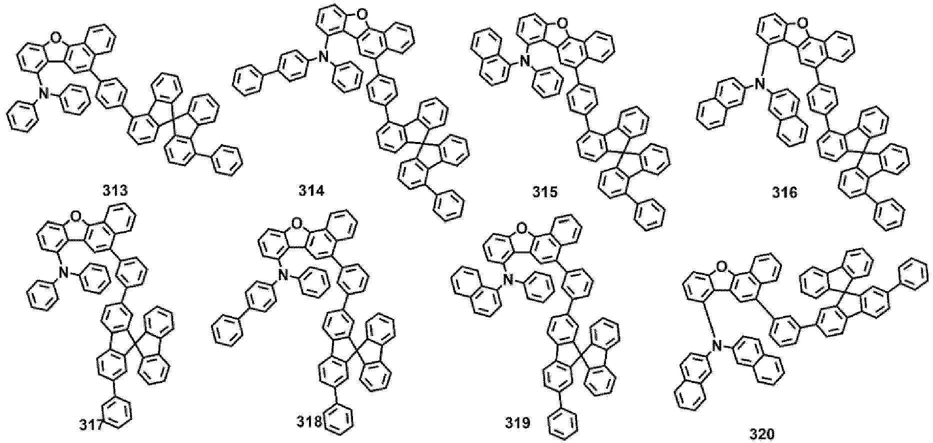

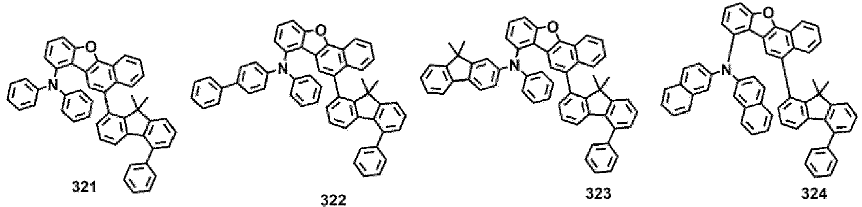

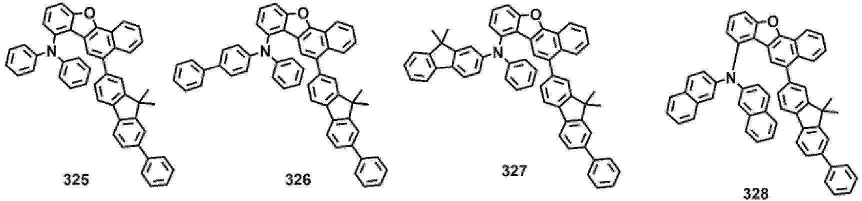

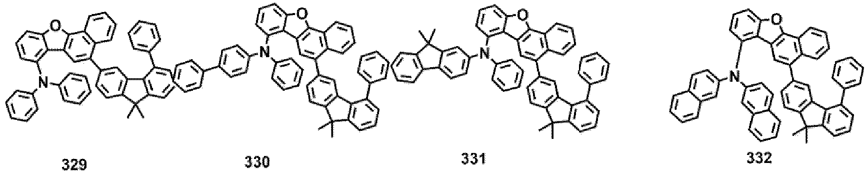

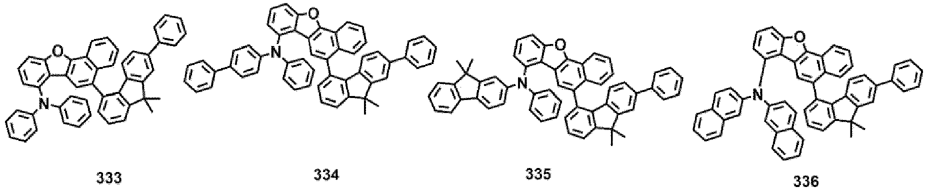

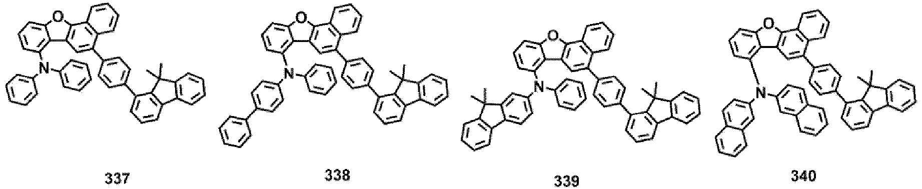

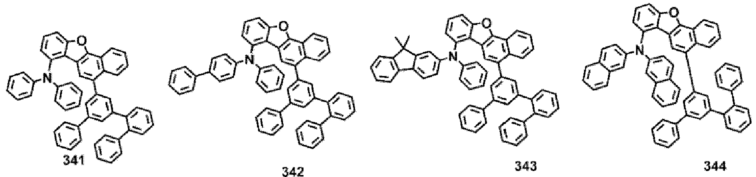

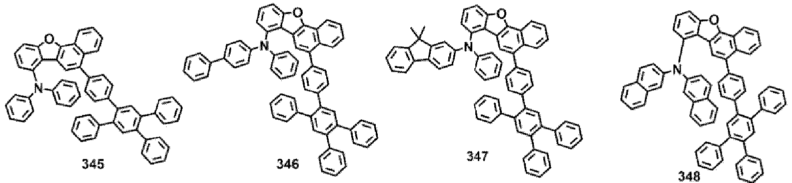

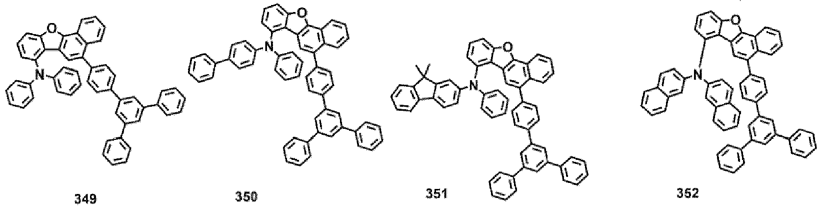

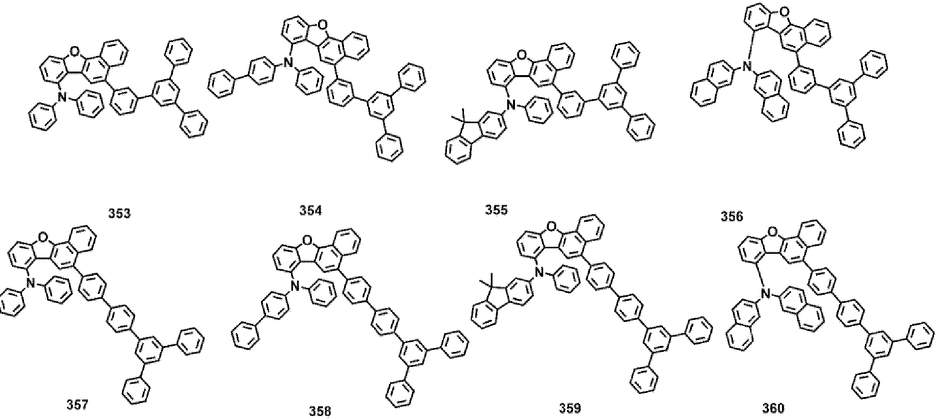

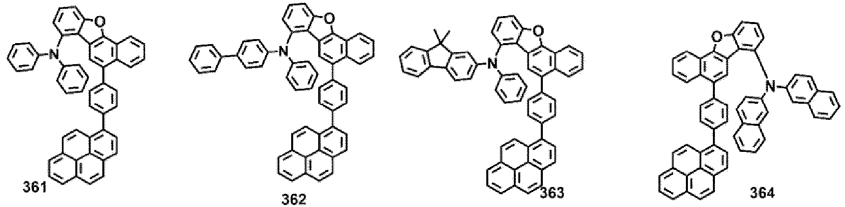

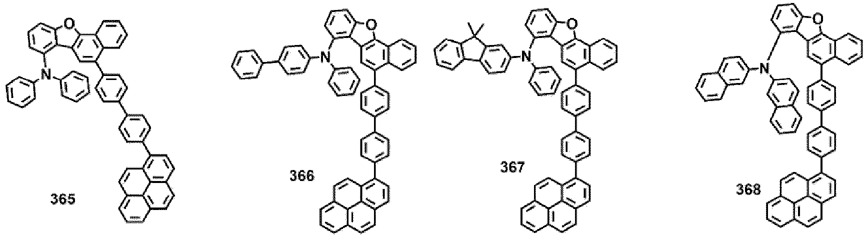

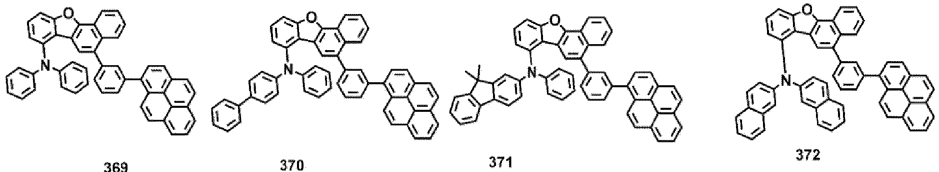

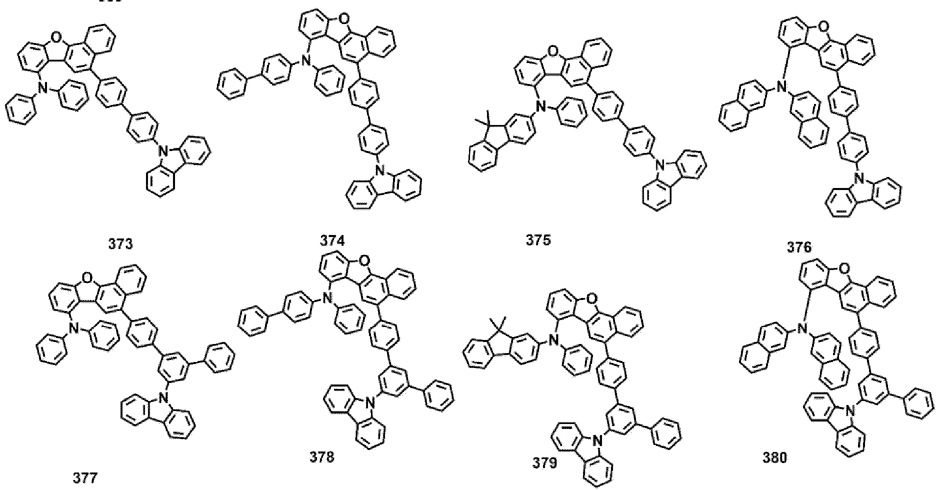

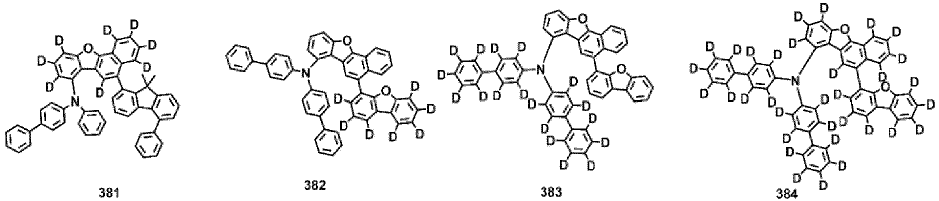

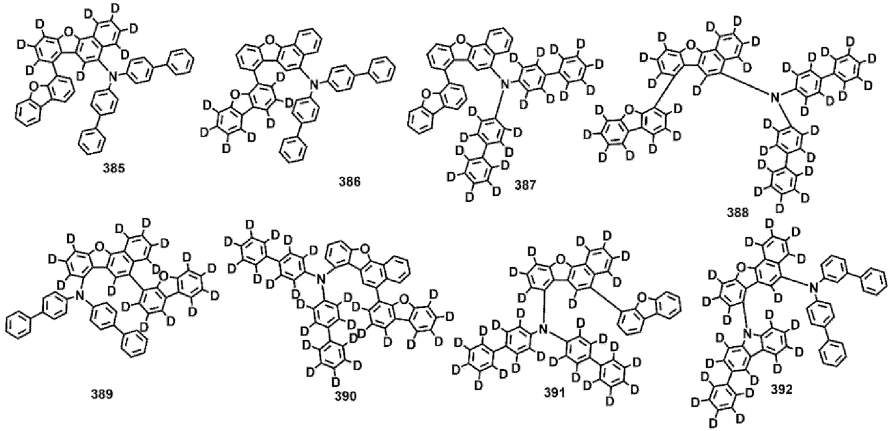

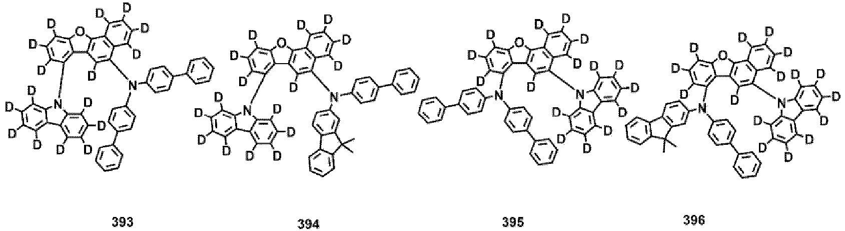

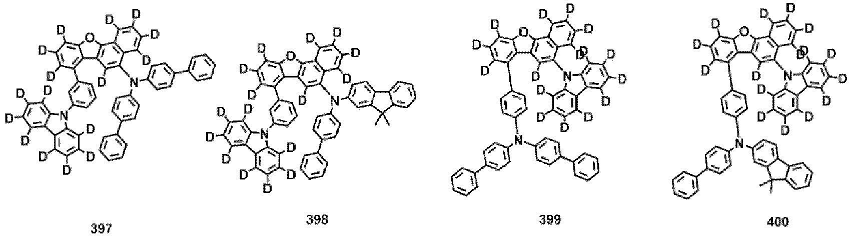

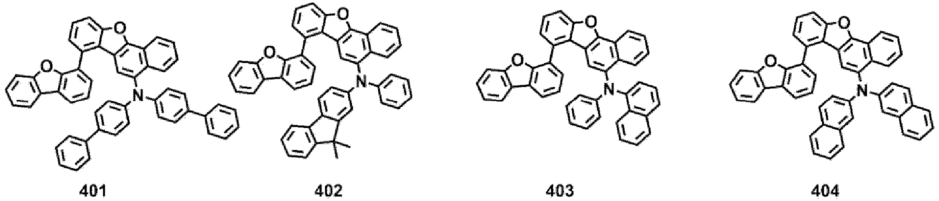

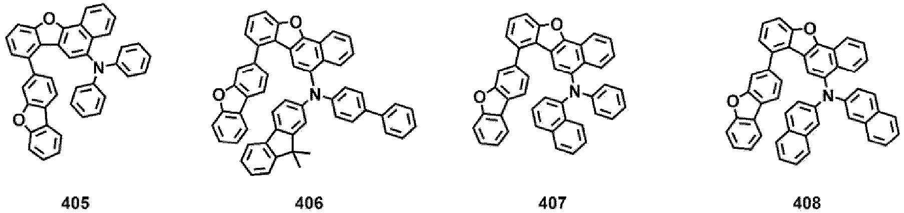

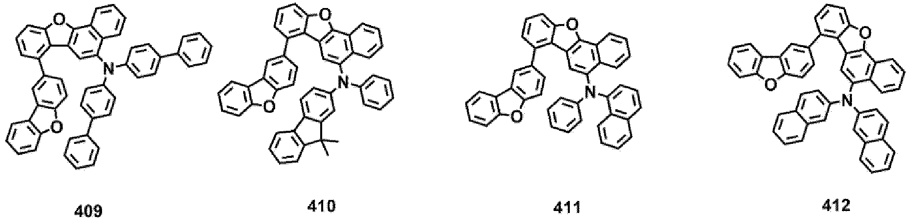

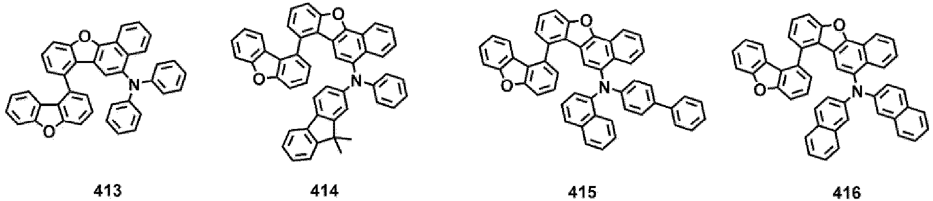

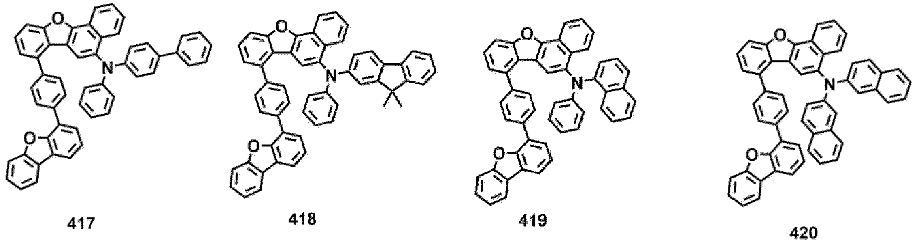

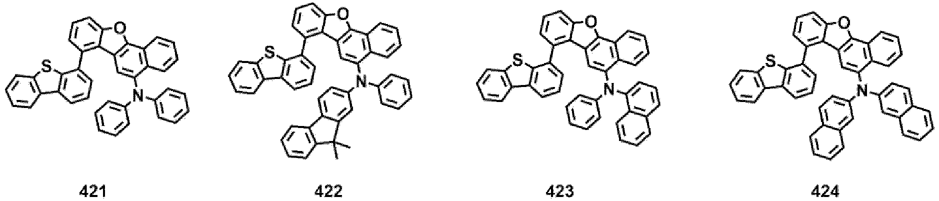

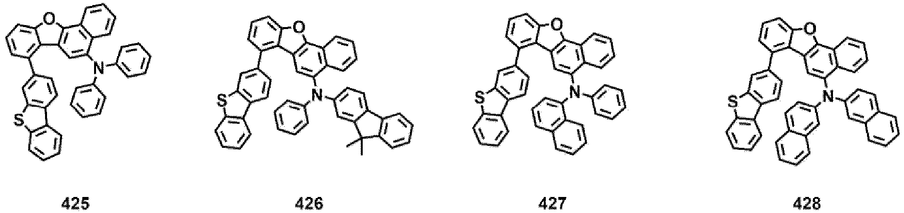

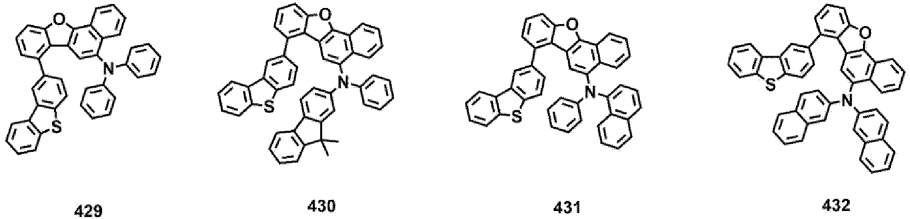

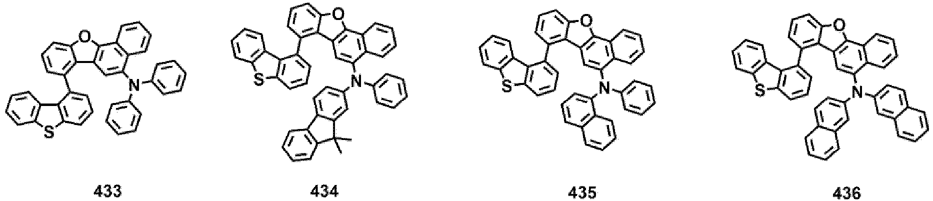

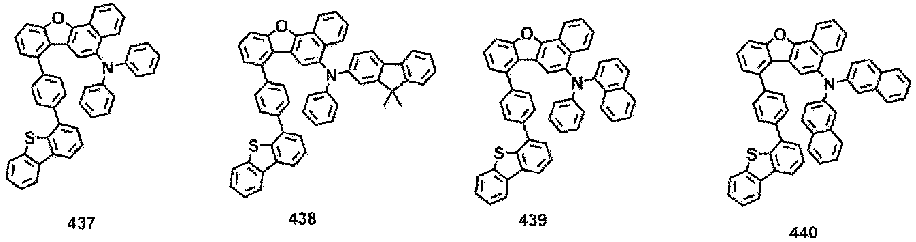

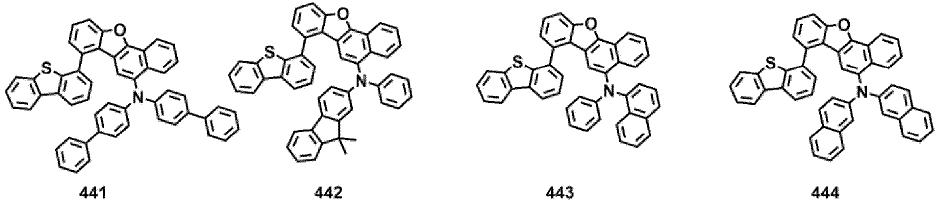

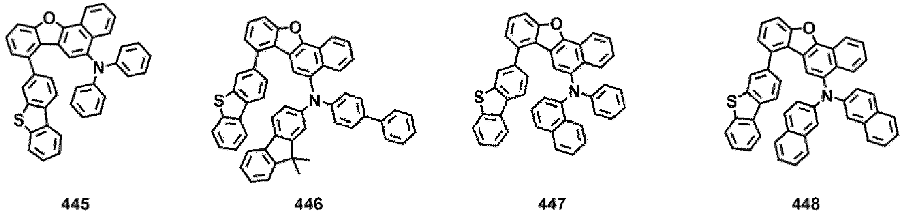

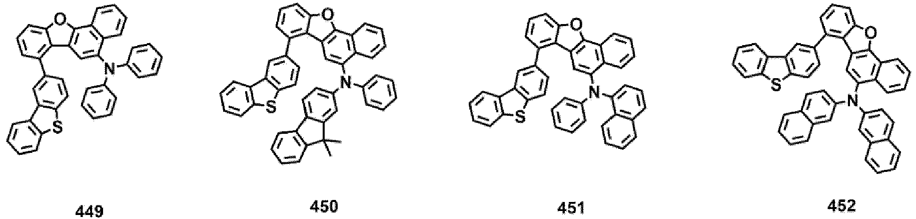

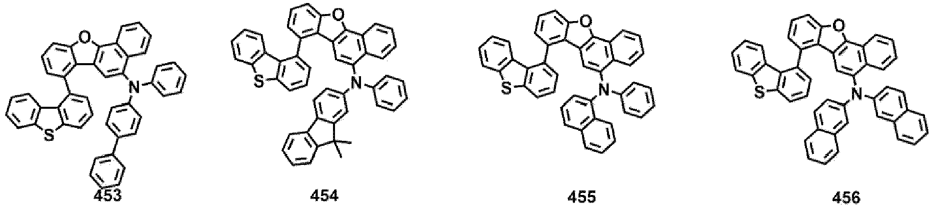

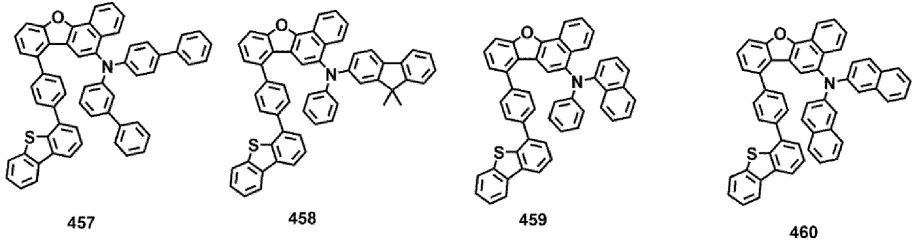

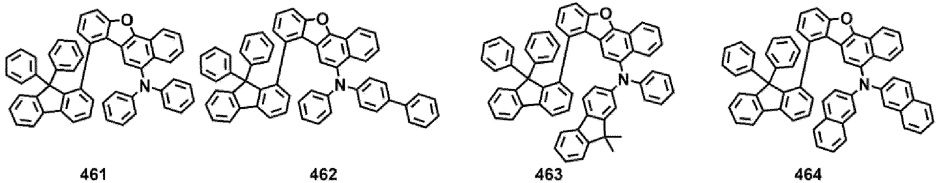

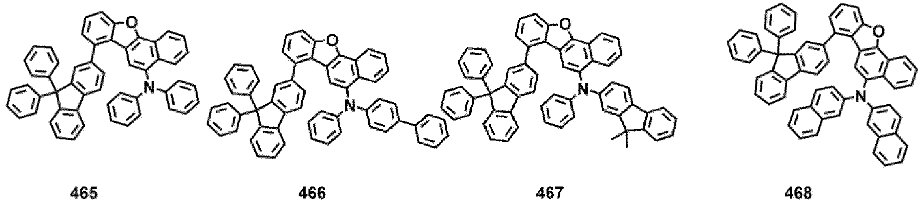

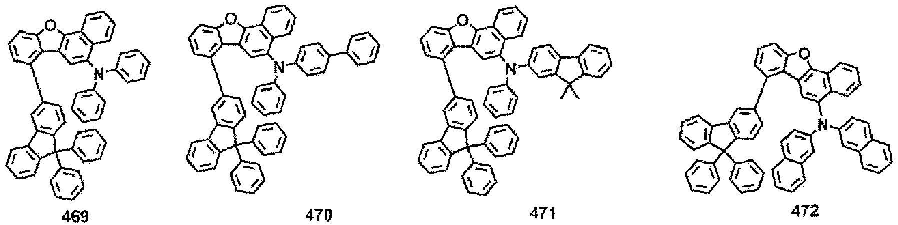

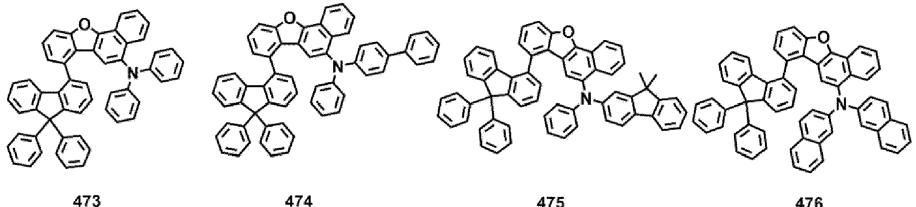

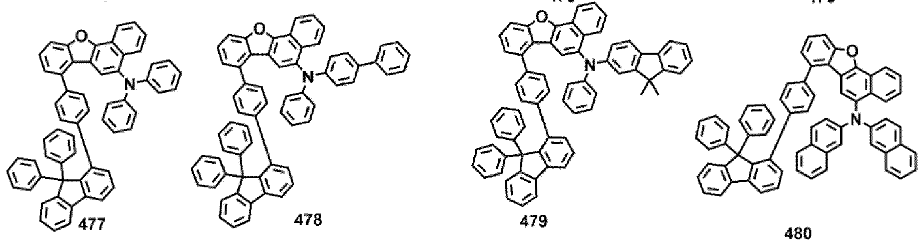

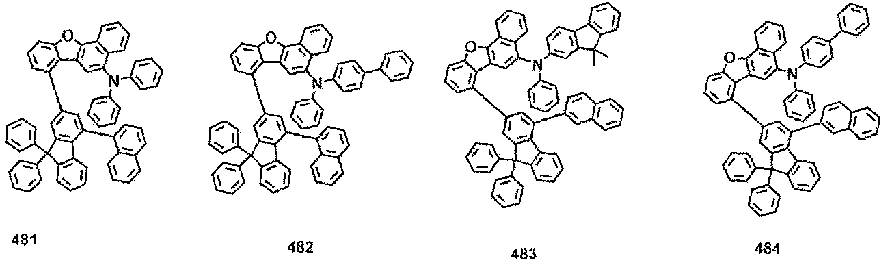

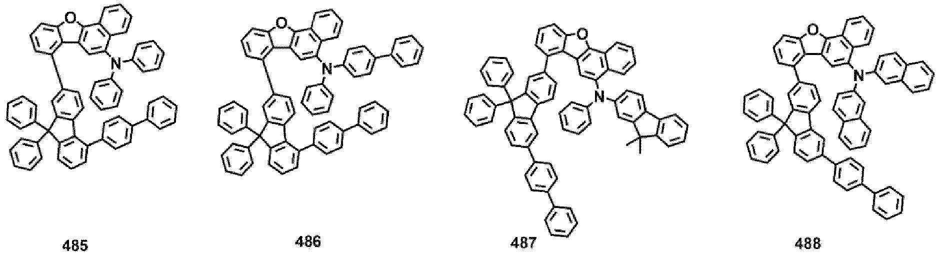

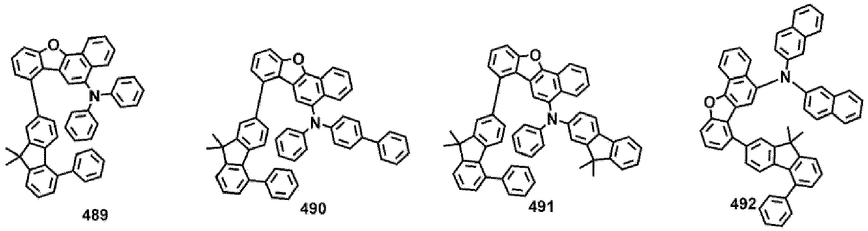

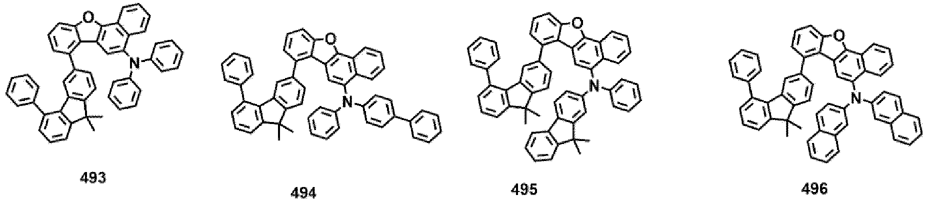

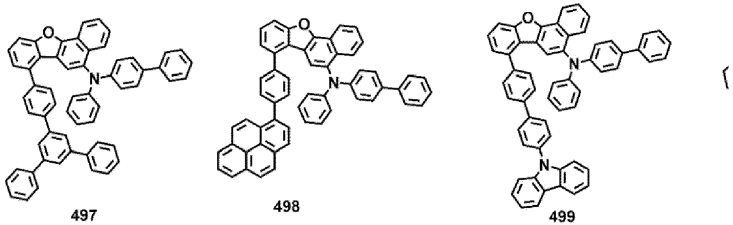

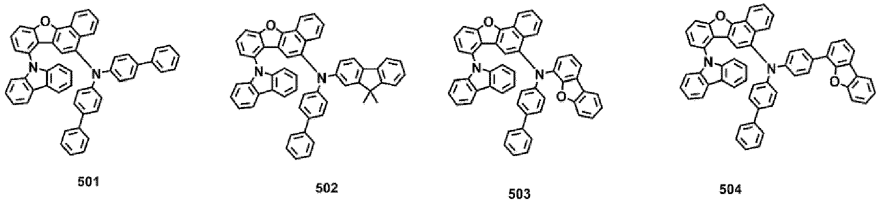

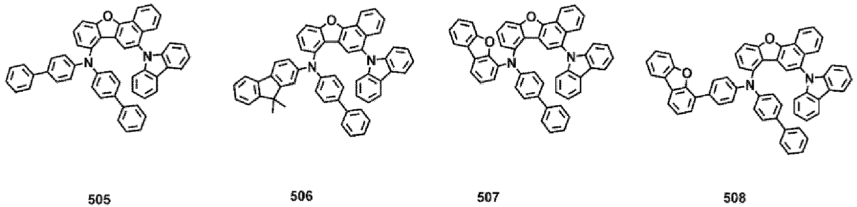

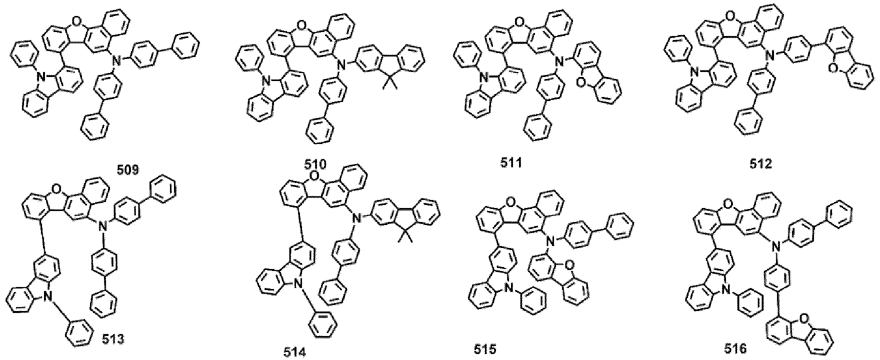

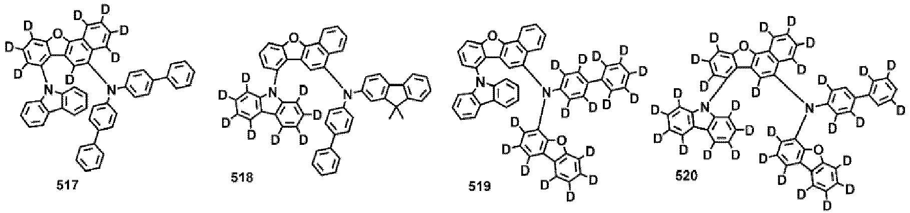

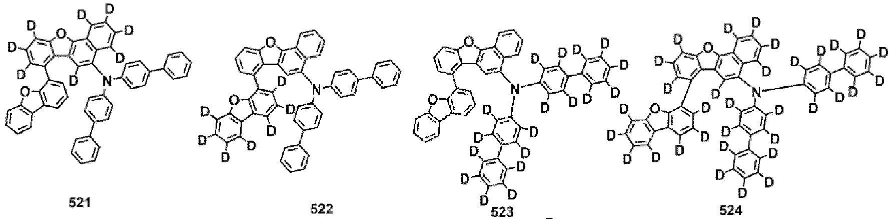

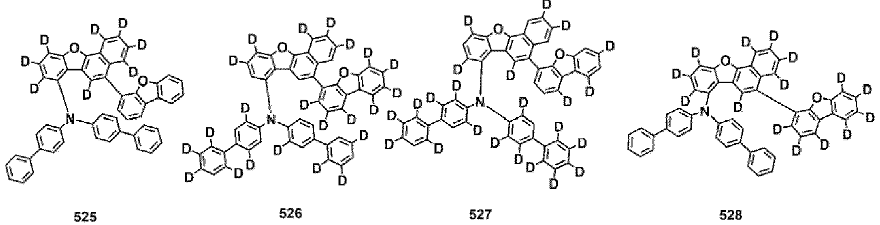

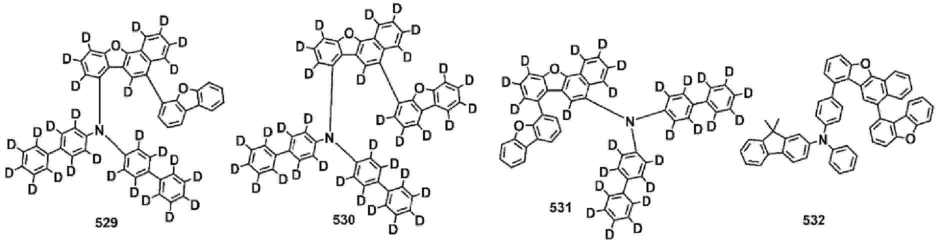

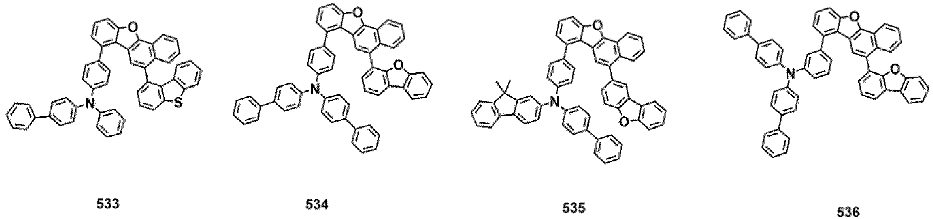

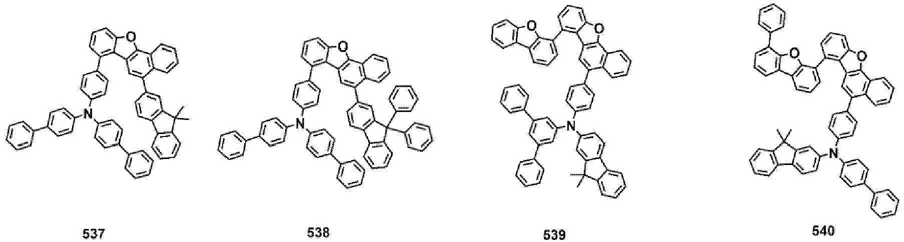

2. Organische lichtemittierende Vorrichtung, umfassend eine erste Elektrode, eine zweite Elektrode und eine organische Materialschicht mit einer oder mehreren Schichten, die zwischen der ersten Elektrode und der zweiten Elektrode bereitgestellt sind, wobei eine oder mehrere Schichten der organischen Materialschicht die heterocyclische Verbindung nach Anspruch 1 umfassen.

3. Organische lichtemittierende Vorrichtung nach Anspruch 2, wobei die organische Materialschicht eine Lochtransportschicht mit einer oder mehreren Schichten umfasst und die Lochtransportschicht die heterocyclische Verbindung umfasst.

4. Organische lichtemittierende Vorrichtung nach Anspruch 2, wobei die organische Materialschicht eine Lochtransporthilfsschicht mit einer oder mehreren Schichten umfasst und die Lochtransporthilfsschicht die heterocyclische Verbindung umfasst.

5. Organische lichtemittierende Vorrichtung nach Anspruch 2, ferner umfassend eine oder zwei oder mehrere Schichten, ausgewählt aus der Gruppe bestehend aus einer lichtemittierenden Schicht, einer Lochinjektionsschicht, einer Lochtransportschicht, einer Elektroneninjektionsschicht, einer Elektronentransportschicht, einer Lochhilfsschicht und einer Lochblockierschicht.

## Revendications

1. Composé hétérocyclique représenté par l'un des composés suivants :

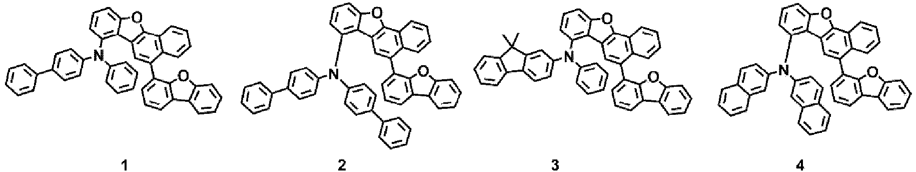

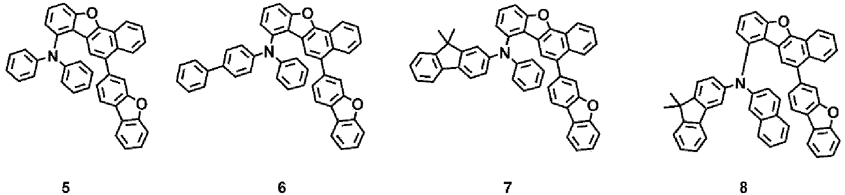

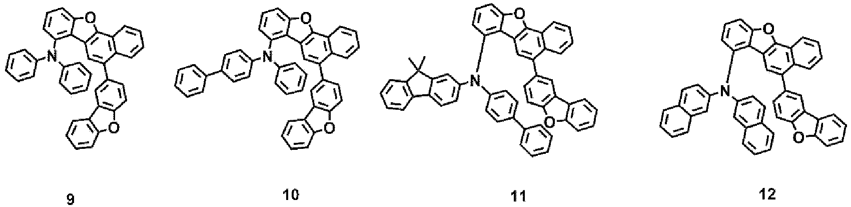

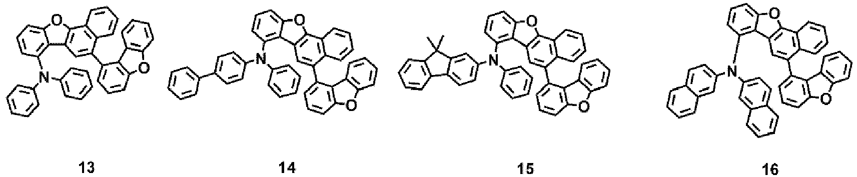

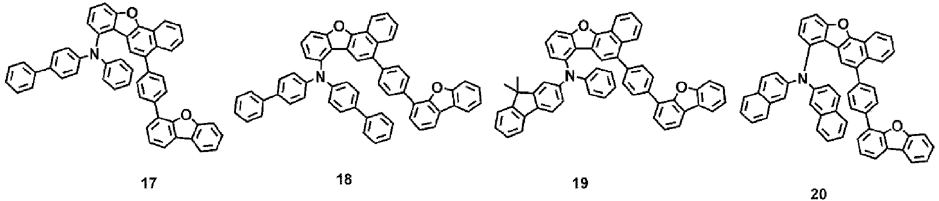

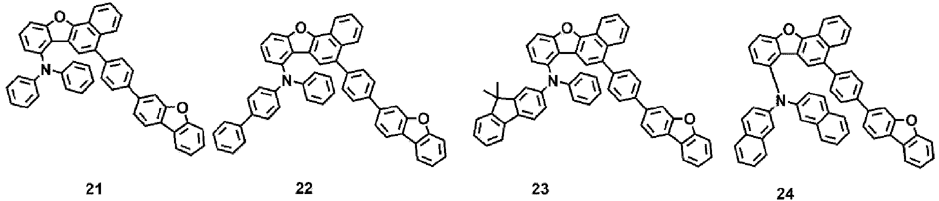

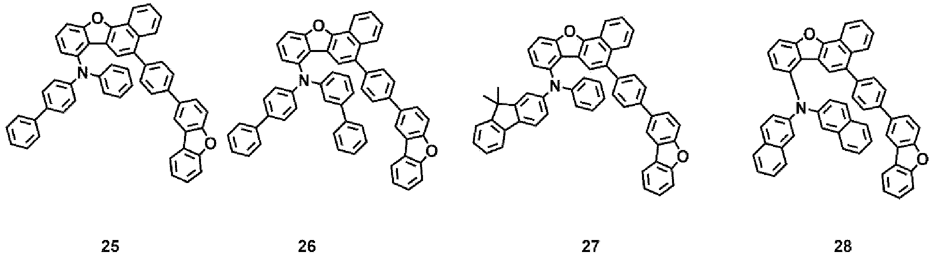

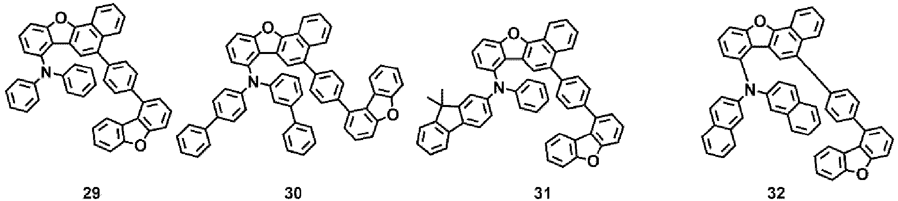

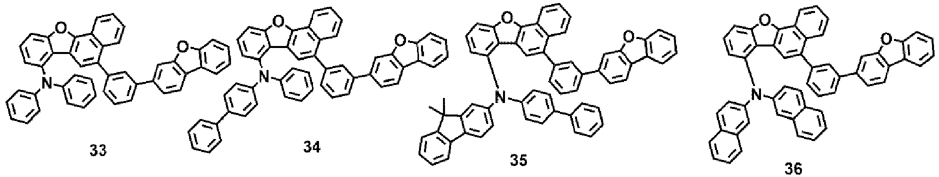

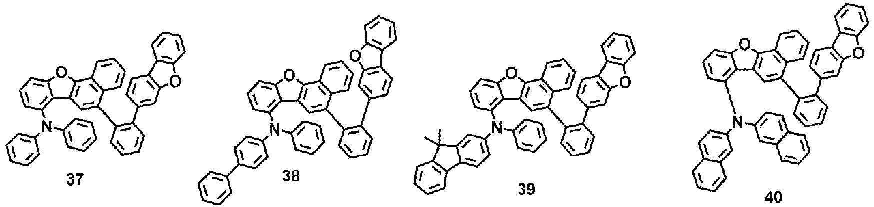

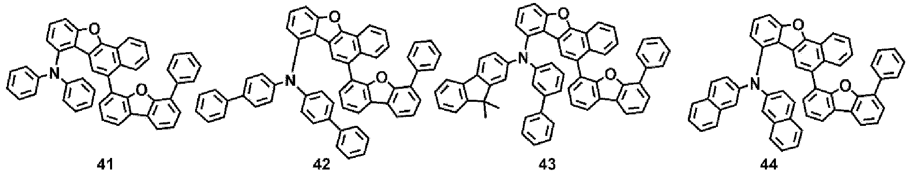

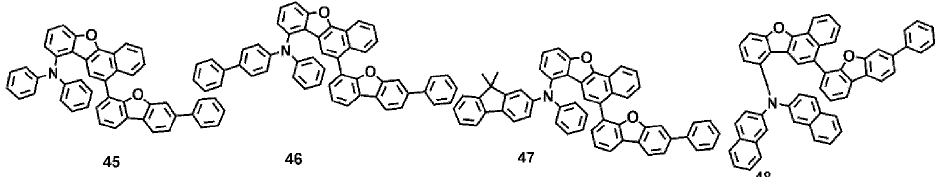

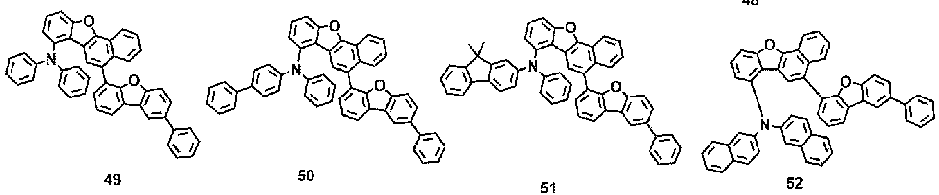

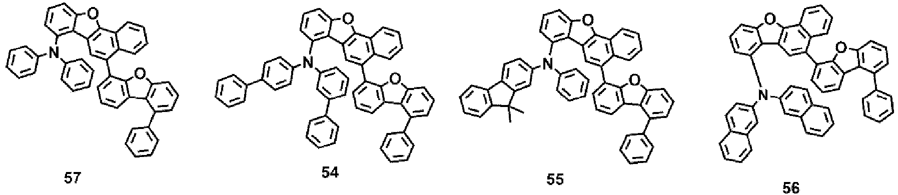

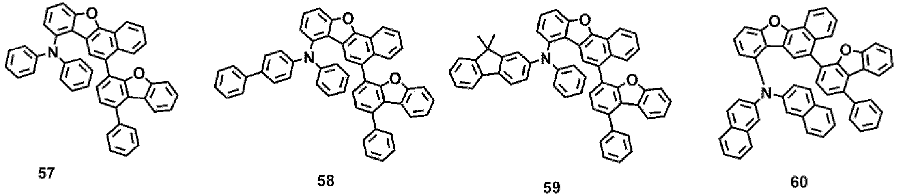

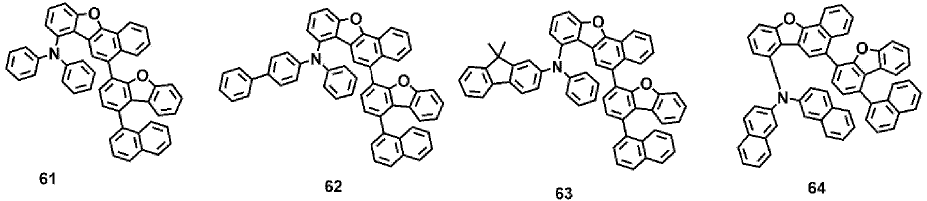

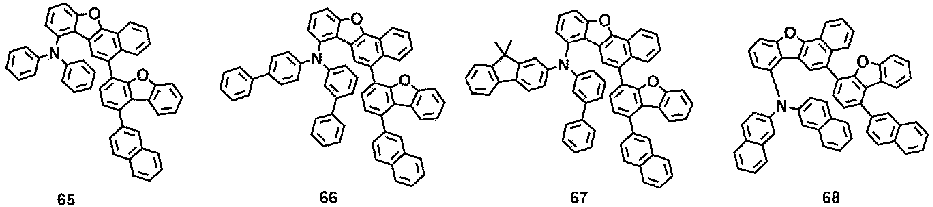

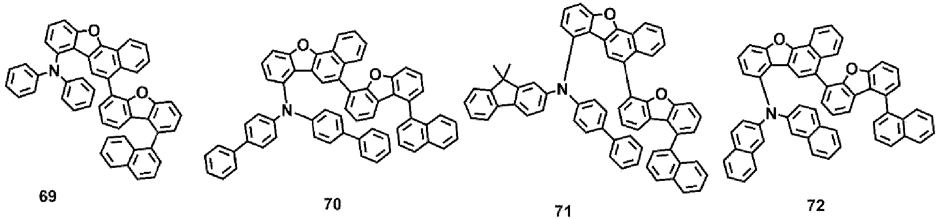

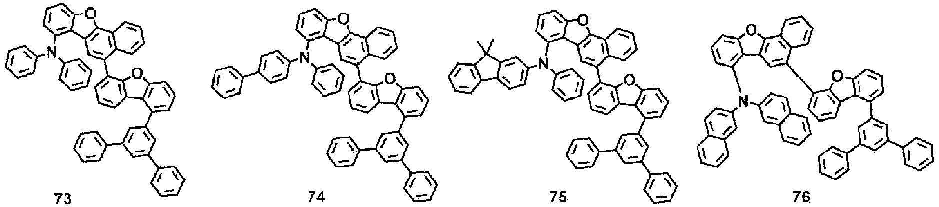

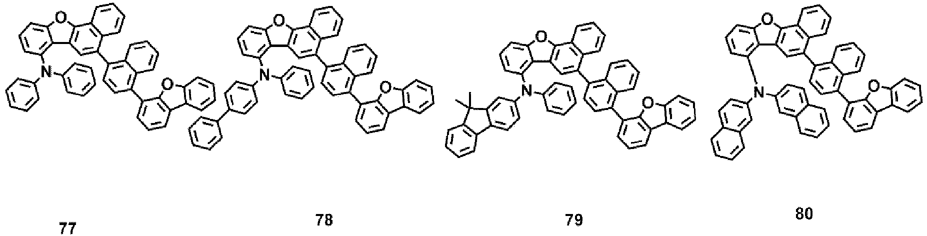

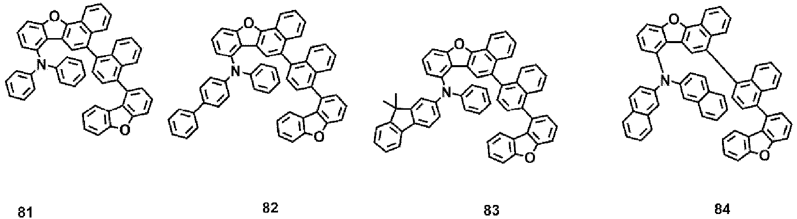

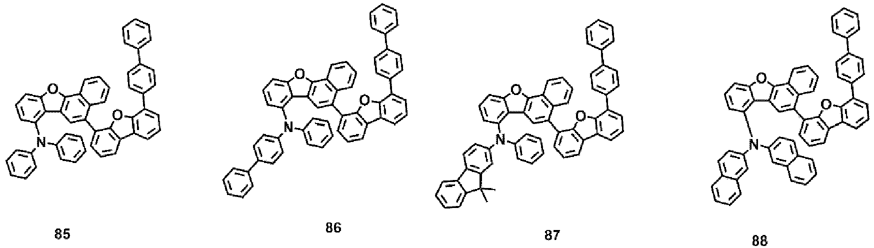

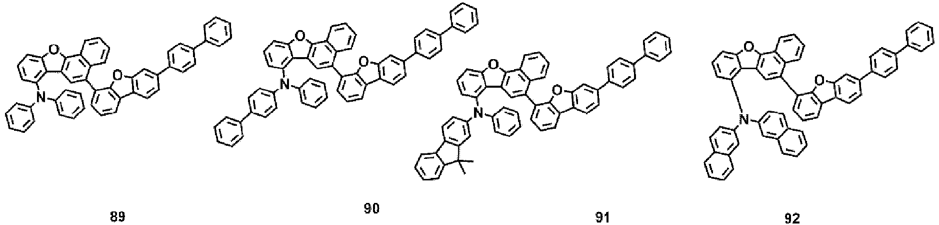

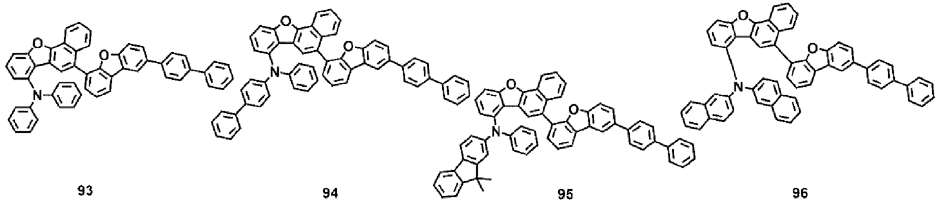

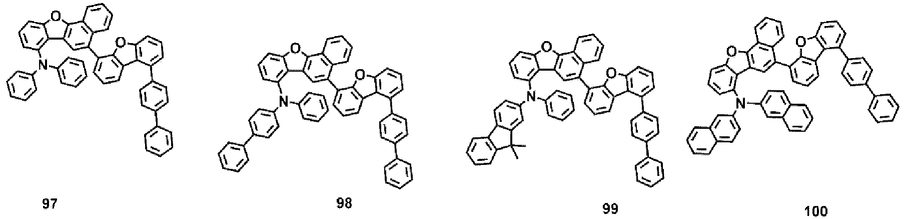

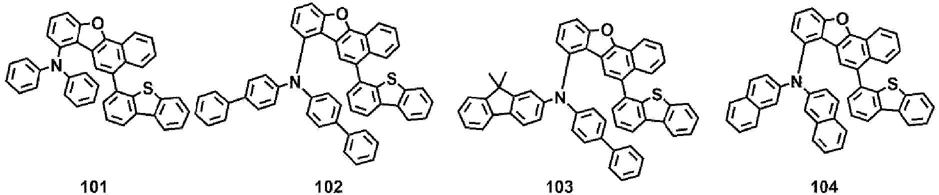

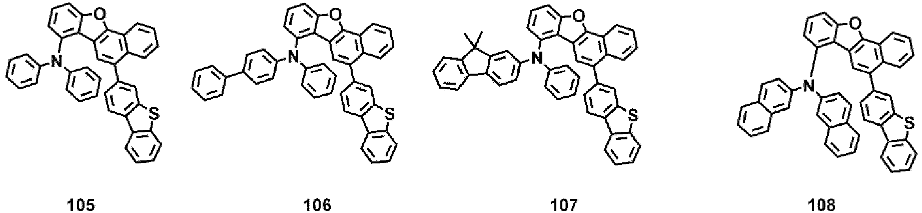

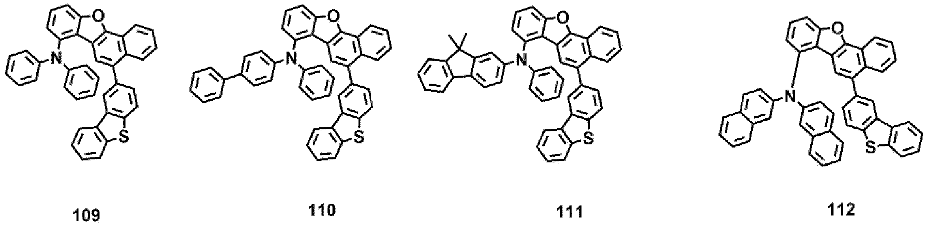

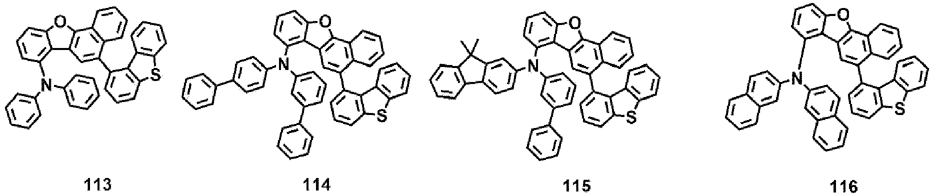

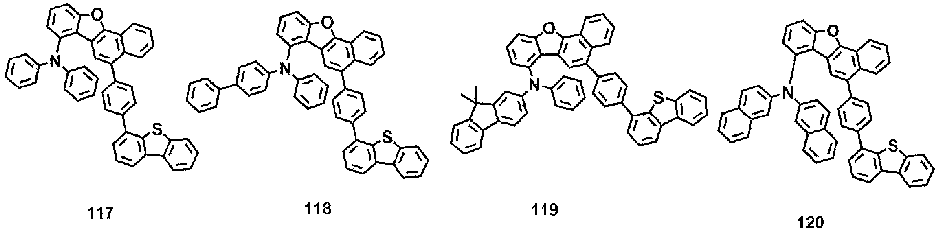

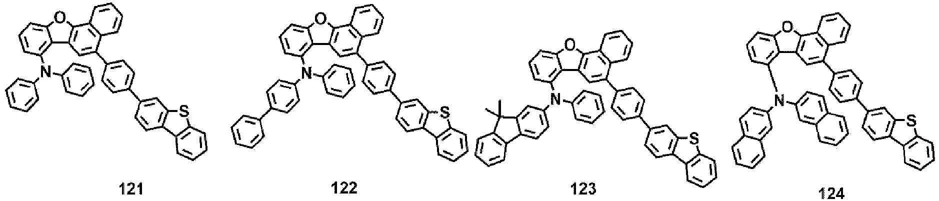

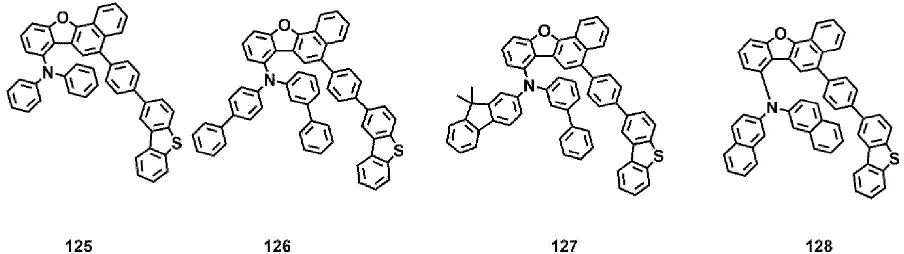

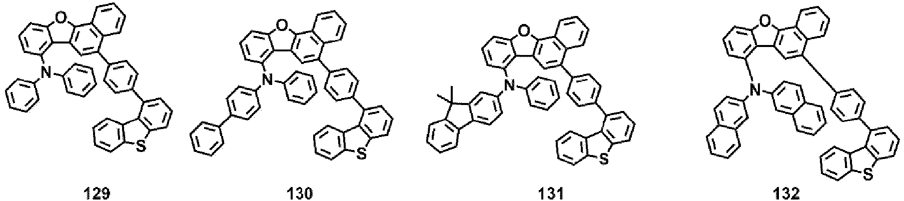

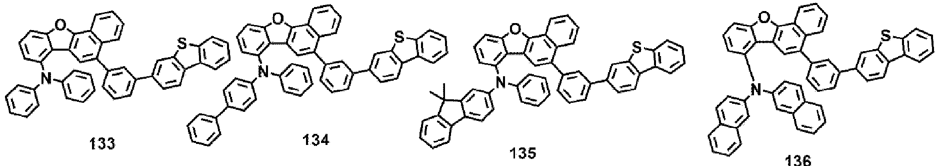

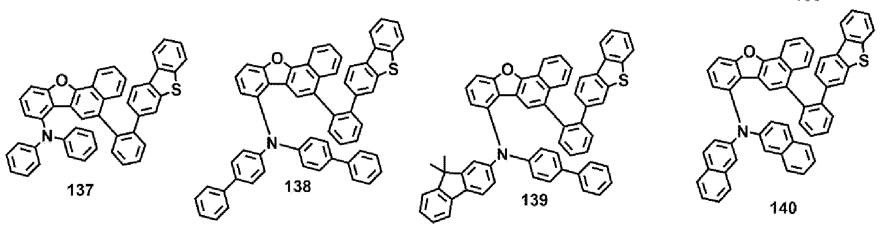

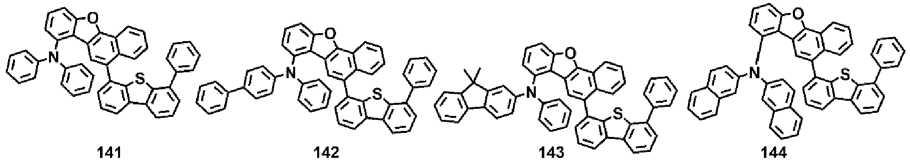

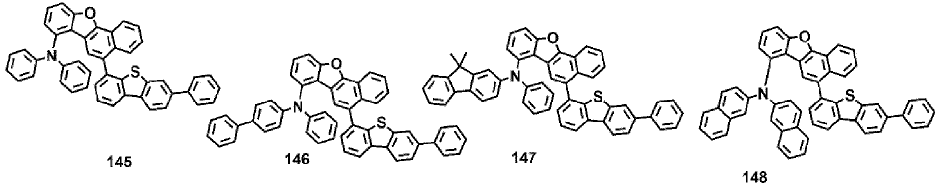

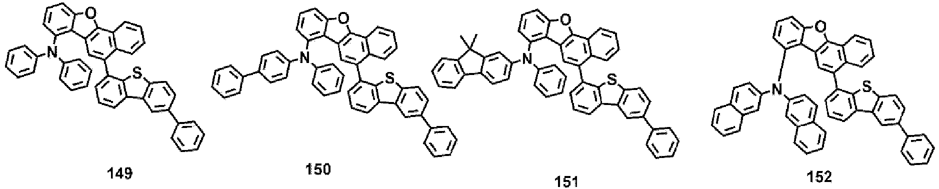

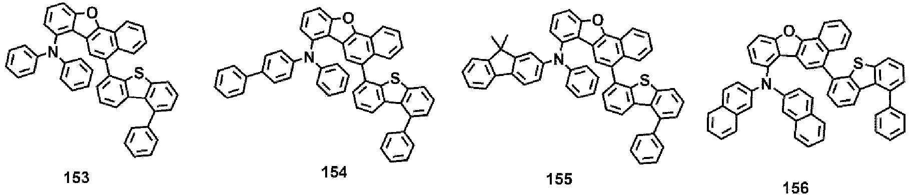

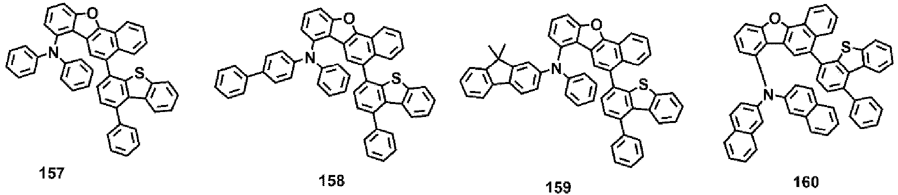

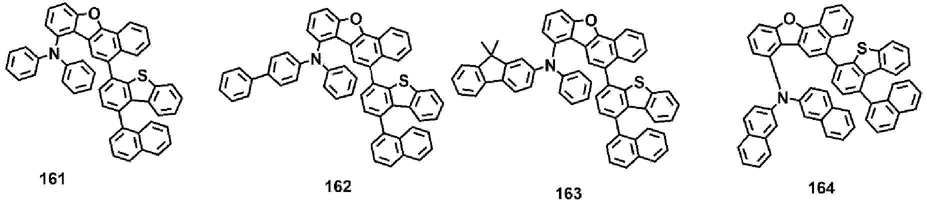

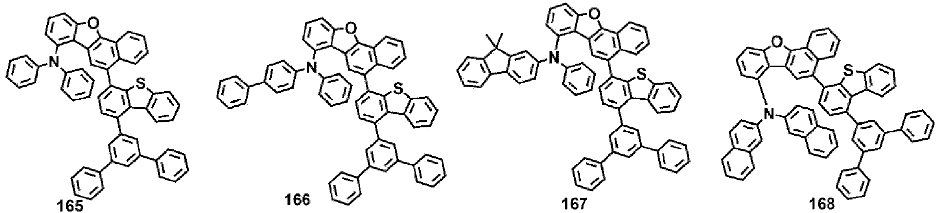

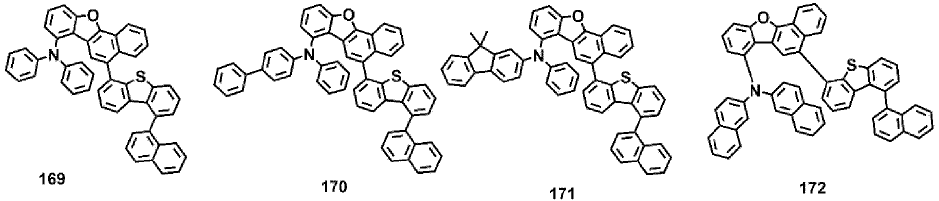

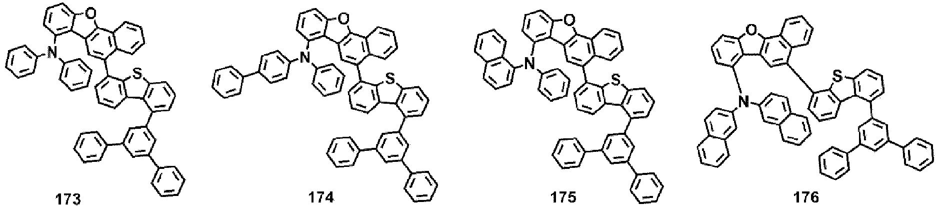

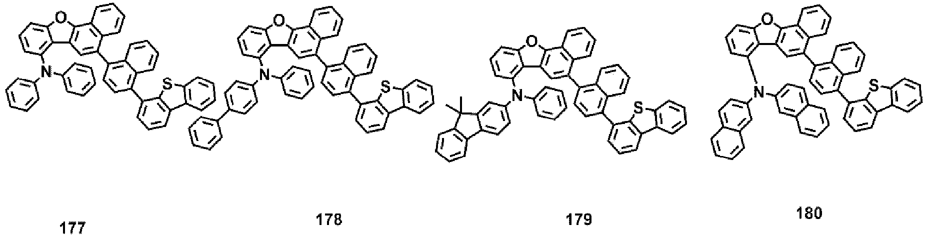

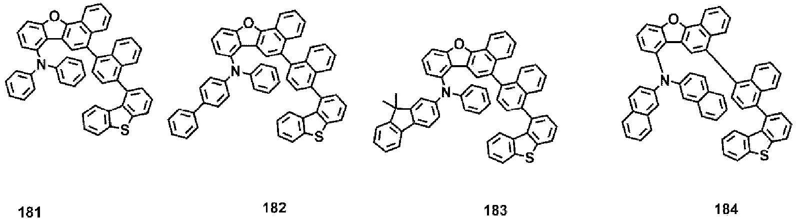

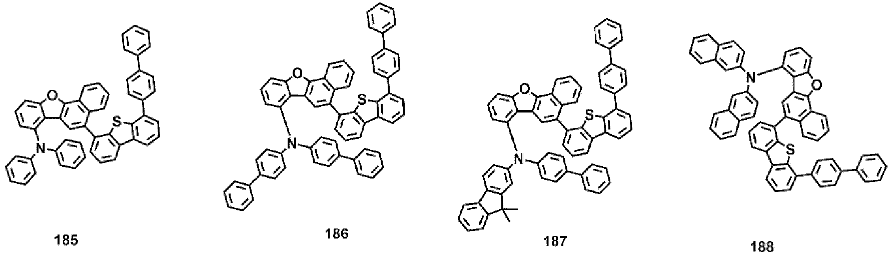

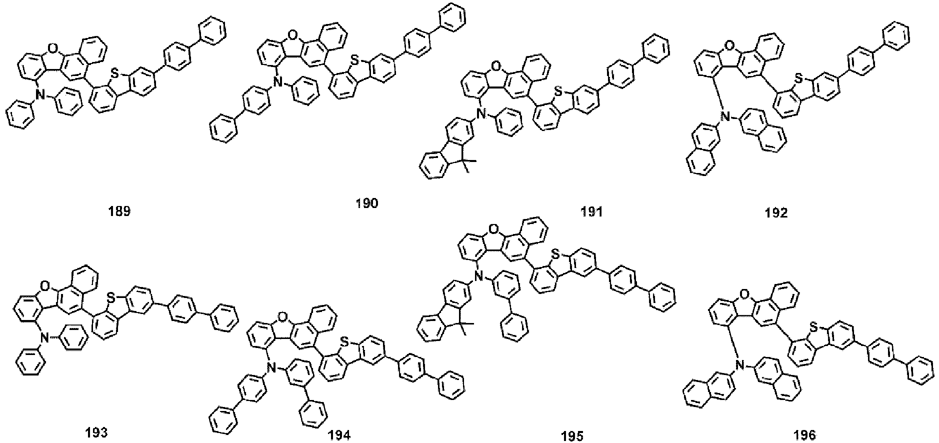

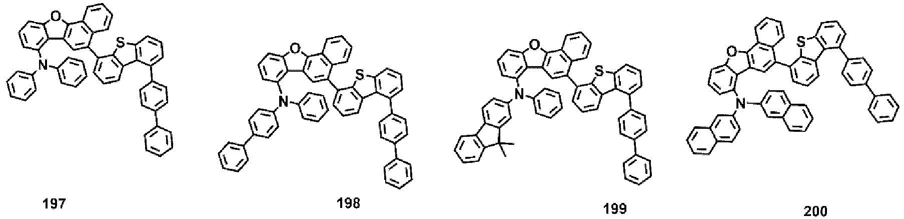

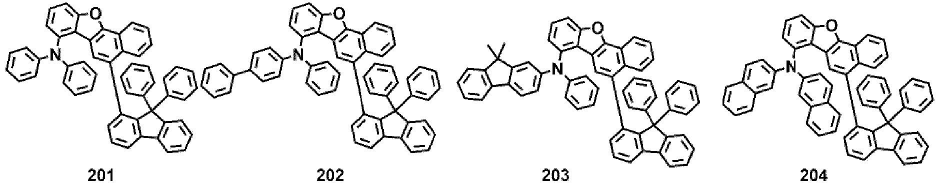

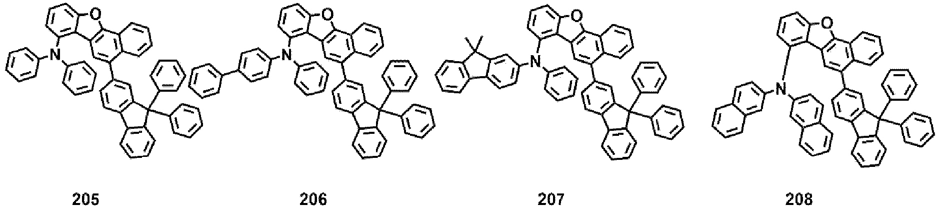

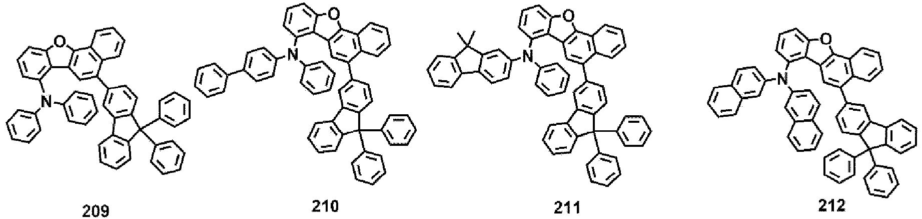

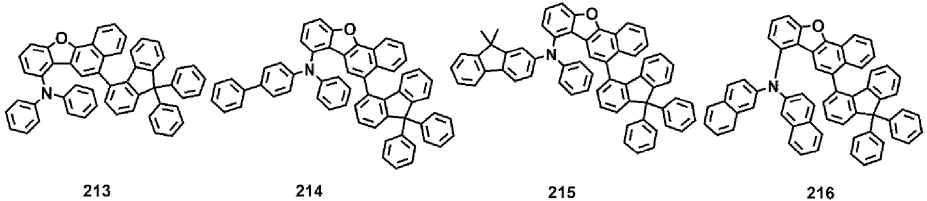

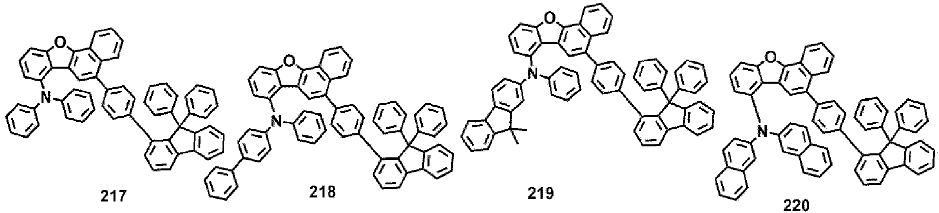

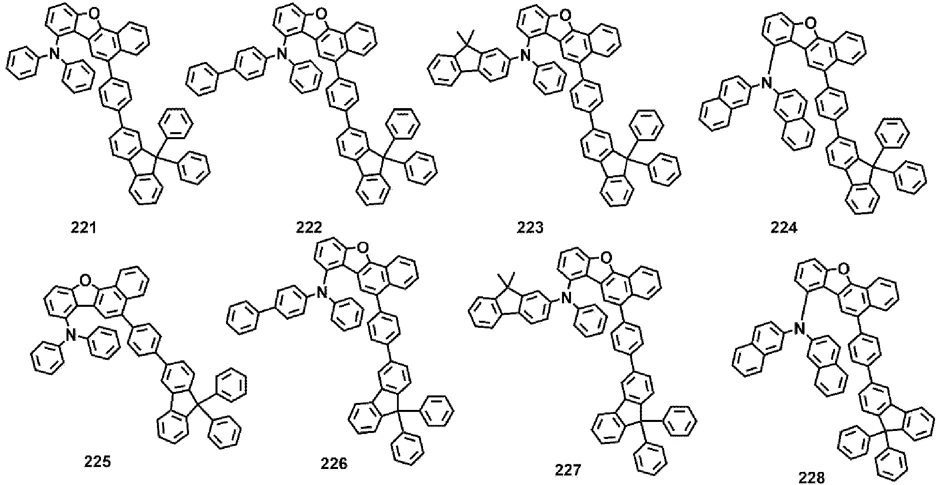

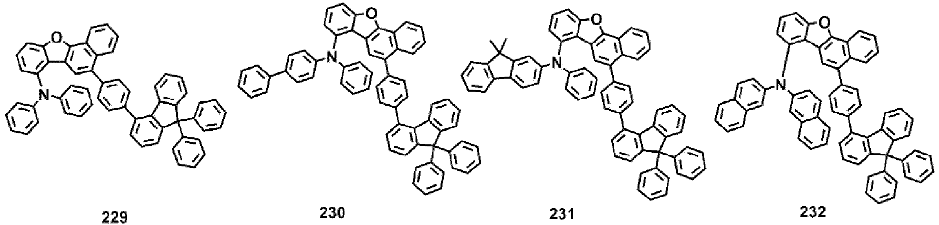

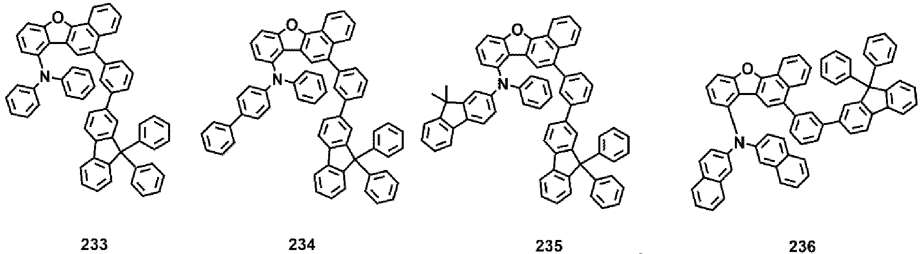

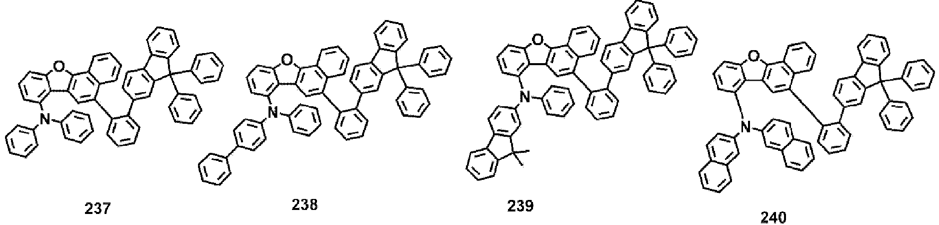

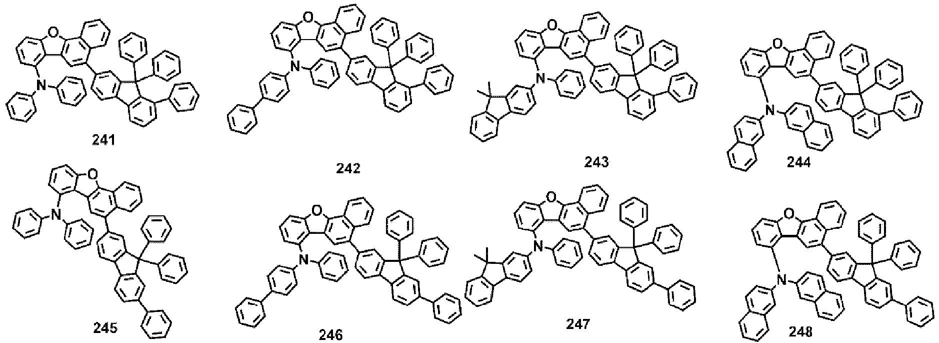

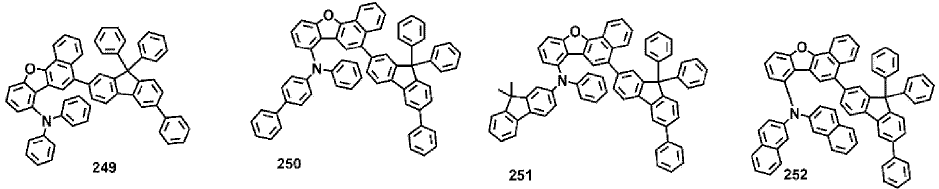

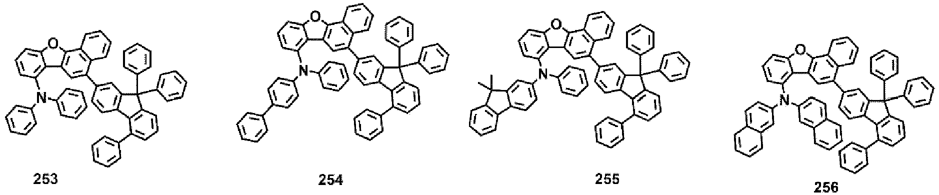

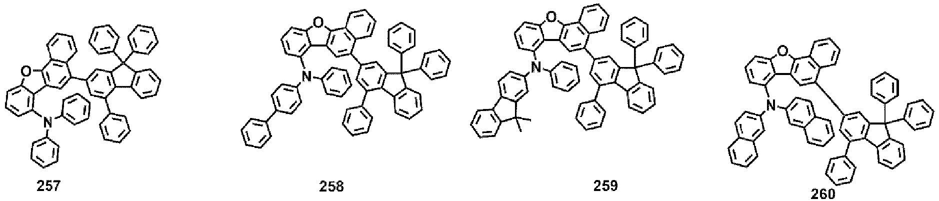

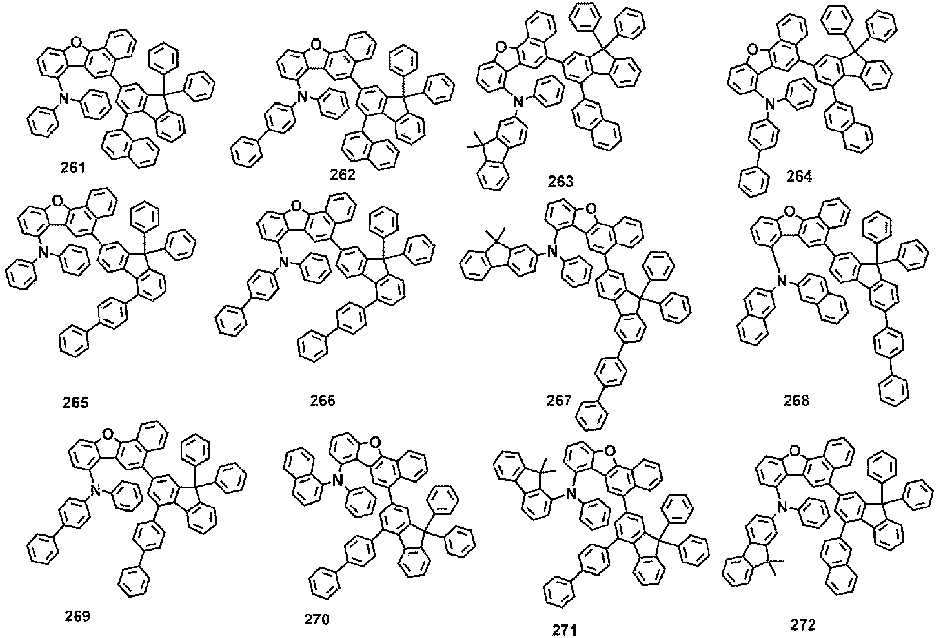

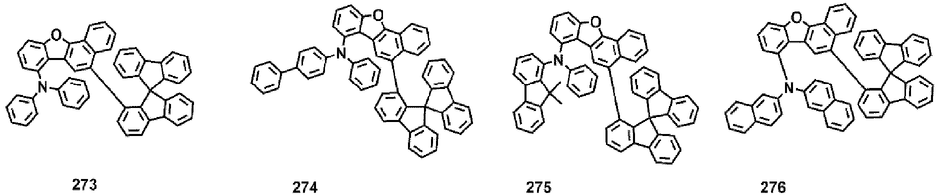

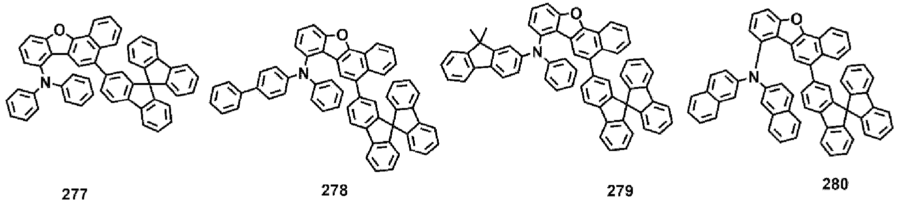

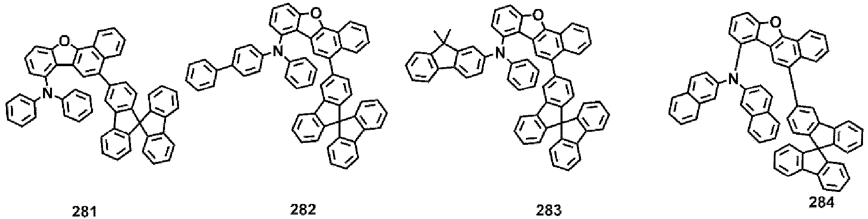

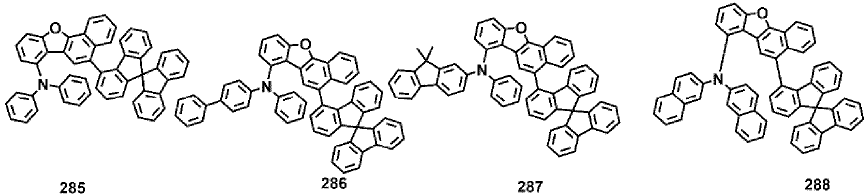

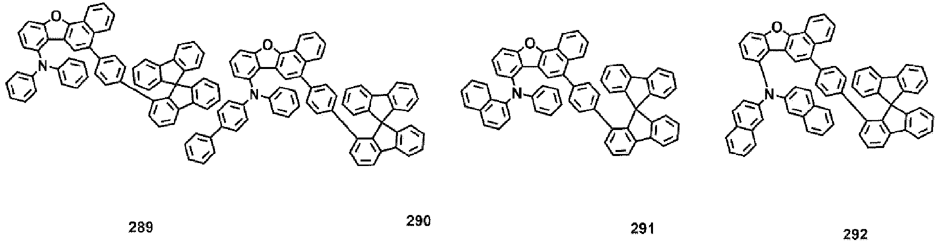

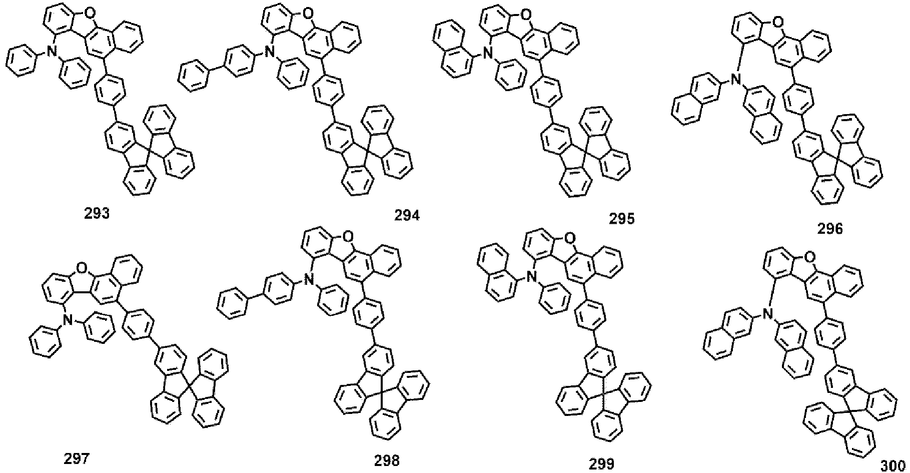

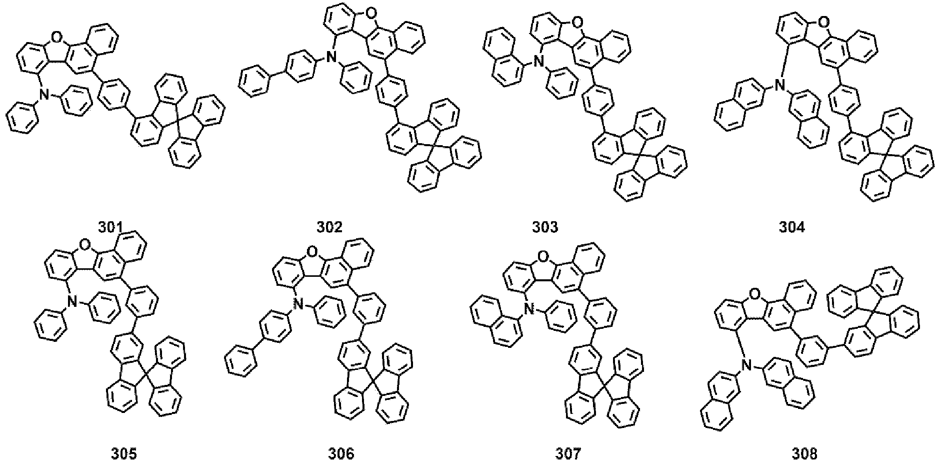

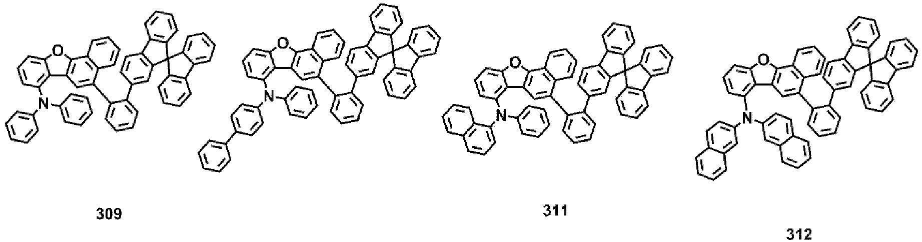

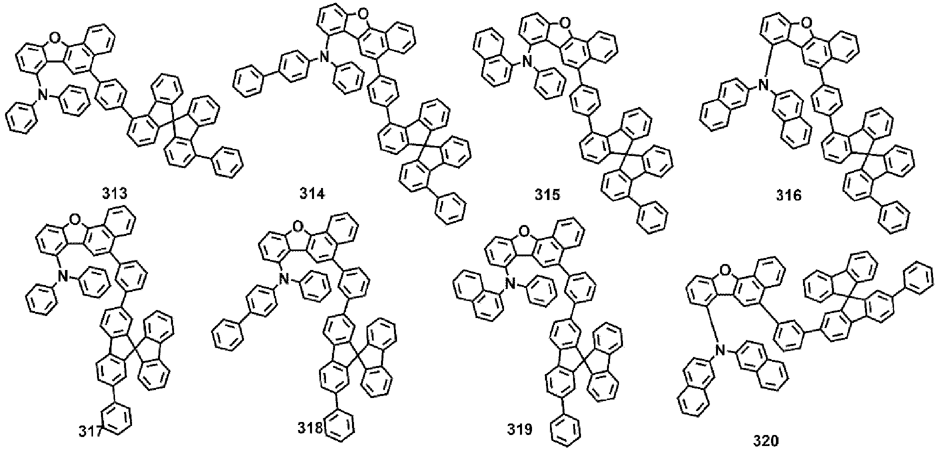

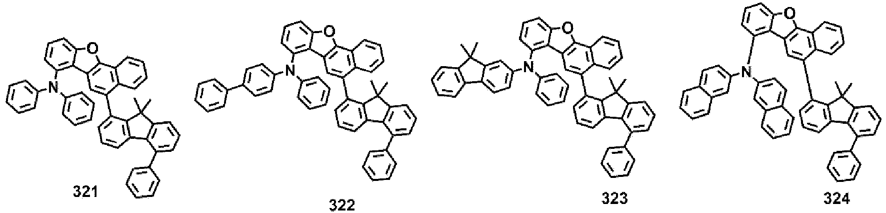

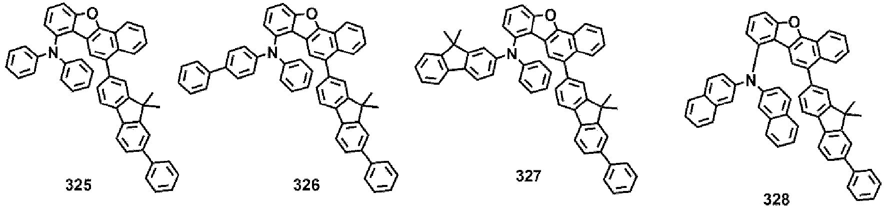

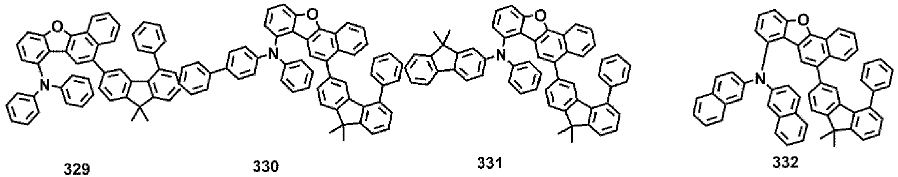

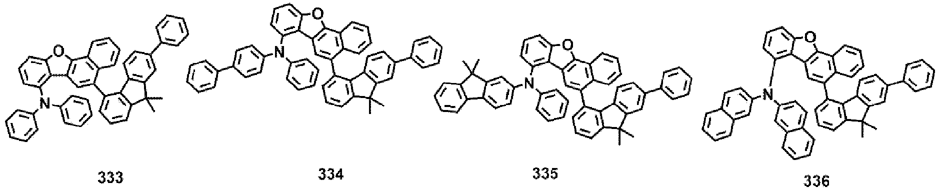

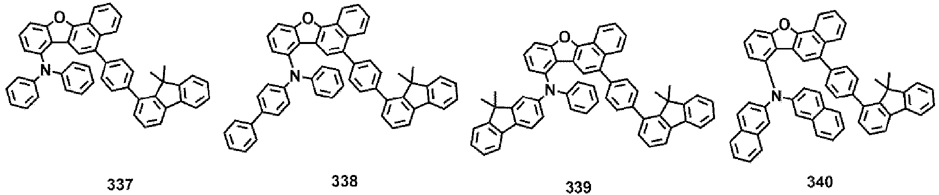

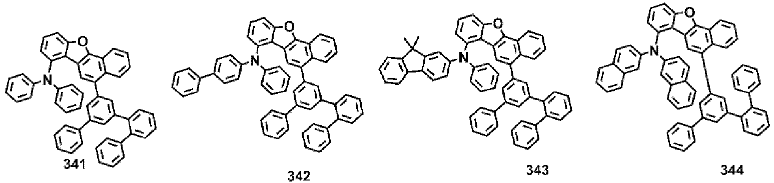

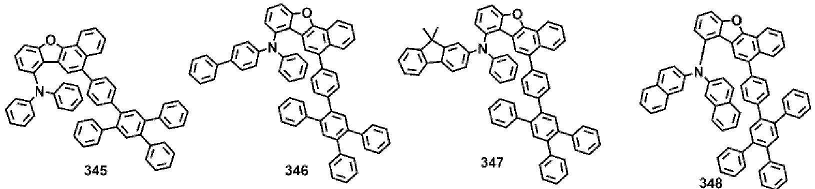

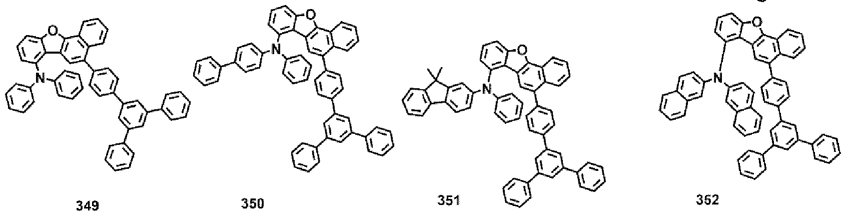

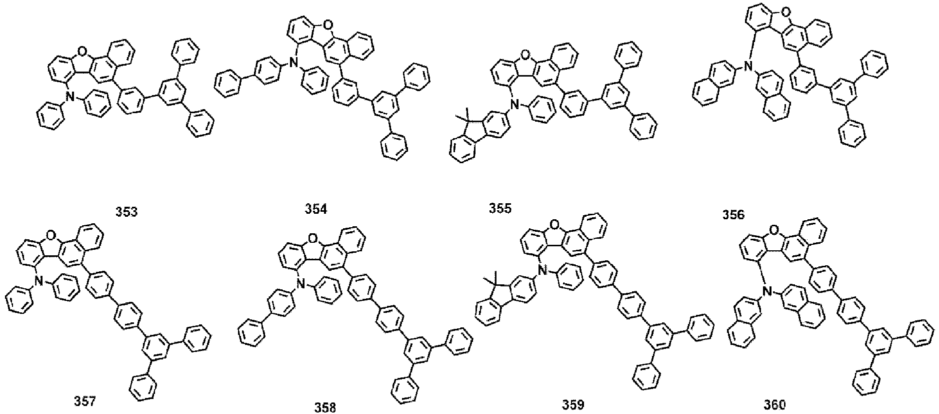

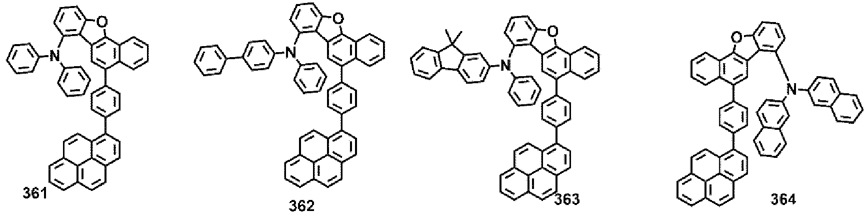

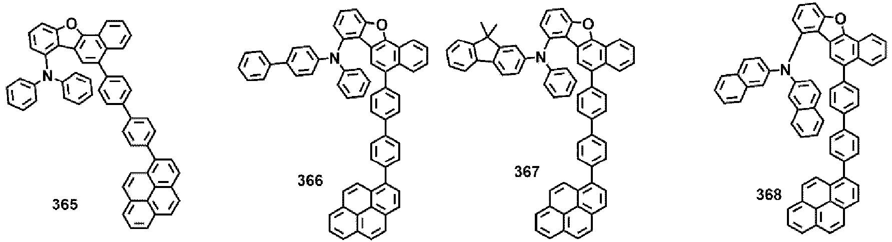

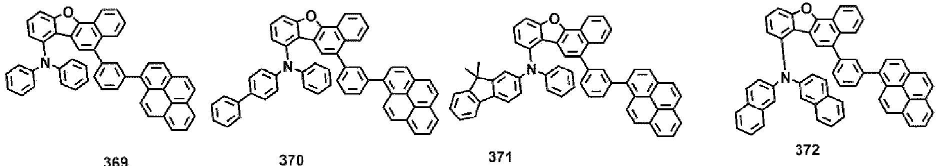

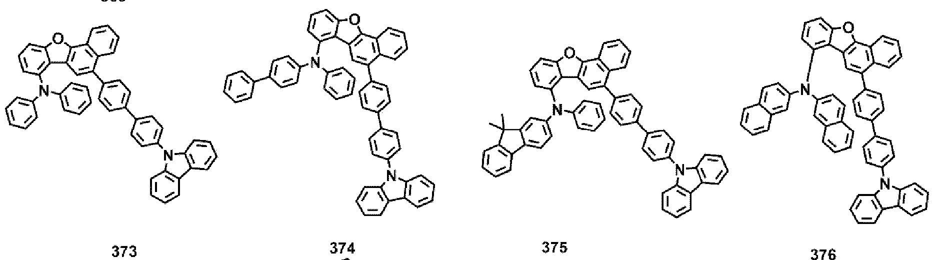

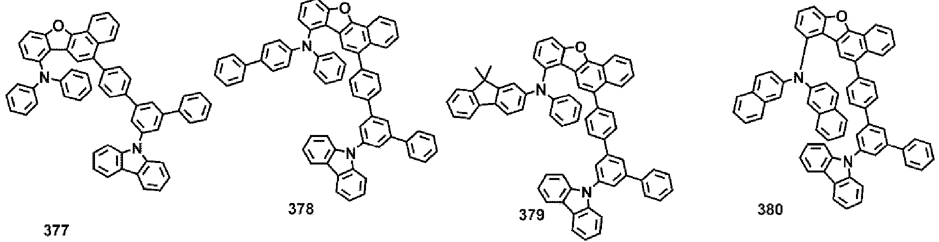

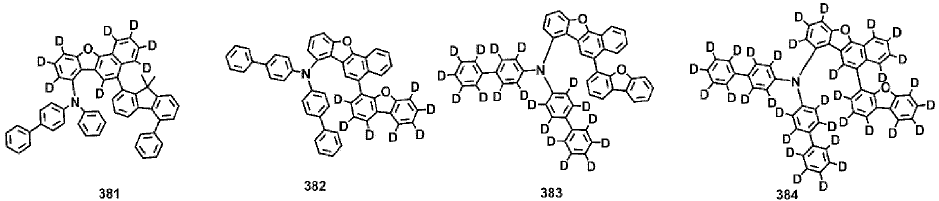

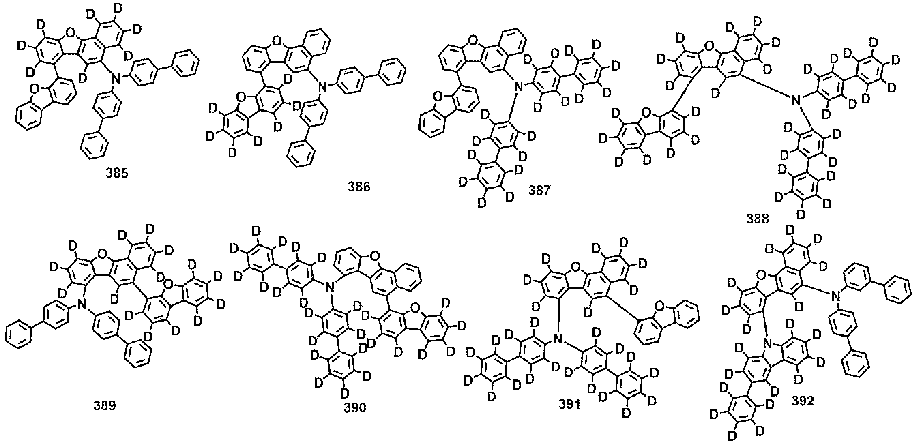

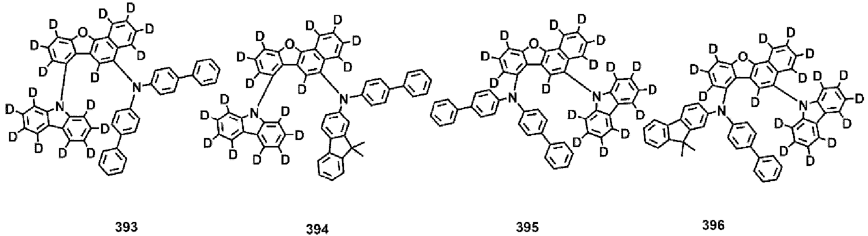

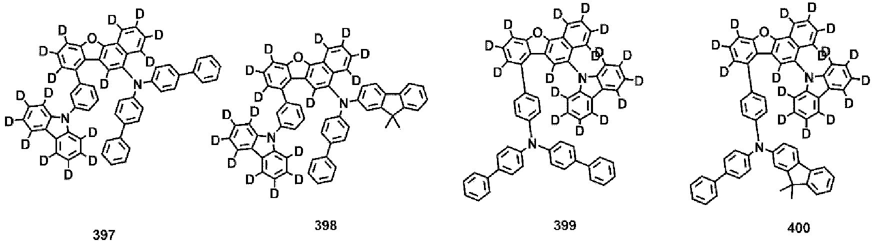

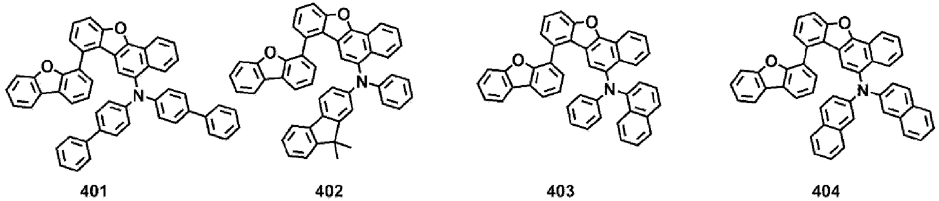

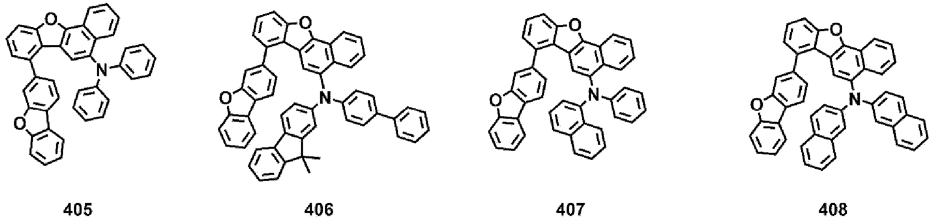

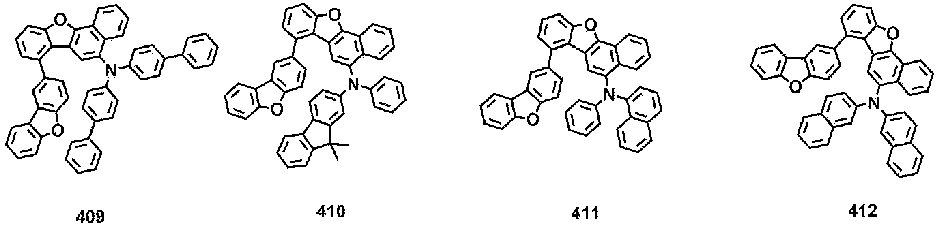

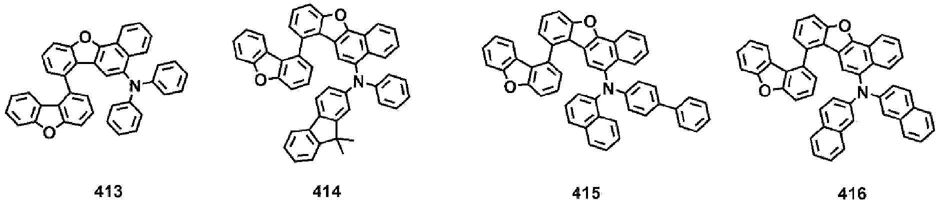

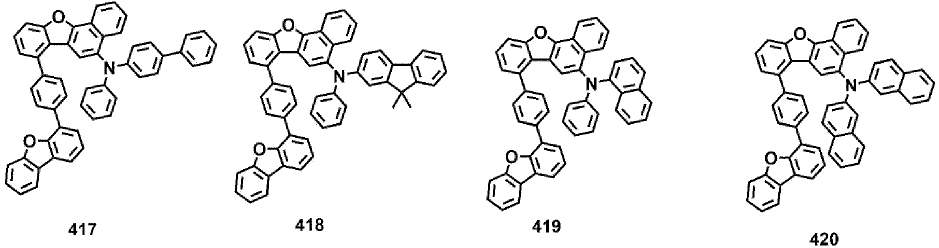

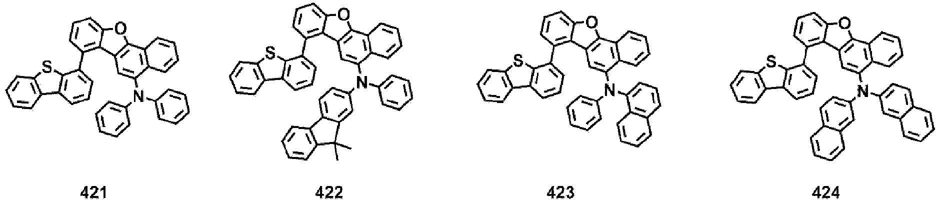

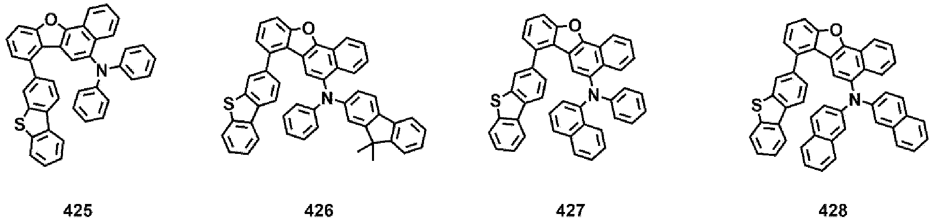

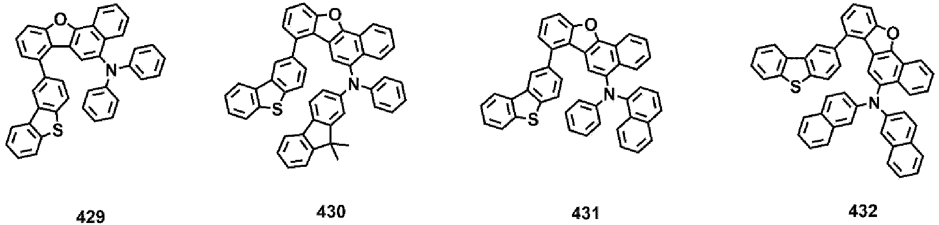

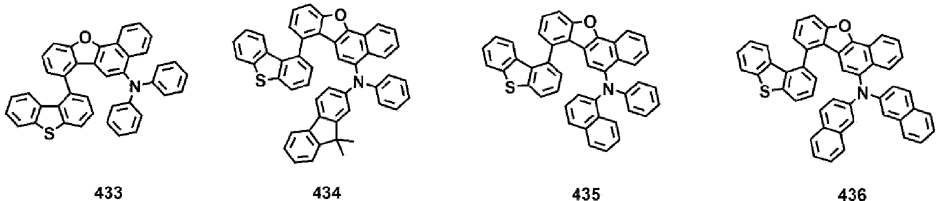

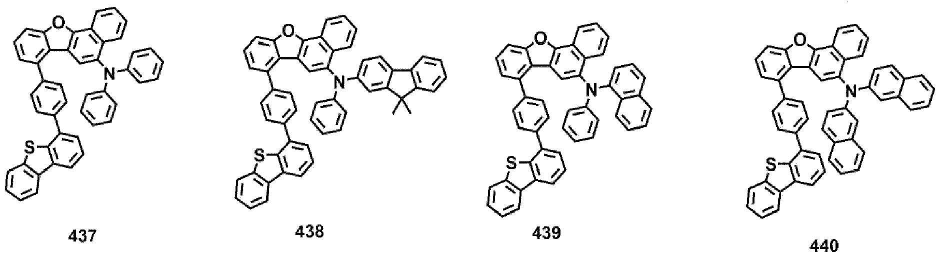

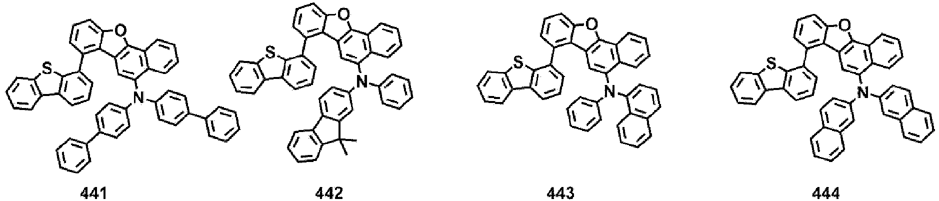

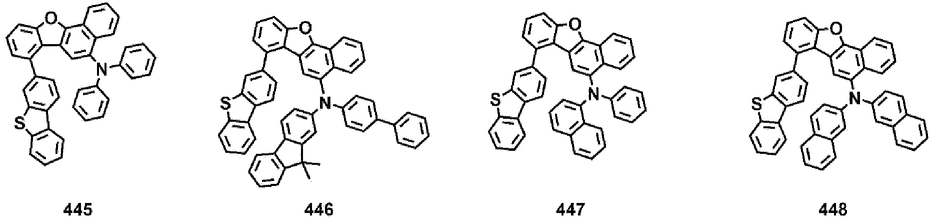

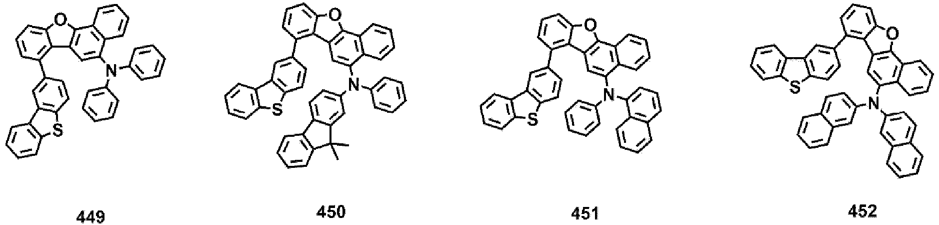

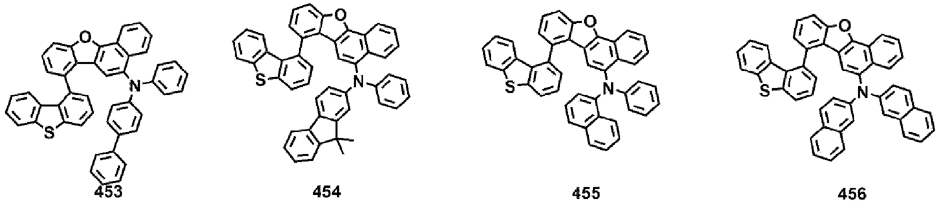

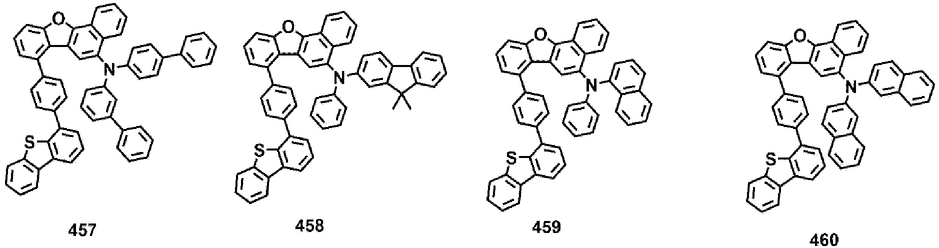

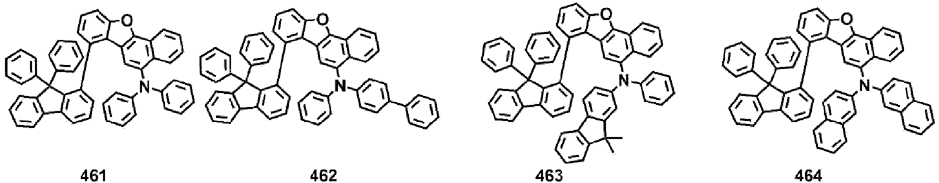

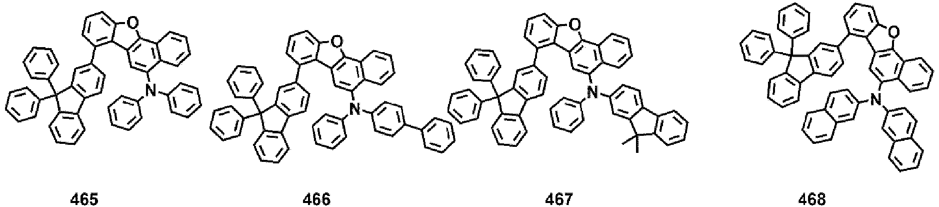

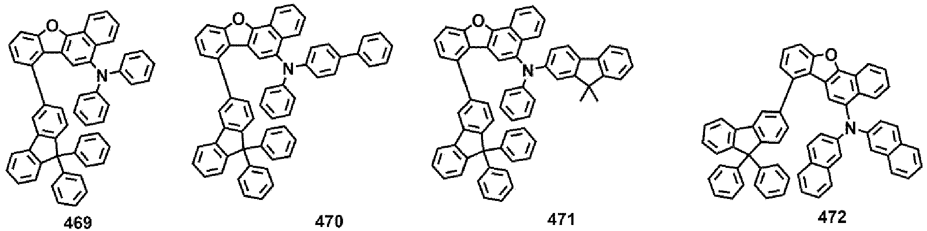

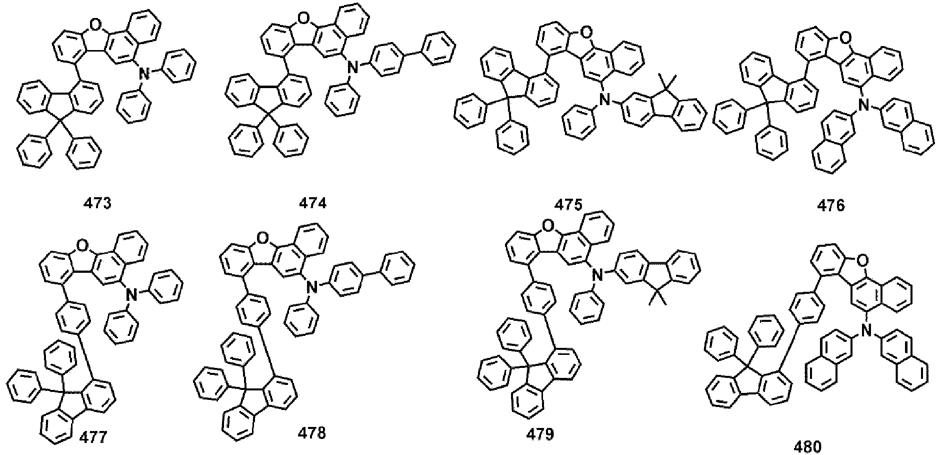

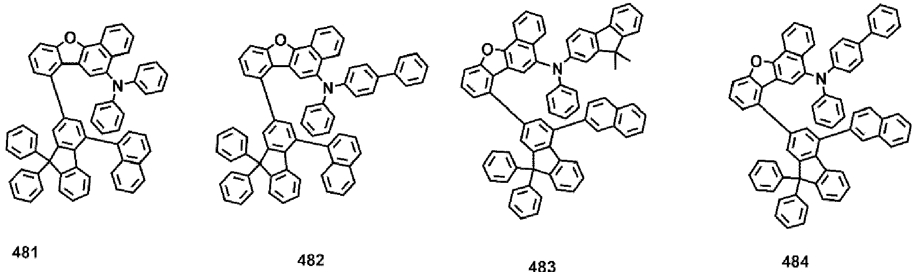

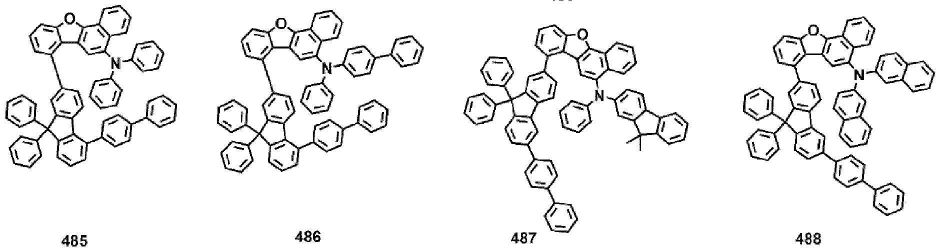

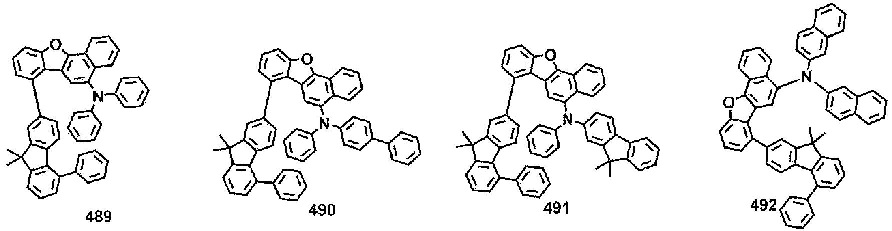

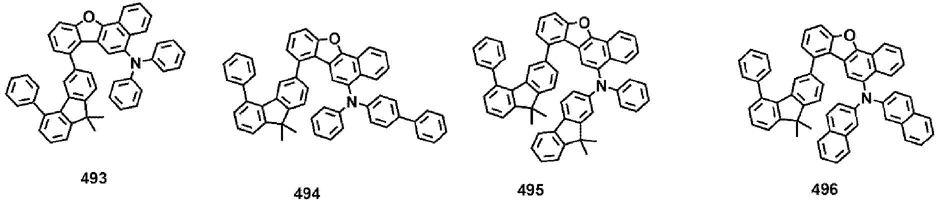

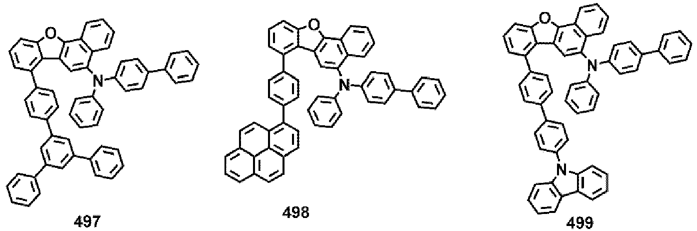

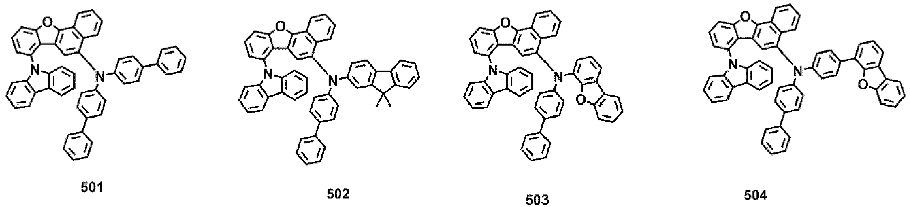

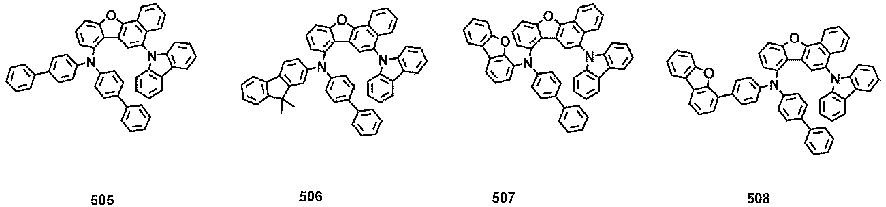

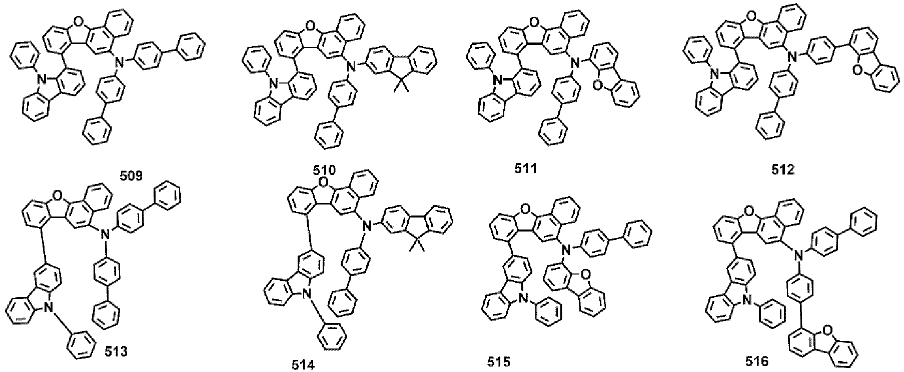

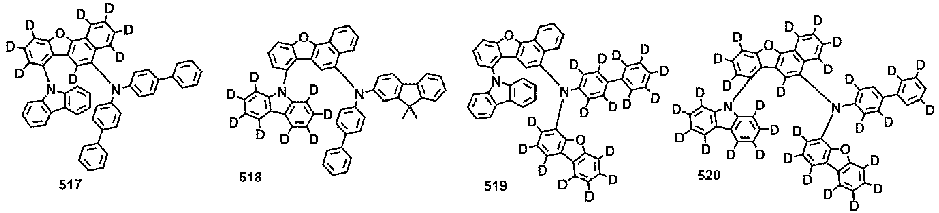

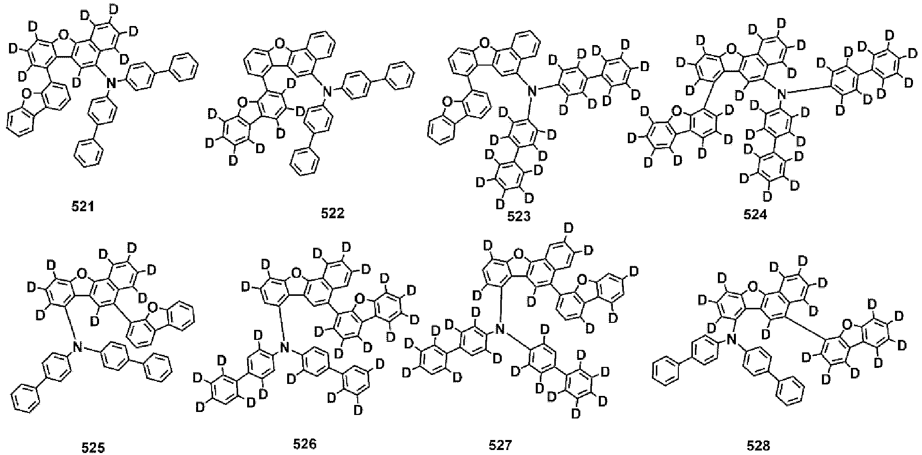

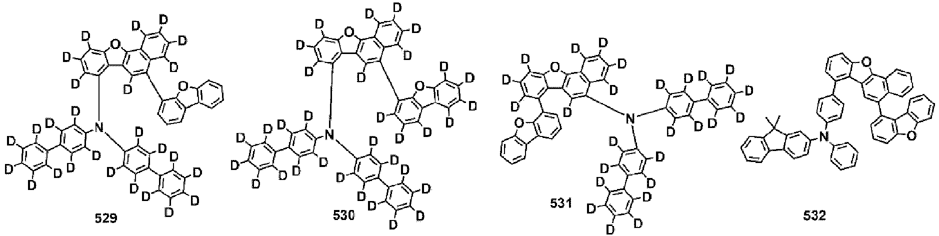

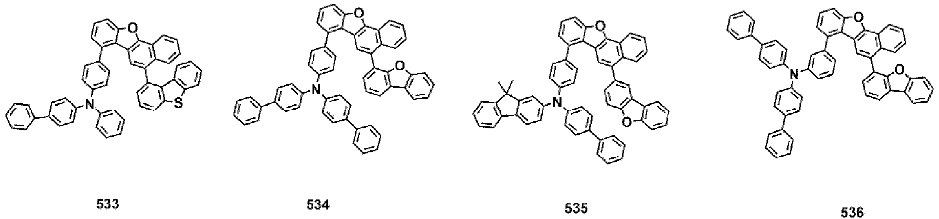

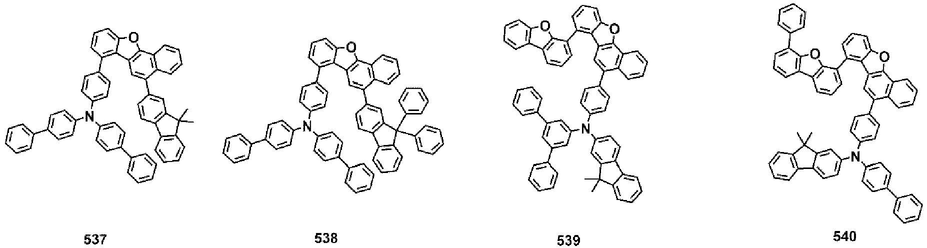

2. Dispositif électroluminescent organique comprenant une première électrode, une deuxième électrode et une couche de matériau organique ayant une ou plusieurs couches fournies entre la première électrode et la deuxième électrode, dans lequel une ou plusieurs couches de matériau organique comprennent le composé hétérocyclique selon la revendication 1.

3. Dispositif électroluminescent organique selon la revendication 2, dans lequel la couche de matériau organique comprend une couche de transfert de trous ayant une ou plusieurs couches, et la couche de transfert de trous comprend le composé hétérocyclique.

4. Dispositif électroluminescent organique selon la revendication 2, dans lequel la couche de matériau organique comprend une couche de transfert de trous auxiliaire ayant une ou plusieurs couches, et la couche de transfert de trous auxiliaire comprend le composé hétérocyclique.

5. Dispositif électroluminescent organique selon la revendication 2, comprenant en outre une ou deux ou plus de deux couches sélectionnées dans le groupe constitué d'une couche électroluminescente, une couche d'injection de trous, une couche de transfert de trous, une couche de transfert d'électrons, une couche de trous auxiliaire et une couche de blocage de trous.
